# EUROPÄISCHE PATENTSCHRIFT

(11) **EP 3 675 809 B1**
(45) Veröffentlichungstag und Bekanntmachung des Hinweises auf die Patenterteilung: **27.09.2023**
(21) Anmeldenummer: 18759965.9
(22) Anmeldetag: 31.08.2018
(51) Int. Cl.: A61K 8/49, A61K 31/165, A61K 31/34, C07D 405/12, A23L 33/10, A61K 31/341, A61K 31/36, A61K 31/381, A61K 31/4155, A61K 31/443, A61K 31/444, C07C 323/41, C07D 213/75, C07D 407/12, A61K 31/4436, C07D 231/40, C07D 307/14, C07D 307/22, C07D 333/36, C07D 405/14

(54) **VERWENDUNG PHYSIOLOGISCHER KÜHLWIRKSTOFFE UND MITTEL ENTHALTEND SOLCHE WIRKSTOFFE**
USE OF PHYSIOLOGICAL COOLING INGREDIENTS AND AGENTS CONTAINING SUCH ACTIVE INGREDIENTS
UTILISATION DE SUBSTANCES ACTIVES DE REFROIDISSEMENT PHYSIOLOGIQUES ET MOYEN COMPORTANT DES TELLES SUBSTANCES ACTIVES

(30) Priorität: 31.08.2017 EP 17188719
(43) Veröffentlichungstag der Anmeldung: 08.07.2020
(73) Patentinhaber: BASF SE, 67056 Ludwigshafen am Rhein (DE); Symrise AG, 37603 Holzminden (DE)
(72) Erfinder: JOIN, Benoît, 37603 Holzminden (DE); ONGOUTA, Jekaterina, 37627 Stadtoldendorf (DE); BACKES, Michael, 37603 Holzminden (DE); BRODHAGE, Rahim, 37671 Höxter (DE); MACHINEK, Arnold, 37603 Holzminden (DE); LOGES, Hubert, 37671 Höxter (DE); MUNDT, Susanne, 37603 Holzminden (DE); SOMERS, Tom, 37603 Holzminden (DE); SUBKOWSKI, Thomas, 69198 Schriesheim (DE); WITTENBERG, Jens, 67117 Limburgerhof (DE); WEISEL, Martin, 68165 Mannheim (DE); SIEGEL, Wolfgang, 67117 Limburgerhof (DE); BOLLSCHWEILER, Claus, 69118 Heidelberg (DE); PELZER, Ralf, 37699 Fürstenberg (DE)
(74) Vertreter: Eisenführ Speiser
(86) Internationale Anmeldenummer: PCT/EP2018/073486
(87) Internationale Veröffentlichungsnummer: WO 2019/043164

(56) Entgegenhaltungen:
- US-A1- 2015 086 491
- US-A1- 2016 317 532
- US-A1- 2017 096 418

## Beschreibung

Die Erfindung betrifft primär neuartige Modulatoren des Kälte-Menthol-Rezeptors TRPM8, Verfahren zur Modulation des TRPM8-Rezeptors unter Verwendung dieser Modulatoren, die Verwendung der Modulatoren zur Induktion von Kältegefühl sowie die unter Verwendung dieser Modulatoren hergestellten Mittel.

In einem speziellen Aspekt betrifft die Erfindung ein Mittel umfassend wenigstens einen solchen Modulator zum Erzielen einer Kühlwirkung auf Haut oder Schleimhaut, wobei vorteilhafterweise die Kühlwirkung verglichen mit bekannten Kühlwirkstoffen, z.B. mit Menthan-3-carbonsäure-N-ethylamid (WS3), länger anhaltend ist und/oder verglichen mit bekannten Kühlwirkstoffen, z.B. FEMA 4809 oder FEMA 4496, früher einsetzt.

Ferner betrifft ein spezieller Aspekt der Erfindung Mittel, die solche ausgewählte TRPM8-Rezeptor-Modulatoren umfassen, wobei die Mittel zu bestimmten Zwecken dienen.

Außerdem betrifft die Erfindung auch ein Verfahren zum Erreichen einer physiologischen Kühlwirkung auf Haut oder Schleimhaut, bei dem die genannten Mittel eingesetzt werden.

Weitere Aspekte der vorliegenden Erfindung ergeben sich aus der nachfolgenden Beschreibung, den Beispielen sowie insbesondere den beigefügten Patentansprüchen.

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Mischungen verwendet werden. Dabei ist zu berücksichtigen, dass nicht alle Verbindungen, die *in vitro* Rezeptoren beeinflussen, die (auch) an der Vermittlung einer physiologischen Kühlwirkung beteiligt sind, tatsächlich einen solchen Effekt *in vivo* auf der Haut oder auf Schleimhäuten erzeugen. Insbesondere wird ein solcher Effekt nicht immer identisch verlaufen. Das bedeutet zum Beispiel, dass die Stärke der vermittelten physiologischen Kühlwirkung sowie der Verlauf der Stärke der Kühlwirkung gegen Zeitdauer nicht alleine aus der Tatsache geschlossen werden können, dass eine bestimmte Verbindung ein Agonist eines an der Vermittlung eines Kühleindruckes beteiligten Rezeptors ist.

Der bekannteste physiologisch wirksame Kühlwirkstoff ist L-Menthol, der aber einige Nachteile besitzt, z. B. starker Geruchseindruck, eine hohe Flüchtigkeit und in höheren Konzentrationen einen bitteren und/oder scharfen Eigengeschmack, bzw. eine Haut reizende Wirkung.

Es wurde daher schon früher nach starken Kühlwirkstoffen gesucht, die nicht die nachteiligen Eigenschaften des L-Menthols aufweisen. So wurden z.B. Milchsäureester von Menthol(en) gemäß DE 2 608 226 und gemischte Carbonate mit Menthol(en) und Polyolen gemäß DE 4 226 043 und Menthonketale gemäß EP 0 507 190 beschrieben.

Menthylmonoester von Disäuren nach US 5,725,865 und US 5,843,466 sind zwar interessante natürlich vorkommende Alternativen, können aber in sensorischen Tests nicht die Stärke der vorher beschriebenen Kühlwirkstoffe erreichen.

In J. Soc. Cosmet. Chem. 1978, 29, 185-200 wurden die Ergebnisse einer Studie an ca. 1200 Verbindungen präsentiert, bei der die Verbindungen L-Menthancarbonsäure-*N*-ethylamid ("WS3") und insbesondere *N*^{α}-(L-Menthancarbonyl)-glycinethylester ("WS5") als die stärksten Kühlwirkstoffe gefunden wurden. Letzterer hat bei starker Wirkung aber den Nachteil, hydrolyseempfindlich zu sein und dabei die entsprechende freie Säure *N*^{α}-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlwirkstoffen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten (z.B. die Menthancarbonsäure-N-(alkyloxyalkyl)amide nach JP 2004059474) oder sind zusätzlich stark reizend (WS5: N-[[5-Methyl-2-(1-methylethyl)cyclohexyl]carbonyl]glycinethylester, US 2005/0222256). *N*^{α}-(Menthancarbonyl)alkyloxyalkylamide wurden in JP 2004059474 beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar.

Des Weiteren sind Menthylglyoxylate und ihre Hydrate in JP 2005343795 als Kühlsubstanzen beschrieben worden.

Übersichten zu den bisher hergestellten und verwendeten Kühlwirkstoffen findet man bei M. Erman, Perfumer & Flavorist 32(10), 20-35 (2007**)** und M. L. Dewis in D. J. Rowe, Chemistry and Technology of Flavors and Fragrances, Blackwell Publishing Ltd, Oxford 2005, p. 212 - 222.

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca²⁺ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, A TRP channel that senses cold stimuli and menthol.Cell. 2002 Mar 8;108(5):705-15; McKemy DD et al. Identification of a cold receptor reveals a general role for TRP channels in thermosensation Nature 2002 Mar 7; 416 (6876): 52-8; Zuker CS. Neurobiology: A cool ion channel Nature 2002 Mar 7; 416 (6876): 27-8).

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt (McKemy D.D., Molecular Pain 1, 2005, 16; McKemy D.D., Nature 416, 2002, 52-58; Peier A.M., Cell 108, 2002, 705-715; Dhaka A., Annu. Rev. Neurosci. 29, 2006, 135-161). Durch Applikation von Menthol wird TRPM8 aktiviert, wodurch ein Ca²⁺-Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (British Patent 1971 # 1315761 Watson H.R., J. Soc. Cosmet. Chem. 29, 1978, 185-200; Furrer S.M., Chem. Percept. 1, 2008, 119-126). Es gibt auch vereinzelte, strukturell mit Menthol nicht verwandte Verbindungen, die eine signifikante TRPM8-Modulation bewirken, wie z. B. Icilin (Wei E.T., J. Pharm. Pharmacol. 35, 1983, 110-112; WO 2004/026840), WS-23 oder in der Patentanmeldung WO 2007/019719 aufgeführte Verbindungen.

Weitere Wirkungen von Substanzen, die den TRPM8-Rezeptor bzw. seine Insektenanaloga modulieren, sind eine Repellent-Wirkung auf Insekten (WO 2002/015692; WO 2004/000023, US 2004/0028714), sowie Aktivität in der Antitumortherapie (z.B. eine Beeinflussung von Prostatatumoren), Aktivität bei der Behandlung von inflammatorischen Schmerzen / Hyperalgesie und eine Wirkung als TRPM8-Antagonisten in der Therapie von Blasensyndrom bzw. überaktiver Blase (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Dissertation Behrendt H.J. 2004, Universität Bochum; Lashinger E.S.R. Am. J. Physiol. Renal Physiol. Am J Physiol Renal Physiol. 2008 Jun 18. [Epub ahead of print]; PMID: 18562636).

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit und /oder Flüchtigkeit auf.

In der älteren Internationalen Patentanmeldung PCT/EP2009/061019 der Anmelderin werden einzelne Verbindungen zur Modulation des TRPM8-Rezeptors vorgeschlagen. So sind beispielsweise die folgenden Verbindungen offenbart:

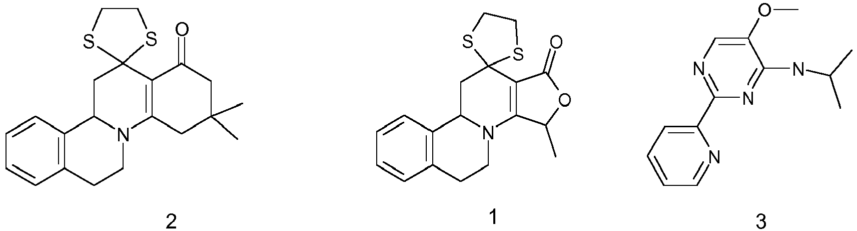

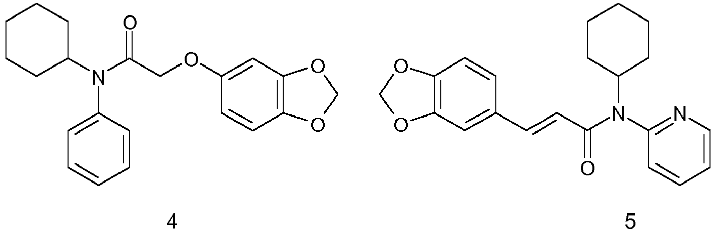

Diese Verbindungen sind auch kommerziell erhältlich:
Verbindung 1 unter CAS Nummer: 99602-94-5 (3R-cis-Form)
Verbindung 2 unter CAS Nummer: 165753-08-2
Verbindung 3 unter CAS Nummer: 338771-57-6
Verbindung 4 unter CAS Nummer: 878942-21-3
Verbindung 5 unter CAS Nummer: 748783-13-3 (ohne Stereochemie)

Des Weiteren wird an dieser Stelle auf die Internationale Patentanmeldung PCT/EP2010/067936 der Anmelderin verwiesen, worin ebenfalls Verbindungen zur Modulation des TRPM8-Rezeptors vorgeschlagen werden.

Weitere TRPM8-Rezeptor-Modulatoren und deren Verwendung zur Induktion eines Kältegefühls werden in US 2017/0096418 A1, US 2016/0317532 A1 oder US 2015/0086491 A1 genannt.

Primäre Aufgabe der vorliegenden Erfindung war es, neue Substanzen zu identifizieren, die eine besondere physiologische Kühlwirkung besitzen, vorzugsweise solche, die zu einer Modulation des TRPM8-Rezeptors führen, welche als Alternativen, vorzugsweise als geeignetere Mittel, zu den bisher bekannten Modulatoren einsetzbar sind. Solche Verbindungen sollten sich insbesondere auch für Anwendungen im Bereich Kosmetik (z.B. hair care, skin care, oral care), Ernährung (feed/food), Textil, OTC-Produkte (z.B. Brandsalbe), Pharmaka (z.B. Tumorbehandlung, Blasenschwäche) oder Verpackungen eignen. Die anzugebenden Verbindungen bzw. Mischungen von Verbindungen sollten vorzugsweise einen möglichst schwachen Eigengeschmack zeigen, insbesondere wenig oder gar nicht bitter schmecken sowie möglichst nicht reizend sein.

Für die Lösung der erfindungsgemäßen Aufgabe waren dabei vor allem Mittel mit Wirkstoffen gesucht, die eine besonders langandauernde Kühlempfindung vermitteln können. Bevorzugt sollten diese Mittel darüber hinaus besonders intensive und/oder schnell einsetzende Kühleindrücke vermitteln können.

Auf der Mundschleimhaut zeigen viele der oben erwähnten konventionellen, im Stand der Technik bekannten Kühlsubstanzen alle ein mehr oder weniger identisches Kühlverhalten. Das durch sie vermittelte kühlende Frischempfinden setzt nach etwa 0,5 Minuten ein, flacht dann aber nach einem Höhepunkt bei 3 bis 5 Minuten wieder relativ schnell ab, wobei die Kühlung insgesamt für höchstens 30 Minuten deutlich wahrnehmbar ist und erfahrungsgemäß in Intensität und Dauer nur wenig durch eine Veränderung der Dosierung zu beeinflussen ist. Auf Seiten der Verbraucher besteht aber der Wunsch nach einer insbesondere lange andauernden Kühlwirkung, die für den Verwender mit einem entsprechenden Frische- und Wohlgefühl verbunden ist.

Es hat sich überraschenderweise gezeigt, dass die erfindungsgemäß zu verwendenden Verbindungen die gemeinsame Eigenschaft haben, *in vivo* eine besonders lange Kühlwirkung auf Haut oder Schleimhaut zu erzielen. Dies war weder für die in dieser Anmeldung erwähnten TRPM8-Agonisten vorhersagbar, noch trifft es auf alle dieser Agonisten zu.

Bisher gab es im Stand der Technik keine Hinweise dazu, dass speziell die erfindungsgemäß zu verwendenden Verbindungen eine besonders geeignete Kühlwirkung zu vermitteln in der Lage sind. Um die langandauernde Kühlwirkung zu quantifizieren, können Vergleichsversuche mit Menthan-3-carbonsäure-N-ethylamid durchgeführt werden. Für diese Vergleichsversuche tauscht der Fachmann die erfindungsgemäß einzusetzende Verbindung oder die Verbindungen durch Menthan-3-carbonsäure-N-ethylamid (auch WS3), vorzugsweise in gleicher Konzentration, aus. Dann werden die Kühlwirkungen der jeweiligen Mittel miteinander verglichen. Sofern die erfindungsgemäß einzusetzenden Verbindungen im zu untersuchenden Mittel in einer Konzentration von höher als 100 ppm enthalten sind, ist es bevorzugt, dass für die Evaluierung, ob die Kühlwirkung gegenüber WS3 verlängert ist, das zu testende Mittel so zu verdünnen, dass die erfindungsgemäßen einzusetzenden Verbindungen in einer Konzentration von 100 ppm vorliegen. Selbstverständlich ist der gleiche Verdünnungsschritt auch für das Vergleichsmittel vorzunehmen, das WS3 enthält.

In diesem Zusammenhang ist bevorzugt, dass in den entsprechenden Vergleichen die Kühlwirkung der Mittel mit den erfindungsgemäß einzusetzenden Verbindungen vorzugsweise um wenigstens 10 Minuten, bevorzugt um wenigstens 15 Minuten, weiter bevorzugt wenigstens 20 Minuten und besonders bevorzugt wenigstens 30 Minuten gegenüber den Vergleichsversuchen verlängert ist.

Die primäre Aufgabe der vorliegenden Erfindung wird erfindungsgemäß gelöst durch ein nichttherapeutisches Verfahren zur Modulation, vorzugsweise zur *in vitro* und/oder *in vivo* Modulation, des Kälte-Menthol-Rezeptors TRPM8, wobei man den Rezeptor mit wenigstens einem Modulator in Kontakt bringt, der ausgewählt ist aus der Gruppe bestehend aus Verbindungen des folgenden Strukturtyps 1: 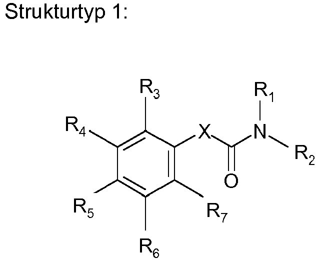 worin
R₁ aus folgender Struktur besteht: 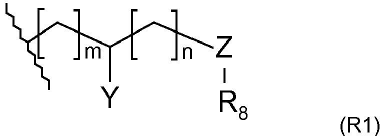 worin
   Z ausgewählt ist aus der Gruppe bestehend aus O und S;
   n gleich 0 oder 1 ist;
   m
      - gleich 1 ist und Y Wasserstoff ist und R8 ein lineares oder verzweigtes C₁ bis C₅ Alkan, bevorzugt ein C₅ Alkan, ist, oder Y NR₉ oder CR₉ ist und R₉ mit R₈ einen teilweise oder vollständig gesättigten 5- oder 6-gliedrigen Ring ausbildet, oder
      - gleich 0, 2 oder 3 ist und Y Wasserstoff ist und R₈ ein lineares oder verzweigtes C₁ bis C₅ Alkan, bevorzugt ein C₅ Alkan, ist, oder Y NR₉ oder CR₉ ist und R₉ mit R₈ einen aromatischen, einen teilweise oder einen vollständig gesättigten 5- oder 6-gliedrigen Ring ausbildet;
R₂ ein linearer oder verzweigter C₁ bis C₅ Alkylrest, ein 5- oder 6-gliedriger Cycloalkylrest oder ein 5- oder 6-gliedriger Arylrest ist, wobei der Alkylrest, der Cycloalkylrest oder der Arylrest optional ein oder zwei (Ring-)Heteroatome beinhalten.
R₃, R₄, R₅, R₆ und R₇ gleich oder verschieden sind und ausgewählt sind aus der Gruppe bestehend aus
   Wasserstoff;
   Halogenen;
   linearen oder verzweigten C₁-bis C₆-Alkylgruppen, die gegebenenfalls 1, 2, 3 oder 4 gleiche oder verschiedene Substituenten tragen, die ausgewählt sind aus der Gruppe bestehend aus NH₂, OH, SH, Halogenen oder linearen oder verzweigten C₁- bis C₆-Alkylgruppen;
   linearen oder verzweigten C₁- bis C₆-Alkoxygruppen, die gegebenenfalls 1, 2, 3 oder 4 gleiche oder verschiedene Substituenten tragen, die ausgewählt sind aus der Gruppe bestehend aus NH₂, OH, SH, Halogenen oder C₁- bis C₆-Alkoxygruppen;
   ein- oder mehrkernige Aryl-, Arylalkyl- und Heteroarylgruppen, die gegebenenfalls 1, 2, 3 oder 4 gleiche oder verschiedene Substituenten tragen, die ausgewählt sind aus der Gruppe bestehend aus NH₂, OH, SH, Halogenen, linearen oder verzweigten C₁- bis C₆-Alkylgruppen; wobei die Heteroarylgruppen 1, 2, 3 oder 4 Ring-Heteroatome aufweisen, die gleich oder verschieden sind und ausgewählt sind aus der Gruppe bestehend aus O, N und S, oder
zwei benachbarte Reste R₃, R₄, R₅, R₆ und R₇ zusammen mit den Kohlenstoffatomen, an die sie gebunden sind, einen 4-, 5-, 6- oder 7-gliedrigen, ein- oder mehrfach ungesättigten heterocyclischen Rind bilden, der gegebenenfalls 1, 2, 3, 4 oder 5 gleiche oder verschiedene Substituenten trägt, die ausgewählt sind aus der Gruppe bestehend aus linearen oder verzweigten C₁- bis C₆-Alkylgruppen, und der 1, 2 oder 3 Ring-Heteroatome aufweist, die gleich oder verschieden sind und ausgewählt sind aus der Gruppe bestehend aus O, N und S;
X
   - ausgewählt ist aus der Gruppe bestehend aus
      -C₁- bis C₄-Alkylengruppen; -C₂- bis C₄-Alkenylengruppen, sowie -A-C₁- bis C₄- oder -C₁- bis C₄-A- Alkylengruppen oder -A-C₂- bis C₄- oder -C₂- bis C₄-A-Alkenylengruppen, worin A für O, S oder NH steht; oder
   - für eine chemische Einfachbindung steht;

sowie Salzen dieser Verbindungen, insbesondere Säureadditionssalzen mit anorganischen oder insbesondere organischen, ein- oder insbesondere mehrwertigen Carbonsäuren;
und gegebenenfalls dieser Verbindungen in Stereoisomeren-reiner Form oder Gemischen von Stereoisomeren davon.

In der Literatur gibt es verschiedene Synonyme für "TRPM8": TRPP8, LTRPC6, CMR1, MGC2849, transient receptor potential cation channel subfamily M member 8. Im Sinne der vorliegenden Erfindung sind alle Bezeichnungen mit umfasst. Mit umfasst sind auch alle funktionalen Modifikationen des Rezeptors, wie insbesondere Splicevarianten, Isoformen, wie z.B. TRPM8 CRA_a, TRPM8 CRA_b und alle analogen Rezeptoren aus verschiedenen Organismen, wie Mensch, Maus, Ratte. Die Nukleotid- bzw. Aminosäuresequenzen der verschiedenen Rezeptoren sind an sich bekannt und in Sequenzdatenbanken hinterlegt. So ist z.B. die Sequenzinformation für hTRPM8 unter der Nummer NM_024080 eingetragen.

Ein "Modulator" im Sinne der Erfindung stellt eine Verbindung dar, die als Agonist und/oder Antagonist des TRPM8-Rezeptors *in vivo* und/oder *in vitro,* insbesondere *in vivo* wirken kann.

Geeignete Modulatoren können dabei entweder nur als Antagonist oder Agonist, insbesondere nur als Agonist, oder sowohl als Antagonist als auch als Agonist agieren. Dabei können sich insbesondere eine agonistische oder eine antagonistische Wirkung in Abhängigkeit von der jeweiligen gewählten Modulatorkonzentration einstellen.

Ein "Agonist" ist dabei eine Verbindung, welche eine Aktivierung des TRPM8-Rezeptors vermittelt, also einen Ca²⁺ -Einstrom in die kälte-sensitiven Neuronen induziert und damit ein Kältegefühl vermittelt.

Ein "Antagonist" ist dagegen eine Verbindung, welche dieser Aktivierung des TRPM8-Rezeptors entgegenwirken kann.

Die erfindungsgemäßen Modulatoren können ihre Wirkung dadurch ausüben, dass sie reversibel oder irreversibel, spezifisch oder unspezifisch an ein TRPM8-Rezeptormolekül binden. Gewöhnlich erfolgt die Bindung nicht-kovalent über ionische und/oder nichtionische, wie z.B. hydrophobe, Wechselwirkungen mit dem Rezeptormolekül. "Spezifisch" umfasst dabei sowohl ausschließliche Wechselwirkung mit einem oder mehreren verschiedenen TRPM8-Rezeptormolekülen (wie z.B. TRPM8-Molekülen verschiedenen Ursprungs oder verschiedenen Isoformen). "Unspezifisch" ist dagegen eine Wechselwirkung des Modulators mit mehreren verschiedenen Rezeptormolekülen unterschiedlicher Funktion und/oder Sequenz, wobei sich jedoch als Folge eine gewünschte agonistische und/oder antagonistische Modulation (wie oben beschrieben) des TRPM8-Rezeptors feststellen lässt.

Ein erfindungsgemäßer Modulator dient insbesondere zur Induktion von Kältegefühl, bei Mensch und/oder Tier. Eine "Induktion von Kältegefühl" liegt vorzugsweise dann vor, wenn die Verbindung im hierin beschriebenen zellulären Aktivitätstest einen agonistischen Effekt auf hTRPM8 zeigt.

Erfindungsgemäße Mittel enthalten neben den für das jeweilige Mittel üblichen Bestandteilen eine wirksame Menge wenigstens eines erfindungsgemäßen Modulators. "Wirksam" bedeutet in diesem Zusammenhang eine Konzentration des Modulators, die ausreicht, um bei Applikation des Mittels (z.B. Auftragung auf die Haut) den gewünschten Effekt, wie z.B. pharmakologischen Effekt, oder sensorischen Effekt wie z.B. den olfaktorischen Kälteeffekt zu vermitteln.

Werden keine anderslautenden Angaben gemacht, so gelten im Rahmen der vorliegenden Erfindung die folgenden allgemeinen Bedeutungen:
**Halogen:** F, CI, Br oder J
**Alkyl sowie alle Alkylteile in davon abgeleiteten Resten, wie z,B. Alkoxy, Alkylthio, Alkoxyalkyl, Alkoxyalkoxy, Alkylamino und Dialkylamino:** gesättigte, lineare oder verzweigte Kohlenwasserstoffreste mit 1 bis 4, 1 bis 6, 1 bis 8 oder 1 bis 10 Kohlenstoffatomen, z. B.
   - **C₁- bis C₆-Alkyl** wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.
   - **C₁- bis C₆-Alkoxy,** umfassend **C₁- bis C₄-Alkoxy,** wie z.B. Methoxy, Ethoxy, n-Propoxy, 1-Methylethoxy, Butoxy, 1-Methylpropoxy, 2-Methylpropoxy oder 1,1-Dimethylethoxy;
sowie z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
**Cycloalkyl (Ring):** carbocyclische Reste mit 3 bis 20 Kohlenstoffatomen, wie z.B.
   - **C₃- bis C₁₂-Cycloalkyl,** wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutylethyl, Cyclopentyl-methyl, Cyclopentyl-ethyl, Cyclohexyl-methyl oder
   - **C₃- bis C₇-Cycloalkyl,** wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann.
**Alkylen:** lineare oder ein- oder mehrfach verzweigte Kohlenwasserstoff-Brückengruppen mit 1 bis 20 Kohlenstoffatomen, wie z.B.
   - **C₁- bis C₇-Alkylengruppen** ausgewählt unter -CH₂-, -(CH₂)₂-, -(CH₂)₃-,-(CH₂)₄-, -(CH₂)₂-CH(CH₃)-, -CH₂-CH(CH₃)-CH₂- , (CH₂)₄-, -(CH₂)₅-, -(CH₂)₆, -(CH₂)₇-, -CH(CH₃)-CH₂-CH₂-CH(CH₃)- oder -CH(CH₃)-CH₂-CH₂-CH₂-CH(CH₃)- oder
   - **C₁- bis C₄-Alkylengruppen** ausgewählt unter -CH₂-, -(CH₂)₂-, -(CH₂)₃-,-(CH₂)₄-, -(CH₂)₂-CH(CH₃)-, -CH₂-CH(CH₃)-CH₂-.
**Alkenylen:** die ein- oder mehrfach, insbesondere einfach ungesättigten Analoga obiger Alkylengruppen mit 2 bis 20 Kohlenstoffatomen, insbesondere für **C₂- bis C₇-Alkenylene** oder **C₂- bis C₄**-**Alkenylen,** wie -CH=CH-, -CH=CH-CH₂-, -CH₂-CH=CH-, -CH=CH-CH₂-CH₂-, -CH₂-CH=CH-CH₂-, -CH₂-CH₂-CH=CH-, -CH(CH₃)-CH=CH-, -CH₂-C(CH₃)=CH-,
**Aryl:** ein- oder mehrkernige, vorzugsweise ein- oder zweikernige, gegebenenfalls substituierte aromatische Reste mit 6 bis 20, insbesondere 6 bis 10 Ring-Kohlenstoffatomen, wie z.B. Phenyl, Biphenyl, Naphthyl wie 1- oder 2-Naphthyl, Tetrahydronaphthyl, Fluorenyl, Indenyl und Phenanthrenyl. Diese Arylreste können gegebenenfalls 1, 2, 3, 4, 5 oder 6 gleiche oder verschiedene Substituenten tragen.
**Arylalkyl:** die Aryl-substituierten Analoga obiger Alkylreste, wobei Aryl ebenfalls die oben angegebenen Bedeutungen besitzt, wie z.B. Phenyl-C₁- bis C₄-Alkylreste ausgewählt unter Phenyl-methyl oder Phenyl-ethyl.
**Heterocyclyl (heterocyclischer Ring):** fünf- bis siebengliedriger gesättigter, partiell ungesättigter oder aromatische Heterocyclen bzw. Heterocyclylreste, enthaltend ein, zwei, drei oder vier Heteroatome aus der Gruppe O, N oder S, wie z.B.
   - 5- oder 6-gliedriges gesättigtes oder einfach ungesättigtes Heterocyclyl, enthaltend ein bis zwei Stickstoffatome und/oder ein Sauerstoff- oder Schwefelatom oder ein oder zwei Sauerstoff- und/oder Schwefelatome als Ringglieder, z. B. 2-Tetrahydrofuranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
   - 5-gliedriges aromatisches Heterocyclyl (= **Heteroaryl** bzw. **Hetaryl),** enthaltend neben Kohlenstoffatomen ein, zwei oder drei Stickstoffatome oder ein oder zwei Stickstoffatome und ein Schwefel- oder Sauerstoffatom als Ringglieder, z. B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
   - 5-gliedriges aromatisches Heterocyclyl (= **Heteroaryl** bzw. **Hetaryl),** das 1, 2, 3 oder 4 Stickstoffatome als Ringglieder aufweist, wie 1-, 2- oder 3-Pyrrolyl, 1-, 3- oder 4-Pyrazolyl, 1-, 2- oder 4-Imidazolyl, 1,2,3-[1H]-Triazol-1-yl, 1,2,3-[2H]-Triazol-2-yl, 1,2,3-[1H]-Triazol-4-yl, 1,2,3-[1H]-Triazol-5-yl, 1,2,3-[2H]-Triazol-4-yl, 1,2,4-[1H]-Triazol-1-yl, 1,2,4-[1H]-Triazol-3-yl, 1,2,4-[1H]-Triazol-5-yl, 1,2,4-[4H]-Triazol-4-yl, 1,2,4-[4H]-Triazol-3-yl, [1H]-Tetrazol-1-yl, [1H]-Tetrazol-5-yl, [2H]-Tetrazol-2-yl und [2H]-Tetrazol-5-yl;
   - 5-gliedriges aromatisches Heterocyclyl (= **Heteroaryl** bzw. **Hetaryl),** das 1 unter Sauerstoff und Schwefel ausgewähltes Heteroatom und gegebenenfalls 1, 2 oder 3 Stickstoffatome als Ringglieder aufweist, beispielsweise 2-Furyl, 3-Furyl, 2-Thienyl, 3-Thienyl, 3- oder 4-Isoxazolyl, 3- oder 4-Isothiazolyl, 2-, 4- oder 5-Oxazolyl, 2-, 4 oder 5-Thiazolyl, 1,2,4-Thiadiazol-3-yl, 1,2,4-Thiadiazol-5-yl, 1,3,4-Thiadiazol-2-yl, 1,2,4-Oxadiazol-3-yl, 1,2,4-Oxadiazol-5-yl und 1,3,4-Oxadiazol-2-yl;
   - 6-gliedriges Heterocyclyl (= **Heteroaryl** bzw. **Hetaryl),** enthaltend neben Kohlenstoffatomen ein oder zwei bzw. ein, zwei oder drei Stickstoffatome als Ringglieder, z. B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,2,4-Triazin-3-yl; 1,2,4-Triazin-5-yl, 1,2,4-Triazin-6-yl und 1,3,5-Triazin-2-yl;
**Substituenten,** wie insbesondere für obige Reste, sind insbesondere ausgewählt unter Ketogruppen, -COOH, -COO-Alkyl, -OH, -SH, -CN, Amino, -NO₂, Alkyl, oder, wobei in den Alkylgruppen ein oder mehrere H-Atome durch Halogen, ersetzt sein können.

In einer bevorzugten Ausführungsform des erfindungsgemäßen Verfahrens sind die Verbindungen des Strukturtyps 1 ausgewählt aus der Gruppe A, bestehend aus den folgenden Verbindungen:

| Struktur | Name |
|---|---|
| 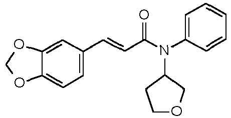 | (E)-3-(1,3-benzodioxol-5-yl)-N-phenyl-N-tetrahydrofuran-3-yl-prop-2-enamid |
| 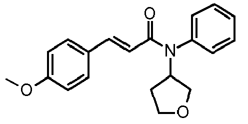 | (E)-3-(4-Methoxyphenyl)-N-phenyl-N-tetrahydrofuran-3-yl-prop-2-enamid |
| 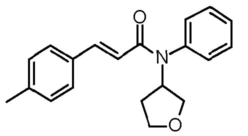 | (E)-N-phenyl-3-(p-tolyl)-N-tetrahydrofuran-3-yl-prop-2-enamid |
| 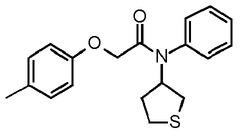 | 2-(4-Methylphenoxy)-N-phenyl-N-tetrahydrothiophen-3-yl-acetamid |
| 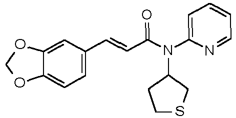 | (E)-3-(1,3-benzodioxol-5-yl)-N-(2-pyridyl)-N-tetrahydrothiophen-3-yl-prop-2-enamid |
| 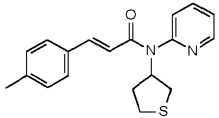 | (E)-3-(p-tolyl)-N-(2-pyridyl)-N-tetrahydrothiophen-3-yl-prop-2-enamid |
| 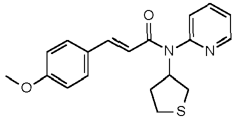 | (E)-3-(4-methoxyphenyl)-N-(2-pyridyl)-N-tetrahydrothiophen-3-yl-prop-2-enamid |
| 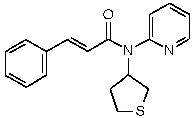 | (E)-3-phenyl-N-(2-pyridyl)-N-tetrahydrothiophen-3-yl-prop-2-enamid |
| 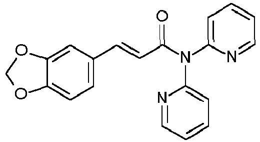 | (E)-3-(1,3-Benzodioxol-5-yl)-N,N-bis(2-pyridyl)prop-2-enamid |
| 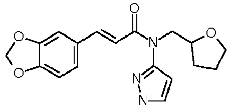 | (E)-3-(1,3-benzodioxol-5-yl)-N-(1H-pyrazol-3-yl)-N-(tetrahydrofuran-2-ylmethyl)prop-2-enamid |
| 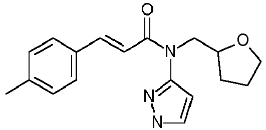 | (E)-3-(p-tolyl)-N-(1H-pyrazol-3-yl)-N-(tetrahydrofuran-2-ylmethyl)prop-2-enamid |
| 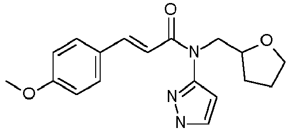 | (E)-3-(4-methoxyphenyl)-N-(1H-pyrazol-3-yl)-N-(tetrahydrofuran-2-ylmethyl)prop-2-enamid |
| 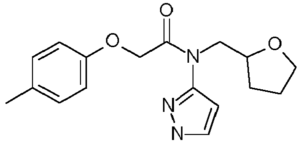 | 2-(4-methylphenoxy)-N-(1H-pyrazol-3-yl)-N-(tetrahydrofuran-2-ylmethyl)acetamid |
| 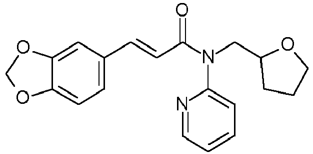 | (E)-3-(1,3-benzodioxol-5-yl)-N-(2-pyridyl)-N-(tetrahydrofuran-2-ylmethyl)prop-2-enamid |
| 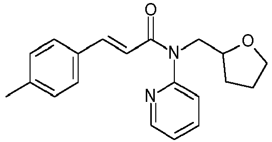 | (E)-3-(p-tolyl)-N-(2-pyridyl)-N-(tetrahydrofuran-2-ylmethyl)prop-2-enamid |
| 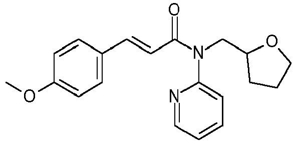 | (E)-3-(4-methoxyphenyl)-N-(2-pyridyl)-N-(tetrahydrofuran-2-ylmethyl)prop-2-enamid |
| 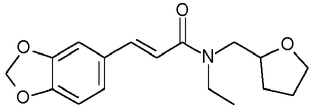 | (E)-3-(1,3-benzodioxol-5-yl)-N-ethyl-N-(tetrahydrofuran-2-ylmethyl)prop-2-enamid |
| 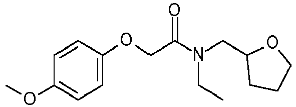 | N-ethyl-2-(4-methoxyphenoxy)-N-(tetrahydrofuran-2-ylmethyl)acetamid |
| 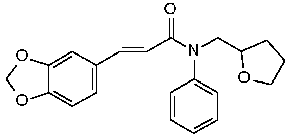 | (E)-3-(1,3-benzodioxol-5-yl)-N-phenyl-N-(tetrahydrofuran-2-ylmethyl)prop-2-enamid |
| 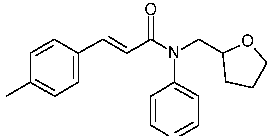 | (E)-N-phenyl-3-(p-tolyl)-N-(tetrahydrofuran-2-ylmethyl)prop-2-enamid |
| 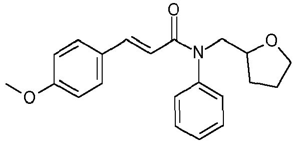 | (E)-3-(4-methoxyphenyl)-N-phenyl-N-(tetrahydrofuran-2-ylmethyl)prop-2-enamid |
| 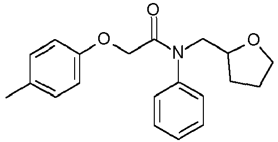 | 2-(4-methylphenoxy)-N-phenyl-N-(tetrahydrofuran-2-ylmethyl)acetamid |
| 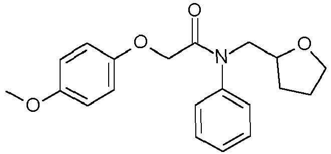 | 2-(4-methoxyphenoxy)-N-phenyl-N-(tetrahydrofuran-2-ylmethyl)acetamid |
| 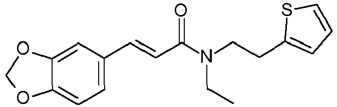 | (E)-3-(1,3-benzodioxol-5-yl)-N-ethyl-N-[2-(2-thienyl)ethyl]prop-2-enamid |
| 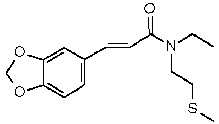 | (E)-3-(1,3-benzodioxol-5-yl)-N-ethyl-N-(2-methylsulfanylethyl)prop-2-enamid |
| 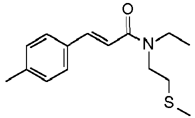 | (E)-N-ethyl-N-(2-methylsulfanylethyl)-3-(p-tolyl)prop-2-enamid |
| 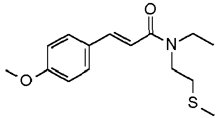 | (E)-N-ethyl-3-(4-methoxyphenyl)-N-(2-methylsulfanylethyl)prop-2-enamid |
| 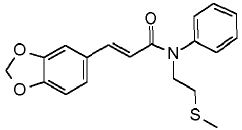 | (E)-3-(1,3-benzodioxol-5-yl)-N-(2-methylsulfanylethyl)-N-phenyl-prop-2-enamid |
| 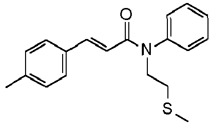 | (E)-N-(2-methylsulfanylethyl)-N-phenyl-3-(p-tolyl)prop-2-enamid |
| 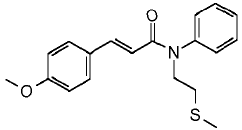 | (E)-3-(4-methoxyphenyl)-N-(2-methylsulfanylethyl)-N-phenyl-prop-2-enamid |
| 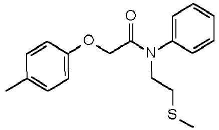 | 2-(4-methylphenoxy)-N-(2-methylsulfanylethyl)-N-phenyl-acetamid |
| 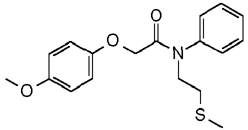 | 2-(4-methoxyphenoxy)-N-(2-methylsulfanylethyl)-N-phenyl-acetamid |
| 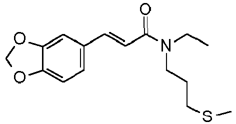 | (E)-3-(1,3-benzodioxol-5-yl)-N-ethyl-N-(3-methylsulfanylpropyl)prop-2-enamid |
| 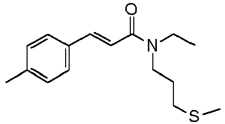 | (E)-N-ethyl-N-(3-methylsulfanylpropyl)-3-(p-tolyl)prop-2-enamid |
| 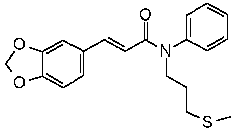 | (E)-3-(1,3-benzodioxol-5-yl)-N-(3-methylsulfanylpropyl)-N-phenyl-prop-2-enamid |
| 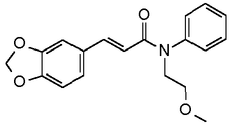 | (E)-3-(1,3-benzodioxol-5-yl)-N-(2-methoxyethyl)-N-phenyl-prop-2-enamid |
| 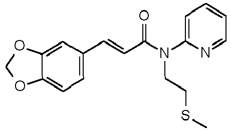 | (E)-3-(1,3-benzodioxol-5-yl)-N-(2-methylsulfanylethyl)-N-(2-pyridyl)prop-2-enamid |
| 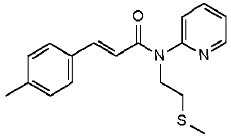 | (E)-N-(2-methylsulfanylethyl)-3-(p-tolyl)-N-(2-pyridyl)prop-2-enamid |
| 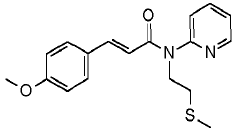 | (E)-3-(4-methoxyphenyl)-N-(2-methylsulfanylethyl)-N-(2-pyridyl)prop-2-enamid |
| 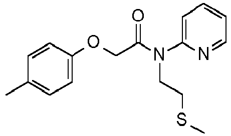 | 2-(4-methylphenoxy)-N-(2-methylsulfanylethyl)-N-(2-pyridyl)acetamid |
| 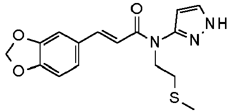 | (E)-3-(1,3-benzodioxol-5-yl)-N-(2-methylsulfanylethyl)-N-(1H-pyrazol-3-yl)prop-2-enamid |
| 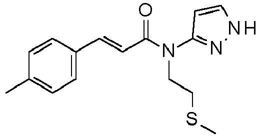 | (E)-N-(2-methylsulfanylethyl)-3-(p-tolyl)-N-(1H-pyrazol-3-yl)prop-2-enamid |
| 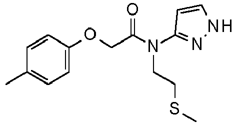 | 2-(4-methylphenoxy)-N-(2-methylsulfanylethyl)-N-(1H-pyrazol-3-yl)acetamid |
| 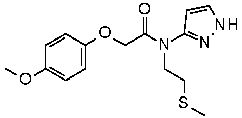 | 2-(4-methoxyphenoxy)-N-(2-methylsulfanylethyl)-N-(1H-pyrazol-3-yl)acetamid |

Sofern Differenzen zwischen der Namensbezeichnung und dem jeweiligen Formelbild bestehen sollten, ist die Aussage des Formelbildes maßgebend.

Besonders bevorzugt sind dabei die Verbindungen der Gruppe A ausgewählt aus der Gruppe B, bestehend aus den folgenden Verbindungen:

| Struktur | Name |
|---|---|
| 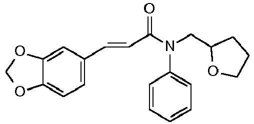 | (E)-3-(1,3-benzodioxol-5-yl)-N-phenyl-N-(tetrahydrofuran-2-ylmethyl)prop-2-enamid |
| 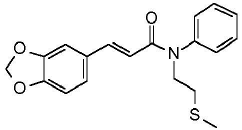 | (E)-3-(1,3-benzodioxol-5-yl)-N-(2-methylsulfanylethyl)-N-phenyl-prop-2-enamid |
| 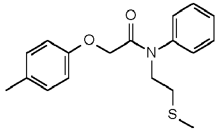 | 2-(4-methylphenoxy)-N-(2-methylsulfanylethyl)-N-phenyl-acetamid |
| 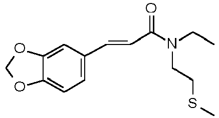 | (E)-3-(1,3-benzodioxol-5-yl)-N-ethyl-N-(2-methylsulfanylethyl)prop-2-enamid |
| 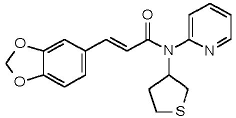 | (E)-3-(1,3-benzodioxol-5-yl)-N-(2-pyridyl)-N-tetrahydrothiophen-3-yl-prop-2-enamid |
| 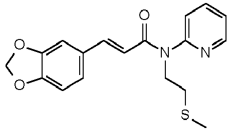 | (E)-3-(1,3-benzodioxol-5-yl)-N-(2-methylsulfanylethyl)-N-(2-pyridyl)prop-2-enamid |
| 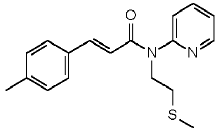 | (E)-N-(2-methylsulfanylethyl)-3-(p-tolyl)-N-(2-pyridyl)prop-2-enamid |
| 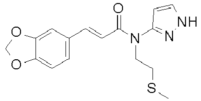 | (E)-3-(1,3-benzodioxol-5-yl)-N-(2-methylsulfanylethyl)-N-(1H-pyrazol-3-yl)prop-2-enamid |
| 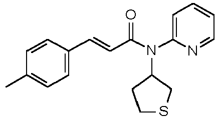 | (E)-3-(p-tolyl)-N-(2-pyridyl)-N-tetrahydrothiophen-3-yl-prop-2-enamid |
| 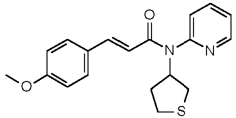 | (E)-3-(4-methoxyphenyl)-N-(2-pyridyl)-N-tetrahydrothiophen-3-yl-prop-2-enamid |
| 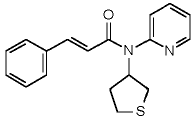 | (E)-3-phenyl-N-(2-pyridyl)-N-tetrahydrothiophen-3-yl-prop-2-enamid |
| 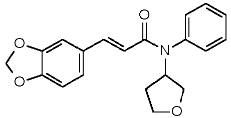 | (E)-3-(1,3-benzodioxol-5-yl)-N-phenyl-N-tetrahydrofuran-3-yl-prop-2-enamid |
| 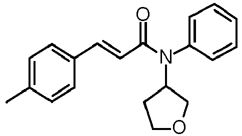 | (E)-N-phenyl-3-(p-tolyl)-N-tetrahydrofuran-3-yl-prop-2-enamid |

Weiter bevorzugt sind die Verbindungen der Gruppen A und B ausgewählt aus der Gruppe C, bestehend aus den folgenden Verbindungen:

| Struktur | Name |
|---|---|
| 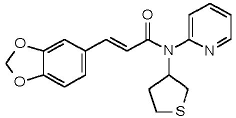 | (E)-3-(1,3-benzodioxol-5-yl)-N-(2-pyridyl)-N-tetrahydrothiophen-3-yl-prop-2-enamid |
| 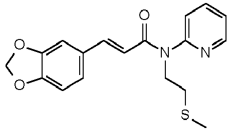 | (E)-3-(1,3-benzodioxol-5-yl)-N-(2-methylsulfanylethyl)-N-(2-pyridyl)prop-2-enamid |
| 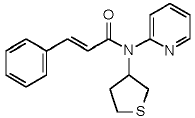 | (E)-3-phenyl-N-(2-pyridyl)-N-tetrahydrothiophen-3-yl-prop-2-enamid |
| 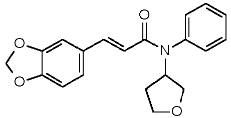 | (E)-3-(1,3-benzodioxol-5-yl)-N-phenyl-N-tetrahydrofuran-3-yl-prop-2-enamid |

Gemäß einer Ausgestaltung der vorliegenden Erfindung sind die Verbindungen des Strukturtyps 1 nicht ausgewählt aus den Verbindungen wie in WO2011/061330 beschrieben.

In einer bevorzugten Ausführungsform der vorliegenden Erfindung sind die Verbindungen des Strukturtyps 1 daher nicht ausgewählt aus der Gruppe D, bestehend aus den folgenden Verbindungen: 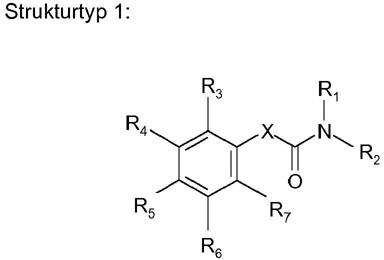 worin
R₁ aus folgender Struktur besteht: 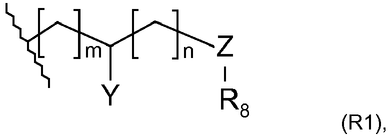 worin
   Z ausgewählt ist aus der Gruppe bestehend aus O und S;
   n gleich 0 oder 1 ist;
   m gleich 0 ist und Y NR₉ oder CR₉ ist und R₉ mit R₈ einen aromatischen oder einen vollständig gesättigten 5- oder 6-gliedrigen Ring ausbildet;
   und
worin R₂ ein linearer oder verzweigter C₁ bis C₅ Alkylrest, ein 5- oder 6-gliedriger Cycloalkylrest oder ein 5- oder 6-gliedriger Arylrest ist, wobei der Cycloalkylrest oder der Arylrest optional ein oder zwei (Ring-)Heteroatome beinhalten, die gleich oder verschieden sind und ausgewählt sind unter O, N und S;
R₃, R₄, R₅, R₆ und R₇ gleich oder verschieden sind und ausgewählt sind aus der Gruppe bestehend aus
   Wasserstoff;
   Halogenen;
   linearen oder verzweigten C₁- bis C₆-Alkylgruppen, die gegebenenfalls 1, 2, 3 oder 4 gleiche oder verschiedene Substituenten tragen, die ausgewählt sind aus der Gruppe bestehend aus NH₂, OH, SH, Halogenen oder linearen oder verzweigten C₁- bis C₆-Alkylgruppen;
   linearen oder verzweigten C₁- bis C₆-Alkoxygruppen, die gegebenenfalls 1, 2, 3 oder 4 gleiche oder verschiedene Substituenten tragen, die ausgewählt sind aus der Gruppe bestehend aus NH₂, OH, SH, Halogenen oder C₁- bis C₆-Alkoxygruppen;
   ein- oder mehrkernige Aryl-, Arylalkyl- und Heteroarylgruppen, die gegebenenfalls 1, 2, 3 oder 4 gleiche oder verschiedene Substituenten tragen, die ausgewählt sind aus der Gruppe bestehend aus NH₂, OH, SH, Halogenen, linearen oder verzweigten C₁- bis C₆-Alkylgruppen; wobei die Heteroarylgruppen 1, 2, 3 oder 4 Ring-Heteroatome aufweisen, die gleich oder verschieden sind und ausgewählt sind aus der Gruppe bestehend aus O, N und S, oder
   zwei benachbarte Reste R₃, R₄, R₅, R₆ und R₇ zusammen mit den Kohlenstoffatomen, an die sie gebunden sind, einen 4-, 5-, 6- oder 7-gliedrigen, ein- oder mehrfach ungesättigten heterocyclischen Rind bilden, der gegebenenfalls 1, 2, 3, 4 oder 5 gleiche oder verschiedene Substituenten trägt, die ausgewählt sind aus der Gruppe bestehend aus linearen oder verzweigten C₁- bis C₆-Alkylgruppen, und der 1, 2 oder 3 Ring-Heteroatome aufweist, die gleich oder verschieden sind und ausgewählt sind aus der Gruppe bestehend aus O, N und S;
   X
      - ausgewählt ist aus der Gruppe bestehend aus
         -C₁- bis C₄-Alkylengruppen; -C₂- bis C₄-Alkenylengruppen, sowie -A-C₁- bis C₄- oder -C₁-bis C₄-A- Alkylengruppen oder -A-C₂- bis C₄- oder -C₂- bis C₄-A-Alkenylengruppen, worin A für O, S oder NH steht; oder
      - für eine chemische Einfachbindung steht;
sowie Salzen dieser Verbindungen, insbesondere Säureadditionssalzen mit anorganischen oder insbesondere organischen, ein- oder insbesondere mehrwertigen Carbonsäuren;
und gegebenenfalls dieser Verbindungen in Stereoisomeren-reiner Form oder Gemischen von Stereoisomeren davon.

In einer weiteren, bevorzugten Ausführungsform der vorliegenden Erfindung sind die Verbindungen des Strukturtyps 1 nicht ausgewählt aus der Gruppe E, bestehend aus den folgenden Verbindungen:

| Struktur | Name |
|---|---|
| 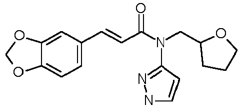 | (E)-3-(1,3-benzodioxol-5-yl)-N-(1H-pyrazol-3-yl)-N-(tetrahydrofuran-2-ylmethyl)prop-2-enamid |
| 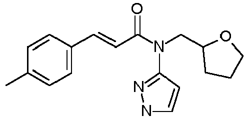 | (E)-3-(p-tolyl)-N-(1H-pyrazol-3-yl)-N-(tetrahydrofuran-2-ylmethyl)prop-2-enamid |
| 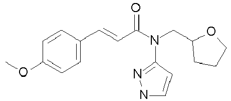 | (E)-3-(4-methoxyphenyl)-N-(1H-pyrazol-3-yl)-N-(tetrahydrofuran-2-ylmethyl)prop-2-enamid |
| 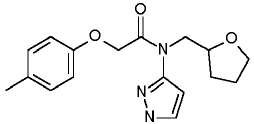 | 2-(4-methylphenoxy)-N-(1H-pyrazol-3-yl)-N-(tetrahydrofuran-2-ylmethyl)acetamid |
| 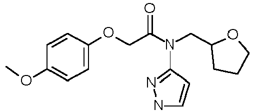 | 2-(4-methoxyphenoxy)-N-(1H-pyrazol-3-yl)-N-(tetrahydrofuran-2-ylmethyl)acetamid |
| 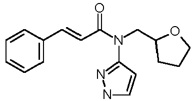 | (E)-3-phenyl-N-(1H-pyrazol-3-yl)-N-(tetrahydrofuran-2-ylmethyl)prop-2-enamid |
| 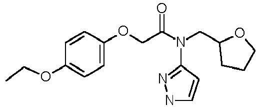 | 2-(4-ethoxyphenoxy)-N-(1H-pyrazol-3-yl)-N-(tetrahydrofuran-2-ylmethyl)acetamid |
| 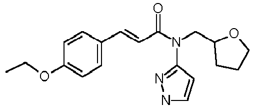 | (E)-3-(4-ethoxyphenyl)-N-(1H-pyrazol-3-yl)-N-(tetrahydrofuran-2-ylmethyl)prop-2-enamid |
| 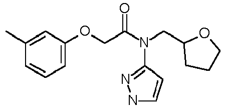 | 2-(3-methylphenoxy)-N-(1H-pyrazol-3-yl)-N-(tetrahydrofuran-2-ylmethyl)acetamid |
| 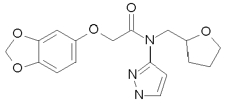 | 2-(1,3-benzodioxol-5-yloxy)-N-(1H-pyrazol-3-yl)-N-(tetrahydrofuran-2-ylmethyl)acetamid |
| 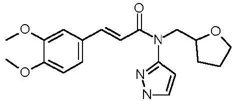 | (E)-3-(3,4-dimethoxyphenyl)-N-(1H-pyrazol-3-yl)-N-(tetrahydrofuran-2-ylmethyl)prop-2-enamid |
| 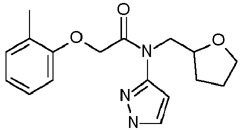 | 2-(2-methylphenoxy)-N-(1H-pyrazol-3-yl)-N-(tetrahydrofuran-2-ylmethyl)acetamid |
| 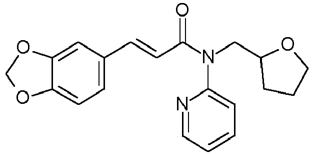 | (E)-3-(1,3-benzodioxol-5-yl)-N-(2-pyridyl)-N-(tetrahydrofuran-2-ylmethyl)prop-2-enamid |
| 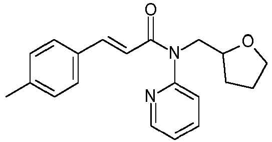 | (E)-3-(p-tolyl)-N-(2-pyridyl)-N-(tetrahydrofuran-2-ylmethyl)prop-2-enamid |
| 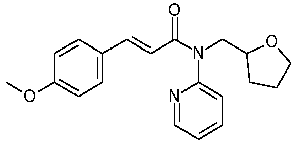 | (E)-3-(4-methoxyphenyl)-N-(2-pyridyl)-N-(tetrahydrofuran-2-ylmethyl)prop-2-enamid |
| 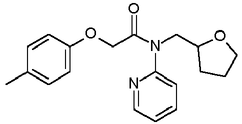 | 2-(4-methylphenoxy)-N-(2-pyridyl)-N-(tetrahydrofuran-2-ylmethyl)acetamid |
| 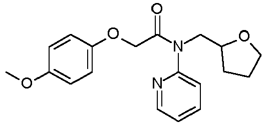 | 2-(4-methoxyphenoxy)-N-(2-pyridyl)-N-(tetrahydrofuran-2-ylmethyl)acetamid |
| 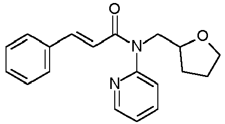 | (E)-3-phenyl-N-(2-pyridyl)-N-(tetrahydrofuran-2-ylmethyl)prop-2-enamid |
| 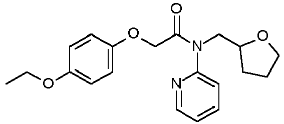 | 2-(4-ethoxyphenoxy)-N-(2-pyridyl)-N-(tetrahydrofuran-2-ylmethyl)acetamid |
| 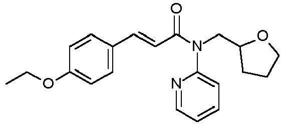 | (E)-3-(4-ethoxyphenyl)-N-(2-pyridyl)-N-(tetrahydrofuran-2-ylmethyl)prop-2-enamid |
| 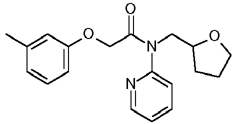 | 2-(3-methylphenoxy)-N-(2-pyridyl)-N-(tetrahydrofuran-2-ylmethyl)acetamid |
| 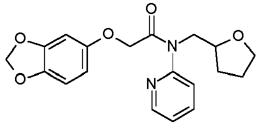 | 2-(1,3-benzodioxol-5-yloxy)-N-(2-pyridyl)-N-(tetrahydrofuran-2-ylmethyl)acetamid |
| 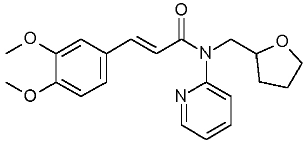 | (E)-3-(3,4-dimethoxyphenyl)-N-(2-pyridyl)-N-(tetrahydrofuran-2-ylmethyl)prop-2-enamid |
| 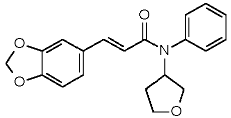 | (E)-3-(1,3-benzodioxol-5-yl)-N-phenyl-N-tetrahydrofuran-3-yl-prop-2-enamid |
| 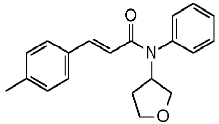 | (E)-N-phenyl-3-(p-tolyl)-N-tetrahydrofuran-3-yl-prop-2-enamid |
| 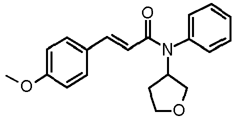 | (E)-3-(4-Methoxyphenyl)-N-phenyl-N-tetrahydrofuran-3-yl-prop-2-enamid |
| 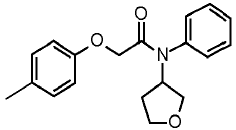 | 2-(4-Methylphenoxy)-N-phenyl-N-tetrahydrofuran-3-yl-acetamid |
| 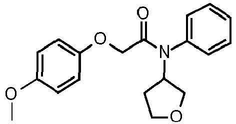 | 2-(4-methoxyphenoxy)-N-phenyl-N-tetrahydrofuran-3-yl-acetamid |
| 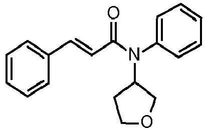 | (E)-N,3-diphenyl-N-tetrahydrofuran-3-yl-prop-2-enamid |
| 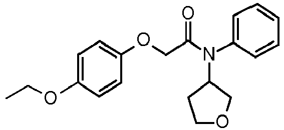 | 2-(4-ethoxyphenoxy)-N-phenyl-N-tetrahydrofuran-3-yl-acetamid |
| 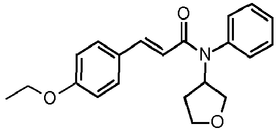 | (E)-3-(4-ethoxyphenyl)-N-phenyl-N-tetrahydrofuran-3-yl-prop-2-enamid |
| 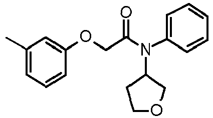 | 2-(3-methylphenoxy)-N-phenyl-N-tetrahydrofuran-3-yl-acetamid |
| 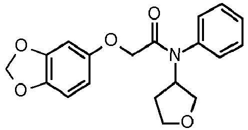 | 2-(1,3-benzodioxol-5-yloxy)-N-phenyl-N-tetrahydrofuran-3-yl-acetamid |
| 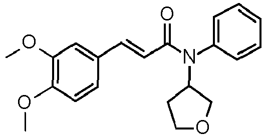 | (E)-3-(3,4-dimethoxyphenyl)-N-phenyl-N-tetrahydrofuran-3-yl-prop-2-enamid |
| 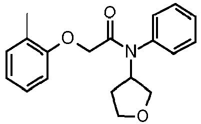 | 2-(2-methylphenoxy)-N-phenyl-N-tetrahydrofuran-3-yl-acetamid |
| 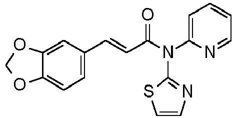 | (E)-3-(1,3-benzodioxol-5-yl)-N-(2-pyridyl)-N-thiazol-2-yl-prop-2-enamid |
| 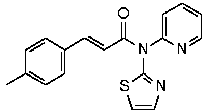 | (E)-3-(p-tolyl)-N-(2-pyridyl)-N-thiazol-2-yl-prop-2-enamid |
| 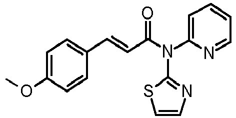 | (E)-3-(4-methoxyphenyl)-N-(2-pyridyl)-N-thiazol-2-yl-prop-2-enamid |
| 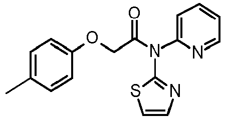 | 2-(4-methylphenoxy)-N-(2-pyridyl)-N-thiazol-2-yl-acetamid |
| 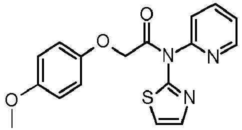 | 2-(4-methoxyphenoxy)-N-(2-pyridyl)-N-thiazol-2-yl-acetamid |
| 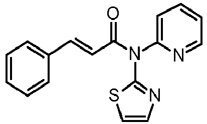 | (E)-3-phenyl-N-(2-pyridyl)-N-thiazol-2-yl-prop-2-enamid |
| 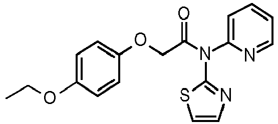 | 2-(4-ethoxyphenoxy)-N-(2-pyridyl)-N-thiazol-2-yl-acetamid |
| 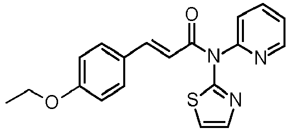 | (E)-3-(4-ethoxyphenyl)-N-(2-pyridyl)-N-thiazol-2-yl-prop-2-enamid |
| 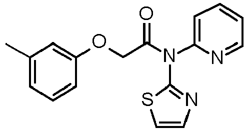 | 2-(3-methylphenoxy)-N-(2-pyridyl)-N-thiazol-2-yl-acetamid |
| 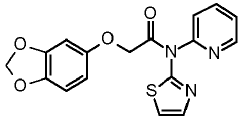 | 2-(1,3-benzodioxol-5-yloxy)-N-(2-pyridyl)-N-thiazol-2-yl-acetamid |
| 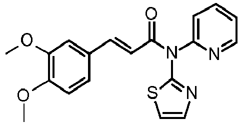 | (E)-3-(3,4-dimethoxyphenyl)-N-(2-pyridyl)-N-thiazol-2-yl-prop-2-enamid |
| 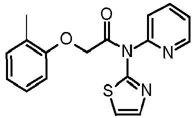 | 2-(2-methylphenoxy)-N-(2-pyridyl)-N-thiazol-2-yl-acetamid |
| 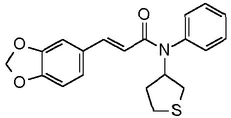 | (E)-3-(1,3-benzodioxol-5-yl)-N-phenyl-N-tetrahydrothiophen-3-yl-prop-2-enamid |
| 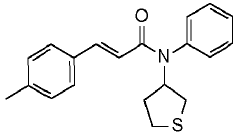 | (E)-N-phenyl-3-(p-tolyl)-N-tetrahydrothiophen-3-yl-prop-2-enamid |
| 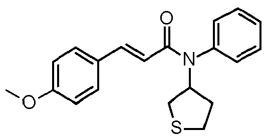 | (E)-3-(4-Methoxyphenyl)-N-phenyl-N-tetrahydrothiophen-3-yl-prop-2-enamid |
| 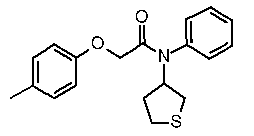 | 2-(4-Methylphenoxy)-N-phenyl-N-tetrahydrothiophen-3-yl-acetamid |
| 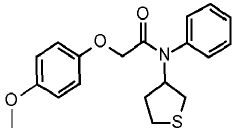 | 2-(4-methoxyphenoxy)-N-phenyl-N-tetrahydrothiophen-3-yl-acetamid |
| 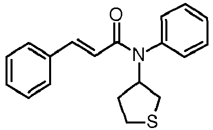 | (E)-N,3-diphenyl-N-tetrahydrothiophen-3-yl-prop-2-enamid |
| 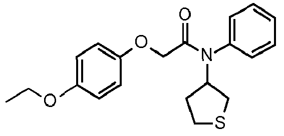 | 2-(4-ethoxyphenoxy)-N-phenyl-N-tetrahydrothiophen-3-yl-acetamid |
| 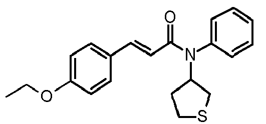 | (E)-3-(4-ethoxyphenyl)-N-phenyl-N-tetrahydrothiophen-3-yl-prop-2-enamid |
| 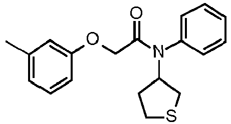 | 2-(3-methylphenoxy)-N-phenyl-N-tetrahydrothiophen-3-yl-acetamid |
| 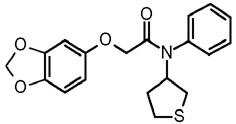 | 2-(1,3-benzodioxol-5-yloxy)-N-phenyl-N-tetrahydrothiophen-3-yl-acetamid |
| 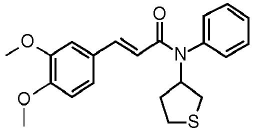 | (E)-3-(3,4-dimethoxyphenyl)-N-phenyl-N-tetrahydrothiophen-3-yl-prop-2-enamid |
| 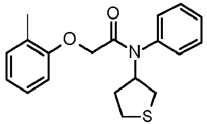 | 2-(2-methylphenoxy)-N-phenyl-N-tetrahydrothiophen-3-yl-acetamid |
| 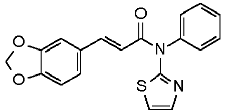 | (E)-3-(1,3-benzodioxol-5-yl)-N-phenyl-N-thiazol-2-yl-prop-2-enamid |
| 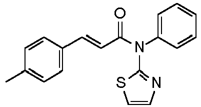 | (E)-N-phenyl-3-(p-tolyl)-N-thiazol-2-yl-prop-2-enamid |
| 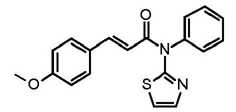 | (E)-3-(4-methoxyphenyl)-N-phenyl-N-thiazol-2-yl-prop-2-enamid |
| 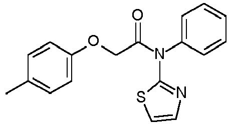 | 2-(4-Methylphenoxy)-N-phenyl-N-thiazol-2-yl-acetamid |
| 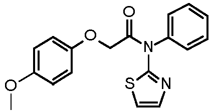 | 2-(4-methoxyphenoxy)-N-phenyl-N-thiazol-2-yl-acetamid |
| 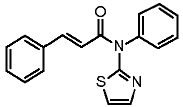 | (E)-N,3-diphenyl-N-thiazol-2-yl-prop-2-enamid |
| 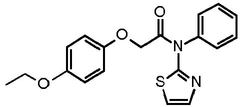 | 2-(4-ethoxyphenoxy)-N-phenyl-N-thiazol-2-yl-acetamid |
| 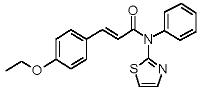 | (E)-3-(4-ethoxyphenyl)-N-phenyl-N-thiazol-2-yl-prop-2-enamid |
| 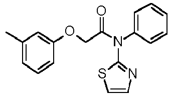 | 2-(3-methylphenoxy)-N-phenyl-N-thiazol-2-yl-acetamid |
| 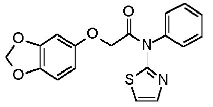 | 2-(1,3-benzodioxol-5-yloxy)-N-phenyl-N-thiazol-2-yl-acetamid |
| 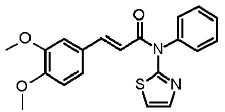 | (E)-3-(3,4-dimethoxyphenyl)-N-phenyl-N-thiazol-2-yl-prop-2-enamide |
| 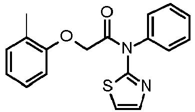 | 2-(2-methylphenoxy)-N-phenyl-N-thiazol-2-yl-acetamid |
| 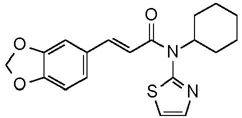 | (E)-3-(1,3-benzodioxol-5-yl)-N-cyclohexyl-N-thiazol-2-yl-prop-2-enamid |
| 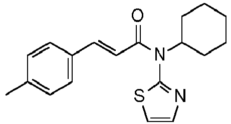 | (E)-N-cyclohexyl-3-(p-tolyl)-N-thiazol-2-yl-prop-2-enamid |
| 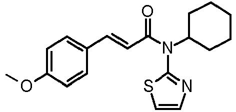 | (E)-N-cyclohexyl-3-(4-methoxyphenyl)-N-thiazol-2-yl-prop-2-enamid |
| 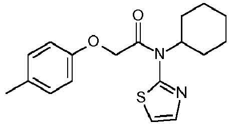 | N-cyclohexyl-2-(4-methylphenoxy)-N-thiazol-2-yl-acetamid |
| 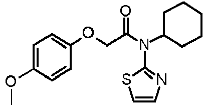 | N-cyclohexyl-2-(4-methoxyphenoxy)-N-thiazol-2-yl-acetamid |
| 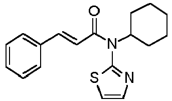 | (E)-N-cyclohexyl-3-phenyl-N-thiazol-2-yl-prop-2-enamid |
| 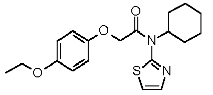 | N-cyclohexyl-2-(4-ethoxyphenoxy)-N-thiazol-2-yl-acetamid |
| 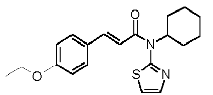 | (E)-N-cyclohexyl-3-(4-ethoxyphenyl)-N-thiazol-2-yl-prop-2-enamid |
| 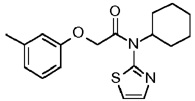 | N-cyclohexyl-2-(3-methylphenoxy)-N-thiazol-2-yl-acetamid |
| 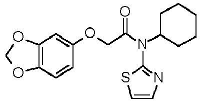 | 2-(1,3-benzodioxol-5-yloxy)-N-cyclohexyl-N-thiazol-2-yl-acetamid |
| 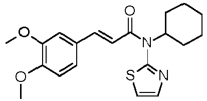 | (E)-N-cyclohexyl-3-(3,4-dimethoxyphenyl)-N-thiazol-2-yl-prop-2-enamid |
| 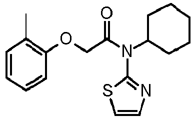 | N-cyclohexyl-2-(2-methylphenoxy)-N-thiazol-2-yl-acetamid |
| 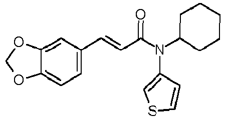 | (E)-3-(1,3-benzodioxol-5-yl)-N-cyclohexyl-N-(3-thienyl)prop-2-enamid |
| 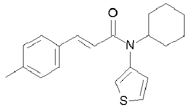 | (E)-N-cyclohexyl-3-(p-tolyl)-N-(3-thienyl)prop-2-enamid |
| 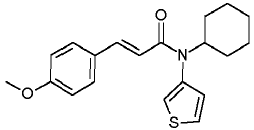 | (E)-N-Cyclohexyl-3-(4-methoxyphenyl)-N-(3-thienyl)prop-2-enamid |
| 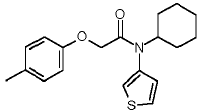 | N-Cyclohexyl-2-(4-methylphenoxy)-N-(3-thienyl)acetamid |
| 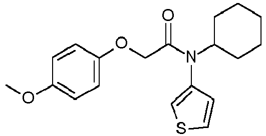 | N-Cyclohexyl-2-(4-methoxyphenoxy)-N-(3-thienyl)acetamid |
| 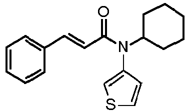 | (E)-N-cyclohexyl-3-phenyl-N-(3-thienyl)prop-2-enamid |
| 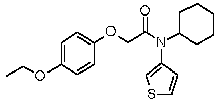 | N-cyclohexyl-2-(4-ethoxyphenoxy)-N-(3-thienyl)acetamid |
| 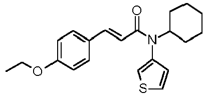 | (E)-N-cyclohexyl-3-(4-ethoxyphenyl)-N-(3-thienyl)prop-2-enamid |
| 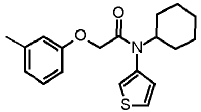 | N-cyclohexyl-2-(3-methylphenoxy)-N-(3-thienyl)acetamid |
| 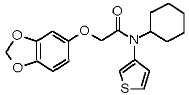 | 2-(1,3-benzodioxol-5-yloxy)-N-cyclohexyl-N-(3-thienyl)acetamid |
| 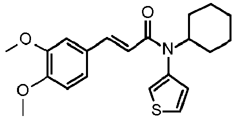 | (E)-N-cyclohexyl-3-(3,4-dimethoxyphenyl)-N-(3-thienyl)prop-2-enamid |
| 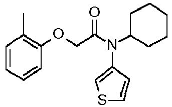 | N-cyclohexyl-2-(2-methylphenoxy)-N-(3-thienyl)acetamid |
| 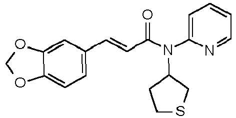 | (E)-3-(1,3-benzodioxol-5-yl)-N-(2-pyridyl)-N-tetrahydrothiophen-3-yl-prop-2-enamid |
| 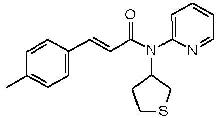 | (E)-3-(p-tolyl)-N-(2-pyridyl)-N-tetrahydrothiophen-3-yl-prop-2-enamid |
| 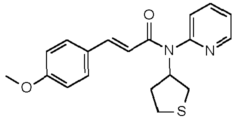 | (E)-3-(4-methoxyphenyl)-N-(2-pyridyl)-N-tetrahydrothiophen-3-yl-prop-2-enamid |
| 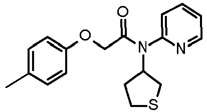 | 2-(4-methylphenoxy)-N-(2-pyridyl)-N-tetrahydrothiophen-3-yl-acetamid |
| 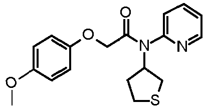 | 2-(4-methoxyphenoxy)-N-(2-pyridyl)-N-tetrahydrothiophen-3-yl-acetamid |
| 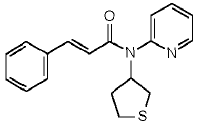 | (E)-3-phenyl-N-(2-pyridyl)-N-tetrahydrothiophen-3-yl-prop-2-enamid |
| 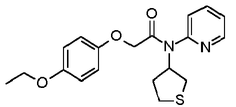 | 2-(4-ethoxyphenoxy)-N-(2-pyridyl)-N-tetrahydrothiophen-3-yl-acetamid |
| 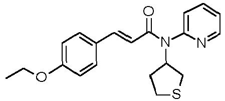 | (E)-3-(4-ethoxyphenyl)-N-(2-pyridyl)-N-tetrahydrothiophen-3-yl-prop-2-enamid |
| 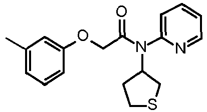 | 2-(3-methylphenoxy)-N-(2-pyridyl)-N-tetrahydrothiophen-3-yl-acetamid |
| 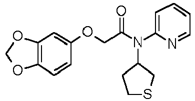 | 2-(1,3-benzodioxol-5-yloxy)-N-(2-pyridyl)-N-tetrahydrothiophen-3-yl-acetamid |
| 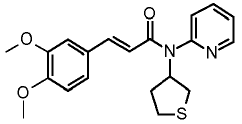 | (E)-3-(3,4-dimethoxyphenyl)-N-(2-pyridyl)-N-tetrahydrothiophen-3-yl-prop-2-enamid |
| 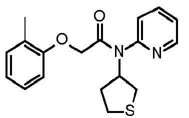 | 2-(2-methylphenoxy)-N-(2-pyridyl)-N-tetrahydrothiophen-3-yl-acetamid |
| 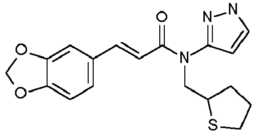 | (E)-3-(1,3-benzodioxol-5-yl)-N-(1H-pyrazol-3-yl)-N-(tetrahydrothiophen-2-ylmethyl)prop-2-enamid |
| 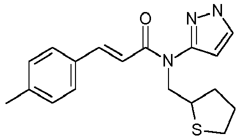 | (E)-3-(p-Tolyl)-N-(1H-pyrazol-3-yl)-N-(tetrahydrothiophen-2-ylmethyl)prop-2-enamid |
| 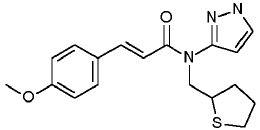 | (E)-3-(4-methoxyphenyl)-N-(1H-pyrazol-3-yl)-N-(tetrahydrothiophen-2-ylmethyl)prop-2-enamid |
| 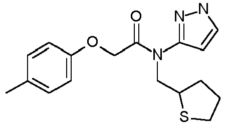 | 2-(4-Methylphenoxy)-N-(1H-pyrazol-3-yl)-N-(tetrahydrothiophen-2-ylmethyl)acetamid |
| 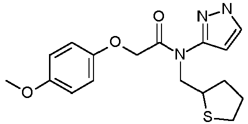 | 2-(4-Methoxyphenoxy)-N-(1H-pyrazol-3-yl)-N-(tetrahydrothiophen-2-ylmethyl)acetamid |
| 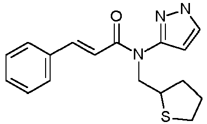 | (E)-3-phenyl-N-(1H-pyrazol-3-yl)-N-(tetrahydrothiophen-2-ylmethyl)prop-2-enamid |
| 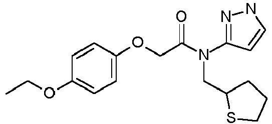 | 2-(4-ethoxyphenoxy)-N-(1H-pyrazol-3-yl)-N-(tetrahydrothiophen-2-ylmethyl)acetamid |
| 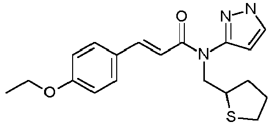 | (E)-3-(4-ethoxyphenyl)-N-(1H-pyrazol-3-yl)-N-(tetrahydrothiophen-2-ylmethyl)prop-2-enamid |
| 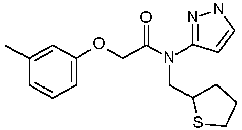 | 2-(3-methylphenoxy)-N-(1H-pyrazol-3-yl)-N-(tetrahydrothiophen-2-ylmethyl)acetamid |
| 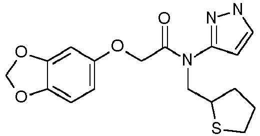 | 2-(1,3-benzodioxol-5-yloxy)-N-(1H-pyrazol-3-yl)-N-(tetrahydrothiophen-2-ylmethyl)acetamid |
| 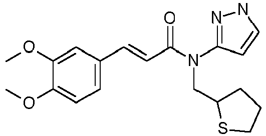 | (E)-3-(3,4-dimethoxyphenyl)-N-(1H-pyrazol-3-yl)-N-(tetrahydrothiophen-2-ylmethyl)prop-2-enamid |
| 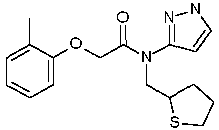 | 2-(2-methylphenoxy)-N-(1H-pyrazol-3-yl)-N-(tetrahydrothiophen-2-ylmethyl)acetamid |
| 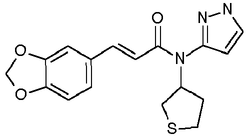 | (E)-3-(1,3-benzodioxol-5-yl)-N-(1H-pyrazol-3-yl)-N-tetrahydrothiophen-3-yl-prop-2-enamid |
| 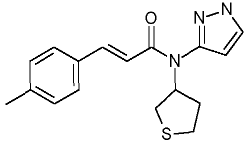 | (E)-3-(p-Tolyl)-N-(1H-pyrazol-3-yl)-N-tetrahydrothiophen-3-yl-prop-2-enamid |
| 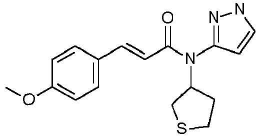 | (E)-3-(4-Methoxyphenyl)-N-(1H-pyrazol-3-yl)-N-tetrahydrothiophen-3-yl-prop-2-enamid |
| 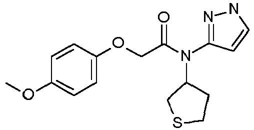 | 2-(4-Methoxyphenoxy)-N-(1H-pyrazol-3-yl)-N-tetrahydrothiophen-3-yl-acetamid |
| 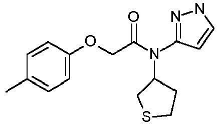 | 2-(4-methylphenoxy)-N-(1H-pyrazol-3-yl)-N-tetrahydrothiophen-3-yl-acetamid |
| 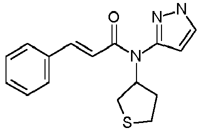 | (E)-3-phenyl-N-(1H-pyrazol-3-yl)-N-tetrahydrothiophen-3-yl-prop-2-enamid |
| 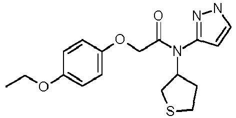 | 2-(4-ethoxyphenoxy)-N-(1H-pyrazol-3-yl)-N-tetrahydrothiophen-3-yl-acetamid |
| 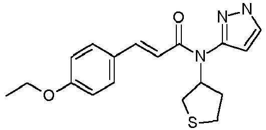 | (E)-3-(4-ethoxyphenyl)-N-(1H-pyrazol-3-yl)-N-tetrahydrothiophen-3-yl-prop-2-enamide |
| 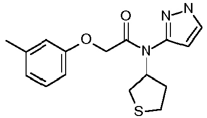 | 2-(3-methylphenoxy)-N-(1H-pyrazol-3-yl)-N-tetrahydrothiophen-3-yl-acetamid |
| 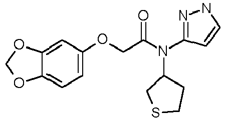 | 2-(1,3-benzodioxol-5-yloxy)-N-(1H-pyrazol-3-yl)-N-tetrahydrothiophen-3-yl-acetamid |
| 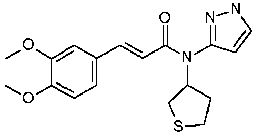 | (E)-3-(3,4-dimethoxyphenyl)-N-(1H-pyrazol-3-yl)-N-tetrahydrothiophen-3-yl-prop-2-enamid |
| 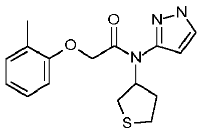 | 2-(2-methylphenoxy)-N-(1H-pyrazol-3-yl)-N-tetrahydrothiophen-3-yl-acetamid |
| 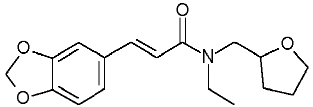 | (E)-3-(1,3-benzodioxol-5-yl)-N-ethyl-N-(tetrahydrofuran-2-ylmethyl)prop-2-enamide |
| 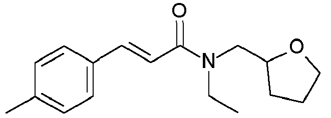 | (E)-N-ethyl-3-(p-tolyl)-N-(tetrahydrofuran-2-ylmethyl)prop-2-enamid |
| 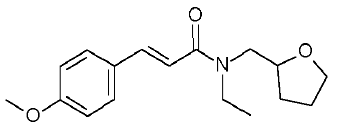 | (E)-N-ethyl-3-(4-methoxyphenyl)-N-(tetrahydrofuran-2-ylmethyl)prop-2-enamid |
| 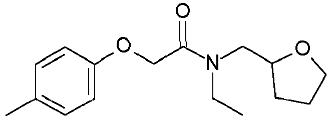 | N-ethyl-2-(4-methylphenoxy)-N-(tetrahydrofuran-2-ylmethyl)acetamide |
| 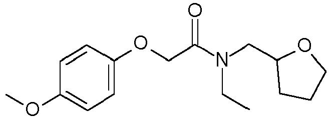 | N-ethyl-2-(4-methoxyphenoxy)-N-(tetrahydrofuran-2-ylmethyl)acetamid |
| 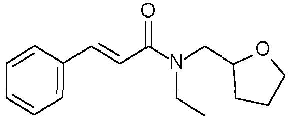 | (E)-N-ethyl-3-phenyl-N-(tetrahydrofuran-2-ylmethyl)prop-2-enamid |
| 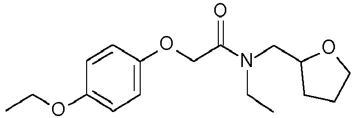 | 2-(4-ethoxyphenoxy)-N-ethyl-N-(tetrahydrofuran-2-ylmethyl)acetamid |
| 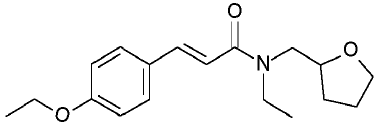 | (E)-3-(4-ethoxyphenyl)-N-ethyl-N-(tetrahydrofuran-2-ylmethyl)prop-2-enamid |
| 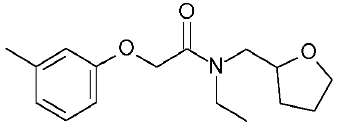 | N-ethyl-2-(3-methylphenoxy)-N-(tetrahydrofuran-2-ylmethyl)acetamid |
| 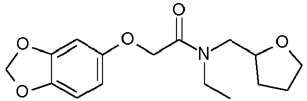 | 2-(1,3-benzodioxol-5-yloxy)-N-ethyl-N-(tetrahydrofuran-2-ylmethyl)acetamid |
| 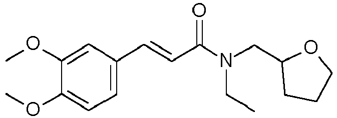 | (E)-3-(3,4-dimethoxyphenyl)-N-ethyl-N-(tetrahydrofuran-2-ylmethyl)prop-2-enamid |
| 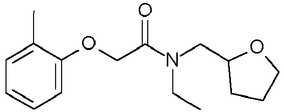 | N-ethyl-2-(2-methylphenoxy)-N-(tetrahydrofuran-2-ylmethyl)acetamid |
| 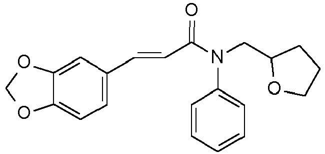 | (E)-3-(1,3-benzodioxol-5-yl)-N-phenyl-N-(tetrahydrofuran-2-ylmethyl)prop-2-enamid |
| 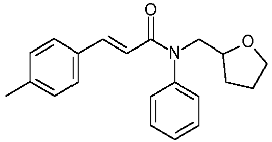 | (E)-N-phenyl-3-(p-tolyl)-N-(tetrahydrofuran-2-ylmethyl)prop-2-enamid |
| 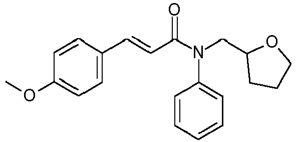 | (E)-3-(4-methoxyphenyl)-N-phenyl-N-(tetrahydrofuran-2-ylmethyl)prop-2-enamid |
| 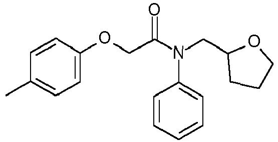 | 2-(4-methylphenoxy)-N-phenyl-N-(tetrahydrofuran-2-ylmethyl)acetamid |
| 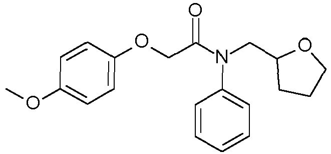 | 2-(4-methoxyphenoxy)-N-phenyl-N-(tetrahydrofuran-2-ylmethyl)acetamid |
| 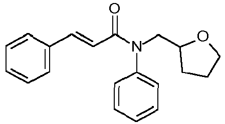 | (E)-N,3-diphenyl-N-(tetrahydrofuran-2-ylmethyl)prop-2-enamid |
| 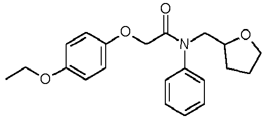 | 2-(4-ethoxyphenoxy)-N-phenyl-N-(tetrahydrofuran-2-ylmethyl)acetamid |
| 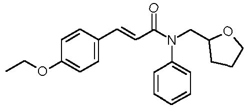 | (E)-3-(4-ethoxyphenyl)-N-phenyl-N-(tetrahydrofuran-2-ylmethyl)prop-2-enamid |
| 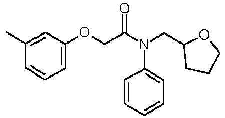 | 2-(3-methylphenoxy)-N-phenyl-N-(tetrahydrofuran-2-ylmethyl)acetamid |
| 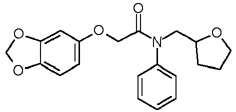 | 2-(1,3-benzodioxol-5-yloxy)-N-phenyl-N-(tetrahydrofuran-2-ylmethyl)acetamid |
| 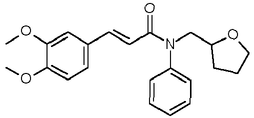 | (E)-3-(3,4-dimethoxyphenyl)-N-phenyl-N-(tetrahydrofuran-2-ylmethyl)prop-2-enamid |
| 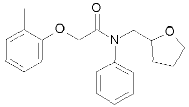 | 2-(2-methylphenoxy)-N-phenyl-N-(tetrahydrofuran-2-ylmethyl)acetamid |

Gemäß einer alternativen Ausführungsform der vorliegenden Erfindung sind die Verbindungen der Gruppe D, wie oben beschrieben, bevorzugt im Rahmen der vorliegenden Erfindung einzusetzende Verbindungen.

Gemäß einer weiteren alternativen Ausführungsform sind die Verbindungen der Gruppe E, wie oben beschrieben, bevorzugt im Rahmen der vorliegenden Erfindung einzusetzende Verbindungen. Vorzugsweise wird in einem erfindungsgemäßen Verfahren, insbesondere in seiner bevorzugten Ausgestaltung der Rezeptor mit wenigstens einem Modulator in Kontakt gebracht, welcher in einem zellulären Aktivitätstest unter Verwendung von Zellen, welche den humanen TRPM8-Rezeptor rekombinant exprimieren, die Permeabilität dieser Zellen für Ca²⁺ Ionen modulieren.

In einem erfindungsgemäßen Verfahren, vorzugsweise in einer vorstehend als bevorzugt bezeichneten Ausführungsform, wirkt der Modulator auf die zelluläre Ca²⁺ lonen-Permeabilität agonistisch oder antagonistisch.

Besonders bevorzugt ist eine Ausführung eines erfindungsgemäßen Verfahrens, insbesondere einer bevorzugten Ausgestaltung, wobei der Modulator ein TRPM8-Rezeptor-Agonist ist.

Ein weiterer Aspekt der vorliegenden Erfindung umfasst die Verwendung eines erfindungsgemäßen Modulators, insbesondere in einem oder mehreren der bevorzugten Verfahren, zur nicht-therapeutischen Induktion von Kältegefühl bei Mensch und/oder Tier.

Vorzugsweise wird ein solcher erfindungsgemäßer Modulator in oben beschriebener Verwendung bevorzugt zur nicht-therapeutischen Induktion von Kältegefühl durch eine den Modulator enthaltende Verpackung oder ein den Modulator enthaltenes Textil verwendet.

Gemäß einer Alternative der erfindungsgemäßen Verwendung wird bevorzugt ein Mittel eingesetzt, umfassend wenigstens eine, zwei, drei oder mehr der erfindungsgemäßen Modulatoren, insbesondere der bevorzugten Ausgestaltungen, vorzugsweise in einer (Gesamt-)Menge von 0,1 ppm bis 10 Gew.- %, bezogen auf das Gesamtgewicht des Mittels, zum Erzielen einer Kühlwirkung auf Haut oder Schleimhaut, die vorzugsweise verglichen mit der Kühlwirkung eines Mittels gleicher Zusammensetzung, bei dem lediglich der/die Modulatoren gegen Menthancarbonsäure-N-ethylamid, vorzugsweise in gleicher Konzentration, ausgetauscht ist/sind, verlängert ist, bevorzugt um wenigstens 10 Minuten verlängert ist, und/oder verglichen mit der Kühlwirkung eines Mittels gleicher Zusammensetzung, bei dem lediglich der/die Modulatoren gegen FEMA 4809 oder FEMA 4496, vorzugsweise in gleicher Konzentration, ausgetauscht ist/sind, früher einsetzt.

Um die langandauernde Kühlwirkung der erfindungsgemäß zu verwendenden Verbindungen zu quantifizieren, werden vorzugsweise Vergleichsversuche mit Menthan-3-carbonsäure-N-ethylamid (wie oben beschrieben) durchgeführt.

Vorzugsweise ist/sind der/die erfindungsgemäße(n) Modulator(en) zu einer der als bevorzugt beschriebenen Verwendungen ausgewählt aus der jeweils bereits beschriebenen Gruppe A, Gruppe B oder Gruppe C, oder gemäß einer anderen Ausführungsform Gruppe D oder Gruppe E.

Ein weiterer Aspekt der vorliegenden Erfindung betrifft einen Modulator wie vorangehend beschrieben zur Verwendung als Modulator des TRPM8 Rezeptors, vorzugsweise zur Anwendung in einem therapeutischen Verfahren zur Modulation des TRPM8 Rezeptors.

Zudem betrifft die vorliegende Erfindung neue Verbindungen des Strukturtyps 1 *per se.* Insbesondere betrifft die vorliegende Erfindung Verbindungen ausgewählt aus Gruppe A, Gruppe B oder Gruppe C bzw. Gruppe D oder Gruppe E wie jeweils hierin beschrieben, oder ein Salz einer solchen Verbindung, insbesondere ausgewählt aus Säureadditionssalzen mit anorganischen oder insbesondere organischen, ein- oder insbesondere mehrwertigen Carbonsäuren.

Die vorliegende Erfindung betrifft zudem, unabhängig von den hierin beschriebenen Verwendungen, Mittel umfassend neue Verbindungen des Strukturtyps 1, insbesondere Verbindungen ausgewählt aus Gruppe A, Gruppe B oder Gruppe C bzw. Gruppe D oder Gruppe E wie jeweils hierin beschrieben.

Ein weiterer Aspekt der vorliegenden Erfindung betrifft ein Mittel enthaltend wenigstens einen Modulator wie zuvor beschrieben zur Verwendung als Modulator des TRPM8 Rezeptors, vorzugsweise zur Anwendung in einem therapeutischen Verfahren zur Modulation des TRPM8 Rezeptors.

In einer bevorzugten Ausführungsform ist ein solches erfindungsgemäßes Mittel zur Verwendung als Modulator des TRPM8 Rezeptors, vorzugsweise zur Anwendung in einem therapeutischen Verfahren zur Modulation des TRPM8 Rezeptors, ausgewählt aus der Gruppe bestehend aus
a) Nahrungsmitteln, vorzugsweise Speiseeis, Mousse, Creme, Getränke und Süßwaren,
b) Mundpflegemitteln, vorzugsweise Zahnpasta, Mundwasser und Kaugummi,
c) Körperpflegemitteln, vorzugsweise Haut- oder Haarpflegemitteln, zum Beispiel Sonnencreme, Sonnenbrandcreme, Lotionen und Shampoos, und Pflaster,
d) Schäumen und Gelen.

In einer besonders bevorzugten Ausführungsform ist ein erfindungsgemäßes Mittel zur Verwendung als Modulator des TRPM8 Rezeptors, vorzugsweise zur Anwendung in einem therapeutischen Verfahren zur Modulation des TRPM8 Rezeptors, insbesondere in einer vorstehend als bevorzugt bezeichneten Ausführungsform, ausgewählt aus der Gruppe bestehend aus Aromamischungen und der Ernährung, der Mundhygiene oder dem Genuss dienenden oder kosmetischen Zubereitungen, umfassend eine, zwei, drei oder mehr der Modulatoren wie vorangehend beschrieben, wobei diese(r) Modulator(en) in einer (Gesamt-)Menge von 0,05 ppm bis 10 Gew.-%, vorzugsweise von 0,1 ppm bis 10 Gew.-% bezogen auf das Gesamtgewicht der Aromamischung oder Zubereitung, enthalten ist bzw. sind.

Vorzugsweise umfasst ein solches erfindungsgemäßes Mittel zur Verwendung als Modulator des TRPM8 Rezeptors, vorzugsweise zur Anwendung in einem therapeutischen Verfahren zur Modulation des TRPM8 Rezeptors, zudem:
(1) Einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
   und/oder
(2) einen oder mehrere Aromastoffe ohne physiologische Kühlwirkung
   und/oder
(3) einen oder mehrere trigeminal oder mundwässernd wirksame Stoffe ohne physiologische Kühlwirkung
   und/oder
(4) (iii) eine oder (iv) mehrere Verbindungen, die im Falle (iv) unabhängig voneinander oder gemeinsam zusätzlich einen geschmacksmodulierenden Effekt und/oder einen trigeminalen und/oder einen mundwässernden Reiz verursachen.

Weitere Stoffe mit physiologischer Kühlwirkung umfassen beispielsweise solche wie beschrieben in WO 2014/090293 A1, vorzugsweise Menthol, Mentholverbindungen, Optamint oder Minzöle.

Mentholverbindungen sind vorzugsweise ausgewählt aus der Gruppe bestehend aus Menthol, Menthyl Methyl Ether, Menthone Glyceryl Acetal (Frescolat^{®} MGA, FEMA GRAS 3807), Menthone Glyceryl Ketal (FEMA GRAS 3808), Menthyl Lactate (Frescolat^{®} ML, FEMA GRAS 3748), Menthol Ethylene Glycol Carbonate (Fresolcat^{®} MGC, FEMA GRAS 3805), Menthol Propylene Glycol Carbonate (Frescolat^{®} MPC, FEMA GRAS 3806), Menthyl-N-ethyloxamat (Frescolat^{®} , Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849) sowie Menthancarbonsäureestern und -amiden wie z.B. WS-3 (FEMA GRAS 3455), WS-5 (FEMA GRAS 4309), WS-12 (Frescolat^{®} SC-1, FEMA GRAS 4681) und WS-23 (FEMA GRAS 3804) sowie deren Gemische.

Optamint ist eine Mischung aus mehr als 50 verschiedenen natürlichen ätherischen Ölen und natürlichen oder natur-identischen Aromastoffen. Optaminte weisen variable Zusammensetzungen von verschiedenen (teilweise fraktionierten) Ölen auf, welche vorzugsweise eine Mischung aus beispielsweise unterschiedlichen Pfefferminzöle und Krauseminzöle, sowie Eucalyptus Globulus Öl, Sternanisöl, Menthol, Menthon, Isomenthon, Menthylacetat, Anethol, Eucalyptol usw. darstellt. Eine exakte Wiedergabe der Zusammensetzung der Optaminte ist daher nicht möglich. Die Produktserie Optamint^{®} ist kommerziell erhältlich bei der Firma Symrise AG.

In einer bevorzugten Ausführungsform der Verwendung eines erfindungsgemäßen Modulators, insbesondere in einem oder mehreren der bevorzugten Verfahren, zur nicht-therapeutischen Induktion von Kältegefühl bei Mensch und/oder Tier zum Erreichen einer physiologischen Kühlwirkung auf der Haut und/oder Schleimhaut, ist die Kühlwirkung verglichen mit der Kühlwirkung eines Mittels gleicher Zusammensetzung, bei dem lediglich der/die Modulator(en) gegen Menthancarbonsäure-N-ethylamid, vorzugsweise in gleicher Konzentration, ausgetauscht ist/sind, um wenigstens 10 Minuten verlängert, und/oder verglichen mit der Kühlwirkung eines Mittels gleicher Zusammensetzung, bei dem lediglich der/die Modulator(en) gegen FEMA 4809 oder FEMA 4496, vorzugsweise in gleicher Konzentration ausgetauscht ist/sind, früher einsetzend, mit folgendem Schritt:
- Applizieren einer zum Erreichen einer physiologischen Kühlwirkung ausreichenden Menge eines Mittels wie zuvor beschrieben, insbesondere in einer oder mehrerer bevorzugter Ausführungsformen, auf die Haut und/oder Schleimhaut.

Besonders bevorzugt ist/sind der/die Modulator(en) in einer solchen erfindungsgemäßen Verwendung ausgewählt aus der oben beschriebenen Gruppe A.

Die erfindungsgemäßen Wirkstoffe besitzen ein breites Anwendungsgebiet in der Humankosmetik- und -pflege, insbesondere der Haut- und Haarpflege, sind aber auch pharmakologisch einsetzbar, sowie in Nahrungsmitten und Textilprodukten, aber auch als Repellents und als Bestandteil von insektizid wirkenden Zusammensetzungen verwendbar.

Bei den erfindungsgemäßen Mitteln kann es sich insbesondere um hautkosmetische, haarkosmetische, dermatologische, hygienische oder pharmazeutische Mittel handeln. Insbesondere werden die erfindungsgemäßen, insbesondere kühlend wirkenden Wirkstoffe für die Haut-, und/oder Haarkosmetik oder als Mundpflegemittel angewendet.

Die erfindungsgemäßen haar- oder hautpflegenden Mittel bzw. Zubereitungen liegen insbesondere in Form einer Emulsion, einer Dispersion, einer Suspension, in Form einer wässrigen Tensidzubereitung, einer Milch, einer Lotion, einer Creme, eines Balsams, einer Salbe, eines Gels, eines Granulats, eines Puders, eines Stiftpräparates, wie z. B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und Wasser-in-Öl-Emulsionen oder Mikroemulsionen in Frage.

Im Regelfall wird die haar- oder hautkosmetische Zubereitung zur Applikation auf der Haut (topisch) oder dem Haar verwendet. Unter "topischen Zubereitungen" sind dabei solche Zubereitungen zu verstehen, die dazu geeignet sind, die Wirkstoffe in feiner Verteilung, wie z.B. in einer durch die Haut resorbierbaren Form auf die Haut aufzubringen. Hierfür eignen sich z. B. wässrige und wässrigalkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Nach einer Ausführungsform des erfindungsgemäßen kosmetischen Mittels enthält dieses einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Die erfindungsgemäße Lehre umfasst auch die Verwendung der hierin beschriebenen Wirkstoffe in pharmazeutischen Mitteln zur Behandlung eines Individuums, vorzugsweise eines Säugers, insbesondere eines Menschen, Nutz- oder Haustieres. Die Wirkstoffe werden dazu in Form von pharmazeutischen Zusammensetzungen verabreicht, die einen pharmazeutisch verträglichen Exzipienten mit wenigstens einem erfindungsgemäßen Wirkstoff und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen bzw. Zusammensetzungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Inhibitoren verwendet werden. Ferner können auch Liposomen, Mikrosphären oder Polymermatrizes zur Anwendung kommen.

Bei der Herstellung erfindungsgemäßer Mittel bzw. Zubereitungen bzw. Zusammensetzungen werden erfindungsgemäße Wirkstoffe gewöhnlich mit einem Exzipienten vermischt oder verdünnt. Exzipienten können feste, halbfeste oder flüssige Materialien sein, die als Vehikel, Träger oder Medium für den Wirkstoff dienen. Der Wirkstoffgehalt (eines oder mehrerer gleichzeitig enthaltener erfindungsgemäßer Wirkstoffe) kann dabei in einem weiten Bereich variieren und liegt etwa, jeweils bezogen auf das Gesamtgewicht der Zusammensetzung, im ppm-Bereich von etwa 0,05 ppm bis 10 Gew.-%, bevorzugt 0,1 ppm bis 10 Gew.-%.

Zu geeigneten Exzipienten gehören beispielsweise Lactose, Dextrose, Sucrose, Sorbitol, Mannitol, Stärken, Akaziengummi, Calciumphosphat, Alginate, Traganth, Gelatine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, oder Hager's Handbuch der Pharmazeutischen Praxis, Springer Verlag, Heidelberg dargestellt ist.

Die erfindungsgemäßen Mittel können neben üblichen Zusatz- oder Hilfsstoffen zusätzlich kosmetisch und/oder dermatologisch und/oder pharmakologisch aktive Wirkstoffe enthalten.

Als nicht-limitierende Beispiele geeigneter weiterer Wirkstoffe sind zu nennen:
Geeignete kosmetisch und/oder dermatologisch aktive Wirkstoffe sind z.B. färbende Wirkstoffe, Haut- und Haarpigmentierungsmittel, Tönungsmittel, Bräunungsmittel, Bleichmittel, keratinhärtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder - feuchthaltende Stoffe, rückfettende Wirkstoffe, antierythimatös oder antiallergisch aktive Wirkstoffe, verzweigte Fettsäuren, wie 18-Methyleicosansäure, und Mischungen davon.

Künstlich hautbräunende Wirkstoffe, die geeignet sind, die Haut ohne natürliche oder künstliche Bestrahlung mit UV-Strahlen zu bräunen; dies sind z. B. Dihydroxyaceton, Alloxan und Walnussschalenextrakt. Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat etc.

Antimikrobielle Wirkstoffe, die eingesetzt werden, um Mikroorganismen zu zerstören bzw. ihr Wachstum zu hemmen. Sie dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure etc. Derartige desodorierend wirkende Stoffe sind z. B. Zinkricinoleat, Triclosan, Undecylensäurealkylolamide, Citronensäuretriethylester, Chlorhexidin etc.

Geeignete Hilfs- und Zusatzstoffe für die Herstellung von haarkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1, entnommen werden. Die Hilfs- und Zusatzstoffe sind bevorzugt kosmetisch und/oder pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidanzien, Antireizstoffe, Chelatbildner, Emulsionsstabilisatoren, Filmbildner, Gelbildner, Geruchsmaskierungsmittel, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilanzien, Treibmittel, Trocknungsmittel, Trübungsmittel, Verdickungsmittel, Wachse, Weichmacher, Weißöl. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Weitere geeignete Zusatzstoffe sind ausgewählt unter Parfümölen, Haarpolymeren, Haar- und Hautkonditionern, Pfropfpolymeren, wasserlöslichen oder dispergierbaren silikonhaltigen Polymeren, Lichtschutzmitteln, Bleichmitteln, Pflegemitteln, Färbemitteln, Tönungsmitteln, Bräunungsmitteln, Farbstoffen, Konsistenzgebern, Feuchthaltemitteln, Rückfettern, Collagen, Eiweißhydrolysaten, Lipiden, Antioxidanzien, Entschäumern, Antistatika, Emollienzien, Weichmachern, Peroxidzersetzern.

Als Beispiele geeigneter Hilfs- und Zusatzstoffe sind zu nennen:
(1) Antioxidanzien, ausgewählt unter Aminosäuren (z.B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximine, Buthioninsulfone, Penta-, Hexa-, Heptathioninsulfoximin), insbesondere in sehr geringen, verträglichen Dosierungen (z. B. pmol bis µmol/kg Bereich), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und dessen Derivaten (z. B. Natriumascorbat, Ascorbylpalmitat, Mg-Ascorbylphosphat, Ascorbylacetat), Tocopherol und Derivate (z. B. Vitamin-E-Acetat, Tocotrienol), Vitamin A und Derivate (Vitamin-A-Palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, Trans-Stilbenoxid).
(2) Peroxidzersetzer, d. h. Verbindungen, die in der Lage sind Peroxide, besonders bevorzugt Lipidperoxide, zu zersetzen. Darunter sind organische Substanzen zu verstehen, wie z. B. Pyridin-2-thiol-3-carbonsäure, 2-Methoxy-pyrimidinol-carbonsäuren, 2-Methoxy-pyridincarbonsäuren, 2-Dimethylamino-pyrimidinolcarbonsäuren, 2-Dimethylamino-pyridincarbonsäuren.
(3) Verdickungsmittel, wie vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Insbesondere werden nichtionische Verdicker eingesetzt.
(4) Konservierungsmittel, die mit ihrer E-Nummer nachfolgend aufgeführt sind

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E215 | p-Hyd roxybenzoesäu reethylester-Na-Salz | E 238 | Calciumformiat |
| E 216 | p-Hydroxybenzoesäu re-n-propylester | E 239 | Hexamethylentetramin |
| E 217 | p-Hydroxybenzoesäu re-n-propylester-Na-Salz | E 249 | Kaliumnitrit |
| E 218 | p-Hydroxybenzoesäu remethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konservierungsmittel oder Konservierungshilfsstoffe geeignet, wie Dibromdicyanobutan (2-Brom-2-brommethylglutarodinitril), 3-Jod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Formaldehydabspalter.

Ferner sind Phenylhydroxyalkylether, insbesondere die unter der Bezeichnung Phenoxyethanol bekannte Verbindung, aufgrund ihrer bakteriziden und fungiziden Wirkungen auf eine Anzahl von Mikroorganismen als Konservierungsmittel geeignet.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden. Ebenso können auch antimikrobielle Polypeptide eingesetzt werden.

(5) Lichtfilterwirkstoffe, die UV-Strahlen im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl, und Mischungen davon. Geeignet sind weiterhin p-Aminobenzoesäureester, Zimtsäureester, Benzophenone, Kampferderivate sowie UV-Strahlen abhaltende Pigmente, wie Titandioxid, Talkum und Zinkoxid.

Als UV-Filtersubstanzen kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2`,4,4`-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethyl-ester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Die erfindungsgemäßen kosmetischen und dermatologischen Mittel bzw. Zubereitungen können vorteilhafterweise außerdem UV-Strahlen abhaltende anorganische Pigmente auf Basis von Metalloxiden und/oder anderen in Wasser schwer löslichen oder unlöslichen Metallverbindungen, ausgewählt aus der Gruppe der Oxide des Zinks (ZnO), Titan (TiOz), Eisens (z. B. Fe₂O₃), Zirkoniums (ZrOz), Siliciums (SiOz), Mangans (z.B. MnO), Aluminiums (Al₂O₃), Cers (z. B. Ce₂O₃), Mischoxiden der entsprechenden Metalle sowie Abmischungen aus solchen Oxiden enthalten.

Die anorganischen Pigmente können dabei in gecoateter Form vorliegen, d. h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.
(6) Repellentwirkstoffe, d. h. Verbindungen, die in der Lage sind, bestimmte Tiere, insbesondere Insekten, vom Menschen abzuhalten oder zu vertreiben. Dazu gehört z. B. 2-Ethyl-1, 3-hexandiol, N, N-Diethyl-m-toluamid etc.
(7) Geeignete hyperemisierend wirkende Stoffe, welche die Durchblutung der Haut anregen, sind z. B. ätherische Öle, wie Latschenkieferextrakt, Lavendelextrakt, Rosmarinextrakt, Wacholderbeerextrakt, Rosskastanienextrakt, Birkenblätterextrakt, Heublumenextrakt, Ethylacetat, Kampfer, Menthol, Pfefferminzöl, Rosmarinextrakt, Eukalyptusöl etc.
(8) Geeignete keratolytisch und keratoplastisch wirkende Stoffe sind z. B. Salicylsäure, Kalziumthioglykolat, Thioglykolsäure und ihre Salze, Schwefel etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion etc.
(9) Geeignete Antiphlogistika, die Hautreizungen entgegenwirken, sind z. B. Allantoin, Bisabolol, Dragosantol, Kamillenextrakt, Panthenol etc.
(10) Kosmetisch oder pharmazeutisch akzeptable Polymere, wie kationische, amphotere und neutrale Polymere.

Geeignete Polymere sind z. B. kationische Polymere mit der Bezeichnung Polyquaternium nach INCI, z. B. Copolymere aus Vinylpyrrolidon/N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat PQ 11), Copolymere aus N-Vinylcaprolactam/N-Vinylpyrrolidon/N-Vinylimidazoliumsalzen (Luviquat E Hold), kationische Cellulosederivate (Polyquaternium-4 und -10), Acrylamidocopolymere (Polyquaternium-7) und Chitosan.

Geeignete kationische (quaternisierte) Polymere sind auch Merquat (Polymer auf Basis von Dimethyldiallylammoniumchlorid), Gafquat (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar-Marken der Firma Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und deren Derivate. Dazu zählt beispielsweise Luviflex ^{®} Swing (teilverseiftes Copolymerisat von Polyvinylacetat und Polyethylenglykol, Firma BASF).

Geeignete Polymere sind auch nichtionische, wasserlösliche bzw. wasserdispergierbare Polymere oder Oligomere, wie Polyvinylcaprolactam, z. B. Luviskol ^{®} Plus (BASF), oder Polyvinylpyrrolidon und deren Copolymere, insbesondere mit Vinylestern, wie Vinylacetat, z. B. Luviskol ^{®} VA 37 (BASF), Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer (National Starch) erhältlichen Octylacrylamid / Methylmethacrylat / tert.-Butylaminoethylmethacrylat-Hydroxypropylmethacrylat-Copolymere sowie zwitterionische Polymere, wie sie beispielsweise in den deutschen Patentanmeldungen DE39 29 973, DE 21 50 557, DE28 17 369 und DE 3708 451 offenbart sind. Acrylamidopropyltrimethylammoniumchlorid/Acrylsäure- bzw. Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette (AMERCHOL) im Handel erhältlich sind, und Copolymere aus Hydroxyethylmethacrylat, Methylmethacrylat, N, N-Dimethylaminoethylmethacrylat und Acrylsäure (Jordapon (D)).

Geeignete Polymere sind auch nichtionische, siloxanhaltige, wasserlösliche oder dispergierbare Polymere, z. B. Polyethersiloxane, wie Tegopren ^{®} (Firma Goldschmidt) oder Besi (Firma Wacker).

Im Folgenden werden einzelne besondere Anwendungsformen erfindungsgemäßer Wirkstoffe beispielhaft näher erläutert.

### Kühlende Haut- und Haarpflegemittel:

Nach einer bevorzugten Ausführungsform handelt es sich bei den erfindungsgemäßen Mitteln um ein kühlendes Haut- oder Haarpflege- oder -reinigungsmittel.

Bevorzugte Haut- oder Haarreinigungsmittel sind Seifen von flüssiger bis gelförmiger Konsistenz, wie Transparentseifen, Luxusseifen, Deoseifen, Cremeseifen, Babyseifen, Hautschutzseifen, abrasive Seifen und Syndets, pastose Seifen, Schmierseifen und Waschpasten, Peelingseifen, Feuchtigkeitstücher, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungs-gemäßen Mitteln um ein Duschgel, eine Shampoo-Formulierung oder ein Badepräparat. Solche Formulierungen enthalten wenigstens einen erfindungsgemäßen Wirkstoff sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Grundsätzlich kann der Wirkstoffgehalt über einen weiten Bereich, wie z.B. 0,05 ppm bis 10 Gew.-%, bevorzugt 0,1 ppm bis 10 Gew.-% variieren.

### i) Spezielle Ausgestaltungen für Mittel zur Auftragung auf die Haut:

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die erfindungsgemäßen dermatologischen Mittel verwendet werden in Nose-Strips zur Porenreinigung, in Antiaknemitteln, Repellents, Rasiermitteln, After- und Pre-Shave-Pflegemitteln, After-Sun-Pflegemitteln, Haarentfernungsmitteln, Haarfärbemitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Sonnenschutzcremes, Feuchthaltecremes, Bleichcremes, Selbstbräunungscremes, Vitamincremes, Hautlotionen, Pflegelotionen und Feuchthaltelotionen.

Hautkosmetische und dermatologische Mittel enthalten insbesondere wenigstens einen erfindungsgemäßen Wirkstoff in einem Anteil von etwa 0,05 ppm bis 10 Gew.-%, bevorzugt 0,1 ppm bis 10 Gew.-%, bezogen auf das Gesamtgewicht des Mittels.

Je nach Anwendungsgebiet können die erfindungsgemäßen Hautkosmetikmittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den erfindungsgemäßen Wirkstoffen und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Enzyme, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Silikonöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆- bis C₃₀-Fettsäuren, Wachsester, wie z. B. Jojobaöl, Fettalkohole, Vaseline, hydriertes Lanolin und acetyliertes Lanolin sowie Mischungen davon.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die Herstellung der kosmetischen oder dermatologischen Zubereitungen erfolgt nach üblichen, dem Fachmann bekannten Verfahren.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halbfeste oder flüssige Materialien sein, die als Vehikel, Träger oder Medium für den Wirkstoff dienen können.

Die Zumischung weiterer Hilfsstoffe erfolgt gewünschtenfalls in der dem Fachmann bekannten Weise. Weiterhin sind die Polymere und Dispersionen geeignet, als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Bevorzugt liegen die kosmetischen und dermatologischen Mittel in Form von Emulsionen insbesondere als Wasser-in-Öl (W/O)- oder Öl-in-Wasser (O/W)-Emulsionen vor. Es ist aber auch möglich, andere Formulierungsarten zu wählen, beispielsweise, Gele, Öle, Oleogele, multiple Emulsionen, beispielsweise in Form von W/O/W- oder O/W/O-Emulsionen, wasserfreie Salben bzw. Salbengrundlagen usw. Auch emulgatorfreie Formulierungen wie Hydrodispersionen, Hydrogele oder eine Pickering-Emulsion sind vorteilhafte Ausführungsformen.

Die Herstellung von Emulsionen erfolgt nach bekannten Methoden. Die Emulsionen enthalten neben wenigstens einem erfindungsgemäßen Wirkstoff in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion als W/O-Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Polyelektrolytkomplex eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Rizinusöl, Sesamöl, Olivenöl, Jojobaöl, Karite-Öl, Hoplostethus-Öl, mineralische Öle, deren Destillationsbeginn unter Atmosphärendruck bei ca. 250°C und deren Destillationsendpunkt bei 410°C liegt, wie z. B. Vaselinöl, Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Silikonöle, wie Dimethylpolysiloxan, Methylphenylpolysiloxan und das Silikonglykol-Copolymer , Fettsäuren und Fettalkohole enthalten.

Neben den erfindungsgemäßen Wirkstoffen können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungs-gemäßen Mitteln um ein Duschgel, eine Shampooformulierung oder ein Badepräparat.

Solche Formulierungen enthalten wenigstens einen erfindungsgemäßen Wirkstoff sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidanzien sowie Verdickern/Gelbildnern, Hautkonditioniermitteln und Feuchthaltemitteln.

Diese Formulierungen enthalten insbesondere 2 bis 50 Gew.-%, wie 5 bis 40 Gew.-%, oder 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen.

Dazu zählen z. B. Natriumlaurylsulfat, Ammoniumtaurytsulfat, Natriumlaurylethersulfat, Ammoniumlaurylethersulfat, Natriumlaurylsarkosinat, Natriumoleylsuccinat, Ammoni-umlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono-oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampooformulierungen Verdicker, wie z. B. Kochsalz, PEG-55, Propylenglykol-Oleat, PEG-120-Methylglucosedioleat und andere sowie Konservierungsmittel, weitere Wirk- und Hilfsstoffe und Wasser enthalten.

### ii) Spezielle Ausgestaltungen für Mittel zur Auftragung auf das Haar:

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten insbesondere wenigstens einen erfindungsgemäßen Wirkstoff in einer Menge im Bereich von etwa 0,05 ppm bis 10 Gew.-%, bevorzugt 0,1 ppm bis 10 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-) Spray, (Aerosol-) Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 ppm bis 20 Gew.- %. Diese Mikrodispersionen benötigen in der Regel keine Emulgatoren oder Tenside zu ihrer Stabilisierung.

Die erfindungsgemäßen haarkosmetischen Formulierungen enthalten in einer speziellen Ausführungsform a) 0,0001 bis 50 Gew.-% oder 0,001 bis 10, oder 0,005 bis 1 Gew.-% wenigstens eines erfindungsgemäßen Wirkstoffs, b) 20 bis 99,95 Gew.-% Wasser und/oder Alkohol, c) 0 bis 50 Gew.-% wenigstens eines Treibgases, d) 0 bis 5 Gew.-% wenigstens eines Emulgators, e) 0 bis 3 Gew.-% wenigstens eines Verdickers, sowie bis zu 25 Gew.-% weitere Bestandteile.

Unter Alkohol sind alle in der Kosmetik üblichen Alkohole zu verstehen, z. B. Ethanol, Isopropanol, n-Propanol.

Weiterhin zählen hierzu alle in der Kosmetik bekannten Styling- und Conditioner-Polymere, die in Kombination mit den erfindungsgemäßen Wirkstoffen eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon-Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Sprayzubereitungen a) 0,0001 bis 50 Gew.-% oder 0,001 bis 10, oder 0,005 bis 1 Gew.-% wenigstens eines erfindungsgemäßen Wirkstoffs, b) 20 bis 99,9 Gew.-% Wasser und/oder Alkohol, c) 0 bis 70 Gew.-% wenigstens eines Treibmittel, d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält a) 0,0001 bis 50 Gew.-% oder 0,001 bis 10, oder 0,005 bis 1 Gew.-% wenigstens eines erfindungsgemäßen Wirkstoffs, b) 55 bis 99,8 Gew.-% Wasser und/oder Alkohol, c) 5 bis 20 Gew.-% eines Treibmittel, d) 0,1 bis 5 Gew.-% eines Emulgators, e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4 ; Cetethe, z. B. Cetheth-1, Polyethylenglycolcetylether, Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein: a) 0,0001 bis 50 Gew.-% oder 0,001 bis 10, oder 0,005 bis 1 Gew.-% wenigstens eines erfindungsgemäßen Wirkstoffs b) 80 bis 99,85 Gew.-% Wasser und/oder Alkohol, c) 0 bis 3 Gew.-%, bevorzugt 0,05 bis 2 Gew.-%, eines Gelbildners, d) 0 bis 20 Gew.-% weitere Bestandteile.

Der Einsatz von Gelbildnern kann von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid / AcrylamidCopolymere, Steareth-10-Allylether, Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Spezielle Shampooformulierungen enthalten a) 0,0001 bis 50 Gew.-% oder 0,001 bis 10, oder 0,005 bis 1 Gew.-% wenigstens eines erfindungsgemäßen Wirkstoffs, b) 25 bis 94,95 Gew.-% Wasser, c) 5 bis 50 Gew.-% Tenside, c) 0 bis 5 Gew.-% eines weiteren Konditioniermittels, d) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten, im Molekül aufweisen.

Geeignet sind zum Beispiel Natriumlaurylsulfat, Ammoniumlaurysulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Wirkstoffen eingesetzt werden.

Hierzu zählen beispielsweise die zuvor genannten kationischen Polymere mit der Bezeichnung Polyquaternium nach INCI, insbesondere Copolymere aus Vinylpyrrolidon/ N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat D PQ 11), Copolymere aus N-Vinylcaprolactam/N-Vinylpyrrolidon/N-Vinylimidazoliumsalzen (Luviquat D Hold), kationische Cellulosederivate (Polyquaternium-4 und -10), Acrylamidcopolymere (Polyquaternium-7). Ferner können Eiweißhydrolysate verwendet werden sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon-Kopolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

### Kühlende Mundpflegemittel:

Erfindungsgemäße Mundpflegemittel können in an sich bekannter Weise, z.B. als Zahnpasta, Zahngel, oder wässrige oder wässrig-alkoholische Mundpflegemittel (Mundwasser) formuliert sein.

Erfindungsgemäße Mundpflegezusammensetzungen enthalten, bezogen auf das Gewicht der Zusammensetzung, vorzugsweise 0,05 ppm bis 10 Gew.-%, bevorzugt 0,1 ppm bis 10 Gew.-% der Gesamtmenge wenigstens eines erfindungsgemäßen Wirkstoffs.

Darüber hinaus können die Mundpflegemittel, insbesondere Zahnpasten, auch Abrasiva enthalten, wie Siliziumoxidhydrat, Dicalciumphosphatdihydrat, Calciumcarbonat, Natriumhydrogencarbonat, Calciumpyrophosphat und Aluminiumoxid. Beispielsweise kann auch eine Mischung aus zur Zähflüssigkeit neigendem gefällten Silizium und scheuernden gefällten Silizium [Handbook of Pharmaceutical Excipients, The Pharmaceutical Society of Great Britain, 1 Lambeth High Street, London SE 1 7JN, England, pages 253-256] verwendet werden. Das zuerst genannte wird auf Grund seiner thixotropen Eigenschaften, das zweite wegen seiner besseren Wirksamkeit beim Beseitigen der an den Zahnflächen anhaftenden Substanzen verwendet. Der Einsatz dieser Produkte gewährleistet eine geringe Scheuerwirkung, denn es handelt sich um amorphe Feststoffe mittlerer Härte, die gleichzeitig voll und ganz mit dem als Mineralisierungsmittel benutzten Fluorid kompatibel sind, da sie keine Kalksalze enthalten, welche ihre Unlöslichkeit bewirken und ihre Bioverfügbarkeit verringern würden.

Die Rezeptur der erfindungsgemäßen Mundpflegemittel, wie z.B. Zahnpasta, kann auch geeignete Zusätze und Vehikel enthalten, um deren Eigenschaften zu verbessern und die Herstellung zu erleichtern. Diese sind z.B. unter Bindemitteln, Verdickungsmittel, Duftstoffen, Farbstoffen, Konservierungsmitteln, Benetzungsmitteln oder Feuchthaltemittel, Tensiden, Schmiermitteln, Trübungsmitteln, Remineralisierungsstoffen, Tensiden, Puffern, Alkoholen, Vitaminen, Wasser, zusätzlichen Wirkstoffen und deren Mischungen ausgewählt.

Als Bindemittel kann jedes normalerweise bei der Herstellung dieser Art von Rezepturen verwendete Mittel eingesetzt werden, z. B. Tragantgummi. Das Bindemittel kann in der Rezeptur in einer Menge von 0,5-1,5 Gew.-% der Gesamtmenge enthalten sein.

In dem Mundpflegemittel können auch organische Verdickungsmittel eingearbeitet werden, wie Natriumcarboxymethylcellulose, Celluloseether, Xanthangummi, Karageenane, Natriumalginat und Carbopole. Es können auch anorganische Verdickungsmittel, wie Siliziumoxid-Verdickungsmittel, Natriumaluminiumsilikate und Tone, zur Bereitstellung der entsprechenden Rheologie verwendet werden. Das Verdickungsmittel kann in der Rezeptur in einer Menge von 0,5-5 Gew.-% der Gesamtmenge enthalten sein.

Die Zahnpasta kann durch Zusatz eines geeigneten üblichen Duftstoffes, z. B. eines Pfefferminzaromas, aromatisiert werden. Ätherische Öle einschließlich u.a. Nelkenöl, Zimtöl, Pfefferminzöl und Krauseminzöl sind ebenfalls geeignet. Der Duftstoff kann in der Rezeptur in einer Menge von 0,5-15 Gew.-% der Gesamtmenge enthalten sein.

Als Farbstoff kann jeder der üblicherweise bei der Herstellung von Zahnpasta verwendete Farbstoff eingesetzt werden zum Beispiel Brillantblau FCF, C.42090 [KIRSCH PHARMA]. Der Farbstoff kann in der Rezeptur in einer Menge von 0,001 - 0,005 Gew.-% der Gesamtmenge enthalten sein.

Bei dem Konservierungsmittel kann es sich um jedes übliche Mittel, wie etwa ein Derivat der Benzoesäure, z. B. p-Hydroxy-Methylbenzoat handeln. Das Konservierungsmittel kann in der Rezeptur in einer Menge von 0,1- 0,3 Gew.-% der Gesamtmenge enthalten sein.

Als Süßstoff kann man z. B. Natriumsaccharin oder Zyklaminsäure und deren Derivate, z. B. Natriumcyclamat, verwenden. Der Süßstoff kann in der Rezeptur in einer Menge von 0,08-0,15 Gew.-% der Gesamtmenge enthalten sein.

Das zur Verhinderung des Austrocknens und der Härtung der Zahnpasta verwendete Benetzungs-oder Feuchthaltemittel ist insbesondere ausgewählt unter Glycerin, Sorbit, Propylenglykol, Xylit und flüssige Polyethylenglykolen, insbesondere eine Mischung aus Sorbit, Glycerin und Xylit, z.B in einem Anteil von 1-60 Gew.-% der Gesamtmenge.

Als Schmiermittel kann jedes der üblicherweise in der Rezeptur einer Zahnpasta verwendeten Mittel sein, z. B. Dimethikon (Polymer des Dimethylpolysiloxans), bei dem es sich um ein oberflächenaktives Mittel handelt, das dazu beiträgt, der erfindungsgemäßen Zahnpasta gute rheologische Eigenschaften zu verleihen. Das Schmiermittel kann in der Rezeptur in einer Menge von 0,25 bis 0,75 Gew.-% der Gesamtmenge enthalten sein.

Als Trübungsmittel kann jedes der üblicherweise eingesetzten Mittel verwendet werden, z. B. Titandioxyd. Das Trübungsmittel kann in der Rezeptur in einer Menge von 0,05 bis 1 Gew.-% der Gesamtmenge enthalten sein.

Als Reminalisierungsmittel verwendet man eine Fluoridquelle, z. B. Natriumfluorid, Zinn(II)-fluorid und Natriummonofluorophosphat, da man auf diese Weise 100% eines aktiven Fluorids als Mittel zur Remineralisierung der weißen Läsionen, verursacht durch die organischen Säuren, die eine Folge des bakteriellen Stoffwechsels sind. Das Remineralisierungsmittel kann in der Rezeptur in einer Menge von 0,2 bis 0,4 Gew.-% der Gesamtmenge enthalten sein.

Typischerweise können des Weiteren übliche Bestandteile enthalten sein, wie anionische Tenside, wie z.B. Natriumlaurylsulphat, Natrium-N-laurylsarcosinat, Natriumlaurylsulphoacetat und Natriumalkylglycerylethersulphonat. Das Tensid kann in der Rezeptur in einer Menge von 0,05 bis 5 Gew.-% der Gesamtmenge enthalten sein.

Wenn gewünscht, kann die durch die Erfindung vorgeschlagene Zahnpasta auch Vitamin enthalten, das aus der von Vitamin A, Vitamin B5, Vitamin C und Vitamin E gebildeten Gruppe und deren Mischungen ausgewählt wird. Im Fall einer Verwendung kann jedes Vitamin in der Rezeptur in einer Menge von 0,1 bis 5 Gew.-% der Gesamtmenge enthalten sein. Diese Vitamine können als solche, in Form von Provitaminen oder in Form akzeptierbarer pharmazeutischer Salze eingesetzt werden. Das Vitamin A, das in der Regel in Form seines Palmitatsalzes verwendet wird, fördert die Epithelisierung der Mundschleimhaut und schützt das Zahnfleisch. Das Vitamin B5, genauer gesagt das D- Panthenol, wirkt schmerzstillend, heilend und entzündungshemmend, schützt die Epithelschleimhaut, fördert die Epithelisierung der Verletzungen und glättet die Narben; es ist für die Behandlung von Verletzungen geeignet, welche infolge von Zahn-extraktionen, Gingivitis, Stomatitis, Schmerzen nach dem Einsetzen von Zahnprothesen, Geschwüren, traumatischen Lädierungen der Schleimhaut, chronischen und rückläufigen Aphthen entstehen. Das Vitamin C regeneriert das Epithel der Mundschleimhaut, fördert die Kollagensynthese und das Immunsystem (Entzündungs-mechanismus) und erhöht die Schutzfähigkeit der Phagozyten gegen Bakterien. Das Vitamin E, das normalerweise in Form seines Azetatsalzes eingesetzt wird, wirkt schmerzstillend und entzündungshemmend, schützt die Mundschleimhaut gegen die Fettüberoxidierung infolge der Bildung freier Radikale und gegen kontaminisierende Substanzen der Umwelt (Ozon, Zigarettenrauch usw.) und begünstigt die Heilung der Verletzungen. Durch die Zugabe eines oder einiger dieser Vitamine bietet die Erfindung eine Zahnpasta, die neben den zuvor genannten Eigenschaften auch entzündungshemmende Merkmale und schmerzstillende Auswirkungen besitzt, welche die Schutzfähigkeit der Membranen der Mundschleimhaut erhöhen und den Index der Zahnbelags- und Zahnsteinbildung sowie den der bakteriellen Kontamination verringern.

Zusätzliche Wirkstoffe sind z.B. antimikrobielle und Plaque-penetrierende Mittel, wie beta-Naphthol, Thymol, Chlorthymol und Hexylresorcin; oder keimtötende Verbindungen, wie quaternäre Ammoniumverbindungen.; Zahnsteinbekämpfungsmittel, wie Tetranatriumpyrophosphat, GANTREZ-Polymer^{®} S-70, Natriumtripolyphosphat und Zinkcitrat; Peroxidverbindungen, wie Wasserstoffperoxid und anorganischen Peroxide.

Gegebenenfalls kann auch ein Puffer verwendet werden, der in für die Aufrechterhaltung eines pH-Werts von etwa 6-8 geeigneten Konzentrationen enthalten ist, wie z.B Alkalimetallphosphatpuffer. Die Anwesenheit von Kaliumionen übt zudem eine die Überempflindichkeit lindernde Wirkung aus.

Wasser oder Alkohol können in einem Anteil von 1 bis 20 Gew.-% der Gesamtmenge des Mittels enthalten sein.

In Kombination mit dem Alkohol oder anstelle des Alkohols können auch Glykolverbindungen verwendet werden, wie Glycerin, Sorbit oder Propylenglykol.

Das erfindungsgemäße Mundpflegemittel kann leicht durch Mischung geeigneter Mengen der verschiedenen Bestandteile in einem z.B. mit Rührschaufeln ausgestatteten Reaktor hergestellt werden.

### Kühlende Pflaster:

Grundsätzlich kann der Wirkstoffgehalt über einen weiten Bereich, wie z.B. 0,05 ppm bis 10 Gew.-%, bevorzugt 0,1 ppm bis 10 Gew.-% variieren.

Erfindungsgemäße Pflaster können in beliebiger Weise aufgebaut sein, beispielsweise nach dem Matrixsystem, dem Membransystem oder dem Vliessystem (Drug Dev. Ind. Pharm. 14 (1988), 183-209; Drug Dev. Ind. Pharm. 13 (1987), 589-651; Drugs of Today 23 (1987), 625-646.

Das Matrixsystem besteht in einfachster Weise aus 3 Teilen: der flexiblen Stützfolie, der den Wirkstoff enthaltenden Klebematrix und einer Abziehfolie. Falls eine nichtklebende Matrix verwendet wird, muss zur Haftung auf der Haut eine Randzone der Stützfolie mit Klebstoff versehen werden.

Ein Membransystem weist hingegen mindestens 5 Teile auf: eine flexible Stützfolie, ein Reservoir mit gelöstem oder suspendiertem Wirkstoff, eine Membran zur Steuerung der Wirkstofffreigabe, eine auf die Membran aufgezogene Klebeschicht und eine Abziehfolie.

Beim Vliessystem besteht die den Wirkstoff enthaltende Schicht aus einem saugfähigen Vlies oder porösen Polymer, das mit einer Wirkstofflösung oder -suspension getränkt ist. Diese Schicht, die fest mit der Stützfolie verbunden ist, wird durch eine Abziehfolie abgedeckt. Der Rand der Stützfolie ist, zur Applikation auf die Haut, mit Klebstoff versehen.

Grundsätzlich sind alle erfindungsgemäßen Wirkstoffe in dieser Weise formulierbar.

Die zu verwendenden Hilfsstoffe sind die für die Herstellung von Pflastern üblichen. Neben dem klebenden Agens, in der Regel ein Polymer mit einer Glastemperatur zwischen -70 und -10, insbesondere -55 und -25 °C, sowie einer Trägerfolie, die mit diesem klebenden Agens beschichtet wird, und dem Wirkstoff werden häufig Emulgatoren, Verdickungsmittel sowie Stoffe, die die Wirkstofffreigabe beeinflussen sollen, und andere Hilfsmittel zugesetzt.

Die klebrigen Polymeren mit den oben genannten niedrigen Glastemperaturen sind bekannt, beispielsweise aus den US-Patenten 2 973 282 und 3 307 544. Die selbstklebenden Bänder und Folien sollen auf bloßen Kontakt auf der menschlichen Haut kleben, doch soll die Kohäsion der Klebschicht und deren Adhäsion an der Trägerfolie größer sein als die Adhäsion an der Haut, so dass sie sich weitgehend rückstandslos wieder abziehen lassen. Es handelt sich in der Regel um Copolymerisate auf der Basis von Acryl- und Methacrylsäureestern von Alkoholen mit 2 bis 12, insbesondere 4 bis 8 Kohlenstoffatomen, die zahlreiche andere Comonomere einpolymerisiert enthalten können, beispielsweise (Meth)acrylsäure, (Meth)acrylnitril, (Meth)acrylamid, N-tert.-Butyl-(meth-)acrylamid, Vinylester wie Vinylacetat, -propionat oder -butyrat, andere Vinylverbindungen wie Styrol, ferner Butadien. Besonders hervorgehoben seien Butylacrylat und 2-Ethylhexylacrylat. Die Polymeren können durch Zusatz geringer Mengen Comonomerer mit 2 oder mehr copolymerisierbaren Doppelbindungen, also beispielsweise von Diacrylaten, wie Butandioldiacrylat, oder Divinylverbindungen, wie Divinylbenzol, oder durch Zusatz anderer Vernetzungsmittel, z.B. Melamin-Formaldehydharze, vernetzt sein. Als klebrige Polymere können ferner Polyisobutylene und Polyvinylether unterschiedlichen Molekulargewichts verwendet werden.

Die Teilchengröße der Dispersionen soll zwischen 50 und 500 nm, insbesondere zwischen 50 und 200 nm liegen. Die Teilchengröße und der Vernetzungsgrad können in bekannter Weise in Abhängigkeit von den Polymerisationsbedingungen und den Comonomeren eingestellt werden. Kleinere Teilchengrößen und ein erhöhter Vernetzungsgrad können eine Steigerung der Wirkstofffreisetzung bewirken.

Matrix-Pflaster können in üblicher Weise hergestellt werden, indem man den Wirkstoff in einer geeigneten Polymerlösung löst oder fein dispergiert und anschließend diese wirkstoffhaltige Selbstklebemasse mittels Walzen- oder Rakelauftragsverfahren zum Film auszieht. In einigen Fällen ist es zweckmäßig, den Wirkstoff vor der Zugabe zu der Polymerlösung in einem organischen Lösungsmittel, z.B. Ethanol oder Aceton, aufzulösen oder feinst zu dispergieren. Dadurch kann eine bessere Verteilung des Wirkstoffes im Polymer erzielt werden.

Die Pflaster können auch nach der deutschen Patentanmeldung P 38 07 283.1 hergestellt werden, indem man den Wirkstoff in feinpulvriger Form (Teilchengröße unter 200, insbesondere unter 50 µm) in die wässrige Latexdispersion einarbeitet, oder in einer wässrigen Emulgatorlösung dispergiert oder löst und diese Mischung der wässrigen Latexdispersion bei einer Temperatur von 10 bis 80, insbesondere 30 bis 70 °C zumischt. Daneben kann auch das Salz eines Wirkstoffs in wässriger Lösung mit der Polymerdispersion bei einem pH-Wert gemischt werden, bei dem der Wirkstoff vorwiegend in der wasserlöslichen ionisierten Form vorliegt. Durch pH-Verschiebung wird dann der Wirkstoff in die ungeladene wasserunlösliche Form gebracht und simultan in die Dispersion einemulgiert.

Zweckmäßig legt man den Wirkstoff vor, gibt den Emulgator und Wasser zu und mischt dann mit der Polymerdispersion. Die so erhaltene wirkstoffhaltige Dispersion wird gegebenenfalls mit weiteren Hilfsstoffen versehen und, wie erwähnt, in an sich bekannter Weise auf einer Stützfolie zu einem Film ausgezogen und getrocknet. Die Trocknungstemperatur kann hierbei zwischen Raumtemperatur und 100°C liegen, wobei ein Optimum zwischen angestrebter schneller Trocknung und zu vermeidender Blasenbildung im Film sowie thermischer Belastung des Wirkstoffs im Allgemeinen bei 35 bis 45°C liegt.

Dieses Verfahren hat den großen Vorteil der Vermeidung von organischen Lösungsmitteln. Jedoch kommen im Prinzip auch alle anderen üblichen Herstellverfahren für Matrix-Pflaster in Betracht.

Die resultierenden Filme haben Dicken von 10 bis 800, bevorzugt 50 bis 300 µm. Die Filmherstellung kann kontinuierlich oder diskontinuierlich erfolgen. Das Auftrageverfahren kann mehrmals wiederholt werden, bis der Film die gewünschte Dicke erreicht hat. Die klebrige Polymerschicht enthält den Wirkstoff in einer Konzentration im Bereich von 1 bis 40, insbesondere 5 bis 25 Gew.-%. Die gleiche Konzentration gilt auch für die Reservoirflüssigkeit beim Membransystem und für die Wirkstofflösung oder -Dispersion, mit der beim Vliessystem das Vlies oder das poröse Polymere getränkt wird.

Als Emulgatoren sowohl für die Wirkstoffe wie auch die Polymeren werden die hierfür üblichen Tenside verwendet, wie das Natriumsalz von längerkettigen Fettsäuren und des Schwefelsäurehalbesters eines (gegebenenfalls oxethylierten) Fettalkohols als Beispiele für anionische Tenside sowie polyoxethylierte Alkylphenole und längerkettige Fettalkohole (z.B. Hexadecan-(1)-ol) und Glycerin-Fettsäurepartialester als Beispiele für nichtionische Tenside und Coemulgatoren.

Die gewünschte Viskosität der ausziehfertigen Masse kann z.B. mit Polyacrylsäuren oder Cellulosederivaten eingestellt werden.

Als zusätzliche Vernetzungsmittel die die Kohäsion und damit die Klebeeigenschaften der Filme verbessern, können z.B. Melamin-Formaldehydharze verwendet werden.

Im Sinne einer Verbesserung der Wirkstofffreisetzung wirken Quellstoffe wie Polyvinylpyrrolidon, Cellulosederivate oder Polyacrylate, da der Film vermehrt Wasser aufnehmen kann und dadurch der Diffusionswiderstand sinkt. Die Freisetzung der Wirkstoffe kann ferner verbessert werden durch den Zusatz von hydrophilen Weichmachern wie Glycerin, 1, 2-Propandiol der Polyethylenglykolen und lipophilen Weichmachern wie Triacetin, Dibutylphthalat oder Isopropylmyristat.

Matrixpflaster ergeben üblicherweise eine Wirkstofffreisetzung 1. Ordnung. Durch die Verwendung von Füllstoffen, die den Wirkstoff adsorbieren, wie Aerosil, mikrokristalline Cellulose oder Lactose, resultiert annähernd eine Freisetzung 0. Ordnung.

Die Stützfolie, auf die die wirkstoffhaltige Selbstklebemasse aufgetrocknet wird, ist zweckmäßig sowohl für den Wirkstoff wie für Wasserdampf praktisch undurchlässig. Sie kann z.B. aus einer Aluminium-Kunststoff-Verbundfolie, einer metallisierten Kunststofffolie, einer Kunststofffolie, die zur Wirkstoffseite hin mit einer Sperrschicht aus z.B. Polyvinylidenchlorid versehen ist, oder aus einer einfachen Kunststofffolie, z.B. Polyesterfolie, bestehen.

Die erfindungsgemäßen Pflaster, die nach dem Membransystem aufgebaut sind, werden ebenfalls in üblicher Weise hergestellt (z.B. EP 0 186 071 A2, US 4 262 003).

Die Herstellung der nach dem Vliessystem aufgebauten Pflaster erfolgt dadurch, dass auf der Stützfolie befestigte Vliese oder poröse Polymere mit einer Lösung oder Dispersion des Wirkstoffes in einem hydrophilen oder lipophilen Lösungsmittel oder Lösungsmittelgemisch getränkt werden. Anschließend wird die undurchlässige Abziehfolie aufgebracht.

### Kühlende Nahrungsmittel:

Erfindungsgemäße kühlende Nahrungsmittel können in (bei Umgebungstemperatur) fester, flüssiger, halbfester, pastöser, cremiger order geschäumter Form vorliegen. Sie enthalten neben herkömmlichen Nahrungsbestandteilen wenigstens eine wirksame (d.h. als kühlend wirkende) Menge wenigstens eines erfindungsgemäßen Wirkstoffs.

Typische Bestandteile sind dabei Fette, Kohlenhydrate, Proteine, Ballaststoffe, Wasser, Alkohol und dergleichen.
Der Proteinanteil kann z.B. 0 bis 50 Gew.-%, bezogen auf das Gesamtgewicht des Nahrungsmittels betragen;
der Fettanteil kann z.B. der 0 bis 50 Gew.-%, bezogen auf das Gesamtgewicht des Nahrungsmittels betragen;
der Kohlenhydratanteil kann z.B. 0 bis 90 Gew.-%, bezogen auf das Gesamtgewicht des Nahrungsmittels betragen;
der Ballaststoffanteil kann z.B. 0 bis 90 Gew.-%, bezogen auf das Gesamtgewicht des Nahrungsmittels betragen;
der Wasseranteil kann z.B. 0 bis 95 Gew.-%, bezogen auf das Gesamtgewicht des Nahrungsmittels betragen;
der Alkoholanteil kann z.B. 0 bis 15 Gew.-%, bezogen auf das Gesamtgewicht des Nahrungsmittels betragen;
der Anteil erfindungsgemäßer Wirkstoffe kann z.B. im Bereich von 0,05 ppm bis 10 Gew.-%, bevorzugt 0,1 ppm bis 10 Gew.-%, bezogen auf das Gesamtgewicht des Nahrungsmittels liegen.

Beispiele für Kohlehydrate sind z.B. Mono- und Disaccharide, Glukose, Galaktose, Mannose, Laktose, Maltose, und Saccharose; Fruktose und Mannose; Polysaccharide wie z. B. Stärken. Maltodextrine, Mehl.

Der Begriff "Ballaststoff" bezieht sich auf lösliche, unlösliche, fermentierbare, nicht fermentierbare oder eine beliebige Kombination solcher Ballaststoffe. Bei dem Ballaststoff kann es sich z. B. um Sojafasern, Pektin, bestimmte resistente Stärken, Oligofruktose, Inuline, Haferfasern, Erbsenfasern, Guargummi, Gum acazia, modifizierte Cellulose handeln.

Bei dem Fettbestandteil kann es sich um ein beliebiges Lipid oder Fett handeln, dessen Eignung zur Verwendung in Nährmitteln bekannt ist. Typische Fette sind unter anderem Milchfett, Distelöl, Canolaöl, Eidotterlipid, Olivenöl, Baumwollsamenöl, Kokosnussöl, Palmöl, Palmkernöl, Sojaöl, Sonnenblumenöl, Fischöl und Fraktionen aller vorstehenden Öle, die sich davon ableiten, wie Palmolein, mittelkettige Triglyceride (MCT), und Fettsäureester, wobei es sich bei den Fettsäuren z. B. um Arachidonsäure, Linolsäure, Palmitinsäure, Stearinsäure, Docosahexaensäure, Eicosapentaensäure, Linolensäure, Ölsäure, Laurinsäure, Caprinsäure, Caprylsäure, Capronsäure handelt. Hochölsäurehaltige Formen verschiedener Öle werden auch als geeignet für die vorliegende Verwendung erachtet, wie hochölsäurehaltiges Sonnenblumenöl und hochölsäurehaltiges Distelöl.

Bei dem Protein kann es sich um ein beliebiges Protein und/oder Aminosäuregemisch handeln, dessen Eignung zur Verwendung in Nährmitteln bekannt ist. Typische Proteine sind tierische Proteine, pflanzliche Proteine wie Sojaprotein, Milchprotein wie Magermilchprotein, Molkenprotein und Casein, und Aminosäuren (oder Salze davon) wie Isoleucin, Phenylalanin, Leucin, Lysin, Methionin, Threonin, Tryptophan, Arginin, Glutamin, Taurin, Valin. Bevorzugte Proteinquellen sind Molkenprotein, Natriumcaseinat oder Calciumcaseinat, das gegebenenfalls mit Aminosäuren versetzt ist. Für einige Anwendungen ist eine bevorzugte Proteinquelle hydrolysiertes Protein (Proteinhydrolysat) das gegebenenfalls mit Aminosäuren versetzt ist.

Bei dem Proteinhydrolysat kann es sich um ein beliebiges geeignetes Proteinhydrolysat handeln, das in einem Nährmittel verwendet wird, wie Sojaproteinhydrolysat, Caseinhydrolysat, Molkenproteinhydrolysat, andere tierische und pflanzliche Proteinhydrolysate und Gemische davon. Das Proteinhydrolysat der erfindungsgemäßen Zusammensetzung ist vorzugsweise ein Sojaprotein-, Molkenprotein-oder ein Caseinproteinhydrolysat, das kurze Peptide und Aminosäuren umfasst und gegebenenfalls mit zusätzlichen Aminosäuren versetzt ist. In einer bevorzugten Ausführungsform enthält das erfindungsgemäß geeignete Proteinhydrolysat einen hohen Anteil freier Aminosäuren (z. B. mehr als 40%) und niedermolekularer Peptidfragmente.

Das hydrolysierte Protein der erfindungsgemäßen Zusammensetzung ist auch vorzugsweise versetzt mit verschiedenen freien Aminosäuren, um einen ernährungsmäßig ausgewogenen Aminosäuregehalt bereitzustellen. Beispiele für solche freien Aminosäuren sind unter anderem L-Tryptophan, L-Methionin, L-Cystin, L-Tyrosin und L-Arginin.

Die erfindungsgemäßen Nährmittel enthalten gegebenenfalls auch Vitamine und Mineralien. Dem Fachmann ist geläufig, dass Minimalerfordernisse für bestimmte Vitamine und Mineralien aufgestellt worden sind, die für die normale physiologische Funktion notwendig sind. Der Fachmann weiß außerdem, dass den Nährmitteln angemessene zusätzliche Mengen von Vitamin- und Mineralbestandteilen zugesetzt werden müssen, um gewisse Verluste bei der Verarbeitung und Lagerung derartiger Zusammensetzungen zu kompensieren. Die erfindungsgemäße Zusammensetzung enthält gegebenenfalls ernährungsmäßig signifikante Mengen Vitamine und Mineralien.

Beispiele für Mineralien, Vitamine und andere Nährstoffe, die gegebenenfalls in der erfindungsgemä-ßen Zusammensetzung vorliegen, sind unter anderem Vitamin A, Vitamin B₆, Vitamin B₁₂, Vitamin E, Vitamin K, Vitamin C, Vitamin D, Inositol, Taurin, Folsäure, Thiamin, Riboflavin, Niacin, Biotin, Pantothensäure, Cholin, Calcium, Phosphor, lod, Eisen, Magnesium, Kupfer, Zink, Mangan, Chlorid, Kalium, Natrium, Beta-Carotin, Nucleotide, Selen, Chrom, Molybdän und L-Carnitin. Mineralien werden üblicherweise in Salzform zugesetzt.

Die erfindungsgemäße Zusammensetzung enthält gegebenenfalls auch üblicherweise Emulgatoren und/oder Stabilisatoren wie Lecithin (z. B. aus Ei oder Soja), modifiziertes Lecithin (z. B. enzymatisch oder acetyliert), Carrageenan, Xanthangummi, Mono- und Diglyceride, Guargummi, Carboxymethylcellulose, Stearoyllactylate, succinylierte Monoglyceride, Saccharoseester von Fettsäuren, Diacetylweinsäureester von Monoglyceriden, Polyglycerolester von Fettsäuren oder beliebige Gemische davon.

Die erfindungsgemäße Zusammensetzung enthält wahlweise ein oder mehrere natürliche oder künstliche Geschmacksträger zur Verbesserung der Schmackhaftigkeit. Man kann jeden auf dem Sektor verwendeten Geschmacksträger verwenden, wie Erdbeer, Kirsch, Schokolade, Orange, Kokosnuss, Vanille; Gewürze wie Muskat und Zimt; oder Citronensäure. In einigen Fällen, in denen natürliche Geschmacksträger, wie Kokosnussstücke, verwendet werden, trägt der Bestandteil zum Gesamtnährwertprofil der Zusammensetzung bei, d. h. er trägt zur Qualität und Quantität des Fett-, Protein- und/oder Kohlenhydratbestandteils bei.

Die erfindungsgemäße Zusammensetzung enthält wahlweise auch verschiedene andere Bestandteile, die zum Nährwertprofil der Zusammensetzung beitragen und/oder erwünschte Geschmackseigenschaften, wie Geschmacksverstärkung oder Mundgefühl, verleihen können. Derartige Bestandteile sind unter anderem Erdnüsse, Rosinen, Käsepulver, Essig, Salz, Natriumbicarbonat. Bei Riegeln wird die Zusammensetzung üblicherweise mit einem Schokoladen- oder einem aromatisierten Überzug (z. B. Schokolade, Vanille, Erdbeer usw.) versehen.

Die erfindungsgemäße Zusammensetzung enthält wahlweise auch natürliche oder künstliche Farbstoffe, um den ästhetischen Anreiz zu verbessern.

Die erfindungsgemäßen Zusammensetzungen können in mehreren physikalischen Erscheinungsformen vorliegen, z. B. als flüssige enterale Nährmittel oder Getränke für Erwachsene oder Kinder, in einer halbfesten Form wie Pudding, Creme, Mousse, oder einer festen Form, wie einem Nährmittelriegel oder Keks.

Die erfindungsgemäße Zusammensetzung kann nach bekannten Standardverfahren der Lebensmitteltechnik hergestellt werden, zum Beispiel nach analogen Verfahren zu den in folgenden Druckschriften beschriebenen: US-Patente 4,670,268; 4,497,800; 4,900,566; 5,104,677; 5,389,395; und 5,223,285; Chocolate, Cocoa and Confectionery: Science and Technology, 3. Auflage, Bernard W. Minifie, Van Nostrand Reinhold, New York, 1989, S. 502-506; worauf vollinhaltlich verwiesen wird.

Bei Nährmittelriegeln und Keksen ist es üblicherweise angestrebt, die Zusammensetzung nach der physikalischen Formung zu backen.

Die erfindungsgemäße Zusammensetzung kann gewünschtenfalls nach bekannten Verfahren sterilisiert werden, zum Beispiel durch Wärmebehandlung wie Autoklavieren oder Sterilisieren oder Bestrahlung, oder mit sterilen Verfahren hergestellt und verpackt werden.

Die erfindungsgemäße Zusammensetzung kann in eine beliebige Art von Behälter oder Verpackung verpackt werden, deren Eignung zur Aufbewahrung von Lebensmitteln bekannt ist, wie Papier, Glas, beschichteter Karton, Kunststoff oder beschichtete Metalldosen.

Die erfindungsgemäße Zusammensetzung kann ernährungsmäßig ausgewogen sein. Unter dem Begriff "ernährungsmäßig ausgewogen" versteht man, dass die Zusammensetzung angemessene Nährstoffe enthält, um ein gesundes menschliches Leben über ausgedehnte Zeitspannen zu erhalten.

### Textilprodukte, ausgerüstet mit erfindungsgemäßen Wirkstoffen:

Die Ausrüstung von Textilien mit erfindungsgemäßen Wirkstoffen, ist in vielerlei Hinsicht von Interesse.

So findet die Ausrüstung von Textilien mit kühlend wirkenden Verbindungen insbesondere dort Verwendung wo Kleidungsstücke in direkten Kontakt mit der Haut gelangen können, so dass der Wirkstoff durch transdermale Übertragung seine Wirkungen, z.B. lokal oder systemisch, entfalten kann. Kürzlich wurde über Textilien berichtet, die mit sogenannten Wellness-Additiven, d.h. Substanzen, welche das Wohlbefinden fördern, ausgerüstet sind (R. Breier "Megatrend Wellness - Innovative Ideen für die Textilausrüstung", 31. Aachener Textiltage November 2004).

Eine insektizide Ausrüstung wiederum ist im Hinblick auf den Materialschutz, z.B. Ausrüstung des Textils gegen Mottenfraß etc., aber insbesondere auch zur Abwehr von parasitären Insekten, wie Mücken, von Interesse.

Grundlegendes Problem bei der Ausrüstung von Textilien mit Wirkstoffen ist die Anbindung des Wirkstoffs an den textilen Träger, die einerseits eine Permanenz der Ausrüstung gewährleisten muss und andererseits so gewählt werden muss, dass der Wirkstoff seine Wirkung nicht verliert. Hierzu werden im Stand der Technik verschiedene Ansätze vorgeschlagen.

So wurden z.B. Cyclodextrine zur Anbindung von Wirkstoffen an Textilien vorgeschlagen (siehe beispielsweise DE-A-19810951 und EP-A-0 392 608). Cyclodextrine sind cyclische Oligosaccharide, die durch enzymatischen Abbau von Stärke gebildet werden. Die häufigsten Cyclodextrine sind α-, β-und γ-Cydodextrine, die aus sechs, sieben bzw. acht α-1,4-verknüpften Glucose-Einheiten bestehen. Eine charakteristische Eigenschaft der Cyclodextrin-Moleküle ist ihre Ringstruktur mit weitgehend unveränderlichen Abmessungen. Der Innendurchmesser der Ringe beträgt etwa 570 pm für α-Cydodextrin, etwa 780 pm für β-Cyclodextrin und etwa 950 pm für γ-Cyclodextrin. Aufgrund ihrer Struktur sind Cyclodextrine in der Lage, Gastmoleküle, insbesondere hydrophobe Gastmoleküle, in wechselnden Mengen bis zur Sättigung einschließen zu können.

Die EP-A-1710345 beschreibt die Ausrüstung von Textilien mit Duftstoffen und anderen niedermolekularen organischen Wirkstoffen, die über eine amylosehaltige Substanz mit einem Amylosegehalt von wenigstens 30 % an das Textil gebunden sind.

Durch die Amyloseanteile der amylosehaltigen Substanz wird der Wirkstoff auf dem Textil gebunden und kontrolliert abgegeben, so dass die Wirkung über einen langen Zeitraum erhalten bleibt. Man nimmt an, dass der Wirkstoff ähnlich wie bei Cyclodextrinen in den durch die helikale Konformation der Amylose gebildeten Hohlräumen im Sinne einer Einschlussverbindung reversibel gebunden wird, wodurch einerseits eine Fixierung des Wirkstoffs auf der Oberfläche des textilen Trägers erreicht wird und andererseits eine kontrollierte Freisetzung möglich ist.

Für die erfindungsgemäße Ausrüstung von Textilien sind neben Amylose grundsätzlich alle Substanzen, insbesondere amylosehaltigen Stärken, d.h. native Stärken, modifizierte Stärken und Stärkederivate, geeignet, deren Amylosegehalt wenigstens 30 Gew.-% und insbesondere wenigstens 40 Gew.-% beträgt. Die Stärke kann nativ sein, z.B. Maisstärke, Weizenstärke, Kartoffelstärke, Sorghumstärke, Reisstärke oder Marantastärke, durch Teilaufschluss nativer Stärke gewonnen oder chemisch modifiziert sein. Geeignet ist auch reine Amylose als solche, z.B. enzymatisch gewonnene Amylose, z. B. aus Sucrose gewonnene Amylose. Geeignet sind auch Mischungen von Amylose und Stärke, sofern der Gesamtgehalt an Amylose wenigstens 30 Gew.-%, bezogen auf das Gesamtgewicht der Mischung beträgt. Es versteht sich, dass hier und im Folgenden alle Angaben in Gew.-%, welche sich auf Amylose oder amylosehaltige Substanzen beziehen, bei Mischungen aus Amylose und Stärke stets auf das Gesamtgewicht von Amylose + Stärke bezogen sind, sofern nicht ausdrücklich anderes angegeben ist.

Erfindungsgemäß besonders geeignet sind amylosehaltige Substanzen, insbesondere Amylose und amylosehaltige Stärken sowie Amylose/Stärkegemische, deren Amylosegehalt wenigstens 40 Gew.-% und insbesondere wenigstens 45 Gew.-%, bezogen auf das Gesamtgewicht der Substanz beträgt. In der Regel wird der Amylosegehalt 90 Gew.-% und insbesondere 80 Gew.-% nicht überschreiten. Derartige Substanzen sind bekannt und kommerziell erhältlich. Beispielsweise werden amylosehaltige Stärken von den Firmen Cerestar unter der Handelsmarke Amylogel^{®} und National Starch unter den Handelsbezeichnungen HYLON^{®} V und VII vertrieben.

Zur Erreichung der Anbindung des/der Wirkstoff(e) and das Textil, kann man das Textil mit der amylosehaltigen Substanz in der Regel in einer Menge von wenigstens 0,5 Gew.-%, vorzugsweise wenigstens 1 Gew.-% und insbesondere wenigstens 2 Gew.-%, jeweils bezogen auf das Gewicht des Textils, ausrüsten. In der Regel wird man die amylosehaltige Substanz in einer Menge von nicht mehr als 25 Gew.-%, häufig nicht mehr als 20 Gew.-% und insbesondere nicht mehr als 15 Gew.-%, bezogen auf das Gewicht des Textils, einsetzen um die taktilen Eigenschaften des Textils nicht nachteilig zu beeinflussen.

Zunächst wird das textile Material mit der amylosehaltigen Substanz als solcher ausgerüstet und anschließend das so ausgerüstete Textil mit einer geeigneten Aufbereitung des Wirkstoffs behandelt. Hierdurch wird die auf dem textilen Material befindliche amylosehaltige Substanz mit dem Wirkstoff beladen.

Man kann aber auch die amylosehaltige Substanz gemeinsam mit einem Wirkstoff einsetzen, um die Textilie auszurüsten. Hierbei können der Wirkstoff und die amylosehaltige Substanz sowohl als Mischung separater Komponenten als auch in der bereits vorgefertigten Form des Amylose-Wirkstoff-Komplexes angewandt werden.

In der Regel wird man den Wirkstoff in einer Menge einsetzen, die für den gewünschten Effekt ausreicht. Die Obergrenze wird durch die maximale Aufnahmekapazität der Amyloseeinheiten der eingesetzten amylosehaltigen Substanz bestimmt und wird in der Regel 20 Gew.-% und häufig 10 Gew.-%, bezogen auf den Amyloseanteil der Substanz, nicht überschreiten. Sofern erwünscht, setzt man den Wirkstoff in der Regel in einer Menge von 0,00001 bis 15 Gew.-%, 0,0001 bis 10 Gew.-%, 0,001 bis 5 Gew.-%, 0,005 bis 1 Gew.-% oder 0,1 bis 10 Gew.-% oder 0,5 bis 5 Gew.-%, bezogen auf den Amyloseanteil der amylosehaltigen Substanz ein.

Zur Textilausrüstung können auch Kombinationen erfindungsgemäßer Wirkstoffen mit anderen an sich bekannten und zur Textilausrüstung geeigneten Wirkstoffen verwendet werden.

Als weitere Wirkstoffe sind grundsätzlich alle organischen Verbindungen und Mischungen organischer Verbindungen geeignet, die als Wirkstoffe bekannt sind und die in Lebewesen wie Menschen und Tieren, einschließlich Mikroorganismen, eine physiologische Wirkung induzieren. Zu nennen sind solche Wirkstoffe, die bekanntermaßen mit Cyclodextrinen Einschlussverbindungen bilden können. Besonders geeignet sind Wirkstoffe, die Kohlenwasserstoffgruppen und insbesondere aliphatische, cycloaliphatische und/oder aromatische Strukturen aufweisen. Das Molekulargewicht der Wirkstoffe liegt typischerweise unterhalb 1000 Dalton und häufig im Bereich von 100 bis 600 Dalton. Geeignet sind außerdem anorganische Verbindungen wie Wasserstoffperoxid, die bekanntermaßen in Cyclodextrinen gebunden werden können (siehe hierzu F. Vögtle, Supramolekulare Chemie, 2. Auflage, B. G. Teubner, Stuttgart 1992, Cyclodextrine und dort zitierte Literatur).

Zu den weiteren Wirkstoffen zählen insbesondere pharmazeutische Wirkstoffe und Wirkstoffe, die das Wohlbefinden von Lebewesen, insbesondere von Menschen fördern und die gemeinhin auch als "Wellness-Additive" bezeichnet werden. Anders als bei pharmazeutischen Wirkstoffen muss bei den Wellness-Additiven nicht zwingend eine therapeutische Wirkung gegeben sein. Vielmehr kann der das Wohlbefinden fördernde Effekt auf einer Vielzahl von Faktoren wie pflegenden, anregenden, kosmetischen oder sonstigen Wirkungen beruhen. Gleichermaßen geeignet sind organische Wirkstoffe, die gegen parasitäre Organismen wirken. Hierzu zählen beispielsweise Wirkstoffe, die gegen Pilze und/oder Mikroorganismen wirken, z.B. Fungizide und Bakterizide, oder die gegen tierische Schädlinge wie Schnecken, Würmer, Milben, Insekten und/oder Nager wirken, z.B. Nematizide, Molluskizide, Insektizide, Akarizide, Rodentizide und Repellent-Wirkstoffe, sowie weiterhin Wirkstoffe gegen Ungräser, d.h. Herbizide, oder Duftstoffe.

Bevorzugte pharmazeutische Wirkstoffe sind solche, die bekanntermaßen über die Haut resorbiert werden können. Hierzu zählen beispielsweise Ibuprofen, Flurbiprofen, Acetylsalicylsäure, Acetamidophen, Apomorphin, butyliertes Hydroxytoluol, Chamzulen, Gujazulen, Chlorthalidon, Cholecalciferol, Dicumarol, Digoxin, Diphenylhydantoin, Furosemid, Hydroflumethiazid, Indomethacin, Iproniazid-Phosphat, Nitroglycerin, Nicotin, Nicotinsäureamid, Oubain, Oxprenolol, Papaverin-Alkaloide wie Papaverin, Laudanosin, Ethaverin und Narcotin sowie Berberin, weiterhin Retionol, trans-Retinolsäure, Pretinol, Spironolacton, Sulpirid, Theophyllin, Theobromin, Corticosteroide und Derivate wie Testosteron, 17-Methyltestosteron, Cortison, Corticosteron, Dexamethason, Triamcinolon, Methylprednisolon, Fludrocortison, Fluocortolon, Prednison, Prednisolon, Progesteron, u.a. Estrogene und Gestagene wie Estradiol, Estriol, Ethinylestradiol-3-methylether, Norethisteron und Ethisteron, sowie Phenethylamin und Derivate wie Tyramin, Adrenalin, Noradrenalin und Dopamin.

Beispiele für erfindungsgemäß geeignete Wirkstoffe mit einer Wirkung gegen parasitäre Organismen sind die unter www.reith-pfister.de/w.list.html sowie unter www.hclrss.demon.co.uk/class_pesticides.html genannten Nematizide, Bakterizide, Fungizide, Insektizide, Insekten-Repellents, Akarizide und Molluskizide.

Beispiele für bakterizide und fungizide Substanzen umfassen:
Antibiotioka, z.B. Cycloheximid, Griseofulvin, Kasugamycin, Natamycin, Polyoxin, Streptomycin, Penizillin oder Gentamycin;
Organische Verbindungen und Komplexe biozider Metalle, z.B. Komplexe des Silbers, Kupfers, Zinns und/oder Zinks wie Bis-(tributyllzinn)oxid, Kupfer-, Zink- und Zinn-Naphthenate, Oxine-Kupfer wie Cu-8, Tris-N-(cyclohexyldiazeniumdioxy)-aluminium, N-(Cyclohexyldiazeniumdioxy)-tributylzinn, Bis-N-(cyclohexyldiazeniumdioxy)-kupfer;
Quarternäre Ammoniumsalze, z.B. Benzyl-Cs- bis C₁₈-alkyldimethylammoniumhalogenide, insbesondere Chloride (Benzalkoniumchloride);
aliphatische Stickstofffungizide und Bactericide wie Cymoxanil, Dodin, Dodicin, Guazidine, Iminoctadin, Dodemorph, Fenpropimorph, Fenpropidin, Tridemorph;
Substanzen mit Peroxidgruppen wie Wasserstoffperoxid, und organische Peroxide wie Dibenzoylperodid;
Organische Chlorverbindungen wie z. B. Chlorhexidin;
Triazolfungizide wie Azaconazol, Cyproconazol, Diclobutrazol, Difenoconazol, Diniconazol, Epoxiconazo, Fenbuconazol, Fluquinconazol, Flusilazol, Flutriafol, Hexaconazol, Metconazol, Propiconazol, Tetraconazol, Tebuconazol und Triticonazol;
Strobilurine wie Dimoxystrobin, Fluoxastrobin, Kresoxim-methyl, Metominostrobin, Orysastrobin, Picoxystrobin, Pyraclostrobin und Trifloxystrobin;
Sulfonamide wie Tolylfluanid und Diclofluanid;
lodverbindungen wie Diiodmethyl-p-tolylsulfon, Napcocide 3-lod-2-propinylalkohol, 4-Chlorphenyl-3-iodpropargylformal, 3-Brom-2,3-diiod-3-propenylethylcarbonat, 2,3,3-Triiodallylalkohol, 3-lod-2-propinyl-n-hexylcarbamat, 3-Brom-2,3-diiod-2-propenylalkohol, 3-lod-2-propinylphenylcarbamat, 3-lod-2-propinyl-n-butylcarbamat, O-1-(6-lod-3-oxohex-5-inyl)phenylcarbamat, O-1-(6-lod-3-oxohex-5-inyl)butylcarbamat;
Isothiazolinone wie *N*-Methylisothiazolin-3-on, 5-Chloro-*N*-methylisothiazolin-3-on, 4,5-Dichloror-*N-*octylisothiazolin-3-on, 1,2-Benzisothiazol-3(2H)on, 4,5-Trimethylisothiazol-3-on und *N*-Octyl-isothiazolin-3-on.
Beispiele für Insektizide und Acarizide sind
Organophosphate wie Acephate, Azamethiphos, Azinphos-methyl, Chlorpyrifos, Chlorpyriphos-methyl, Chlorfenvinphos, Diazinon, Dichlorvos, Dicrotophos, Dimethoate, Disulfoton, Ethion, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Sulprophos, Triazophos, Trichlorfon;
insbesondere Pyrethroide wie Acrinatrin, Allethrin, Bioallethrin, Barthrin, Bifenthrin, Bioethanomethrin, Cyclethrin, Cycloprothrin, Cyfluthrin, beta-Cyfluthrin, Cyhalothrin, gamma-Cyhalothrin, lambda-Cyhalothrin, Cypermethrin, α-Cypermethrin, β-Cypermethrin, λ-Cypermethrin, zeta-Cypermethrin, Cyphenothrin, Deltamethrin, Dimefluthrin, Dimethrin, Empenthrin, Fenfluthrin, Fenprithrin, Fenpropathrin, Fenvalerat, Esfenvalerat, Flucythrinat, Fluvinate, tau-Fluvinate, Furethrin, Permethrin, Biopermethrin, Trans-Permethrin, Phenothrin, Prallethrin, Profluthrin, Pyresmethrin, Resmethrin, Bioresmethrin, Cismethrin, Tefluthrin, Terallethrin, Tetramethrin, Tralomethrin, Transfluthrin, Etofenprox, Flufenprox, Halfenprox, Protrifenbute und Silafulfen;
Pyrrol- und Pyrazol-Insektizide wie Acetoprole, Ethiprole, Fipronil, Tebufenpyrad, Tolfenpyrad, Chlorfenapyr und Vaniliprole.
Beispiele für Repellent-Wirkstoffe sind insbesondere Anthrachinon, Acridinbasen, Kupfernaphthenat, Butopyronoxyl, Dibutylphthalat, Dimethylphthalat, Dimethylcarbat, Ethohexadiol, Hexamide, Methoquin-Butyl, N-Methylneodecanamid, Kampfer, Bergamotte-Öl, Pyrethrum, Nelkenöl, Geraniumöl, Thymianöl und insbesondere Diethyl-m-toluamid und 1-Piperidincarboxylsäure-2-(2-hydroxyethyl)-1-methylpropylester (Picardin).
Beispiele für Wellness-Additive sind insbesondere die nachfolgend aufgeführten Substanzen und Substanzgemische, z.B.
Fette, vorzugsweise pflanzlichen Ursprungs, z.B. Lecithine,
Pflanzenöle wie Jojoba-Öl, Teebaumöl, Nelkenöl, Nachtkerzenöl, Mandelöl, Kokosöl, Avocadoöl, Sojaöl und dergleichen,
Fettsäuren, z.B. ω-6-Fettsäuren, Linolensäure, Linolsäure,
Wachse tierischen oder planzlichen Ursprungs wie Bienenwachs, Candelillawachs, Sheabutter, Shoreabutter, Mangokernbutter, Japanwachs und dergleichen,
Vitamine, insbesondere fettlösliche Vitamine, z. B. Tocopherole, Vitamin E, Vitamin A und dergleichen,
Cortico-Steroide wie Cortison, Corticosteron, Dexamethason, Triamcinolon, Methylprednisolon, Fludrocortison, Fluocortolon, Prednison, Prednisolon, Progesteron,
Aminosäuren, z.B. Arginin, Methionin,
Pflanzenextrakte wie Algenextrakt, Rosskastanienextrakt, Mangoextrakt und dergleichen.

Zur Verbesserung der Waschpermanenz der erfindungsgemäßen Ausrüstung hat es sich bewährt, wenn man die amylosehaltige Substanz mit einem Bindemittel auf dem Textil fixiert. Als Bindemittel kommen zum einen filmbildende, wasserunlösliche Polymere und zum anderen niedermolekulare reaktive Substanzen, die unter Erwärmen polymerisieren, in Betracht. In der Regel wird man das Bindemittel in einer Menge einsetzten, dass das Gewichtsverhältnis von amylosehaltiger Substanz zu wasserunlöslichem Polymer im Bereich von 1:1 bis 100:1, vorzugsweise im Bereich von 1,5:1 bis 50:1 und insbesondere im Bereich von 2:1 bis 20:1 liegt.

In der Regel werden die filmbildenden Polymere in Form einer wässrigen Dispersion von feinteiligen Polymerteilchen eingesetzt. Die Teilchengröße ist für den erfindungsgemäßen Erfolg von untergeordneter Bedeutung. Sie liegt jedoch in der Regel unterhalb 5 µm (Gewichtsmittel) und beträgt in der Regel 50 nm bis 2 µm.

Das filmbildende Polymer kann insbesondere eine Glasübergangstemperatur T_{G} im Bereich von -40 bis 100 °C, vorzugsweise -30 bis +60 °C, insbesondere -20 bis +40 °C, aufweist. Sofern das polymere Bindemittel mehrere Polymerkomponenten umfasst sollte zumindest der Hauptbestandteil eine Glasübergangstemperatur in diesem Bereich aufweisen. Insbesondere liegt die Glasübergangstemperatur des Hauptbestandteils im Bereich von -30 °C bis +60 °C und besonders bevorzugt im Bereich von -20 °C bis +40 °C. Vorzugsweise weisen alle polymeren Bestandteile eine Glasübergangstemperatur in diesen Bereichen auf. Die angegebenen Glasübergangstemperaturen beziehen sich dabei auf die gemäß ASTM-D 3418-82 mittels DSC bestimmte "midpoint temperature". Im Falle vernetzbare Bindemittel bezieht sich die Glasübergangstemperatur auf den unvernetzten Zustand.

Beispiele für geeignete filmbildende Polymere basieren auf den folgenden Polymerklassen:
(1) Polyurethan-Harze;
(2) Acrylatharze (Reinacrylate: Copolymere aus Alkylacrylaten und Alkylmethacrylaten);
(3) Styrolacrylate (Copolymere aus Styrol und Alkylacrylaten);
(4) Styrol/Butadien-Copolymerisate;
(5) Polyvinylester, insbesondere Polyvinylacetate und Copolymere des Vinylacetats mit Vinylpropionat;
(6) Vinylester-Olefin-Copolymere, z. B. Vinylacetat/Ethylen-Copolymere;
(7) Vinylester-Acrylat-Copolymere, z. B. Vinylacetat/Alkylacrylat-Copolymere sowie Vinylacetat/Alkylacrylat/Ethylen-Terpolymere.

Derartige Polymere sind bekannt und im Handel erhältlich, z. B. Polymere der Klassen (2) bis (7) in Form wässriger Dispersionen unter den Bezeichnungen ACRONAL, STYROFAN, BUTOFAN (BASF-AG), MOWILITH, MOWIPLUS, APPRETAN (Clariant), VINNAPAS, VINNOL (WACKER). Wässrige, für das erfindungsgemäße Verfahren geeignete Polyurethan-Dispersionen (1) sind insbesondere solche, die für die Beschichtung von Textilien verwendet werden (siehe z. B. J. Hemmrich, Int. Text. Bull. 39, 1993, Nr.2, S. 53-56; "Wässrige Polyurethan-Beschichtungssysteme" Chemiefasern/Textilind. 39 91 (1989) T149, T150; W. Schröer, Textilveredelung 22, 1987, S. 459-467). Wässrige Polyurethandispersionen sind im Handel erhältlich, z. B. unter den Handelsbezeichnungen Alberdingk^{®} der Fa. Alberdingk, Impranil^{®} der Fa. BAYER AG, Permutex^{®} der Fa. Stahl, Waalwijk, Niederlande, der Fa. BASF SE oder können nach bekannten Verfahren hergestellt werden, wie sie beispielsweise in "Herstellverfahren für Polyurethane" in Houben-Weyl, "Methoden der organischen Chemie", Band E 20/Makromolekulare Stoffe, S. 1587, D. Dietrich et al., Angew. Chem. 82 (1970), S. 53 ff., Angew. Makrom. Chem. 76, 1972, 85 ff. und Angew. Makrom. Chem. 98, 1981, 133-165, Progress in Organic Coatings, 9, 1981, S. 281-240, bzw. Römpp Chemielexikon, 9. Auflage, Band 5, S. 3575 beschrieben werden.

Die filmbildenden Polymere können selbstvernetzend sein, d. h. die Polymere weisen funktionelle Gruppen (vernetzbare Gruppen) auf, die beim Trocknen der Zusammensetzung, gegebenenfalls beim Erwärmen, miteinander, mit den funktionellen Gruppen der Amylose oder mit einem niedermolekularen Vernetzer unter Bindungsbildung reagieren.

Beispiele für vernetzbare funktionelle Gruppen umfassen aliphatisch gebundene OH-Gruppen, NH-CH₂-OH-Gruppen, Carboxylat-Gruppen, Anhydrid-Gruppen, verkappte Isocyanatgruppen und Aminogruppen. Häufig wird man ein Polymer verwenden, dass noch freie OH-Gruppen als reaktive Gruppen aufweist. In der Regel beträgt der Anteil der reaktiven funktionellen Gruppen 0,1 bis 3 mol/kg Polymer. Die Vernetzung kann innerhalb des Polymers durch Reaktion komplementär-reaktiver funktioneller Gruppen bewirkt werden. Vorzugsweise bewirkt man die Vernetzung des Polymers durch Zugabe eines Vernetzers, der reaktive Gruppen aufweist, welche hinsichtlich ihrer Reaktivität zu den funktionellen Gruppen des Vernetzers komplementär sind. Geeignete Paare funktioneller Gruppen, die eine komplementäre Reaktivität aufweisen sind dem Fachmann bekannt. Beispiele für solche Paare sind OH/COOH, OH/NCO, NH₂/COOH, NH₂/NCO sowie M²⁺/COOH, wobei M²⁺ für ein zweiwertiges Metallion wie Zn²⁺, Ca²⁺, oder Mg²⁺ steht. Beispiele für geeignete Vernetzer sind die nachstehend bei den Polyurethanen genannten Di- oder Polyole; primäre oder sekundäre Diamine, vorzugsweise primäre Diamine, z. B. Alkylendiamine wie Hexamethylendiamin, Diethylentriamin, Triethylentetramin, Tetraethylenpentamin, N,N-Bis[(aminopropyl)amino]-ethan, 3,6-Dioxaoctandiamin, 3,7-Dioxanonandiamin, 3,6,9-Trioxaundecandiamin oder Jeffamine, (4,4'-Diaminodicyclohexyl)methan, (4,4'-Diamino-3,3-dimethyldicyclohexyl)methan; Aminoalkohole wie Ethanolamin, Hydroxypropylamin; ethoxylierte Di- und Oligoamine; Dihydrazide von aliphatischen oder aromatischen Dicarbonsäuren wie Adipinsäuredihydrazid; Dialdehyde wie Glyoxal; teilweise oder vollständig O-methylierte Melamine, sowie Verbindungen oder Oligomere, die im Mittel zwei oder mehr, vorzugsweise drei oder mehr Isocyanatgruppen oder reversibel z. B. Hydrogensulfit blockierte Isocyanatgruppen aufweisen. In diesem Fall wird das Mengenverhältnis von Vernetzer zu polymerem Bindemittel so bemessen, dass das Molverhältnis der reaktiven Gruppen im polymeren Bindemittel (Gesamtmenge der reaktiven Gruppen in den Polymeren) zu den reaktiven Gruppen im Vernetzer in der Regel im Bereich von 1:10 bis 10:1 und vorzugsweise im Bereich von 3:1 bis 1:3 liegt. Üblicherweise liegt das Gewichtsverhältnis von polymerem Bindemittel (gerechnet als Feststoff) zu Vernetzer im Bereich von 100:1 bis 1:1 und insbesondere im Bereich von 50:1 bis 5:1.

Alternativ zur Fixierung der amylosehaltigen Substanz mit wasserunlöslichen Polymeren kann man die Amylose bzw. die amylosehaltigen Substanz auch mit reaktiven Verbindungen, die wenigstens eine, gegenüber den OH-Gruppen der Amylose reaktive Gruppe und wenigstens eine weitere funktionelle Gruppe aufweisen, die gegenüber den funktionellen Gruppen auf den Fasern des textilen Materials, z.B. OH-Gruppen, NH₂-Gruppen oder COOH-Gruppen, reaktiv ist, auf dem textilen Material fixieren. Zu den reaktiven Verbindungen zählen die oben genannten Vernetzer sowie die in DE-A 40 35 378 für die Fixierung von Cyclodextrinen vorgeschlagenen Substanzen, z.B. *N*-Hydroxymethyl- und *N-*Alkoxymethylderivate von Harnstoff oder harnstoffartigen Verbindungen wie Dimethylolharnstoff (Bis(hydroxymethyl)harnstoff), Di(methoxymethyl)harnstoff, Dimethylolalkandioldiurethane wie *N*,*N-*Dimethylolethylenharnstoff (*N*,*N*-Bis(hydroxymethyl)imidazolin-2-on), *N*,*N*-Dimethylol-dihydroxyethylenharnstoff (*N*,*N*-Bis(hydroxymethyl)-4,5-dihydroxyimidazolin-2-on), Dimethylolpropylenharnstoff und dergleichen. Derartige Materialien sind in Form wässrige Formulierungen für die Ausrüstung von Textilien im Handel, z.B. unter den Handelsbezeichnungen Fixapret^{®} und Fixapret^{®}-eco der BASF SE. Zu den reaktiven Materialien, die zur Fixierung der amylosehaltigen Substanz auf dem textilen Material genutzt werden können, zählen insbesondere auch Verbindungen mit 2, 3, 4 oder mehr (gegebenenfalls reversibel blockierten) Isocyanat-Gruppen, speziell die mit Bisulfit oder CH-aciden Verbindungen oder Oximene, z.B. Butanonoxim reversibel blockierten Polyisocyanat-Präpolymere auf Basis von Polyether- und Polyesterurethanen, die in DE 2837851, DE 19919816 und der älteren Europäischen Patentanmeldung 03015121 beschrieben werden. Derartige Produkte sind auch kommerziell erhältlich, beispielsweise unter den Handelsbezeichnungen PROTOLAN^{®}367 und PROTOLAN^{®}357 der Rotta GmbH, Mannheim.

Zur Fixierung der amylosehaltigen Substanz kann auch die für die Fixierung von Cyclodextrinen bekannte Vorgehensweise in analoger Weise genutzt werden, bei der man das Cyclodextrin bzw. im vorliegenden Fall die amylosehaltige Substanz mit Reaktivankern versieht, beispielsweise indem man sie mit Dicarbonsäuren oder Dicarbonsäureanhydriden wie Maleinsäure, Fumarsäure, Maleinsäureanhydrid, Bernsteinsäure, Bernsteinsäureanhydrid oder Adipinsäure, mit Diisocyanaten, z. B. Toluoldiisocyanat, Isophorondiisocyanat, Tetramethylendiisocyanat oder Hexamethylendiisocyanat, oder mit Aminocarbonsäuren in an sich bekannter Weise dergestalt umsetzt, dass nur eine der in diesen Verbindungen vorhandenen Funktionalitäten mit den OH-Gruppen der amylosehaltigen Substanz reagiert und die andere zur Anbindung an die reaktiven Gruppen des Fasermaterials erhalten bleibt. Reaktivanker lassen sich auf der amylosehaltigen Substanz auch durch Umsetzung mit 1 ,3,5-Trichlortriazin, 2,3-Dichlorchinoxalin-5,6-carbonsäurechlorid sowie mit Chlordifluorpyrimidin erzeugen.

Ferner können zur Fixierung der Amylose auch Alkoxysilane, wie Diethoxydimethylsilan, Dimethoxydimethylsilan, Triethoxyphenylsilan, Tetraethoxysilan sowie dimere, trimere und höhere Kondensationsprodukte dieser Verbindungen eingesetzt werden.

Auf diese Weise können grundsätzlich alle textilen Materialien ausgerüstet werden, d.h. nicht konfektionierte Ware als auch konfektionierte Ware. Textile Materialien umfassen hier und im Folgenden Gewebe, Gestricke, Gewirke und Vliese. Die textilen Materialen können aus Naturfasergarnen, Synthesefasergarnen und/oder Mischgarnen aufgebaut sein. Als Fasermaterialien kommen grundsätzlich alle für die Herstellung von Textilien üblicherweise eingesetzten Fasermaterialien in Betracht. Hierzu zählen Baumwolle, Wolle, Hanffaser, Sisalfasern, Flachs, Ramie, Polyacrylnitrilfasern, Polyesterfasern, Polyamidfasern, Viskosefasern, Seide, Acetatfasern, Triacetatfasern, Aramidfasern und dergleichen sowie Mischungen dieser Fasermaterialien.

Das Ausrüsten bzw. Behandeln der textilen Materialien mit der amylosehaltigen Substanz kann in an sich bekannter Weise, z.B. mittels der in DE-A 4035378 für die Ausrüstung von Textilien mit Cyclodextrinen beschriebenen Verfahren erfolgen.

Zu nennen sind beispielsweise Verfahren, bei denen die amylosehaltige Substanz, gegebenenfalls als Komplex mit dem Wirkstoff bereits in die Faser, das Filament und/oder das Garn eingesponnen ist, aus dem das Gewebe hergestellt ist.

Häufig wird man jedoch das textile Material vor oder nach Konfektionierung mit der amylosehaltigen Substanz oder einem Komplex aus amylosehaltiger Substanz und Wirkstoff behandeln. In der Regel wird man hierzu das Textil mit einer wässrigen Flotte behandeln, welche die amylosehaltige Substanz und gegebenenfalls den Wirkstoff in ausreichender Menge enthält. Je nach Art des Auftrags und der gewünschten Menge, in der die amylosehaltiger Substanz aufgebracht werden soll, liegt die Konzentration an amylosehaltiger Substanz in der Flotte im Bereich von 1 bis 40 Gew.-%, insbesondere im Bereich von 2 bis 20 Gew.-% und speziell im Bereich von 4 bis 15 Gew.-%.

Die Art der Behandlung ist von untergeordneter Bedeutung und kann beispielsweise als Minimalauftrag, z.B. durch Sprühauftrag, als Normalauftrag im Foulard oder als Hochfeuchteauftrag erfolgen. Hierbei wird das textile Material mit der wässrigen Flotte getränkt. Gegebenenfalls kann man danach überschüssige Flotte entfernen, z.B. durch Abquetschen auf eine Flottenaufnahme von etwa 30 bis 120 %.

Eine andere Möglichkeit zur Behandlung des Textils mit amylosehaltiger Substanz bzw. Komplex aus amylosehaltiger Substanz und Wirkstoff, ist eine Flotte mit Wasser anzusetzen, in der die gewünschten Menge an amylosehaltiger Substanz und gegebenenfalls Wirkstoff enthalten ist, z.B. 0,5 bis 20 Gew.-% (bezogen auf die Masse der auszurüstenden Textilie). Das textile Material wird über einen gewissen Zeitraum, z.B. 10-60 min mit der Behandlungsflotte in hierfür geeigneten Ausrüstungsaggregaten (z.B. Haspelkufe; Rollenkufe; Paddel; etc.) durchtränkt und danach wie oben angegeben abgequetscht und/oder abgeschleudert. Das Flottenverhältnis liegt hierbei in der Regel im Bereich von 1:2 bis 1:50 und insbesondere im Bereich von 1:3 bis 1:20.

Derartige Verfahren sind dem Fachmann bekannt, beispielsweise aus H.K Rouette, Lexikon der Textilveredlung, Laumann-Verlag, Dülmen 1995, S. 669 ff.

In der Regel schließt sich an die Behandlung mit der Flotte ein Trocknungsvorgang an. Die Temperaturen liegen dabei in der Regel im Bereich von 100 bis 200°C und vorzugsweise im Bereich von 120 bis 180°C. Das Trocknen kann in den hierfür üblichen Vorrichtungen durchführen, bei konfektionierter Ware beispielsweise durch trockentumblen bei den oben angegebenen Temperaturen. Bei nichtkonfektionierter Ware wird man in der Regel im Anschluss an den Auftrag das textile Material über ein oder mehrere Spannrahmen führen.

Sofern die amylosehaltige Substanz zusammen mit einem filmbildenden Polymer eingesetzt wird, führt das Trocknen zu einer Fixierung der amylosehaltigen Substanz auf den Textilfasern. In der Regel wird dann die Trocknungstemperatur 100 nicht unterschreiten und liegt vorzugsweise im Bereich von 120 bis 200°C und insbesondere im Bereich von 140 bis 180°C. Im Allgemeinen erfolgt das Trocknen während einer Zeitdauer von 1 bis 10 min, insbesondere 1 bis 2 min, wobei längere Trockenzeiten ebenfalls geeignet sind.

Für das Behandeln mit einer wässrigen Flotte hat es sich als vorteilhaft erwiesen, wenn die wässrige Flotte neben der amylosehaltigen Substanz und gegebenenfalls dem Wirkstoff wenigstens eine oberflächenaktive Substanz (bzw. grenzflächenaktive Substanz) enthält, welche zur Dispergierung der amylosehaltigen Substanz und dem Wirkstoff in der wässrigen Flotte geeignet ist. Vorzugsweise handelt es sich bei der oberflächenaktiven Substanz um ein oligomeres oder polymeres Dispergiermittel. Der Begriff oligomeres oder polymeres Dispergiermittel umfasst im Unterschied zu niedermolekularen oberflächenaktiven Substanzen solche Dispergiermittel, deren zahlenmittleres Molekulargewicht in der Regel wenigstens 2000 Dalton beträgt, z.B. 2000 bis etwa 100000 Dalton und insbesondere im Bereich von etwa 3000 bis 70000 Dalton liegt.

In der Regel enthält die wässrige Flotte das polymere oder oligomere Dispergiermittel in einer Menge von 0,5 bis 20 Gew.-%, vorzugsweise 1 bis 18 Gew.-% und insbesondere 5 bis 15 Gew.-%, bezogen auf die amylosehaltige Substanz.

Geeignete oligomeres oder polymeres Dispergiermittel sind in Wasser löslich und umfassen sowohl neutrale und amphotere wasserlösliche Polymere als auch kationische und anionische Polymere, wobei letztere bevorzugt sind.

Beispiele für neutrale polymere Dispergiermittel sind Polyethylenoxid, Ethylenoxid/ Propylenoxid-Copolymere, bevorzugt Blockcopolymere, Polyvinylpyrrolidon sowie Copolymere von Vinylacetat mit Vinylpyrrolidon.

Die bevorzugten anionischen oligomeren bzw. polymeren Dispergiermittel zeichnen sich dadurch aus, dass sie Carboxylgruppen und/oder Sulfonsäuregruppen aufweisen und üblicherweise als Salze, z.B. als Alkalimetallsalze oder Ammoniumsalze eingesetzt werden.

Bevorzugte anionische Dispergiermittel sind beispielsweise carboxylierte Derivate der Zellulose wie Carboxymethylcellulose, Homopolymere ethylenisch ungesättigter Cs- bis Cs-Mono- und C₄- bis Cs-Dicarbonsäuren, z.B. der Acrylsäure, der Methacrylsäure, der Maleinsäure, der Itaconsäure, Copolymere wenigstens zweier verschiedener ethylenisch ungesättigter Cs- bis Cs-Mono- und C₄- bis Cs-Dicarbonsäuren wie vorstehend genannt, und Copolymere wenigstens einer der vorgenannten ethylenisch ungesättigten Cs- bis Cs-Mono- oder C₄- bis Cs-Dicarbonsäure mit wenigstens einem neutralen Comonomeren. Beispiele für neutrale Comonomere sind *N*-Vinyllactame wie *N-*Vinylpyrrolidon, Vinylester aliphatischer C₂- bis C₁₆-Carbonsäuren wie Vinylacetat, Vinylpropionat, Amide der vorgenannten ethylenisch ungesättigten Carbonsäuren, wie Acrylamid, Methacrylamid und dergleichen, Hydroxy-C₁- bis C₄-alkyl(meth)acrylate wie Hydroxyethylacrylat und -methacrylat, Ester ethylenisch ungesättigter Cs- bis Cs-Mono- oder C₄- bis Cs-Dicarbonsäuren mit Polyethern, z.B. Ester der Acrylsäure oder der Methacrylsäure mit Polyethylenoxiden oder Ethylenoxid/Propylenoxid-Blockcopolymeren, Vinylaromaten wie Styrol und C₂- bis C₁₆-Olefine wie Ethylen, Propen, 1-Hexen, 1-Octen, 1-Decen, 1-Dodecen und dergleichen. Weiterhin bevorzugt sind Homopolymere ethylenisch ungesättigter Sulfonsäuren wie Styrolsulfonsäure und Acrylamidopropansulfonsäure und deren Copolymer mit den vorgenannten Comonomeren. In den Copolymeren wird der Anteil der ethylenisch ungesättigten Säure in der Regel wenigstens 20 Gew.-% betragen und einen Wert von 90 Gew.-% und insbesondere 80 Gew.-%, jeweils bezogen auf das Gesamtgewicht aller das Polymer konstituierenden Monomere, nicht überschreiten.

Copolymerisate aus mindestens einer der oben genannten Säuren und mindestens einem Comonomer sind für diesen Zweck bekannt und im Handel erhältlich, beispielsweise die Copolymere von Acrylsäure und Maleinsäure als Sokalan-Marken der BASF SE.

Ebenfalls bevorzugte anionische Dispergiermittel sind Phenolsulfonsäure-Formaldehyd-Kondensate und Naphthalinsulfonsäure-Formaldehyd-Kondensate (z.B. die Tamol- und Setamol-Marken der BASF) und Ligninsulfonate.

Brauchbare Dispergiermittel sind außerdem niedermolekulare anionische, nicht-ionische, kationische, ampholytische und zwitterionische Tenside. Geeignete Tenside sind z.B. die Alkalimetall-, Ammonium- oder Aminsalze von Cs- bis C₁₈-Alkylsulfaten, wie Natriumlaurylsulfat; Cs- bis C₁₈-Alkylsulfonaten, wie Dodecylsulfonat; Cs- bis C₁₈-Alkylethersulfaten; sowie C₈- bis C₁₈-Alkylethoxylate; Polyoxyethylensorbitanester; Cs- bis C₁₈-Alkylglycinate; Cs- bis C₁₈-Alkyldimethylaminoxide; Betaine etc. Bevorzugt sind die Alkylsulfate und Alkylsulfonate.

Sofern die amylosehaltige Substanz nicht gemeinsam mit einem filmbildenden, wasserunlöslichen Polymer eingesetzt wird, kann das Textil in einem separaten Arbeitsschritt mit dem Polymer behandelt werden. Insbesondere erfolgt die Behandlung gemeinsam mit der amylosehaltigen Substanz. Dementsprechend betrifft eine besondere Ausführungsform ein Verfahren, bei dem die wässrige Flotte zusätzlich ein dispergiertes, filmbildendes, wasserunlösliches Polymer der oben bezeichneten Art umfasst. Die Menge an filmbildenden Polymer ist dabei so gewählt, dass das Gewichtsverhältnis von amylosehaltiger Substanz zu wasserunlöslichem Polymer im Bereich von 1:1 bis 100:1, vorzugsweise im Bereich von 1,5:1 bis 50:1 und insbesondere im Bereich von 2:1 bis 20:1 liegt.

Die Ausrüstung des Textils mit dem Wirkstoff kann in einem separaten Vorgang oder in einem Arbeitsgang zusammen mit der Ausrüstung mit der amylosehaltigen Substanz erfolgen.

Sofern man das Textil in einem separaten Arbeitsgang mit dem Wirkstoff ausrüstet, wird man das Textil zweckmäßigerweise ebenfalls mit einer wässrigen Flotte des Wirkstoffs behandeln. Hierzu wird man in der Regel den Wirkstoff, der üblicherweise in Wasser nicht löslich ist, in Wasser emulgieren oder dispergieren, gegebenenfalls unter Verwendung geeigneter oberflächenaktiver Substanzen. Geeignete oberflächenaktive Substanzen sind insbesondere die zuvor erwähnten niedermolekularen Tenside und hierunter bevorzugt die nichtionischen Tenside, insbesondere Polyoxyethylensorbitanester, Ester von Mono- oder Oligosacchariden mit C₆- bis C₁₈-Fettsäuren und besondere bevorzugt Cs- bis C₁₈-Alkylethoxylate, insbesondere solche mit einem Ethoxylierungsgrad im Bereich von 6 bis 50. In der Regel enthält die wässrige Flotte den Wirkstoff in einer Menge von 0,1 bis 10 Gew.-% und insbesondere in einer Menge von 0,2 bis 5 Gew.-%. Die Menge an oberflächenaktiver Substanz liegt in der Regel im Bereich von 0,5 bis 50 Gew.-% und insbesondere im Bereich von 3 bis 30 Gew.-%, bezogen auf den Wirkstoff. Das Aufbringen des Wirkstoffs aus wässriger Flotte kann mit den hierfür üblichen Methoden erfolgen, z.B. mittels eines Foulards.

Man kann aber auch die Ausrüstung mit Wirkstoff und amylosehaltiger Substanz in einem Arbeitsgang durchführen. Dabei kann man grundsätzlich wie für die Ausrüstung mit der amylosehaltigen Substanz beschrieben vorgehen, wobei die wässrige Flotte der amylosehaltigen Substanz nunmehr zusätzlich den wenigstens einen Wirkstoff enthält. Der Wirkstoff kann dabei separat der Flotte zugegeben werden oder in Form einer Einschlussverbindung, d.h. in Form eines Wirt-Gast-Komplexes mit der amylosehaltigen Substanz.

Die vorliegende Erfindung kann zur Ausrüstung beliebiger Textilien, einschließlich Gewebe, Gewirke, Vliese und dergleichen genutzt werden. Die Art des textilen Materials richtet sich in erster Linie nach dem gewünschten Anwendungszweck.

Bei den auszurüstenden Textilien kann es sich um fertig konfektionierte Produkte wie Bekleidung, einschließlich Unterwäsche und Oberbekleidung, wie z.B. Hemden, Hosen, Jacken, Outdoor-, Trecking- und Militärausrüstungen, Dächer, Zelte, Netze, z.B. Insektenschutznetze und Vorhänge, Hand- und Badetücher, Bettwäsche und dergleichen handeln.

In gleicher Weise kann die Ausrüstung auf der Rohware in Ballen- oder Rollenform erfolgen.

Die mit Wirkstoffen gegen parasitäre Organismen wie Insekten und Acariden ausgerüsteten Textilien sind neben dem Schutz des Menschen auch besonders im Tierschutz zum Schutz gegen Zecken, Milben, Flöhe und dergleichen geeignet.

Die textilen Materialen können aus Naturfasergarnen, Synthesefasergarnen und/oder Mischgarnen aufgebaut sein, wobei die Gewebe üblicherweise ein Flächengewicht im Bereich von 10 bis 500 g/m² vorzugsweise 20 bis 250 g/m² aufweisen. Als Fasermaterialien kommen grundsätzlich alle für die Herstellung von Textilien üblicherweise eingesetzten Fasermaterialien in Betracht. Hierzu zählen Baumwolle, Wolle, Hanffaser, Sisalfasern, Flachs, Ramie, Polyacrylnitrilfasern, Polyesterfasern, Polyamidfasern, Viskosefasern, Seide, Acetatfasern, Triacetatfasern, Aramidfasern und dergleichen sowie Mischungen dieser Fasermaterialien. Geeignet sind auch Glasfasern sowie Mischungen der vorgenannten Fasermaterialien mit Glasfasern z. B. Glasfaser/Kevlar-Mischungen.

Mit einer oben beschriebenen amylose-basierten Wirkstoff-Ausrüstung verbleiben die Wirkstoffe auch nach mehreren Wäschen in den damit ausgerüsteten Textilien. Zudem zeichnen sich die so ausgerüsteten Textilien durch einen angenehmen Griff aus, was insbesondere für den Tragekomfort von aus diesen Textilien hergestellter Bekleidung von Vorteil ist.

### Kühlende Tabakprodukte:

Grundsätzlich kann der Wirkstoffgehalt über einen weiten Bereich, wie z.B. 0,05 ppm bis 10 Gew.-%, bevorzugt 0,1 ppm bis 10 Gew.-% variieren.

Die erfindungsgemäßen Wirkstoffe können in vorteilhafter Weise auch zur Herstellung von Tabakprodukten verwendet werden. Beispiele für derartige Tabakprodukte umfassen, Zigarren, Zigaretten, Pfeifentabak, Kautabak, und Schnupftabak.

Die Herstellung von Tabakprodukten, welche mit kühlend wirkende Zusätzen ergänzt sind, ist an sich bekannt und z.B. beschrieben in US 3,111,127, US 5,752,529 und US 2005/0000529, worauf hiermit ausdrücklich Bezug genommen wird.

### Kühlende Verpackungsmaterialien:

Die erfindungsgemäßen Wirkstoffe sind auch in vorteilhafter Weise zur Herstellung von Verpackungsmaterialien geeignet.

Die Herstellung erfolgt dabei ebenfalls in an sich bekannter Weise. Die Wirkstoffe können dabei in das Verpackungsmaterial, in freier oder z.B. verkapselter Form, eingearbeitet oder auf das Verpackungsmaterial, in freier oder verkapselter Form aufgebracht werden.

So sind entsprechend ausgerüstete Kunststoffverpackungsmaterialien entsprechend den Angaben in der Literatur zur Herstellung von Polymerfilmen herstellbar (z.B. Ullmann, 6th Edn, 2003. Vol. 36, S. 567). Die Herstellung in geeigneter Weise beschichteter Papiere ist ebenfalls bekannt und z.B. beschrieben in Ullmann, Vol. 25, S. 106 ff, 6th Edn, 2003.

Weitere Aspekte der vorliegenden Erfindung ergeben sich aus den nachfolgenden Beispielen sowie den beigefügten Patentansprüchen.

### Beispiele:

Die Beispiele dienen zur Verdeutlichung der Erfindung, ohne diese damit einzuschränken. Sofern nicht anders angegeben, beziehen sich alle Angaben auf das Gewicht.

### Wirkstoffherstellung:

Die erfindungsgemäß verwendeten Wirkstoffe des Strukturtyps 1 sind entweder an sich bekannte Verbindungen oder können in Anlehnung an bekannte Synthesemethoden vom Fachmann auf dem Gebiet der organischen Synthese hergestellt werden.

### Klonierung von humanem TRPM8:

Ausgangspunkt für die Klonierung des humanen TRPM8 Rezeptors ist eine LnCaP cDNA-Bank. Diese ist beispielsweise kommerziell erhältlich (z. B. Firma BioChain, Hayward, USA) oder aus der androgensensitiven humanen Prostata-Adenokarzinomzelllinie LnCaP (z.B. ATCC, CRL1740 oder ECACC, 89110211) unter Verwendung von Standardkits herstellbar.

Die kodierende TRPM8 Sequenz (siehe z.B. http://www.ncbi.nlm.nih.gov/entrez/viewer.fcgi?db=nuccore&id=109689694) kann unter Verwendung von Standardmethoden PCR-amplifiziert und kloniert werden. Das so isolierte humane TRPM8 Gen wurde zur Herstellung des Plasmids plnd_M8 verwendet.

Alternativ dazu kann das TRPM8 Gen auch synthetisch hergestellt werden.

### Generierung der HEK293-Testzellen:

Als Testzellsystem wurde mit der humanen TRPM8 DNA eine stabil transfizierte HEK293 Zelllinie hergestellt. Bevorzugt wird dabei HEK293, die über das eingebrachte Plasmid die Möglichkeit zur Induktion der TRPM8-Expression mittels Tetrazyklin bietet.

Methoden zur Herstellung geeigneter Testzellsysteme sind dem Fachmann bekannt. So kann man die Herstellung der verwendeten Zellen den Angaben in Behrendt H.J. et al., Br. J. Pharmacol. 141, 2004, 737-745 oder der Dissertation von Behrendt "Vergleichende funktionale Untersuchungen des Hitze-Capsaicin-Rezeptors (TRPV1) und des Kälte-Menthol-Rezeptors (TRPM8) in rekombinanten und nativen Zellssystemen", z.B. zugänglich unter http://www-brs.ub.ruhr-uni-bochum.de/netahtml/HSS/Diss/BehrendtHansJoerg/diss.pdf entnehmen. Auf die Offenbarung dieser Druckschriften wird ausdrücklich Bezug genommen.

### Assay auf TRPM8 Modulatoren:

Es wird ein Test, vergleichbar mit dem bereits in der Literatur beschrieben Test von Behrendt H.J. et al., Br. J. Pharmacol. 141, 2004, 737-745, durchgeführt. Die Agonisierung bzw. Antagonisierung des Rezeptors kann mittels eines Ca²⁺-sensitiven Farbstoffs (z.B. FURA, Fluo-4 etc.) quantifiziert werden. Agonisten bewirken alleine eine Zunahme des Ca²⁺-Signals; Antagonisten bewirken in Gegenwart von z.B. Menthol eine Reduzierung des Ca²⁺-Signals (jeweils detektiert über den Farbstoff Fluo-4, der durch Ca²⁺ andere Fluoreszenzeigenschaften hat).

Zunächst wird in an sich bekannter Weise in Zellkulturflaschen eine frische Kultur transformierter HEK-Zellen hergestellt. Die Testzellen HEK293-TRPM8 werden mittels Trypsin von den Zellkulturflaschen abgelöst und 40.000 Zellen/well werden mit 100 µl Medium in 96-Lochplatten (Greiner # 655948 Poly-D-Lysin-beschichtet) ausgesät. Zur Induktion des Rezeptors TRPM8 wird dem Wachstumsmedium Tetrazyklin beigemischt (DMEM/HG, 10% FCS tetrazyklinfrei, 4 mM L-Glutamin, 15 µg/ml Blasticidin, 100 µg/ml Hygromycin B, 1 µg/ml Tetrazyklin). Am darauffolgenden Tag werden die Zellen mit Fluo-4AM Farbstoff beladen und der Test wird durchgeführt. Dazu wird wie folgt vorgegangen:
- Zugabe von je 100 µl/well Färbelösung Ca-4 Kit (RB 141, Molecular Devices) zu je 100 µl Medium (DMEM/HG, 10% FCS tetrazyklinfrei, 4 mM L-Glutamin, 15 µg/ml Blasticidin, 100 µg/ml Hygromycin B, 1 µg/ml Tetrazyklin)
- Inkubation im Brutschrank, 30 Minuten / 37°C / 5% CO₂, 30 Minuten / RT
- Vorbereitung der Testsubstanzen (unterschiedliche Konzentrationen in 200 µl HBSS-Puffer), sowie von Positivkontrollen (unterschiedliche Konzentrationen von Menthol, Icilin bzw. lonomycin in 200 µl HBSS-Puffer) und Negativkontrollen (nur 200 µl HBSS-Puffer)
- Zugabe der Testsubstanzen in Mengen von 50 µl/well und Messung der Fluoreszenzänderung (z.B. im Assaygerät FLIPR, Molecular Devices oder NovoStar, BMG) bei 485 nm Anregung, 520 nm Emission, und Auswertung der Wirkstärke der verschiedenen Substanzen/Konzentrationen und Bestimmung der EC50-Werte.

Die Testsubstanzen werden in Triplikaten in Konzentrationen von 0,1 - 200 µM im Assay eingesetzt. Normalerweise werden die Verbindungen in DMSO-Lösungen bereitgehalten und für den Assay auf eine maximale DMSO-Konzentration von 2% herunterverdünnt.

Eigene Auswertungen bei Durchführung des beschriebenen Assays zeigten überraschenderweise, dass die erfindungsgemäß zu verwendenden Verbindungen (wie hierin beschrieben) als Agonisten von TRPM8 besonders geeignet sind.

Die ermittelten EC50-Werte beispielhaft ausgewählter erfindungsgemäßer Modulatoren sind in der folgenden Tabelle gezeigt.

| Struktur | Name | Aktivität In-vitro-Assay (EC50) |
|---|---|---|
| 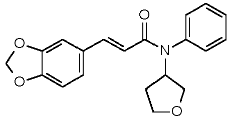 | (E)-3-(1,3-benzodioxol-5-yl)-N-phenyl-N-tetrahydrofuran-3-yl-prop-2-enamid | 25 µM |
| 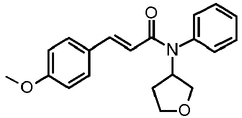 | (E)-3-(4-Methoxyphenyl)-N-phenyl-N-tetrahydrofuran-3-yl-prop-2-enamid | 12,5 µM |
| 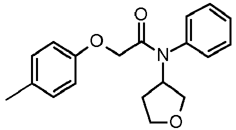 | 2-(4-Methylphenoxy)-N-phenyl-N-tetrahydrofuran-3-yl-acetamid | 25 µM |
| 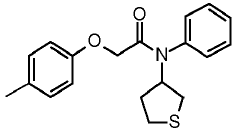 | 2-(4-Methylphenoxy)-N-phenyl-N-tetrahydrothiophen-3-yl-acetamid | 12,5 µM |
| 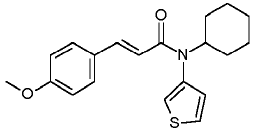 | (E)-N-Cyclohexyl-3-(4-methoxyphenyl)-N-(3-thienyl)prop-2-enamid | 25 µM |
| 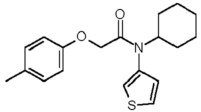 | N-Cyclohexyl-2-(4-methylphenoxy)-N-(3-thienyl)acetamid | 30 µM |
| 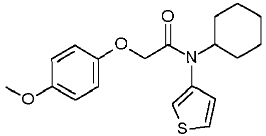 | N-Cyclohexyl-2-(4-methoxyphenoxy)-N-(3-thienyl)acetamid | 50 µM |
| 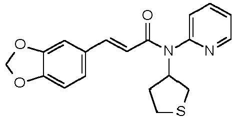 | (E)-3-(1,3-benzodioxol-5-yl)-N-(2-pyridyl)-N-tetrahydrothiophen-3-yl-prop-2-enamid | 6 µM |
| 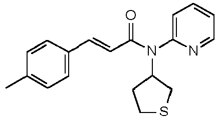 | (E)-3-(p-tolyl)-N-(2-pyridyl)-N-tetrahydrothiophen-3-yl-prop-2-enamid | 25 µM |
| 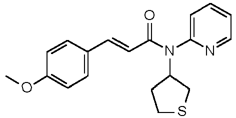 | (E)-3-(4-methoxyphenyl)-N-(2-pyridyl)-N-tetrahydrothiophen-3-yl-prop-2-enamid | 25 µM |
| 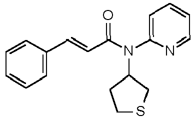 | (E)-3-phenyl-N-(2-pyridyl)-N-tetrahydrothiophen-3-yl-prop-2-enamid | 30 µM |
| 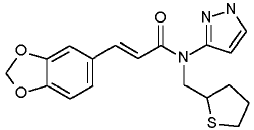 | (E)-3-(1,3-benzodioxol-5-yl)-N-(1H-pyrazol-3-yl)-N-(tetrahydrothiophen-2-ylmethyl)prop-2-enamid | 5 µM |
| 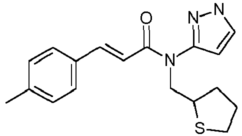 | (E)-3-(p-Tolyl)-N-(1H-pyrazol-3-yl)-N-(tetrahydrothiophen-2-ylmethyl)prop-2-enamid | 50 µM |
| 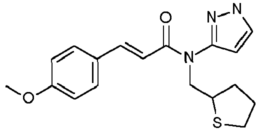 | (E)-3-(4-methoxyphenyl)-N-(1H-pyrazol-3-yl)-N-(tetrahydrothiophen-2-ylmethyl)prop-2-enamid | 50 µM |
| 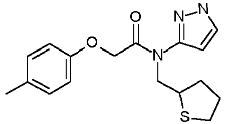 | 2-(4-Methylphenoxy)-N-(1H-pyrazol-3-yl)-N-(tetrahydrothiophen-2-ylmethyl)acetamid | 50 µM |
| 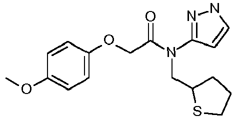 | 2-(4-Methoxyphenoxy)-N-(1H-pyrazol-3-yl)-N-(tetrahydrothiophen-2-ylmethyl)acetamid | 50 µM |
| 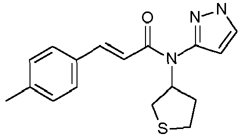 | (E)-3-(p-Tolyl)-N-(1H-pyrazol-3-yl)-N-tetrahydrothiophen-3-yl-prop-2-enamid | 50 µM |
| 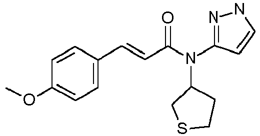 | (E)-3-(4-Methoxyphenyl)-N-(1H-pyrazol-3-yl)-N-tetrahydrothiophen-3-yl-prop-2-enamid | 50 µM |
| 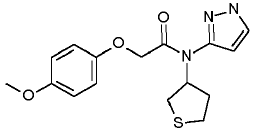 | 2-(4-Methoxyphenoxy)-N-(1H-pyrazol-3-yl)-N-tetrahydrothiophen-3-yl-acetamid | 10 µM |
| 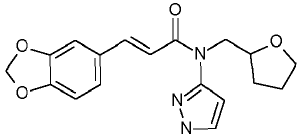 | (E)-3-(1,3-benzodioxol-5-yl)-N-(1H-pyrazol-3-yl)-N-(tetrahydrofuran-2-ylmethyl)prop-2-enamid | 15 µM |
| 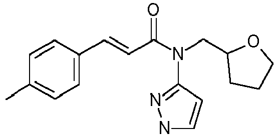 | (E)-3-(p-tolyl)-N-(1H-pyrazol-3-yl)-N-(tetrahydrofuran-2-ylmethyl)prop-2-enamid | 10µM |
| 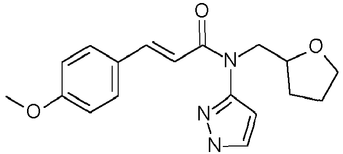 | (E)-3-(4-methoxyphenyl)-N-(1H-pyrazol-3-yl)-N-(tetrahydrofuran-2-ylmethyl)prop-2-enamid | 25µM |
| 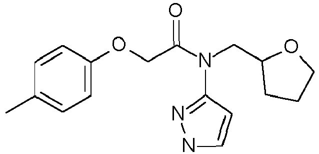 | 2-(4-methylphenoxy)-N-(1H-pyrazol-3-yl)-N-(tetrahydrofuran-2-ylmethyl)acetamid | 10µM |
| 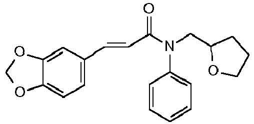 | (E)-3-(1,3-benzodioxol-5-yl)-N-phenyl-N-(tetrahydrofuran-2-ylmethyl)prop-2-enamid | 5µM |
| 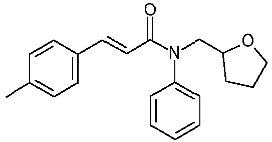 | (E)-N-phenyl-3-(p-tolyl)-N-(tetrahydrofuran-2-ylmethyl)prop-2-enamid | 5µM |
| 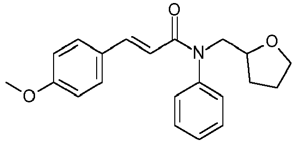 | (E)-3-(4-methoxyphenyl)-N-phenyl-N-tetrahydrofuran-2-ylmethyl)prop-2-enamid | 25 µM |
| 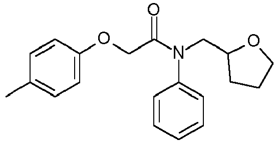 | 2-(4-methylphenoxy)-N-phenyl-N-tetrahydrofuran-2-ylmethyl)acetamid | 50 µM |
| 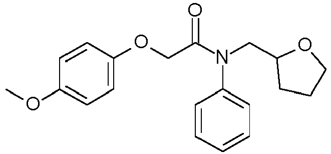 | 2-(4-methoxyphenoxy)-N-phenyl-N-(tetrahydrofuran-2-ylmethyl)acetamid | 50 µM |
| 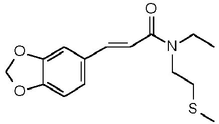 | (E)-3-(1,3-benzodioxol-5-yl)-N-ethyl-N-(2-methylsulfanylethyl)prop-2-enamid | 15 µM |
| 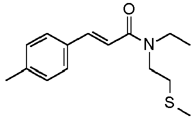 | (E)-N-ethyl-N-(2-methylsulfanylethyl)-3-(p-olyl)prop-2-enamid | 10 µM |
| 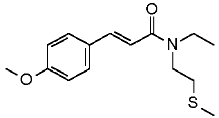 | (E)-N-ethyl-3-(4-methoxyphenyl)-N-(2-methylsulfanylethyl)prop-2-enamid | 50 µM |
| 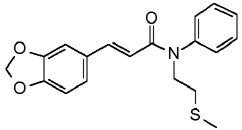 | (E)-3-(1,3-benzodioxol-5-yl)-N-(2-methylsulfanylethyl)-N-phenyl-prop-2-enamid | 50 µM |
| 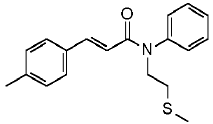 | (E)-N-(2-methylsulfanylethyl)-N-phenyl-3-(p-tolyl)prop-2-enamid | 50 µM |
| 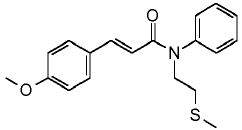 | (E)-3-(4-methoxyphenyl)-N-(2-methylsulfanylethyl)-N-phenyl-prop-2-enamid | 25 µM |
| 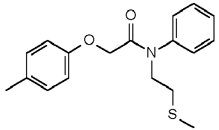 | 2-(4-methylphenoxy)-N-(2-methylsulfanylethyl)-N-phenyl-acetamid | 25 µM |
| 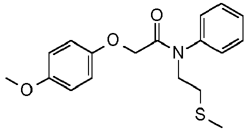 | 2-(4-methoxyphenoxy)-N-(2-methylsulfanylethyl)-N-phenyl-acetamid | 30 µM |
| 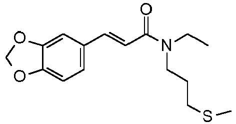 | (E)-3-(1,3-benzodioxol-5-yl)-N-ethyl-N-(3-methylsulfanylpropyl)prop-2-enamid | 30 µM |

### Synthesebeispiele:

### 1) Herstellung von N-ethyl-2-methylsulfanyl-ethanamin

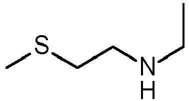

1,1-Dimethoxy-2-(methylthio)ethan (21.5 mL, 161.07 mmol, 1.0 Äq) wird in HCl-Lösung (0.5 M, 323 mL, 1.0 Äq) gelöst und 1 h bei 50 °C gerührt. Das Reaktionsgemisch wird auf RT abgekühlt und mit CH₂Cl₂ extrahiert (500 mL). Die vereinten organischen Phasen werden über Na₂SO₄ getrocknet. Die Aldehyd-Lösung wird mit THF (500 mL) verdünnt, auf 0 °C gekühlt und anschließend mit Ethylamin (70 %-ige Lösung in Wasser, 15.57 mL, 241.61 mmol, 1.5 Äq), Essigsäure (9.1 mL, 161.07 mmol, 1.0 Äq) und Na₂SO₄ (250 g) versetzt. Nach 16 h wird das Reaktionsgemisch durch Na₂SO₄ filtriert und mit Natriumtriacetoxyborhydrid (37.6 g, 177.17 mmol, 1.1 Äq) versetzt. Das Reaktionsgemisch wird bei RT 16 h gerührt. Der Feststoff wird über Kieselgur abfiltriert und die Lösung im Vakuum eingeengt. Das Rohprodukt wird in Vakuum destilliert (7 mbar, 60 °C). Die Verbindung wird ohne weitere Analysen verwendet.

Ausbeute: 23 %.

### 2) Herstellung von N-ethyl-3-methylsulfanyl-propan-1-amin

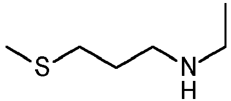

Methional (25.0 g, 240.0 mmol, 1.0 Äq) wird in THF (1000 mL) gelöst, auf 0 °C gekühlt und anschließend mit Ethylamin (70 %-ige Lösung in Wasser, 23.2 mL, 360.0 mmol, 1.5 Äq), Essigsäure (13.6 mL, 240.0 mmol, 1.0 Äq) und Na₂SO₄ (250 g) versetzt. Nach 16 h wird das Reaktionsgemisch durch Na₂SO₄ filtriert und mit Natriumtriacetoxyborhydrid (55.95 g, 264.0 mmol, 1.1 Äq) versetzt. Das Reaktionsgemisch wird bei RT 16 h gerührt. Der Feststoff wird über Kieselgur abfiltriert und die Lösung im Vakuum eingeengt. Das Rohprodukt wird in Vakuum destilliert (7 mbar, 60 °C). Die Verbindung wird ohne weitere Analysen verwendet.

Ausbeute: 31 %.

### 3) Herstellung von N-Pyridin-, N-Phenyl- und N-1H-Pyrazolaminen

1,1-Dimethoxy-2-(methylthio)ethane (1.0 Äq) wird in HCl-Lösung (0.5 M, 1.0 Äq) gelöst und 1 h bei 50 °C gerührt. Das Reaktionsgemisch wird auf RT abgekühlt und mit CH₂Cl₂ extrahiert (insgesamt 3 mL/mmol). Die vereinten organischen Phasen werden über Na₂SO₄ getrocknet. Die Aldehyd-Lösung wird auf 0 °C gekühlt und anschließend mit Amin (1.0 Äq), Natriumtriacetoxyborhydrid (1.5 Äq) und Essigsäure (1.0 Äq) versetzt. Das Reaktionsgemisch wird bei RT 16 h gerührt. Die Reaktion wird durch die Zugabe von NaHCOs-Lösung beendet. Die wässrige Phase wird dreimal mit CH₂Cl₂ extrahiert, die vereinten organischen Phasen werden über Na₂SO₄ getrocknet und im Vakuum eingeengt. Das Rohprodukt wird per Flash-Chromatographie gereinigt. (Hexan:EtOAc = 19:1).

### N-(2-methylsulfanylethyl)pyridin-2-amin

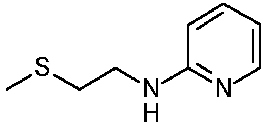

84 % Ausbeute

Die Verbindung wird ohne weitere Analysen verwendet.

### N-(2-methylsulfanylethyl)anilin

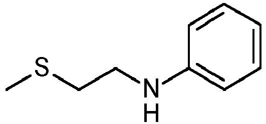

Ausbeute: 85 %

Die Verbindung wird ohne weitere Analysen verwendet.

### N-(2-methylsulfanylethyl)-1H-pyrazol-5-amin

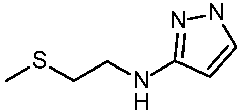

Ausbeute: 84 %

Die Verbindung wird ohne weitere Analysen verwendet.

### 4) Herstellung von N-(2-methoxyethyl)anilin

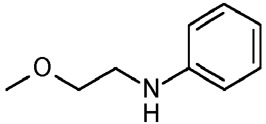

1,1-Dimethoxy-2-(methyloxy)ethan (10 g, 83.23 mmol, 1.0 Äq) wird in HCl-Lösung (0.5 M, 1.0 Äq) gelöst und 1 h bei 50 °C gerührt. Das Reaktionsgemisch wird auf RT abgekühlt und mit CH₂Cl₂ extrahiert (insgesamt 3 mL/mmol). Die vereinten organischen Phasen werden über Na₂SO₄ getrocknet. Die Aldehyd-Lösung wird auf 0 °C gekühlt und anschließend mit Anilin (7.75 g, 83.23 mmol, 1.0 Äq), Natriumcianoborhydrid (5.75 g, 91.55 mmol, 1.1 Äq) und Essigsäure (4.7 mL, 83.23 mmol, 1.0 Äq) versetzt. Das Reaktionsgemisch wird bei RT 16 h gerührt. Die Reaktion wird durch die Zugabe von NaHCOs-Lösung beendet. Die wässrige Phase wird dreimal mit CH₂Cl₂ extrahiert, die vereinten organischen Phasen werden über Na₂SO₄ getrocknet und im Vakuum eingeengt. Das Rohprodukt wird per Flash-Chromatographie gereinigt. (Hexan:EtOAc = 19:1).
Ausbeute: 19 %

Die Verbindung wird ohne weitere Analysen verwendet.

### 5) Herstellung von erfindungsgemäßen Zimtsäure- oder phenoxyessig-säureamiden

### Methode A

Zimt- oder Phenoxyessigsäure (1.5 Äq) und N-MethylFormAnilid (MFA) (1 Tropfen) werden in CH₂Cl₂ (5 mL/mmol) gelöst, auf 0 °C gekühlt und mit Oxalylchlorid (3.0 Äq.) versetzt. Das Reaktionsgemisch wird für 2 h bei RT und 1 h bei 40 °C gerührt und im Vakuum eingeengt. Triethylamin (4.0 Äq.) und jeweiliges Amin (1.0 Äq) werden in CH₂Cl₂ (4 mL/mmol) gelöst und auf 0 °C gekühlt. Das Rohprodukt wird in CH₂Cl₂ (1 mL/mmol) gelöst und langsam zugetropft. Das Reaktionsgemisch wird für 16 h bei RT gerührt. Die Reaktion wird durch die Zugabe von NaHCOs-Lösung beendet. Die wässrige Phase wird mit CH₂Cl₂ (3×) extrahiert, die vereinte organische Phase über Na₂SO₄ getrocknet und im Vakuum eingeengt. Das Rohprodukt wird per Flash-Chromatographie gereinigt.

### Methode B

Zimt- oder Phenoxyessigsäure (2.0 Äq) und MFA (1 Tropfen) werden in CH₂Cl₂ (5 mL/mmol) gelöst, auf 0 °C gekühlt und mit Oxalylchlorid (4.2 Äq.) versetzt. Das Reaktionsgemisch wird für 2 h bei RT und 1 h bei 40 °C gerührt und im Vakuum eingeengt. Pyridin (2.0 Äq.) und jeweiliges Amin (1.0 Äq) werden in CH₂Cl₂ (4 mL/mmol) gelöst und auf 0 °C gekühlt. Das Rohprodukt wird in CH₂Cl₂ (1 mL/mmol) gelöst und langsam zugetropft. Das Reaktionsgemisch wird für 16 h bei RT gerührt. Die Reaktion wird durch die Zugabe von NaHCOs-Lösung beendet. Die wässrige Phase wird mit CH₂Cl₂ (3×) extrahiert, die vereinte organische Phase über Na₂SO₄ getrocknet und im Vakuum eingeengt. Das Rohprodukt wird in Ethanol (15 mL/mmol) gelöst und mit NaOH (5.0 Äq) versetzt. Nach 15 min wird das Lösungsmittel im Vakuum entfernt und das Rohrprodukt mit Wasser versetzt und dreimal mit CH₂Cl₂ extrahiert. Die vereinten organischen Phasen werden über Na₂SO₄ getrocknet und im Vakuum eingeengt. Das Produkt wird per Flash-Chromatographie gereinigt. (EtOAc:Hexan = 25:75 → 45:55).

### 6) Herstellung von (E)-3-(1,3-benzodioxol-5-yl)-N-(2-methylsulfanylethyl)-N-phenyl-prop-2-enamid

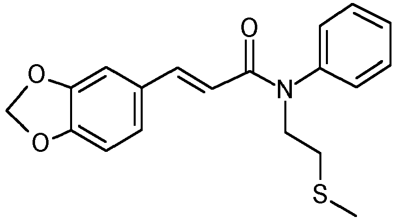

### Methode A: 71 % Ausbeute

¹H NMR (400 MHz, Chloroform-d) δ 7.59 (d, *J* = 15.5 Hz, 1H), 7.49 - 7.41 (m, 2H), 7.41 - 7.35 (m, 1H), 7.28 - 7.22 (m, 2H), 6.85 (dd, J= 8.1, 1.7 Hz, 1H), 6.73 (d, *J* = 8.1 Hz, 1H), 6.73 (d, *J =* 1.7 Hz, 1H), 6.10 (d, *J* = 15.4 Hz, 1H), 5.93 (s, 2H), 4.06 - 3.99 (m, 2H), 2.74 - 2.68 (m, 2H), 2.16 (s, 3H).

### 7) Herstellung von 2-(4-methylphenoxy)-N-(2-methylsulfanylethyl)-N-phenyl-acetamid

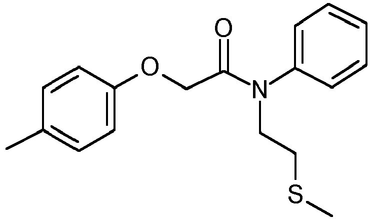

### Methode A: 87 % Ausbeute

¹H NMR (400 MHz, Chloroform-d) δ 7.46 (dd, *J* = 8.3, 6.4 Hz, 2H), 7.43 - 7.37 (m, 1H), 7.30 - 7.23 (m, 2H), 7.01 (d, *J* = 8.4 Hz, 2H), 6.67 (d, *J* = 8.6 Hz, 2H), 4.34 (s, 2H), 3.98 - 3.90 (m, 2H), 2.70 - 2.61 (m, 2H), 2.25 (s, 3H), 2.13 (s, 3H).

### 8) Herstellung von (E)-3-(1,3-benzodioxol-5-yl)-N-ethyl-N-(2-methylsulfanylethyl)prop-2-enamid

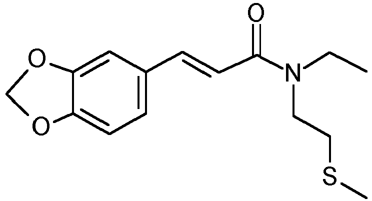

### Methode A: 31 % Ausbeute

¹H NMR (400 MHz, Chloroform-d) δ 7.63 (d, *J* = 15.2 Hz, 2H), 7.06 - 6.96 (m, 4H), 6.81 (d, *J* = 8.0 Hz, 2H), 6.66 (dd, *J* = 15.0, 6.4 Hz, 2H), 6.00 (s, 4H), 3.62 (dd, *J* = 8.6, 6.4 Hz, 4H), 3.52 (q, *J* = 7.2 Hz, 4H), 2.78 - 2.68 (m, 4H), 2.19 (s, 6H), 1.26 (t, *J* = 7.2 HZ, 3H), 1.19 (t, *J* = 7.3 Hz, 3H).

### 9) Herstellung von (E)-3-(1,3-benzodioxol-5-yl)-N-(2-methylsulfanylethyl)-N-(2-pyridyl)prop-2-enamid

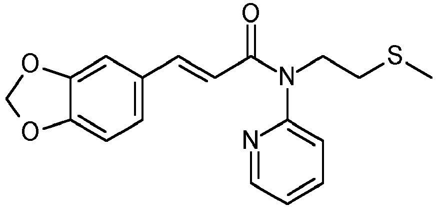

### Methode A: 5 % Ausbeute

¹H NMR (400 MHz, Chloroform-d) δ 8.55 (ddd, *J* = 4.9, 2.0, 0.9 Hz, 1H), 7.75 (td, *J* = 7.7, 2.0 Hz, 1H), 7.64 (d, *J* = 15.4 Hz, 1H), 7.23 (ddd, *J* = 7.4, 2.7, 1.0 Hz, 2H), 6.90 (dd, *J* = 8.2, 1.7 Hz, 1H), 6.81 (d, *J =* 1.7 Hz, 1H), 6.76 (d, *J =* 8.0 Hz, 1H), 6.26 (d, *J* = 15.4 Hz, 1H), 5.96 (s, 2H), 4.26 - 4.16 (m, 2H), 2.89 - 2.75 (m, 2H), 2.15 (s, 3H).

### 10) Herstellung von (E)-N-(2-methylsulfanylethyl)-3-(p-tolyl)-N-(2-pyridyl)prop-2-enamid

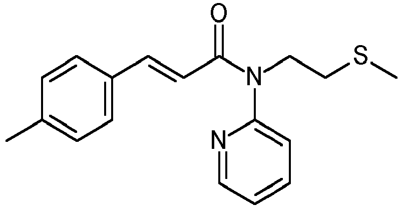

### Methode A: 10 % Ausbeute

¹H NMR (400 MHz, Chloroform-d) δ 8.55 (dd, *J* = 5.4, 2.0 Hz, 1H), 7.75 (td, *J* = 7.8, 2.0 Hz, 1H), 7.71 (d, *J* = 15.4 Hz, 1H), 7.29 - 7.21 (m, 4H), 7.12 (d, *J* = 7.9 Hz, 2H), 6.40 (d, *J* = 15.5 Hz, 1H), 4.30 - 4.16 (m, 2H), 2.87 - 2.74 (m, 2H), 2.33 (s, 3H), 2.15 (s, 3H).

### 11) Herstellung von (E)-3-(1,3-benzodioxol-5-yl)-N-(2-methylsulfanylethyl)-N-(1H-pyrazol-3-yl)prop-2-enamid

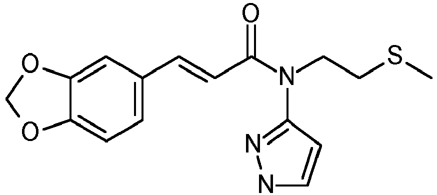

### Methode B: 10 % Ausbeute

¹H NMR (400 MHz, Chloroform-d) δ 7.63 (d, *J* = 15.2 Hz, 1H), 7.61 (d, *J* = 2.4 Hz, 1H), 6.90 (d, *J* = 7.9 Hz, 1H), 6.83 (s, 1H), 6.74 (d, *J* = 8.0 Hz, 1H), 6.35 (d, *J* = 14.7 Hz, 1H), 6.25 (s, 1H), 5.95 (s, 2H), 4.03 (t, *J* = 7.3 Hz, 2H), 2.78 (t, *J* = 7.6 Hz, 2H), 2.16 (s, 3H).

### 12) Herstellung von (E)-N-ethyl-N-(2-methylsulfanylethyl)-3-(p-tolyl)prop-2-enamid

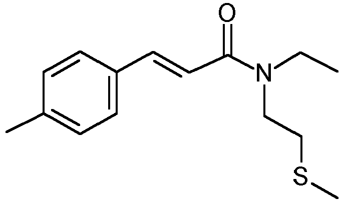

### Methode A: 41 % Ausbeute

Im Spektrum sind zwei Rotamere am Stickstof zu erkennen.

¹H NMR (400 MHz, Chloroform-d) δ 7.69 (d, *J* = 15.3 Hz, 2H), 7.42 (d, *J* = 7.8 Hz, 4H), 7.18 (d, *J* = 7.9 Hz, 4H), 6.79 (dd, *J* = 15.4, 5.7 Hz, 2H), 3.67 - 3.57 (m, 4H), 3.53 (q, *J =* 7.1 Hz, 4H), 2.78 - 2.68 (m, 4H), 2.37 (s, 6H), 2.19 (s, 6H), 1.27 (t, *J* = 7.1 Hz, 3H), 1.20 (t, *J* = 7.1 Hz, 3H).

### 13) Herstellung von (E)-N-ethyl-3-(4-methoxyphenyl)-N-(2-methylsulfanylethyl)prop-2-enamid

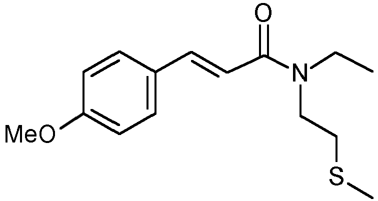

### Methode A: 12 % Ausbeute

¹H NMR (400 MHz, Chloroform-d) δ 7.69 (d, *J* = 15.3 Hz, 1H), 7.53 - 7.44 (m, 2H), 6.96 - 6.83 (m, 2H), 6.70 (d, *J* = 15.3 Hz, 1H), 3.83 (s, 3H), 3.62 (dd, *J* = 8.5, 6.6 Hz, 2H), 3.53 (q, J = 7.1 Hz, 2H), 2.78 - 2.69 (m, 2H), 2.19 (s, 3H), 1.27 - 1.19 (m, 3H).

### 14) Herstellung von (E)-N-(2-methylsulfanylethyl)-N-phenyl-3-(p-tolyl)prop-2-enamid

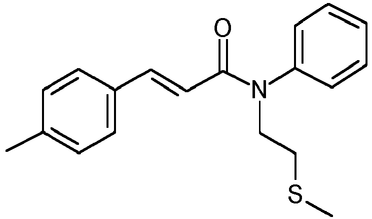

### Methode A: 79 % Ausbeute

¹H NMR (400 MHz, Chloroform-d) δ 7.65 (d, *J* = 15.5 Hz, 1H), 7.48 - 7.41 (m, 2H), 7.41 - 7.35 (m, 1H), 7.29 - 7.23 (m, 2H), 7.19 (d, *J =* 8.1 Hz, 2H), 7.08 (d, *J* = 8.0 Hz, 2H), 6.23 (d, *J* = 15.5 Hz, 1H), 4.07 - 3.99 (m, 2H), 2.75 - 2.67 (m, 2H), 2.31 (s, 3H), 2.16 (s, 3H).

### 15) Herstellung von (E)-3-(4-methoxyphenyl)-N-(2-methylsulfanylethyl)-N-phenyl-prop-2-enamid

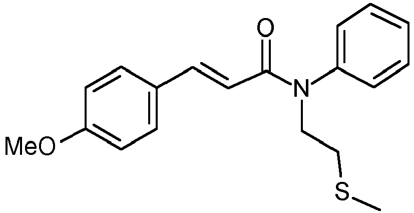

### Methode A: 85 % Ausbeute

¹H NMR (400 MHz, Chloroform-d) δ 7.64 (d, *J* = 15.5 Hz, 1H), 7.48 - 7.41 (m, 2H), 7.42-7.34 (m, 1H), 7.28 - 7.21 (m, 4H), 6.82 - 6.78 (m, 2H), 6.15 (d, *J* = 15.5 Hz, 1H), 4.06 - 4.00 (m, 2H), 3.78 (s, 3H), 2.75 - 2.67 (m, 2H), 2.16 (s, 3H).

### 16) Herstellung von 2-(4-methoxyphenoxy)-N-(2-methylsulfanylethyl)-N-phenyl-acetamid

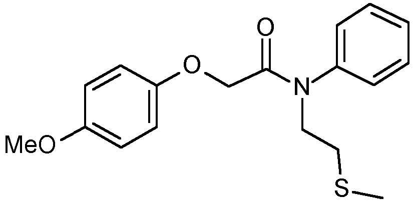

### Methode A: 89 % Ausbeute

¹H NMR (400 MHz, Chloroform-d) δ 7.50 - 7.41 (m, 2H), 7.40 (s, 1H), 7.29 - 7.22 (m, 2H), 6.76 (t, *J* = 9.1 Hz, 2H), 6.71 (s, 2H), 4.32 (s, 2H), 3.99 - 3.88 (m, 2H), 3.73 (s, 3H), 2.72 - 2.59 (m, 2H), 2.13 (s, 3H).

### 17) Herstellung von (E)-N-ethyl-N-(3-methylsulfanylpropyl)-3-(p-tolyl)prop-2-enamid

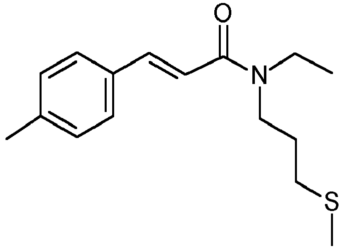

### Methode A: 50 % Ausbeute

Im Spektrum sind zwei Rotamere am Stickstof zu erkennen.

¹H NMR (400 MHz, Chloroform-d) δ 7.69 (d, *J* = 15.3 Hz, 2H), 7.47 - 7.39 (m, 4H), 7.18 (d, *J* = 7.9 Hz, 4H), 6.93 (d, *J* = 15.4 Hz, 1H), 6.79 (d, *J* = 15.4 Hz, 1H), 3.51 (dt, *J* = 14.2, 7.8 Hz, 8H), 2.55 (t, *J* = 7.0 Hz, 4H), 2.37 (s, 6H), 2.11 (s, 6H), 1.99 - 1.87 (m, 4H), 1.39 - 1.08 (m, 6H).

### 18) Herstellung von (E)-3-(1,3-benzodioxol-5-yl)-N-ethyl-N-(3-methylsulfanylpropyl)-prop-2-enamid

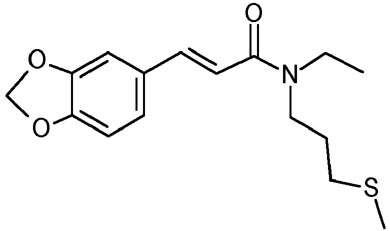

### Methode A: 66 % Ausbeute

Im Spektrum sind zwei Rotamere am Stickstof zu erkennen.

¹H NMR (400 MHz, Chloroform-d) δ 7.62 (d, *J* = 15.3 Hz, 2H), 7.11 - 6.97 (m, 4H), 6.87 - 6.76 (m, 2H), 6.66 (d, *J* = 15.3 Hz, 2H), 5.99 (s, 4H), 3.59 - 3.43 (m, 8H), 2.62 - 2.49 (m, 4H), 2.12 (s, 6H), 1.99 - 1.90 (m, 4H), 1.26 (t, *J* = 7.1 Hz, 3H), 1.19 (t, *J* = 7.1 Hz, 3H).

### 19) Herstellung von (E)-3-(1,3-benzodioxol-5-yl)-N-(3-methylsulfanylpropyl)-N-phenyl-prop-2-enamid

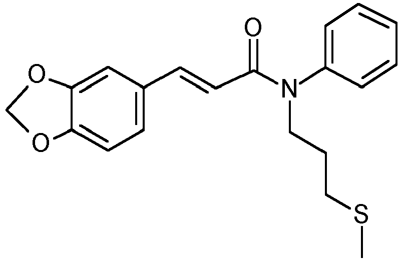

### Methode A: 98 % Ausbeute

¹H NMR (400 MHz, Chloroform-d) δ 7.57 (d, *J* = 15.4 Hz, 1H), 7.48 - 7.42 (m, 2H), 7.41 - 7.34 (m, 1H), 7.23 - 7.18 (m, 2H), 6.85 (dd, *J* = 8.1, 1.7 Hz, 1H), 6.73 - 6.72 (m, 2H), 6.72 (d, *J* = 8.0 Hz, 1H), 6.10 (d, *J* = 15.4 Hz, 1H), 5.93 (s, 2H), 3.95 - 3.89 (m, 2H), 2.56 - 2.49 (m, 2H), 2.06 (s, 3H), 1.94-1.84 (m, 2H).

### 20) Herstellung von (E)-3-(1,3-benzodioxol-5-yl)-N-(2-methoxyethyl)-N-phenyl-prop-2-enamid

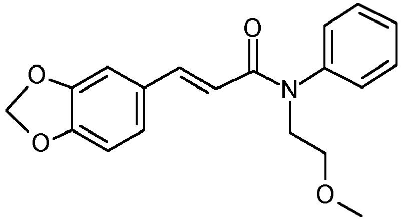

### Methode A: Ausbeute 61 %

¹H NMR (400 MHz, Chloroform-d) δ 7.58 (d, *J* = 15.5 Hz, 1H), 7.46 - 7.40 (m, 2H), 7.40 - 7.33 (m, 1H), 7.29 - 7.24 (m, 2H), 6.85 (dd, *J* = 8.1, 1.6 Hz, 1H), 6.73 (d, *J* = 1.8 Hz, 1H), 6.73 (d, *J =* 8.0 Hz, 1H), 6.12 (d, *J =* 15.4 Hz, 1H), 5.93 (s, 2H), 4.00 (t, *J =* 5.8 Hz, 2H), 3.60 (t, *J* = 5.8 Hz, 2H), 3.33 (s, 3H).

### 21) Herstellung von (E)-3-(4-methoxyphenyl)-N-(2-methylsulfanylethyl)-N-(2-pyridyl)-prop-2-enamid

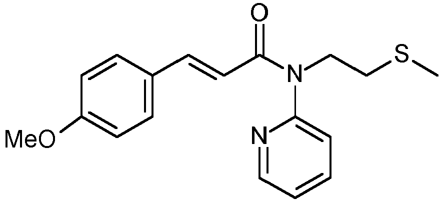

### Methode A: 10 % Ausbeute

¹H NMR (400 MHz, Chloroform-d) δ 8.55 (d, *J* = 5.7 Hz, 1H), 7.75 (td, *J* = 7.7, 2.0 Hz, 1H), 7.69 (d, *J* = 15.5 Hz, 1H), 7.35 - 7.28 (m, 2H), 7.23 (dd, *J* = 7.5, 4.5 Hz, 2H), 6.94 - 6.73 (m, 2H), 6.31 (d, *J* = 15.4 Hz, 1H), 4.26 - 4.18 (m, 2H), 3.80 (s, 3H), 2.85 - 2.76 (m, 2H), 2.15 (s, 3H).

### 22) Herstellung von 2-(4-methylphenoxy)-N-(2-methylsulfanylethyl)-N-(2-pyridyl)-acetamid

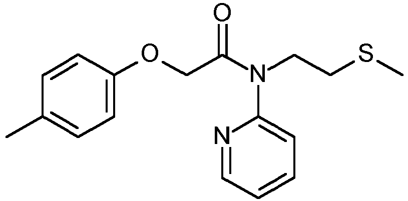

### Methode A: 19 % Ausbeute

¹H NMR (400 MHz, Chloroform-d) δ 8.50 - 8.44 (m, 1H), 7.77 (td, *J* = 7.8, 2.0 Hz, 1H), 7.30 - 7.25 (m, 1H), 7.23 (ddd, *J* = 7.3, 5.0, 0.7 Hz, 1H), 7.01 (d, *J* = 8.3 Hz, 2H), 6.64 (d, *J* = 8.5 Hz, 2H), 4.68 (s, 2H), 4.10 - 4.00 (m, 2H), 2.77 - 2.69 (m, 2H), 2.25 (s, 3H), 2.12 (s, 3H).

### 23) Herstellung von (E)-N-(2-methylsulfanylethyl)-3-(p-tolyl)-N-(1H-pyrazol-3-yl)prop-2-enamid

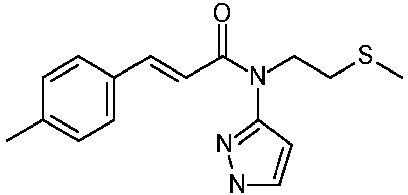

### Methode B: 50 % Ausbeute

¹H NMR (600 MHz, Chloroform-d) δ 10.91 (s, 1H), 7.69 (d, *J* = 15.5 Hz, 1H), 7.60 (d, *J* = 1.6 Hz, 1H), 7.26 (d, *J* = 5.5 Hz, 2H), 7.10 (d, *J* = 7.5 Hz, 2H), 6.46 (d, *J* = 14.6 Hz, 1H), 6.24 (s, 1H), 4.04 (t, *J* = 13.1, 6.2 Hz, 2H), 2.78 (t, *J* = 7.4 Hz, 2H), 2.32 (s, 3H), 2.16 (s, 3H).

### 24) Herstellung von 2-(4-methylphenoxy)-N-(2-methylsulfanylethyl)-N-(1H-pyrazol-3-yl)acetamid

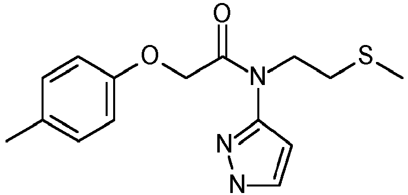

### Methode B: 64 % Ausbeute

¹H NMR (400 MHz, Chloroform-d) δ 7.57 (d, *J* = 2.5 Hz, 1H), 7.00 (d, *J* = 8.2 Hz, 2H), 6.71 (d, *J* = 8.4 Hz, 2H), 6.23 (d, *J* = 2.4 Hz, 1H), 4.53 (s, 2H), 4.01 - 3.83 (m, 2H), 2.77 - 2.62 (m, 2H), 2.23 (s, 3H), 2.11 (s, 3H).

### 25) Herstellung von 2-(4-methoxyphenoxy)-N-(2-methylsulfanylethyl)-N-(1H-pyrazol-3-yl)acetamid

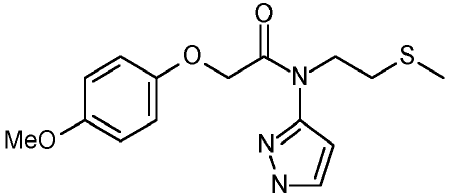

### Methode B: 53 % Ausbeute

¹H NMR (400 MHz, Chloroform-d) δ 10.72 (s, 1H), 7.58 (d, *J* = 2.4 Hz, 1H), 6.77 (s, 4H), 6.24 (d, *J* = 2.4 Hz, 1H), 4.54 (s, 2H), 3.93 (dd, *J* = 8.5, 6.3 Hz, 2H), 3.73 (s, 3H), 2.72 (dd, *J* = 8.6, 6.2 Hz, 2H), 2.13 (s, 3H).

### 26) Herstellung von (E)-3-(1,3-benzodioxol-5-yl)prop-2-enoyl chlorid

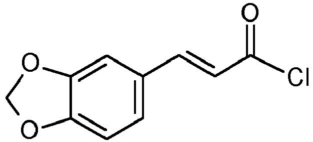

52,7 g (274,2 mmol) 3,4-Dioxymethylenzimtsäure, 529 mL Toluol und 1,60 g DMF werden vorgelegt und auf 57°C aufgeheizt. Innerhalb von 30 min werden bei 57 bis 60°C 44,1 g (371 mmol) Thionylchlorid zugetropft. Die Lösung wird 1 h bei 60 bis 65°C nachgerührt und anschließend auf ein Volumen von ca 300 mL aufkonzentriert. Es werden 3 × 289 mL Toluol zugegeben, die jeweils unter Normaldruck wieder abdestilliert werden.

### 27) Herstellung von (E)-3-(1,3-Benzodioxol-5-yl)-N-phenyl-N-tetrahydrofuran-3-yl-prop-2-enamid

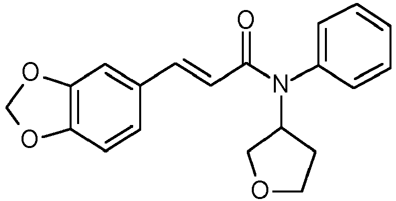

0,72 g (3,4 mmol) 3,4-Methylendioxyzimtsäurechlorid in 7,5 g Toluol werden bei 50°C mit 0,52 g (5,14 mmol) Triethylamin versetzt und anschließend werden 0,52 g (3,19 mmol) N-Phenyloxolan-3-amin in 4 mL Toluol zugegeben. Es wird 8 h bei 60°C gerührt und auf RT abgekühlt. Die organische Phase wird mit Wasser, 1 N Salzsäure, Wasser, gesättigter NaHCOs-Lsg und nochmals Wasser gewaschen, über Na₂SO₄ getrocknet, filtriert und eingeengt.
Ausbeute: 39 %

¹H NMR (500 MHz, CDCl₃) δ 7,56 (d, *J* = 15,4 Hz, 1H); 7,50 - 7,38 (m, 3H); 7,22 - 7,12 (m, 2H); 6,81 (dd, *J* = 8,2, 1,6 Hz, 1H); 6.71 (d, *J* = 8,0 Hz, 1H); 6,67 (d, *J* = 1,6 Hz, 1H); 5,96 - 5,85 (m, 3H); 5,29 (dq, *J* = 8,3, 5,5 Hz, 2H); 4,04 (dd, *J* = 9,3, 6,7 Hz, 1H); 3,79 (dd, J= 9,3, 5,1 Hz, 1H); 3,74 - 3,53 (m, 1H); 2,20 - 2,12 (m, 1H); 1,96 - 1,77 (m, 1H).

### 28) Herstellung von (E)-N-phenyl-3-(p-tolyl)-N-tetrahydrofuran-3-yl-prop-2-enamid

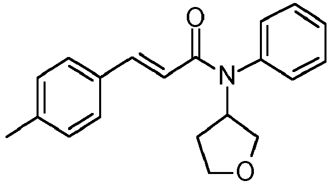

Zu einer Lösung von 0,56 g (3,0 mmol) 4-Methylzimtsäurechlorid in 7,5 mL Toluol werden 0,46 g (4,5 mmol) Triethylamin zugetropft. Anschließend wird innerhalb von 2 min eine Suspension von 0,46 g (2,8 mmol) N-Phenyl-tetrahydrofuran-3-amine in 4 mL Toluol zudosiert. Es wird 46 h bei RT und 11 h bei 50°C gerührt. Zur Aufarbeitung werden 20 mL Wasser zugegeben und die organische Phase abgetrennt und am Rotationsverdampfer eingeengt. Der Rückstand wird aus Essigester-Heptan umkristallisiert.

Ausbeute: 0,32 g Produkt
¹H NMR (500 MHz, CDCl₃) δ 7,63 (d, 1H), 7,50 - 7,40 (m, 3H), 7,22 (d, 2 H), 7,14 (d, 2H), 7,07 (d, 2H), 6,06 (d, 1H), 5,34 - 5,26 (m, 1H), 4,05 (t, 1H), 3,83 - 3,78 (m, 1H), 3,71 (dd, 1H), 3,61 (dd, 1 H), 2,30 (s, 3 H); 2,25 - 2,14 (m, 1 H); 1,90 - 1,81 (m, 1H).

### 29) Herstellung von N-Phenyltetrahydrothiophen-3-amin

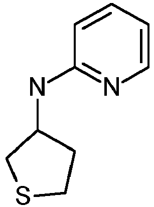

In einem Reaktor werden 91,1 g (0,968 mol) 2-Aminopyridine, 1591 mL Toluol, 98,4 g (0,963 mol) Tetrahydrothiophen-3-on und 81,6 g Essigsäure vorgelegt. Bei Raumtemperatur werden innerhalb von 30 min 288 g (1,36 mol) Natriumtriacetoxyborhydrid portionsweise zugegeben. Es wird mit 115 mL Toluol nachgespült und der Ansatz 21 h bei Raumtemperatur gerührt. Anschließend wird der Ansatz bei 15°C in 4,85 L wässrige 10 % Na₂CO₃-Lösung eindosiert und mit weiteren 500 mL Toluol versetzt. Es wird ca. 20 min bei 15-20°C nachgerührt, die wässrige Phase abgetrennt und diese noch einmal mit 500 ml Toluol extrahiert. Die organischen Phasen werden vereint und mit 2 L Wasser und 280 g 10 % HCl versetzt. Es wird 10 min nachgerührt und die organische Phase abgetrennt und verworfen. Die wässrige Phase wird 2 x mit je 500 mL Toluol nachgewaschen, die ebenfalls verworfen werden. Anschließend wird die wässrige Phase mit 2 L Wasser und 1 L Toluol versetzt und mit 274 mL 10 % NaOH auf pH 10,3 eingestellt. Es wird 10 min nachgerührt, die organische Phase abgetrennt und die wässrige Phase noch einmal mit 250 mL Toluol nachextrahiert. Die vereinten organischen Phasen werden 1 x mit 200 mL Wasser gewaschen, über Na₂SO₄ getrocknet und eingeengt.
Rückstand: 45,3 g (28 %)

### 30) Herstellung von (E)-3-(1,3-benzodioxol-5-yl)-N-(2-pyridyl)-N-tetrahydrothiophen-3-yl-prop-2-enamid

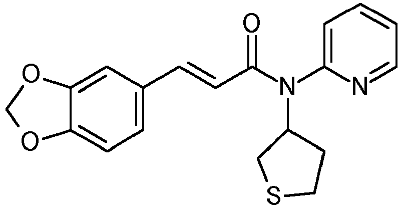

In eine Lösung von ca. 57,7 g (274 mmol) 3,4-Methylendioxyzimtsäurechlorid in ca. 235 mL Toluol werden bei ca. 50°C 41,6 g (0,411 mol) Triethylamin und 46,5 g (258 mmol) N-Phenyltetrahydrothiophen-3-amin in 264 mLToluol zugegeben. Es wird 1 h bei 55°C und anschließend über Nacht bei RT nachgerührt. Die Suspension wird auf 0°C abgekühlt und filtriert. Der Filterrückstand wird in 2 L Essigester aufgenommen und bei 40°C 3 x mit je 500 mL Wasser gewaschen. Es wird bei Normaldruck auf einen Volumenstand von ca. 600 mL aufkonzentriert, innerhalb von 3 h auf ca 0°C abgekühlt und 1 h nachgerührt. Anschließend wird filtriert und der Filterkuchen mit 80 mL eiskaltem Essigester nachgewaschen. Der Filterkuchen wird getrocknet, in 1670 mL Isopropanol aufgenommen und auf Rückfluss erhitzt (Lösung). Es wird auf einen Volumenstand von 670 mL aufkonzentriert, auf 20°C abgekühlt und filtriert. Der Rückstand wird mit 50 mL eiskaltem i-PrOH gewaschen und getrocknet.

### Rückstand: 59,8 g (Ausbeute: 62 %)

¹H NMR (500 MHz, CDCl₃) δ 8,63 (d, 1H); 7,84 (td, *J* = 7,7, 2,0 Hz, 1H); 7,60 (d, *J* = 15,3 Hz, 1H); 7,39 (ddd, *J* = 7,5, 4,9, 0,9 Hz, 1H); 7,22 (d, *J* = 7,8 Hz, 1H), 6,82 (d, 1H); 6,72 (d, *J* = 8,0 Hz, 1H); 6,68 (d, *J* = 1,6 Hz, 1H); 5,94 (s, 2H); 5,80 (d, *J* = 15,3 Hz, 1H); 5,32 - 5,15 (m, 1H); 3,12 (dd, *J* = 10,1, 6,9 Hz, 1H); 2,94 - 2,81 (m, 2H); 2,72 (ddd, *J* = 10,3, 7,8, 2,4 Hz, 1H); 2,35 - 2,25 (m, 1H); 1,90 - 1,79 (m, 1H).

### 31) Herstellung von (E)-3-(p-tolyl)-N-(2-pyridyl)-N-tetrahydrothiophen-3-yl-prop-2-enamid

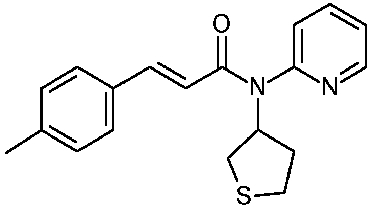

0,74 g (4,1 mmol) 4-Methylzimtsäurechlorid in 10 mL Toluol werden vorgelegt und mit 0,52 g (5,1 mmol) Triethylamin versetzt. Anschließend werden 0,65 g (3,4 mmol) N-Tetrahydrothiophen-3-ylpyridin-2-amin in 4,8 mL Toluol zudosiert und die Mischung 1 h bei 50°C gerührt. Der Ansatz wird bei RT mit 20 mL Wasser gequencht. Der ausgefallene Feststoff wird abfiltriert, mit Wasser und wenig MTBE gewaschen und getrocknet.
Ausbeute: 57 %

¹H NMR (500 MHz, CDCl₃) δ 8,64 (br s, 1H); 7,83 (td, *J* = 7,7, 2,0 Hz, 1H); 7,67 (d, *J* = 15,4 Hz, 1H); 7,39 (dd, *J* = 7,5, 4,9 Hz, 1H); 7,22 (d, *J* = 7,8 Hz, 1H); 7,16 (d, *J* = 8,1 Hz, 2H); 7,07 (d, *J* = 8,0 Hz, 2H); 5,94 (d, *J* = 15,4 Hz, 1H); 5,32 - 5,20 (m, 1H); 3,13 (dd, J = 10,2, 6,9 Hz, 1H); 2,96 - 2,80 (m, 2H); 2,78 - 2,66 (m, 1H); 2,40 - 2,23 (m, 4H); 1,94 - 1,78 (m, 1H).

### 32) Herstellung von (E)-3-(4-methoxyphenyl)-N-(2-pyridyl)-N-tetrahydrothiophen-3-yl-prop-2-enamid

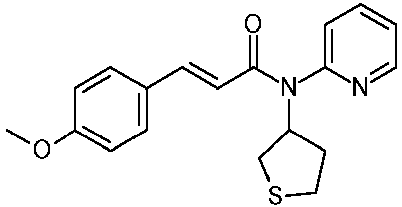

0,81 g (4,1 mmol) 4-Methoxyzimtsäurechlorid in 11 mL Toluol werden vorgelegt und mit 0,52 g (5,1 mmol) Triethylamin versetzt. Anschließend werden 0,65 g (3,4 mmol) N-Tetrahydrothiophen-3-ylpyridin-2-amin in 4,8 mL Toluol zudosiert und die Mischung 1 h bei 50 bis 60°C gerührt. Der Ansatz wird bei RT mit 20 mL Wasser gequencht. Der ausgefallene Feststoff wird abfiltriert, mit Wasser und wenig MTBE gewaschen und getrocknet.
Ausbeute: 69 %

¹H NMR (500 MHz, CDCl₃) δ 8,65 (d, 1H); 7,83 (td, *J* = 7,7, 1,8 Hz, 1H); 7,65 (d, *J* = 15,4 Hz, 1H); 7,39 (dd, *J* = 7,3, 5,0 Hz, 1H); 7,22 (d, *J* = 8,6 Hz, 3H); 6.80 (d, *J* = 8,7 Hz, 2H); 5,85 (d, *J* = 15,4 Hz, 1H); 5,32 - 5,20 (m, 1H), 3,78 (s, 3H); 3,13 (dd, *J* = 10,1, 6,9 Hz, 1H); 2,97 - 2,80 (m, 2H); 2,75 - 2,69 (m, 1H); 2,35 - 2,25 (m, 1H); 1,94 - 1,74 (m, 1H).

### 33) Herstellung von (E)-3-phenyl-N-(2-pyridyl)-N-tetrahydrothiophen-3-yl-prop-2-enamid

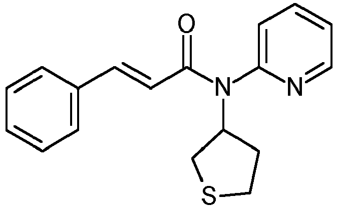

0,68 g (4,1 mmol) Zimtsäurechlorid in 9 mL Toluol werden vorgelegt und mit 0,52 g (5,1 mmol) Triethylamin versetzt. Anschließend werden 0,65 g (3,4 mmol) N-Tetrahydrothiophen-3-ylpyridin-2-amin in 4,8 mL Toluol zudosiert und die Mischung 1 h bei 50 bis 60°C gerührt. Der Ansatz wird bei RT mit 20 ml Wasser gequencht und die organische Phase noch einmal mit 20 mL Wasser nachgewaschen. Die organische Phase wird über Na₂SO₄ getrocknet, filtriert und einrotiert. Anschließend wird aus *n-*Heptan/EtOAc umkristallisiert.
Ausbeute: 50 %

¹H NMR (500 MHz, CDCl₃) δ 8.65 (dd, *J* = 4.8, 1.8 Hz, 1H); 7.84 (td, *J* = 7.7, 2.0 Hz, 1H); 7.70 (d, *J* = 15.4 Hz, 1H); 7.42 - 7.35 (m, 1H); 7.32 - 7.12 (m, 6H); 5.99 (d, *J* = 15.4 Hz, 1H); 5.35 - 5.13 (m, 1H); 3.14 (dd, *J* = 10.2, 6.9 Hz, 1H); 2.97 - 2.81 (m, 2H); 2.72 (m, 1H); 2.31 (m, Hz, 1H); 1.99 - 1.70
(m, 1H).

### 34) Herstellung von N-(tetrahydrofuran-2-ylmethyl)anilin

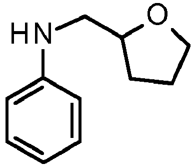

96,1 g (1,0 mol) Furfural werden in 400 g Methylenchlorid vorgelegt. Anschließend werden 111,8 g (1,2 mol) Anilin bei 40 °C zugetropft. Es wird auf 0 °C gekühlt und 400 g Methanol und 56,7 g (1,5 mol) Natrium boronat vorsichtig zugegeben und 1 h bei 0 bis 5 °C nachgerührt. Der Ansatz wird mit Wasser gequencht und Methanol herausdistilliert. Danach wird die Mischung mit 450 g HCl (10%) neutralisiert und mit MTBE extrahiert. Die organische Phase wird getrocknet, kondensiert und das Produkt destilliert und ohne weitere Reinigung benutzt.

100 g Furfurylphenylamin werden in 250 mL Ethanol vorgelegt und 4 g Pd/C zugegeben. Dem Reaktor wird Wasserstoff zugeführt (10 bar) und die Mischung 18 h bei 50 °C gerührt. Der Ansatz wird bei RT filtriert und direkt destilliert.
Ausbeute: 58 %

### Herstellung von (E)-3-81,3-benzodioxo-5-yl)-N-phenyl-N-(tetrahydrofuran-2-ylmethyl)prop-2-enamid

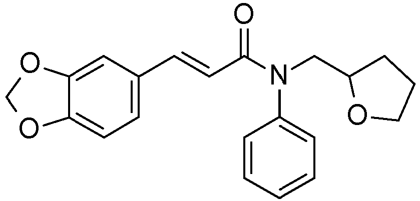

1,14 g (11,3 mmol) Triethylamin und 1,95 g (11 mmol) 2-Tetrahydrofurfurylphenylamin in 30 g Toluol werden vorgelegt. Anschließend werden 4,52 g (21,5 mmol) 3,4-Methylendioxyzimtsäurechlorid zudosiert und die Mischung 2 h bei 100 °C nachgerührt. Der Ansatz wird bei RT auf 50 g Wasser gegossen und der Feststoff abgesaugt. Nach der Phasentrennung wird die organische Phase getrocknet, filtriert und einrotiert. Anschließend wird über Kieselgel (AcOEt) gesäult.
Ausbeute: 76%

¹H NMR (400 MHz, Chloroform-d) δ 7.58 (d, *J* = 15.5 Hz, 1H), 7.47 - 7.39 (m, 2H), 7.39 - 7.33 (m, 2H), 7.33 - 7.28 (m, 1H), 6.84 (dd, *J* = 8.1, 1.7 Hz, 1H), 6.72 (d, *J* = 1.6 Hz, 1H), 6.70 (d, *J* = 7.9 Hz, 1H), 6.13 (d, J = 15.5 Hz, 1H), 5.92 (s, 2H), 4.21 - 4.08 (m, 1H), 4.00 (dd, *J* = 13.7, 4.8 Hz, 1H), 3.89 - 3.81 (m, 1H), 3.84 - 3.75 (m, 1H), 3.77 - 3.68 (m, 1H), 1.98 (ddd, *J* = 6.9, 4.2, 1.9 Hz, 1H), 1.96 - 1.79 (m, 2H), 1.68 - 1.54 (m, 1H).

### 35) Analog zu oben beschrieben Methoden können auch folgende Verbindungen hergestellt werden

### (E)-3-(4-Methoxyphenyl)-N-phenyl-N-tetrahydrofuran-3-yl-prop-2-enamid

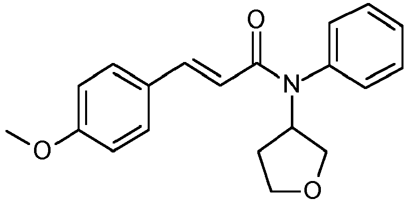

¹H NMR (500 MHz, CD₂Cl₂) δ 7,52 (d, 1H); 7,48 - 7,35 (m, 3H); 7,27 - 7,11 (m, 4H); 6,77 (d, 2H); 5,97 (d, 1H); 5,29 - 5,13 (m, 1H); 3,95 (dd, 1H); 3,74 (s, 3H); 3,70 (dd, 1H); 3,63 (q, 1H); 3,54 (td, 1H); 2,21 - 2,05 (m, 1H); 1,74 - 1,36 (m, 1H).

### 2-(4-Methylphenoxy)-N-phenyl-N-tetrahydrofuran-3-yl-acetamid

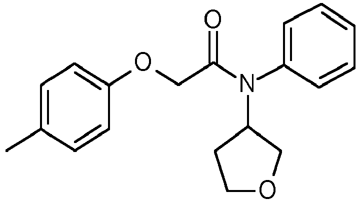

¹H NMR (500 MHz, CDCl₃) δ 7,56 - 7,36 (m, 3H); 7,30 - 7,16 (m, 2H); 7,02 (d, 2H); 6,66 (d, 2H); 5,25 - 5,09 (m, 1H); 4,25 (d, 1H); 4,21 (d, 1H); 3,98 (dd, 1H); 3,78 (dd, 1H); 3,67 (td, 1H); 3,60 - 2,48 (m, 1H), 2,25 (s, 3H); 2,23 - 2,07 (m, 1H); 1,85 - 1,75 (m, 1H).

### 2-(4-Methylphenoxy)-N-phenyl-N-tetrahydrothiophen-3-yl-acetamid

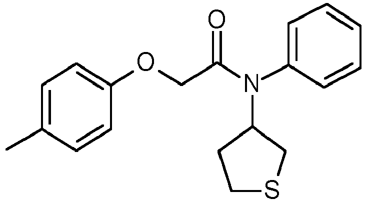

¹H NMR (500 MHz, CDCl₃) δ 7,55 - 7,35 (m, 3H); 7,24 - 7,15 (m, 2H); 7,02 (d, 2H); 6,66 (d, 2H); 5,34 - 4,97 (m, 1H); 4,22 (s, 2H); 3,04 (dd, 1H); 2,84 (td, 1H); 2,74 - 2,50 (m, 2H); 2,37 - 2,10 (m, 4H); 1,65 (qd, 1H).

### (E)-N-Cyclohexyl-3-(4-methoxyphenyl)-N-(3-thienyl)prop-2-enamid

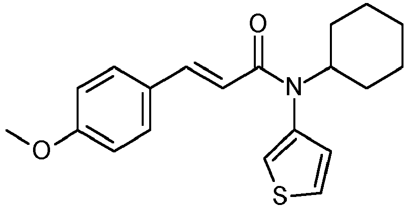

¹H NMR (500 MHz, CDCl₃) δ 7,60 (d, 1H); 7,37 (dd, 1H); 7,25 (d, 2H); 7,08 (dd, 1H); 6,88 (dd, 1H); 6,81 (d, 2H); 6,05 (d, 1H); 4,71 - 4,61 (m, 1H); 3,79 (s, 3H); 1,86 (d, 2H); 1,76 (d, , 2H); 1,61 (d, 1H); 1,44 (qt, 2H); 1,13 (qd, 2H); 1,03-0,90 (m, 1H).

### N-Cyclohexyl-2-(4-methylphenoxy)-N-(3-thienyl)acetamid

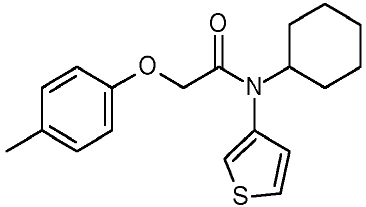

¹H NMR (500 MHz, CDCl₃) δ 7,45 -7,30 (m, 1H); 7,16- 7,08 (m, 1H); 7,02 (d, 2H); 6,87 (dd, 1H); 6,69 (d, 2H); 4,61 - 4,49 (m, 1H); 4,28 (s, 2H); 2,25 (s, 3H); 1,83 (m, 2H); 1,74 (d, 2H); 1,59 (d, 1H); 1,39 (q, 2H); 1,09 (qd, 2H); 0,94 (qt, 1H).

### N-Cyclohexyl-2-(4-methoxyphenoxy)-N-(3-thienyl)acetamid

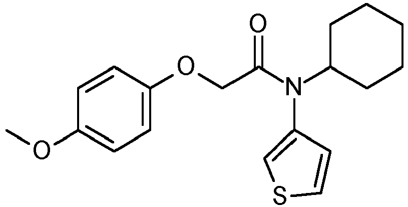

¹H NMR (500 MHz, CDCl₃) δ 7,38 (dd, 1H); 7,11 (dd, 1H); 6,87 (dd, 1H); 6.,82 - 6,62 (m, 4H); 4.,62 - 4.,48 (m, 1H); 4,26 (s, 2H); 3,74 (s, 3H); 1,82 (d, 2H); 1,73 (d, 2H); 1,59 (d, 1H); 1,39 (qt, 2H); 1,09 (qd, 2H); 0,94 (qt, 1H).

### (E)-3-(1,3-benzodioxol-5-yl)-N-(1H-pyrazol-3-yl)-N-(tetrahydrothiophen-2-ylmethyl)prop-2-enamid

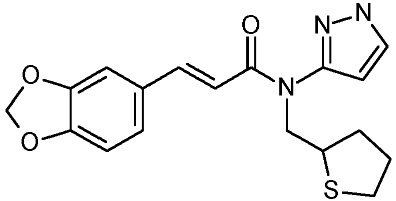

¹H NMR (500 MHz, CDCl₃) δ 7,68 - 7,54 (m, 2H); 6,89 (d, 1H); 6,81 (s, 1H); 6,74 (d, 1H); 6,31 (d, 1H); 6,24 (s, 1H); 5,96 (s, 2H); 4,29 - 4,17 (m, 1H); 3,67 (br s, 2H); 2,92 (dt, 1H); 2,81 (dt, 1H); 2,18 - 1,81 (m, 4H).

### (E)-3-(p-Tolyl)-N-(1H-pyrazol-3-yl)-N-(tetrahydrothiophen-2-ylmethyl)prop-2-enamid

Die Herstellung erfolgt unter Verwendung von N-(Tetrahydrothiophen-2-ylmethyl)-1-(2-trimethylsilylethoxymethyl)pyrazol-3-amin und abschließender Entschützung mit Tetrabutylammoniumfluorid in THF.

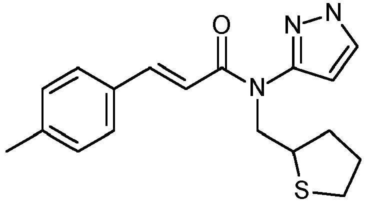

¹H NMR (500 MHz, CDCl₃) δ 7,75 - 7,55 (m, 2H); 7,32 - 7,20 (m, 2H); 7,09 (d, 2H); 6,43 (d, 1H); 6,22 (s, 1H); 4,30 - 4,18 (m, 1H); 3,75 - 3,62 (m, 2H); 2,96 - 2,86 (m, 1H); 2,85 - 2,74 (m, 1H); 2.31 (s, 3H); 2,13 - 1,77 (m, 4H).

### (E)-3-(4-methoxyphenyl)-N-(1H-pyrazol-3-yl)-N-(tetrahydrothiophen-2-ylmethyl)prop-2-enamid

Die Herstellung erfolgt unter Verwendung von N-(Tetrahydrothiophen-2-ylmethyl)-1-(2-trimethylsilylethoxymethyl)pyrazol-3-amin und abschließender Entschützung mit Tetrabutylammoniumfluorid in THF.

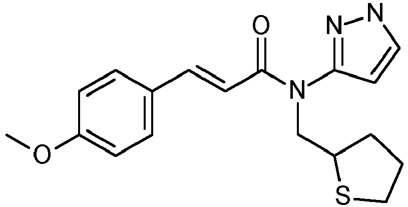

¹H NMR (500 MHz, CDCl₃) δ 7,73 (d, 1H); 7,65 (s, 1H); 7,32 (d, 2H); 6,84 (d, 2H); 6,38 (d 1H); 6,26 (s, 1H); 4,37 - 4,21 (m, 1H); 3,79 (s, 3H); 3,75 - 3,61 (m, 2H); 3,00 - 2,89 (m, 1H); 2,89 - 2,79 (m, 1H); 2,18 - 1,83 (m, 4H).

### 2-(4-Methylphenoxy)-N-(1H-pyrazol-3-yl)-N-(tetrahydrothiophen-2-ylmethyl)acetamid

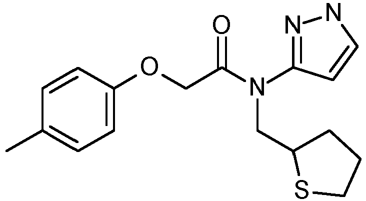

¹H NMR (500 MHz, CDCl₃) δ 7,57 (d, 1H); 7,00 (d, 2H); 6.71 (d, 2H); 6,24 (d, 1H); 4,55 (q, 2H); 4,18 - 4,04 (m, 1H); 3,68 - 3,50 (m, 2H); 2,93 - 2,67 (m, 2H); 2,23 (s, 3H); 2,08 - 1,80 (m, 4H).

### 2-(4-Methoxyphenoxy)-N-(1H-pyrazol-3-yl)-N-(tetrahydrothiophen-2-ylmethyl)acetamid

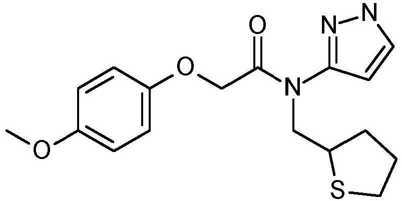

¹H NMR (500 MHz, CDCl₃) δ 7,58 (d, 1H); 6,76 (s, 4H), 6,24 (d, 1H); 4,53 (q, 2H); 4,13 (q, 1H); 3,72 (s, 3H); 3,65 - 3,46 (m, 2H); 2,94 - 2,84 (m, 1H); 2,84 - 2,74 (m, 1H); 2,09 - 1,73 (m, 4H).

### (E)-3-(p-Tolyl)-N-(1H-pyrazol-3-yl)-N-tetrahydrothiophen-3-yl-prop-2-enamid

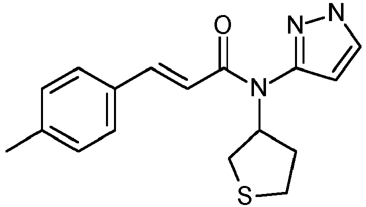

¹H NMR (500 MHz, CDCl₃) δ 7,76 - 7,56 (m, 2H); 7,21 (d, 2H); 7,07 (d, 2H); 6,24 (d, 1H); 6,17 (d, 1H); 5,37 - 5,22 (m, 1H); 3,10 (dd, 1H); 2,92 - 2,80 (m, 1H); 2,76 (t, 1H); 2,69 (ddd, 1H); 2,36 - 2,22 (m, 4H); 1.78 (qd, 1H).

### (E)-3-(4-Methoxyphenyl)-N-(1H-pyrazol-3-yl)-N-tetrahydrothiophen-3-yl-prop-2-enamid

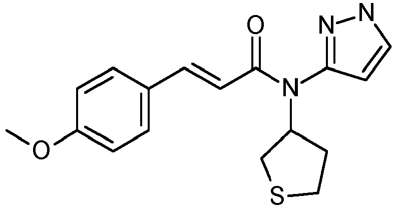

¹H NMR (500 MHz, CDCl₃) δ 7,77 - 7,53 (m, 2H); 7,25 (d, 2H); 6,79 (d, 2H); 6,24 (d, 1H); 6,09 (d, 1H); 5,40 - 5,16 (m, 1H); 3,79 (s, 3H); 3,10 (dd, 1H); 2,92 - 2,82 (m, 1H); 2,76 (t, 1H); 2,75 - 2,65 (m, 1H); 2,31 - 2,22 (m, 1H); 1,85 - 1,70 (m, 1H).

### 2-(4-Methoxyphenoxy)-N-(1H-pyrazol-3-yl)-N-tetrahydrothiophen-3-yl-acetamid

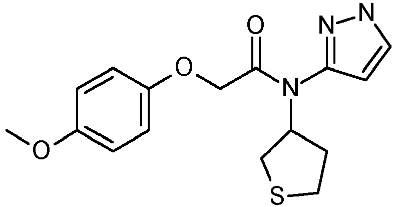

¹H NMR (500 MHz, CDCl₃) δ 7,62 (d, 1H); 6,76 (s, 4H); 6,23 (d, 1H); 5,25 - 5,14 (m, 1H); 4,35 (s, 2H); 3,73 (s, 3H); 3,07 (dd, 1H); 2,84 (dd, 1H); 2,78 - 2,59 (m, 2H); 2,34 - 2,15 (m, 1H); 1,84 - 1,70 (m, 1H).

### (E)-3-(1,3-Benzodioxol-5-yl)-N,N-bis(2-pyridyl)prop-2-enamid

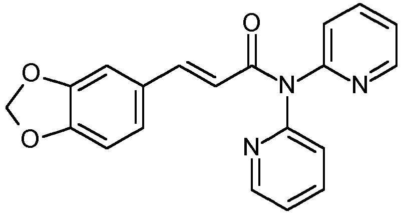

¹H NMR (400 MHz, CDCl₃) δ 8,48 (d, 2H); 7,80 (t, 2H); 7,73 (d, 1H); 7,.52 (d, 2H); 7,25 - 7,16 (m, 2H); 6,92 (d, 1H);, 6,80 (s, 1H); 6,77 (d, 1H); 6,19 (d, 1H); 5,97 (s, 2H).

### (E)-3-(1,3-benzodioxol-5-yl)-N-(1H-pyrazol-3-yl)-N-(tetrahydrofuran-2-ylmethyl)prop-2-enamid

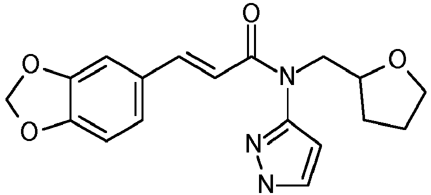

¹H NMR (400 MHz, Chloroform-d) δ 7.61 (d, *J =* 15.5 Hz, 1H), 7.57 (d, *J =* 2.1 Hz, 1H), 6.90 (d, *J* =7.7 Hz, 1H), 6.83 (s, 1H), 6.74 (d, *J* = 8.0 Hz, 1H), 6.42 (d, *J* = 15.2 Hz, 1H), 6.21 (s, 1H), 5.95 (s, 2H),4.22 (qd, *J* = 7.2, 4.2 Hz, 1H), 4.13 (dd, *J* = 13.8, 4.1 Hz, 1H), 3.95 - 3.84 (m, 1H), 3.78 (td, *J* = 7.9, 6.2 Hz, 1H), 3.68 - 3.60 (m, 1H), 2.10 - 1.96 (m, 1H), 1.99 - 1.82 (m, 2H), 1.62 (ddt, *J* = 15.6, 12.0, 8.1 Hz,1H).

### (E)-3-(p-tolyl)-N-(1H-pyrazol-3-yl)-N-(tetrahydrofuran-2-ylmethyl)prop-2-enamid

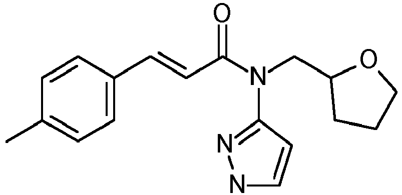

¹H NMR (400 MHz, Chloroform-d) δ 11.26 (s, 1H), 7.68 (d, *J* = 15.5 Hz, 1H), 7.56 (d, *J* = 2.2 Hz, 1H), 7.27 (d, *J* = 8.0 Hz, 2H), 7.10 (d, *J* = 7.8 Hz, 2H), 6.55 (d, *J* = 15.6 Hz, 1H), 6.21 (s, 1H), 4.22 (qd, *J* = 7.1, 4.1 Hz, 1H), 4.18 - 4.10 (m, 1H), 3.90 (dt, *J* = 8.3, 6.8 Hz, 1H), 3.78 (td, *J* = 7.9, 6.1 Hz, 1H), 3.73 - 3.57 (m, 1H), 2.32 (s, 3H), 2.12 - 1.96 (m, 1H), 1.98 - 1.80 (m, 2H), 1.61 (ddt, *J* = 12.0, 8.4, 7.2 Hz, 1H).

### (E)-3-(4-methoxyphenyl)-N-(1H-pyrazol-3-yl)-N-(tetrahydrofuran-2-ylmethyl)prop-2-enamid

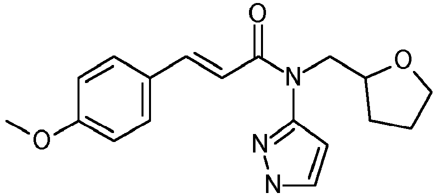

¹H NMR (600 MHz, Chloroform-d) δ 8.15 (d, *J* = 3.0 Hz, 1H), 7.90 (d, *J* = 15.8 Hz, 1H), 7.63 - 7.61 (m, 2H), 7.62 (d, *J* = 15.9 Hz, 1H), 6.93 (d, *J* = 8.7 Hz, 2H), 5.92 (d, *J* = 3.0 Hz, 1H), 4.14 (qd, *J* = 7.1, 3.6 Hz, 1H), 3.97 - 3.87 (m, 1H), 3.85 (s, 3H), 3.83 - 3.73 (m, 1H), 3.57 - 3.47 (m, 1H), 3.32 - 3.25 (m, 1H), 2.04 (dddt, *J* = 9.5, 8.4, 6.9, 4.9 Hz, 1H), 1.98 - 1.88 (m, 2H), 1.69 (ddt, *J* = 12.1, 8.6, 7.1 Hz, 1H).

### 2-(4-methylphenoxy)-N-(1H-pyrazol-3-yl)-N-(tetrahydrofuran-2-ylmethyl)acetamid

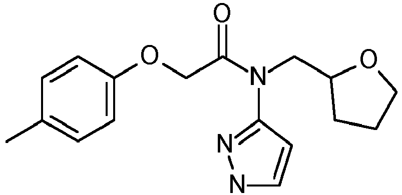

¹H NMR (400 MHz, Chloroform-d) δ 7.54 (d, *J* = 2.4 Hz, 1H), 7.01 (d, *J* = 8.1 Hz, 2H), 6.72 (d, *J* = 8.0 Hz, 2H), 6.28 (s, 1H), 4.66 - 4.48 (m, 2H), 4.2 - 4.1 (m, 1H), 4.0 - 3.9 (m, 1H), 3.9 - 3.8 (m, 1H), 3.8 - 3.7 (m, 1H), 3.64 (dd, *J* = 13.6, 7.7 Hz, 1H), 2.24 (s, 3H), 2.03 - 1.78 (m, 3H), 1.66 - 1.52 (m, 1H).

### (E)-3-(1,3-benzodioxol-5-yl)-N-(2-pyridyl)-N-(tetrahydrofuran-2-ylmethyl)prop-2-enamid

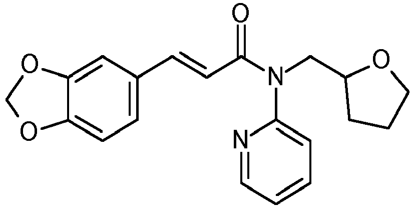

¹H NMR (600 MHz, Chloroform-d) δ 8.53 (ddd, *J* = 4.9, 2.0, 0.8 Hz, 1H), 7.75 (ddd, *J* = 8.1, 7.4, 2.0 Hz, 1H), 7.63 (d, *J* = 15.3 Hz, 1H), 7.33 (dt, *J* = 8.1, 1.0 Hz, 1H), 7.22 (ddd, *J* = 7.4, 4.9, 1.0 Hz, 1H), 6.89 (dd, *J* = 8.1, 1.6 Hz, 1H), 6.80 (d, *J =* 1.7 Hz, 1H), 6.75 (d, *J* = 8.0 Hz, 1H), 6.27 (d, *J* = 15.3 Hz, 1H), 5.95 (s, 2H), 4.24 - 4.18 (m, 2H), 4.04 - 3.98 (m, 1H), 3.73 (ddd, *J* = 8.3, 7.2, 6.5 Hz, 1H), 3.67 (td, *J* = 8.0, 5.9 Hz, 1H), 1.99 (dddd, *J* = 11.9, 8.6, 6.5, 5.2 Hz, 1H), 1.94 - 1.87 (m, 1H), 1.87 - 1.79 (m, 1H), 1.63 (ddt, *J =* 12.2, 8.6, 6.9 Hz, 1H).

### (E)-3-(p-tolyl)-N-(2-pyridyl)-N-(tetrahydrofuran-2-ylmethyl)prop-2-enamid

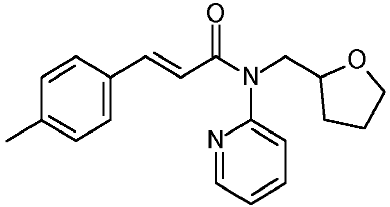

¹H NMR (600 MHz, Chloroform-d) δ 8.53 (dt, *J* = 4.8, 1.9, 0.7 Hz, 1H), 7.74 (td, *J* = 7.7, 2.0 Hz, 1H), 7.69 (d, *J* = 15.5 Hz, 1H), 7.32 (d, *J* = 8.0 Hz, 1H), 7.25 (s, 2H), 7.22 (ddd, *J* = 7.4, 4.9, 0.9 Hz, 1H), 7.11 (d, *J* = 8.0 Hz, 2H), 6.40 (d, *J* = 15.4 Hz, 1H), 4.25 - 4.21 (m, 2H), 4.05 - 3.99 (m, 1H), 3.77 - 3.70 (m, 1H), 3.67 (td, *J* = 8.0, 6.0 Hz, 1H), 2.33 (s, 3H), 2.04 - 1.96 (m, 1H), 1.96 - 1.87 (m, 1H), 1.87 - 1.78 (m, 1H), 1.64 (ddt, J= 12.3, 8.5, 7.0 Hz, 1H).

### (E)-3-(4-methoxyphenyl)-N-(2-pyridyl)-N-(tetrahydrofuran-2-ylmethyl)prop-2-enamid

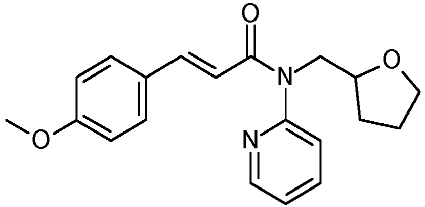

¹H NMR (400 MHz, Chloroform-d) δ 8.53 (ddd, *J* = 5.0, 2.0, 0.8 Hz, 1H), 7.74 (td, *J* = 7.7, 2.0 Hz, 1H), 7.68 (d, *J* = 15.4 Hz, 1H), 7.34 (dd, *J* = 7.5, 1.0 Hz, 1H), 7.32 - 7.28 (m, 2H), 7.21 (ddd, *J* = 7.4, 4.9, 1.0 Hz, 1H), 6.85 - 6.78 (m, 2H), 6.33 (d, *J* = 15.4 Hz, 1H), 4.27 - 4.17 (m, 2H), 4.07 - 3.96 (m, 1H), 3.80 (s, 3H), 3.93 - 3.62 (m, 2H), 2.09 - 1.75 (m, 3H), 1.64 (ddt, *J* = 11.6, 8.2, 6.7 Hz, 1H).

### (E)-3-(1,3-benzodioxol-5-yl)-N-ethyl-N-(tetrahydrofuran-2-ylmethyl)prop-2-enamid

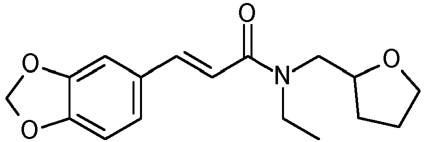

Im Spektrum sind zwei Rotamere am Stickstof zu erkennen.

¹H NMR (400 MHz, Chloroform-d) δ 7.66 - 7.55 (m, 2H), 7.06 - 6.95 (m, 4H), 6.81 - 6.74 (m, 3H), 6.70 (d, *J* = 15.3 Hz, 1H), 5.98 (s, 4H), 4.17 - 4.02 (m, 2H), 3.93 - 3.82 (m, 3H), 3.82 - 3.69 (m, 2H), 3.69 - 3.56 (m, 4H), 3.56 - 3.42 (m, 2H), 3.20 (dd, J= 13.8, 7.3 Hz, 1H), 2.08 - 1.97 (m, 2H), 1.97 - 1.79 (m, 3H), 1.63 - 1.52 (m, 3H), 1.23 (t, *J* = 7.0 Hz, 3H), 1.18 (t, J= 7.1 Hz, 3H).

### N-ethyl-2-(4-methoxyphenoxy)-N-(tetrahydrofuran-2-ylmethyl)acetamid

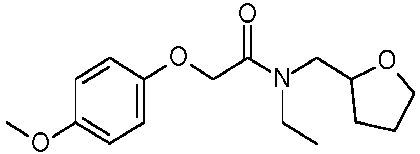

Im Spektrum sind zwei Rotamere am Stickstof zu erkennen.

¹H NMR (400 MHz, Chloroform-d) δ 6.93 - 6.86 (m, 4H), 6.82 (dd, *J* = 9.2, 1.7 Hz, 4H), 4.71 (dd, *J* = 14.6, 1.3 Hz, 2H), 4.66 (dd, *J* = 14.1, 1.0 Hz, 2H), 4.15 - 4.06 (m, 1H), 4.06 - 3.99 (m, 1H), 3.90 - 3.80 (m, 2H), 3.76 (s, 6H), 3.79 - 3.69 (m, 2H), 3.66 - 3.57 (m, 1H), 3.54 (q, *J* = 7.1 Hz, 2H), 3.47 - 3.31 (m, 3H), 3.13 (dd, *J* = 13.9, 7.5 Hz, 2H), 2.07 - 1.94 (m, 2H), 1.94 - 1.78 (m, 4H), 1.58 - 1.44 (m, 2H), 1.21 (t, *J* = 7.1 Hz, 3H), 1.13 (t, *J* = 7.1 Hz, 3H).

### (E)-N-phenyl-3-(p-tolyl)-N-(tetrahydrofuran-2-ylmethyl)prop-2-enamid

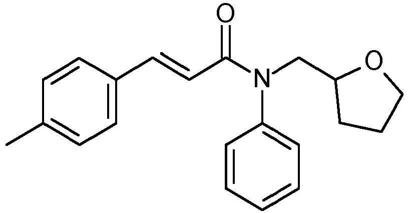

¹H NMR (400 MHz, Chloroform-d) δ 7.65 (d, *J* = 15.5 Hz, 1H), 7.45 - 7.38 (m, 2H), 7.38 - 7.33 (m, 1H), 7.33 - 7.28 (m, 2H), 7.18 (d, *J* = 8.1 Hz, 2H), 7.06 (d, *J* = 8.0 Hz, 2H), 6.26 (d, *J* = 15.5 Hz, 1H), 4.21 - 4.14 (m, 1H), 4.01 (dd, *J* = 13.7, 4.8 Hz, 1H), 3.88 - 3.69 (m, 3H), 2.30 (s, 3H), 2.02 - 1.78 (m, 3H), 1.67 - 1.56 (m, 1H).

### (E)-3-(4-methoxyphenyl)-N-phenyl-N-(tetrahydrofuran-2-ylmethyl)prop-2-enamid

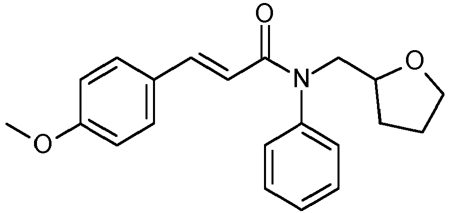

¹H NMR (400 MHz, Chloroform-d) δ 7.63 (d, *J* = 15.5 Hz, 1H), 7.46 - 7.39 (m, 2H), 7.38 - 7.32 (m, 1H), 7.32 - 7.28 (m, 2H), 7.27 - 7.21 (m, 2H), 6.79 (d, *J =* 8.8 Hz, 2H), 6.17 (d, *J* = 15.5 Hz, 1H), 4.17 (qd, *J* = 6.8, 4.9 Hz, 1H), 4.02 (dd, *J* = 13.6, 4.9 Hz, 1H), 3.88 - 3.80 (m, 1H), 3.82 - 3.75 (m, 1H), 3.78 (s, 3H), 3.73 (ddd, *J* = 8.3, 7.4, 5.8 Hz, 1H), 2.06 - 1.94 (m, 1H), 2.03 - 1.78 (m, 2H), 1.68 - 1.56 (m, 1H).

### 2-(4-methylphenoxy)-N-phenyl-N-(tetrahydrofuran-2-ylmethyl)acetamid

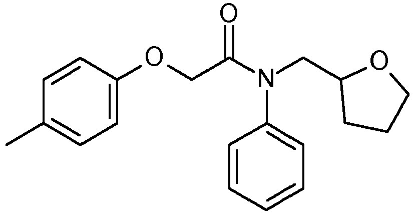

¹H NMR (400 MHz, Chloroform-d) δ 7.43 (dd, *J* = 8.2, 6.4 Hz, 2H), 7.41 - 7.35 (m, 1H), 7.34 - 7.29 (m, 2H), 7.01 (d, *J* = 8.3 Hz, 2H), 6.72 - 6.64 (m, 2H), 4.36 (d, *J* = 14.6 Hz, 1H), 4.31 (d, *J* = 14.7 Hz, 1H), 4.12 (qd, *J* = 6.6, 4.6 Hz, 1H), 3.89 - 3.78 (m, 2H), 3.72 (td, *J* = 7.8, 5.9 Hz, 2H), 2.24 (s, 3H), 2.04 - 1.78 (m, 3H), 1.62 - 1.53 (m, 1H).

### 2-(4-methoxyphenoxy)-N-phenyl-N-(tetrahydrofuran-2-ylmethyl)acetamid

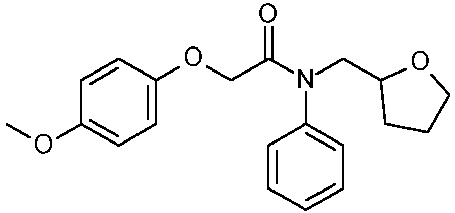

¹H NMR (400 MHz, Chloroform-d) δ 7.47 - 7.40 (m, 2H), 7.40 - 7.34 (m, 1H), 7.34 - 7.28 (m, 2H), 6.77 (d, *J* = 9.3 Hz, 2H), 6.72 (d, *J* = 9.4 Hz, 2H), 4.38 - 4.24 (m, 2H), 4.17 - 4.06 (m, 1H), 3.88 - 3.78 (m, 2H), 3.78 - 3.69 (m, 2H), 3.73 (s, 3H), 2.05 - 1.77 (m, 3H), 1.64 - 1.53 (m, 1H).

### (E)-3-(1,3-benzodioxol-5-yl)-N-ethyl-N-[2-(2-thienyl)ethyl]prop-2-enamid

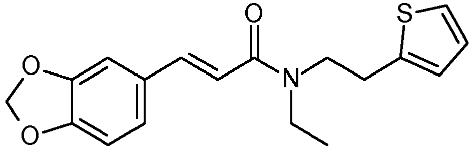

¹H NMR (400 MHz, Chloroform-d) δ 7.65 (d, *J* = 15.2 Hz, 1H), 7.54 (d, *J* = 15.2 Hz, 1H), 7.18 - 7.10 (m, 2H), 7.06 - 6.98 (m, 2H), 6.97 - 6.89 (m, 4H), 6.88 - 6.83 (m, 2H), 6.79 (t, *J* = 8.2 Hz, 2H), 6.66 (d, *J* = 15.3 Hz, 1H), 6.44 (d, *J* = 15.3 Hz, 1H), 5.98 (s, 4H), 3.66 (t, *J* = 7.5 Hz, 4H), 3.50 (q, *J* = 7.1 Hz, 2H), 3.37 (q, *J* = 7.2 Hz, 2H), 3.19 - 3.09 (m, 4H), 1.19 (t, J= 7.1 Hz, 6H).

### Sensorische Bewertung:

### Kühlintensität erfindungsgemäßer Amide im Vergleich zu FEMA 4809:

Testlösungen mit 2 oder 20 ppm der erfindungsgemäßen Amide in einer 5%igen Zuckerlösung wurden von geschulten Panelisten (n = 10 bis 11) sensorisch bewertet und mit einer Lösung mit 2 ppm der Vergleichsverbindung FEMA 4809 verglichen. Dabei wurde die Kühlintensität zu Beginn, die gesamte Kühlintensität und die langanhaltende Wirkung auf einer Skala von 1 (sehr schwach) bis 9 (sehr stark) bewertet.

| **#** | **Dosierung ppm** | **Kühleffekt zu Beginn** | **gesamte Kühlintensität** | **langanhaltende Wirkung** |
|---|---|---|---|---|
| **FEMA 4809** | 2 | 3,9 | 7,3 | 7,5 |
| (E)-3-(1,3-benzodioxol-5-yl)-N-phenyl-N-tetrahydrofuran-3-yl-prop-2-enamid | 20 | 5,8 | 7,8 | 7,9 |
| (E)-3-(1,3-benzodioxol-5-yl)-N-phenyl-N-tetrahydrofuran-3-yl-prop-2-enamid | 2 | 4,1 | 6,1 | 6,9 |
| (E)-N-phenyl-3-(p-tolyl)-N-tetrahydrofuran-3-yl-prop-2-enamid | 20 | 4,3 | 5,7 | 6,1 |
| (E)-3-(1,3-benzodioxol-5-yl)-N-(2-pyridyl)-N-tetrahydrothiophen-3-yl-prop-2-enamid | 20 | 6,6 | 8,3 | 7,9 |
| (E)-3-(p-tolyl)-N-(2-pyridyl)-N-tetrahydrothiophen-3-yl-prop-2-enamid | 20 | 5,3 | 7,0 | 7,4 |
| (E)-3-(4-methoxyphenyl)-N-(2-pyridyl)-N-tetrahydrothiophen-3-yl-prop-2-enamid | 20 | 4,1 | 5,6 | 5,9 |
| (E)-3-phenyl-N-(2-pyridyl)-N-tetrahydrothiophen-3-yl-prop-2-enamid | 20 | 5,3 | 7,8 | 7,8 |
| (E)-3-(1,3-benzodioxol-5-yl)-N-phenyl-N-(tetrahydrofuran-2-ylmethyl)prop-2-enamid | 20 | 3,1 | 5,0 | 5,3 |
| (E)-3-(1,3-benzodioxol-5-yl)-N-(2-methylsulfanylethyl)-N-phenyl-prop-2-enamid | 20 | 2,7 | 4,7 | 4,7 |
| 2-(4-methylphenoxy)-N-(2-methylsulfanylethyl)-N-phenyl-acetamid | 20 | 2,2 | 2,8 | 2,8 |
| (E)-3-(1,3-benzodioxol-5-yl)-N-ethyl-N-(2-methylsulfanylethyl)prop-2-enamid | 20 | 3,8 | 5 | 4,7 |
| (E)-3-(1,3-benzodioxol-5-yl)-N-(2-methylsulfanylethyl)-N-(2-pyridyl)prop-2-enamid | 20 | 6,2 | 7,8 | 7,5 |
| (E)-3-(1,3-benzodioxol-5-yl)-N-(2-methylsulfanylethyl)-N-(2-pyridyl)prop-2-enamid | 2 | 3,4 | 4,6 | 4,3 |
| (E)-N-(2-methylsulfanylethyl)-3-(p-tolyl)-N-(2-pyridyl)prop-2-enamid | 20 | 4 | 5,7 | 5,3 |
| (E)-3-(1,3-benzodioxol-5-yl)-N-(2-methylsulfanylethyl)-N-(1H-pyrazol-3-yl)prop-2-enamid | 20 | 3,7 | 6,6 | 6,6 |
| (E)-3-(1,3-benzodioxol-5-yl)-N-(2-methylsulfanylethyl)-N-(1H-pyrazol-3-yl)prop-2-enamid | 5 | 3,1 | 4,8 | 4,6 |

Bemerkung: Die Verbindungen wurden als 1%ig in Ethanol vorgelöst.

Deutlich zu erkennen ist das schnellere Einsetzen des Kühleffekts, ein klares Kühlprofil, das vergleichbar zur oder besser als die Referenz ist, sowie der deutlich lang anhaltende Kühleffekt bei erfindungsgemäßen Amiden.

### Zeit-Intensitäts-Profil von erfindungsgemäßen Amiden im Vergleich zu Referenzverbindungen (FEMA 4809, 4496) und WS3:

Zur Erstellung eines Zeit-Intensitäts-Profils wurde der Mundraum von geschulten Panelisten (n = 8 bis 11) für 40 Sekunden mit einem Schluck einer Probenlösung (2 ppm FEMA 4809 bzw. 50 ppm FEMA 4496 bzw. 50 ppm 1) bis 5)) gespült. Anschließend wurde die empfundene Kühlintensität in definierten Zeitabständen anhand einer Skala bewertet.

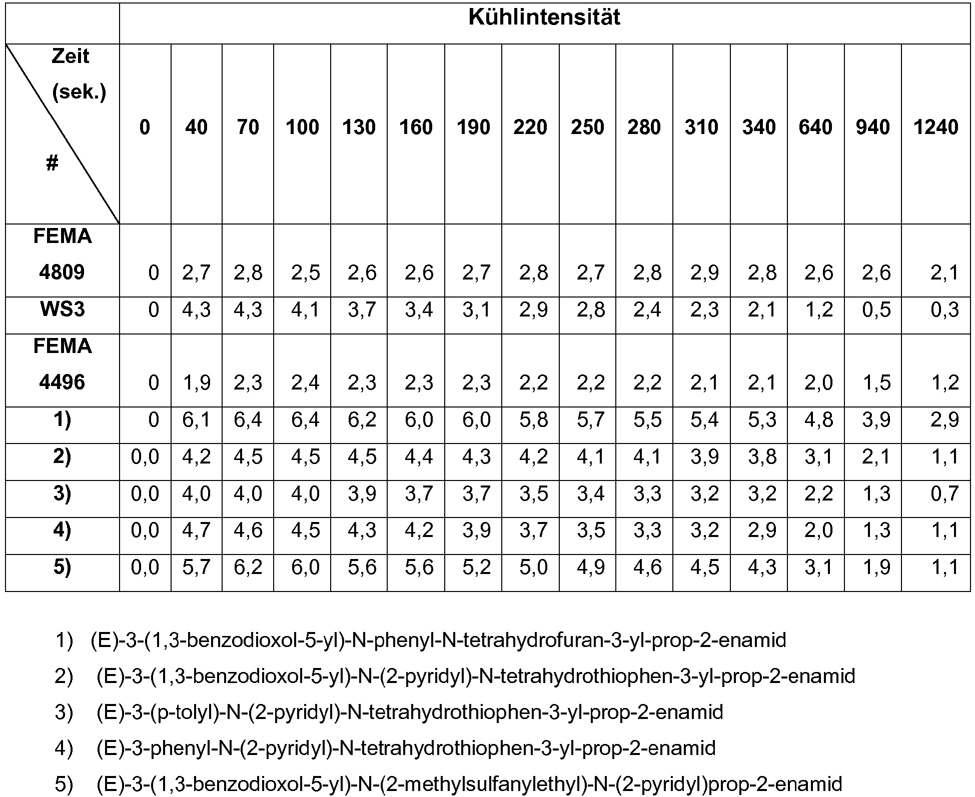

Bei den erfindungsgemäßen Amiden wurde ein deutlich schnelleres oder zumindest ein vergleichbar schnelles Einsetzen von Kühlaktivität gegenüber WS3 festgestellt. Zusätzlich hielt der Kühleffekt länger an als bei WS3 und vergleichbar lang wie bei Referenzverbindungen (FEMA 4809, FEMA 4496), die aber ein deutlich verzögertes Einsetzen der Kühlwirkung zeigten.

### Anwendungsbeispiele:

### Zahnpasta

Geeignete Zahnpasten können nach folgender Basisrezeptur hergestellt werden:

| Gew.-% | Inhaltsstoff-Typ | Inhaltsstoff-Beispiele |
|---|---|---|
| 0,05-0,2% | Fluoride | Natriumfluorid, Zinn(II)-fluorid, Natriummonofluorophosphat; |
| 10-55% | Feuchthaltemittel | Glycerin, Sorbit, Propylenglykol, Polyalkylenglykol |
| 0-50% | Polymer | Polyoxyalkylenblockcopoymere Mw 5000-30000 |
| 10-50% | Wasser | |
| 10-55% | Abrasiva | Calciumpyrophosphat, Dicalciumphosphat, Siliziumoxidhydrat; |
| 2-10% | Bindemittel | Karayagummi, Tragant USP, Natriumalginat, irländisches Moos, Methylcellulose |
| 2-8% | Tensid | Natriumlaurylsulphat, Natrium-N-laurylsarcosinat, Dioctlnatriumsulphosuccinat, Natriumlaurylsulphoacetat |
| 0-10% | Persauerstoffverbindung | Wasserstoffperoxid, anorganischen Peroxiden |
| 0,001-10% | TRPM8-Agonist | |
| 0-10% s.o. | Weitere Zusätze | |

### Evaluierung des zeitlichen Verlaufs der Kühlintensitäten erfindungsgemäß einzusetzender Verbindungen in beispielhafter Zahnpasta:

Die erfindungsgemäß einzusetzenden Kühlsubstanzen wurden wie folgt in Zahnpasta eingearbeitet:

| | | |
|---|---|---|
| Entionisiertes Wasser | 27,52 | |
| Sorbitol 700/0 | 45 | |
| Solbrol M Na-Salz | 0,15 | |
| Trinatriumphosphat | 0,1 | |
| Saccharin | 0,2 | |
| Natriummonofluorphosphat | 1,12 | |
| PEG 1500 | 5 | |
| Sident 9 (Abrasive Silica) | 10 | |
| Sident 22 S (Dickende Silica) | 8 | |
| Natriumcarboxymethylcellul ose | 0,9 | |
| Titan (IV) oxid | 0,5 | |
| Natriumlaurylsulfat (SLS) | 1,5 | |
| jeweilige Kühlsubstanz | 0,01 | |
| Total | | 100 |

### Alle Angaben in Gew.-%

Die sensorischen Eigenschaften der resultierenden Zahnpasta wurden durch ein trainiertes Panel (von 6 Personen) evaluiert. Dazu wurden die Zähne mit der die erfindungsgemäßen Verbindungen enthaltenden Zahnpasta zunächst 30 Sekunden geputzt, dann der Zahnpastaschaum ausgespuckt und der Mund einmal mit Wasser ausgespült. Die Testpersonen beurteilten die Stärke des Kältegefühls auf einer Skala von 0 (kein Kältegefühl) bis 9 (extrem starkes Kältegefühl). Die Beurteilung des Kältegefühls erfolgte jeweils nach 30 Sekunden, 1, 5, 10, 20, 30, 45 und 60 Minuten.

Als erfindungsgemäße Verbindungen wurden exemplarisch die Verbindungen (E)-N-phenyl-3-(p-tolyl)-N-tetrahydrofuran-3-yl-prop-2-enamid, (E)-3-(4-methoxyphenyl)-N-(2-pyridyl)-N-tetrahydrothiophen-3-ylprop-2-enamid, (E)-3-(1,3-benzodioxol-5-yl)-N-phenyl-N-(tetrahydrofuran-2-ylmethyl)prop-2-enamid, (E)-3-(1,3-benzodioxol-5-yl)-N-(2-methylsulfanylethyl)-N-phenyl-prop-2-enamid, 2-(4-methylphenoxy)-N-(2-methylsulfanylethyl)-N-phenylacetamid, (E)-3-(1,3-benzodioxol-5-yl)-Nethyl-N-(2-methylsulfanylethyl)prop-2-enamid und (E)-N-(2-methylsulfanylethyl)-3-(p-tolyl)-N-(2-pyridyl)prop-2-enamid getestet, insbesondere aber die Verbindungen (E)-3-(1,3-benzodioxol-5-yl)-N-(2-pyridyl)-N-tetrahydrothiophen-3-ylprop-2-enamid, (E)-3-(1,3-benzodioxol-5-yl)-N-(2-methylsulfanylethyl)N-(2-pyridyl)prop-2-enamid, (E)-3-(p-tolyl)-N-(2-pyridyl)-N-tetrahydrothiophen-3-yl-prop-2-enamid, (E)-3-phenyl-N-(2-pyridyl)-N-tetrahydrothiophen-3-yl-prop-2-enamid, (E)-3-(1,3-benzodioxol-5-yl)-N-phenyl-N-tetrahydrofuran-3-yl-prop-2-enamid und (E)-3-(1,3-benzodioxol-5-yl)-N-(2-methylsulfanylethyl)-N-(1H-pyrazol-3-yl)prop-2-enamid.

Zum Vergleich wurden Zahnpasten mit gleicher Zusammensetzung getestet, die als konventionelle Kühlsubstanz Menthan-3-carbonsäure-N-ethylamid ("WS 3", s.a. US 4.150.052) enthielten.

Das Ergebnis zeigte, dass die erfindungsgemäßen Kühlsubstanzen den konventionell verwendeten sowohl von einer schnell wahrnehmbaren Intensität her als auch von der Dauer der Kühlwahrnehmung her deutlich überlegen sind. So vermitteln die erfindungsgemäßen Kühlsubstanzen auch noch 1 Stunde nach dem Zähneputzen ein sehr deutlich wahrnehmbares Kältegefühl, während es bei den konventionellen Vergleichsverbindungen nach dieser Zeit schon ganz verschwunden ist.

### Kaugummi

Geeignete Kaugummis können nach folgender Basisrezeptur hergestellt werden:

| Gew.-% | Inhaltsstoff |
|---|---|
| 15 - 25% | Kaugummi-Grundmasse ("gum-base") |
| 20 - 30% | Glucosesirup |
| 50 - 60% | Puderzucker |
| 0,001 - 10% | TRPM8-Agonist |
| 1 - 2% | Weichmacher (z.B. Glycerin) |
| 3 - 6% | Wasser |

Anstelle des Glucosesirups und des Puderzuckers können für "zuckerfreie" Rezepturen auch die Zuckeralkohole Mannit, Xylit und Sorbit, "Palatinit" und andere sowie künstliche Süßstoffe, wie Saccharin, Cyclamat, Acesulfam-K und Aspartame, eingesetzt werden.

### Kosmetische Sonnenschutzzubereitung

In den folgenden Rezepturen wird eine kosmetische Sonnenschutzzubereitung, enthaltend eine Kombination aus mindestens anorganischem Pigment und organischem UV-Filter beschrieben.

Die Herstellung der nachfolgend genannten Formulierungen erfolgt auf übliche, dem Fachmann bekannte Art und Weise.

| | | |
|---|---|---|
| A | 7,50 Uvinul MC 80 | Ethylhexylcinnamat |
| | 2,00 Uvinul M 40 | Benzophenon-3 |
| | 0,80 Rylo PG 11 | Polyglyceryldimersoyat |
| | 1,00 Span 60 | Sorbitanstearat |
| | 0,50 Vitamin E-Acetat | Tocopherylacetat |
| | 3,00 Dracorin 100 SE | Glycerylstearat, PEG-100 Stearat |
| | 1,00 Cremophor CO 410 | PEG-40-hydriertes Rizinusöl |
| B | 3,00 T-Lite SF | Titandioxid, Aluminiumoxidhydrat, Dimethicon- /Methicon Copolymer |
| | 1,00 Cetiol SB 45 | *Butyrospermum parkii* (Shea Butter) |
| | 6,50 Finsolv TN | C₁₂₋₁₅-Alkylbenzoat |
| C | 5,00 Butylenglykol | Butylenglycol |
| | 0,30 Keltrol | Xanthangummi |
| | 0,10 Edeta BD | Dinatrium-EDTA |
| | 0,10 Allantoin | Allantoin |
| | 66,20 Wasser dem. | Aqua dem. |
| D | 1,00 Sepigel 305 | Polyacrylamid, C₁₃₋₁₄-Isoparaffin, Laureth-7 |
| 0,001-10 % TRPM8 Agonist | | |
| | q.s. | Konservierungsmittel |

### Pudding

Rezept (für 100 ml)

| Inhaltsstoff | Menge |
|---|---|
| Fettfreie Trockenmilch | 10,715 g |
| Saccharose | 5 g |
| Novelose Stärke, National Starch | 7 g |
| Pflanzenölgemisch | 2,2 g |
| Carrageenan | 0,016 g |
| Vanillearoma | 0,5 g |
| Natriumstearoyl-2-lactylat | 0,095 g |
| Gelber Farbstoff | 0,189 g |
| Magnesiumphosphat | 0,165 g |
| Vitaminvormischung | 1,84 g |
| Spurenelementvormischung | 0,015 g |
| Wirkstoff | 0,5 g |
| Wasser | 81,94 g |

### Herstellung:

Neun Zehntel des Wassers auf 43,3 °C erhitzen. Magermilchpulver im Wasser auflösen. Öl auf 60 °C erhitzen und Carrageenan und öllösliche Vitamine zum Öl geben. Öl in das Erzeugnis einmischen. Die übrigen Bestandteile außer der modifizierten Stärke, Vanillearoma und Vitaminvormischung hinzufügen. Das Gemisch homogenisieren. Stärke langsam hinzufügen. Wirkstoff, Vitamine und Aroma hinzufügen. Feststoffgehalt standardisieren. In sterilen Einheiten erhitzen und in Dosen verpacken.

### Textilausrüstung mit erfindungsgemäßen Wirkstoffen

Zunächst stellt man eine wässrige Aufschlämmung amylosehaltiger Stärke her, indem man 570 g entionisiertes Wasser wurden mit 10 g eines handelsüblichen Konservierungsmittels versetzt. Hierin löste man 20 g Carboxymethylcellulose, gab anschließend 400 g einer amylosehaltigen Stärke mit einem Amylosegehalt von 50 Gew.-% zu und stellte unter Rühren eine Aufschlämmung her.

Anschließend erfolgt die Herstellung wässriger Flotten mit amylosehaltiger Stärke nach einer der beiden folgenden Methoden:
Methode 1: Die jeweilige Aufschlämmung wird durch Verdünnen mit Wasser auf einen Stärkegehalt von 5 oder 15 Gew.-% eingestellt.
Methode 2: Die jeweilige Aufschlämmung werden zunächst mit Wasser auf einen Stärke-Gehalt von 5 oder 15 Gew.-% verdünnt und anschließend mit 30 g/l einer 30 Gew.-%igen, wässrigen Polyurethandispersion (nicht-ionogen) versetzt.

Anschließend erfolgt die Ausrüstung eines Gewebes mit amylosehaltiger Stärke und erfindungsgemäßem Wirkstoff:
Baumwollgewebemuster mit einem Flächengewicht von 124 g/m² wird mit einer der oben hergestellten Flotten mittels eines Foulards bis zu einer Flottenaufnahme von 80 Gew.-%, bezogen auf das Gewicht des Gewebes behandelt. Anschließend trocknet man 2 min bei 120°C.

Anschließend werden die so ausgerüsteten Gewebemuster mit einer wässrigen Wirkstoff-Formulierung behandelt, indem man eine wässrige Emulsion/Suspension eines erfindungsgemäßen Wirkstoffs mit einem Wirkstoffgehalt von 1 bis 7 Gew.-% bis zu einer Flottenaufnahme von 79 - 80 Gew.-% auf das Gewebemuster auffoulardiert. Anschließend werden die so behandelten Gewebemuster in einem Haushaltstrockner bis zu einer Restfeuchte von 15 % getrocknet.

Die so hergestellten wirkstoffbeladenen Gewebe können dann weiter untersucht werden, wie z.B. auf deren Kühlende Wirkung bei Hautkontakt oder deren Repellent-Wirkung auf Insekten.

### Mundwasser-Aromen

Herstellung von Mundwasser-Aromen mit Kühlwirkung unter Verwendung der erfindungsgemäßen Kühlsubstanzen:
Es wurden vermischt (alle Angaben, soweit nicht anders vermerkt, in Gew-%):

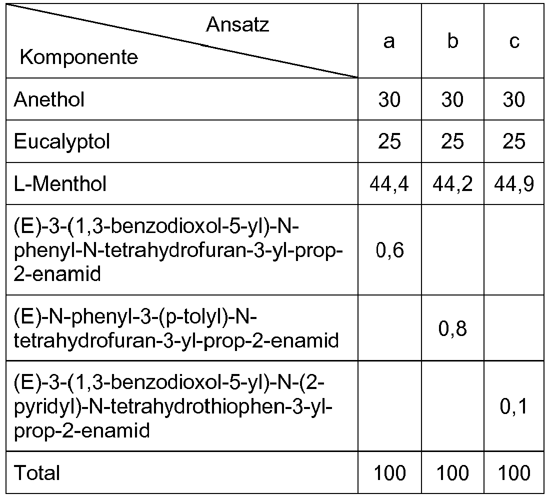

Die Aromen wurden jeweils mit einer Konzentration von 0,15 Gew.% in ein gebrauchsfertiges Mundwasser, bzw. mit einer Konzentration von 3 Gew.% in ein Mundwasserkonzentrat eingearbeitet. Die sensorische Bewertung durch ein geschultes Experten-Panel zeigte, dass die Aromen zu einer schnell einsetzenden, sehr lang anhaltenden Frischewirkung führten, die auch nach Verwendung der Mundwässer noch über eine Zeit von fast 1 h anhielt.

### Kaubonbons

Herstellung eines Kaubonbons mit kühlendem Himbeergeschmack unter Verwendung der erfindungsgemäßen Kühlsubstanzen.

Alle Angaben, soweit nicht anders vermerkt, in Gew-%.

| Bestandteile | 1 | 2 | 3 | 4 | 5 | 6 |
|---|---|---|---|---|---|---|
| Wasser | 7,8 | 7,79 | 7,805 | 7,8 | 7,815 | 7,81 |
| Zucker Raffinade C4 | 42,1 | 42,1 | 42,1 | 42,1 | 42,1 | 42,1 |
| Glucose Sirup Dextrose 40 | 37,3 | 37,3 | 37,3 | 37,3 | 37,3 | 37,3 |
| gehärtetes Pflanzenfett Schmelzpunkt 32-36°C | 6,6 | 6,6 | 6,6 | 6,6 | 6,6 | 6,6 |
| Lecithin Emulgator (Sojalecithin) | 0,3 | 0,3 | 0,3 | 0,3 | 0,3 | 0,3 |
| Gelatine (Schweinegelatine) | 0,8 | 0,8 | 0,8 | 0,8 | 0,8 | 0,8 |
| Fondant Typ - S30 | 4,8 | 4,8 | 4,8 | 4,8 | 4,8 | 4,8 |
| Himbeeraroma | 0,22 | 0,22 | 0,22 | 0,22 | 0,22 | 0,22 |
| Menthyllactat | 0,06 | 0,06 | 0,06 | 0,06 | 0,06 | 0,06 |
| (E)-3-(p-tolyl)-N-(2-pyridyl)-N-tetrahydrothiophen-3-yl-prop-2-enamid | 0,02 | | | | | |
| (E)-3-(4-methoxyphenyl)-N-(2-pyridyl)-N-tetrahydrothiophen-3-yl-prop-2-enamid | | 0,03 | | | | |
| (E)-3-phenyl-N-(2-pyridyl)-N-tetrahydrothiophen-3-yl-prop-2-enamid | | | 0,015 | | | |
| (E)-3-(1,3-benzodioxol-5-yl)-N-phenyl-N-(tetrahydrofuran-2-ylmethyl)prop-2-enamid | | | | 0,02 | | |
| (E)-3-(1,3-benzodioxol-5-yl)-N-(2-methylsulfanylethyl)-N-phenyl-prop-2-enamid | | | | | 0,005 | |
| 2-(4-methylphenoxy)-N-(2-methylsulfanylethyl)-N-phenyl-acetamid | | | | | | 0,01 |

Herstellungshinweise:
a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Himbeeraroma, Menthyllactat und die erfindungsgemäßen Kühlsubstanzen vermischen und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.

Beim Verzehr der Kaubonbons wird während des Kauens ein frischer, kühlender Himbeergeschmack wahrgenommen.

### Fruchtgummis

Herstellung eines Fruchtgummis mit einem lang anhaltenden frischen kühlenden Geschmack unter Verwendung der erfindungsgemäßen Kühlsubstanzen.

Alle Angaben, soweit nicht anders vermerkt, in Gew-%.

| Komponente | 1 | 2 | 3 | 4 | 5 | 6 |
|---|---|---|---|---|---|---|
| Wasser | 23,6 | 23,6 | 23,6 | 23,6 | 23,6 | 23,6 |
| Saccharose | 34,5 | 34,5 | 34,5 | 34,5 | 34,5 | 34,5 |
| Glucosesirup, DE 40 | 31,89 | 31,89 | 31,89 | 31,89 | 31,89 | 31,89 |
| Iso Syrup C* Tru Sweet 01750 (Cerestar GmbH) | 1,5 | 1,5 | 1,5 | 1,5 | 1,5 | 1,5 |
| Gelatine 240 Bloom | 8,2 | 8,2 | 8,2 | 8,2 | 8,2 | 8,2 |
| Gelber und roter Farbstoff | 0,01 | 0,01 | 0,01 | 0,01 | 0,01 | 0,01 |
| Citronensäure | 0,2 | 0,2 | 0,2 | 0,2 | 0,2 | 0,2 |
| (E)-3-phenyl-N-(2-pyridyl)-N-tetrahydrothiophen-3-yl-prop-2-enamid | 0,1 | | | | | |
| (E)-3-(1,3-benzodioxol-5-yl)-N-phenyl-N-(tetrahydrofuran-2-ylmethyl)prop-2-enamid | | 0,1 | | | | |
| (E)-3-(1,3-benzodioxol-5-yl)-N-(2-methylsulfanylethyl)-N-phenyl-prop-2-enamid | | | 0,1 | | | |
| 2-(4-methylphenoxy)-N-(2-methylsulfanylethyl)-N-phenyl-acetamid | | | | 0,1 | | |
| (E)-3-(1,3-benzodioxol-5-yl)-N-phenyl-N-tetrahydrofuran-3-yl-prop-2-enamid | | | | | 0,1 | |
| (E)-N-phenyl-3-(p-tolyl)-N-tetrahydrofuran-3-yl-prop-2-enamid | | | | | | 0,1 |

Die vorstehenden Anwendungsbeispiele lassen sich - gegebenenfalls durch vom Fachmann unschwer vorzunehmende Modifikationen - auf andere Produkte der jeweiligen Produktgruppe übertragen. Dabei ist es für den Fachmann aufgrund der vorliegenden Beschreibung unschwer erkennbar, dass ohne großen Aufwand die erfindungsgemäßen Verbindungen - eventuell unter geringfügigen Modifikationen - untereinander austauschbar sind. Auch die Konzentration der verwendeten erfindungsgemäßen Verbindung oder Mischung ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile ist in der oben stehenden Beschreibung offenbart.

## Patentansprüche

1. Nicht-therapeutisches Verfahren zur Modulation, vorzugsweise zur *in vitro* und/oder *in vivo* Modulation, des Kälte-Menthol-Rezeptors TRPM8, wobei man den Rezeptor mit wenigstens einem Modulator in Kontakt bringt, der ausgewählt ist aus der Gruppe bestehend aus Verbindungen des folgenden Strukturtyps 1: 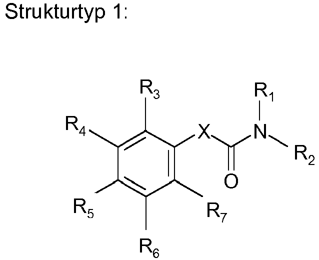 worin
R₁ aus folgender Struktur besteht: 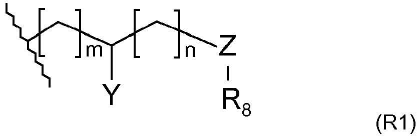 worin
Z ausgewählt ist aus der Gruppe bestehend aus O und S;
n gleich 0 oder 1 ist;
m
- gleich 1 ist und Y Wasserstoff ist und R8 ein linearer oder verzweigter C₁ bis C₅ Alkylrest, bevorzugt ein C₅ Alkylrest, ist, oder Y NR₉ oder CR₉ ist und R₉ mit R₈ einen teilweise oder vollständig gesättigten 5- oder 6-gliedrigen Ring ausbildet, oder
- gleich 0, 2 oder 3 ist und Y Wasserstoff ist und R₈ ein linearer oder verzweigter C₁ bis C₅ Alkylrest, bevorzugt ein C₅ Alkylrest, ist, oder Y NR₉ oder CR₉ ist und R₉ mit R₈ einen aromatischen, einen teilweise oder einen vollständig gesättigten 5- oder 6-gliedrigen Ring ausbildet;
R₂ ein linearer oder verzweigter C₁ bis C₅ Alkylrest, ein 5- oder 6-gliedriger Cycloalkylrest oder ein 5- oder 6-gliedriger Arylrest ist, wobei der Alkylrest, der Cycloalkylrest oder der Arylrest optional ein oder zwei (Ring-)Heteroatome beinhalten;
R₃, R₄, R₅, R₆ und R₇ gleich oder verschieden sind und ausgewählt sind aus der Gruppe bestehend aus
Wasserstoff;
Halogenen;
linearen oder verzweigten C₁- bis C₆-Alkylgruppen, die gegebenenfalls 1, 2, 3 oder 4 gleiche oder verschiedene Substituenten tragen, die ausgewählt sind aus der Gruppe bestehend aus NH₂, OH, SH, Halogenen oder linearen oder verzweigten C₁- bis C₆-Alkylgruppen;
linearen oder verzweigten C₁- bis C₆-Alkoxygruppen, die gegebenenfalls 1, 2, 3 oder 4 gleiche oder verschiedene Substituenten tragen, die ausgewählt sind aus der Gruppe bestehend aus NH₂, OH, SH, Halogenen oder C₁- bis C₆-Alkoxygruppen;
ein- oder mehrkernige Aryl-, Arylalkyl- und Heteroarylgruppen, die gegebenenfalls 1, 2, 3 oder 4 gleiche oder verschiedene Substituenten tragen, die ausgewählt sind aus der Gruppe bestehend aus NH₂, OH, SH, Halogenen, linearen oder verzweigten C₁- bis C₆-Alkylgruppen; wobei die Heteroarylgruppen 1, 2, 3 oder 4 Ring-Heteroatome aufweisen, die gleich oder verschieden sind und ausgewählt sind aus der Gruppe bestehend aus O, N und S, oder
zwei benachbarte Reste R₃, R₄, R₅, R₆ und R₇ zusammen mit den Kohlenstoffatomen, an die sie gebunden sind, einen 4-, 5-, 6- oder 7-gliedrigen, ein- oder mehrfach ungesättigten heterocyclischen Rind bilden, der gegebenenfalls 1, 2, 3, 4 oder 5 gleiche oder verschiedene Substituenten trägt, die ausgewählt sind aus der Gruppe bestehend aus linearen oder verzweigten C₁- bis C₆-Alkylgruppen, und der 1, 2 oder 3 Ring-Heteroatome aufweist, die gleich oder verschieden sind und ausgewählt sind aus der Gruppe bestehend aus O, N und S;
X
- ausgewählt ist aus der Gruppe bestehend aus
-C₁- bis C₄-Alkylengruppen; -C₂- bis C₄-Alkenylengruppen, sowie -A-C₁- bis C₄- oder -C₁- bis C₄-A- Alkylengruppen oder -A-C₂- bis C₄- oder -C₂- bis C₄-A-Alkenylengruppen, worin A für O, S oder NH steht; oder
- für eine chemische Einfachbindung steht;
sowie Salzen dieser Verbindungen, insbesondere Säureadditionssalzen mit anorganischen oder insbesondere organischen, ein- oder insbesondere mehrwertigen Carbonsäuren;
und gegebenenfalls dieser Verbindungen in Stereoisomeren-reiner Form oder Gemischen von Stereoisomeren davon.

2. Verfahren nach Anspruch 1, wobei die Verbindungen des Strukturtyps 1 ausgewählt sind aus der Gruppe A, bestehend aus den folgenden Verbindungen:
| Struktur | Name |
|---|---|
| 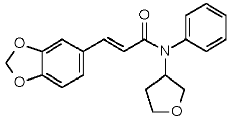 | (E)-3-(1,3-benzodioxol-5-yl)-N-phenyl-N-tetrahydrofuran-3-yl-prop-2-enamid |
| 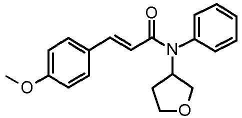 | (E)-3-(4-Methoxyphenyl)-N-phenyl-N-tetrahydrofuran-3-yl-prop-2-enamid |
| 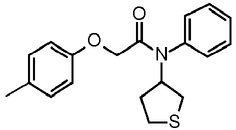 | 2-(4-Methylphenoxy)-N-phenyl-N-tetrahydrothiophen-3-yl-acetam id |
| 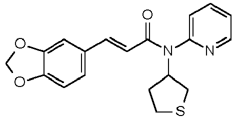 | (E)-3-(1,3-benzodioxol-5-yl)-N-(2-pyridyl)-N-tetrahydrothiophen-3-yl-prop-2-enamid |
| 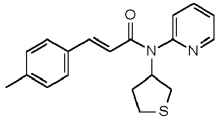 | (E)-3-(p-tolyl)-N-(2-pyridyl)-N-tetrahydrothiophen-3-yl-prop-2-enamid |
| 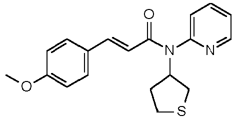 | (E)-3-(4-methoxyphenyl)-N-(2-pyridyl)-N-tetrahydrothiophen-3-yl-prop-2-enamid |
| 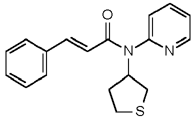 | (E)-3-phenyl-N-(2-pyridyl)-N-tetra-hydrothiophen-3-yl-prop-2-enamid |
| 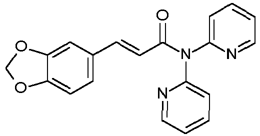 | (E)-3-(1,3-Benzodioxol-5-yl)-N,N-bis(2-pyridyl)prop-2-enamid |
| 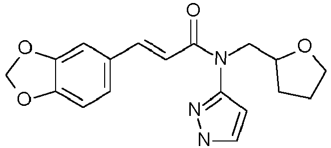 | (E)-3-(1,3-benzodioxol-5-yl)-N-(1H-pyrazol-3-yl)-N-(tetrahydrofuran-2-ylmethyl)prop-2-enamid |
| 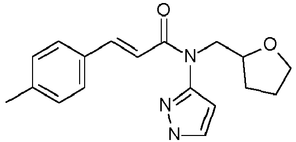 | (E)-3-(p-tolyl)-N-(1H-pyrazol-3-yl)-N-(tetrahydrofuran-2-ylme-thyl)prop-2-enamid |
| 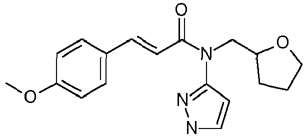 | (E)-3-(4-methoxyphenyl)-N-(1H-pyrazol-3-yl)-N-(tetrahydrofuran-2-ylmethyl)prop-2-enamid |
| 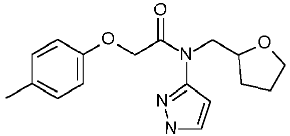 | 2-(4-methylphenoxy)-N-(1H-pyrazol-3-yl)-N-(tetrahydrofuran-2-ylmethyl)acetamid |
| 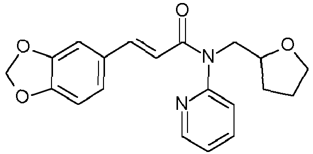 | (E)-3-(1,3-benzodioxol-5-yl)-N-(2-pyridyl)-N-(tetrahydrofuran-2-ylmethyl)prop-2-enamid |
| 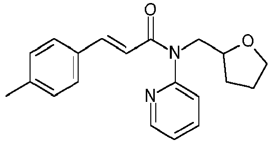 | (E)-3-(p-tolyl)-N-(2-pyridyl)-N-(tet-rahyd rofu ran-2-ylmethyl)prop-2-enamid |
| 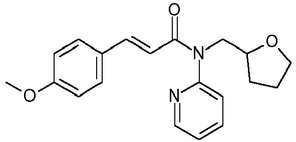 | (E)-3-(4-methoxyphenyl)-N-(2-pyridyl)-N-(tetrahydrofuran-2-ylmethyl)prop-2-enamid |
| 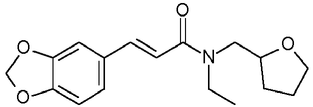 | (E)-3-(1,3-benzodioxol-5-yl)-N-ethyl-N-(tetrahydrofuran-2-ylme-thyl)prop-2-enamid |
| 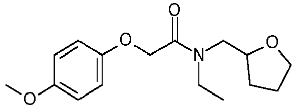 | N-ethyl-2-(4-methoxyphenoxy)-N-(tetrahydrofuran-2-ylmethyl)acetamid |
| 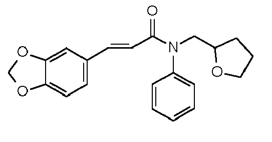 | (E)-3-(1,3-benzodioxol-5-yl)-N-phenyl-N-(tetrahydrofuran-2-ylme-thyl)prop-2-enamid |
| 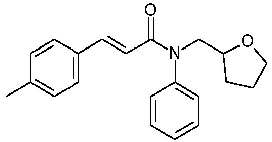 | (E)-N-phenyl-3-(p-tolyl)-N-(tetra-hyd rofu ran-2-ylmethyl)prop-2-enamid |
| 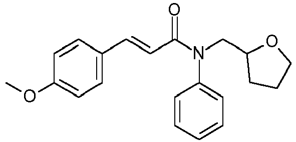 | (E)-3-(4-methoxyphenyl)-N-phenyl-N-(tetrahydrofuran-2-ylme-thyl)prop-2-enamid |
| 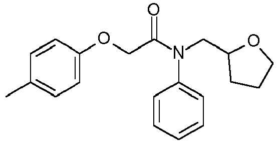 | 2-(4-methylphenoxy)-N-phenyl-N-(tetrahydrofuran-2-ylmethyl)acetamid |
| 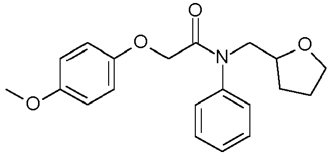 | 2-(4-methoxyphenoxy)-N-phenyl-N-(tetrahydrofuran-2-ylmethyl)acetamid |
| 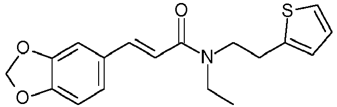 | (E)-3-(1,3-benzodioxol-5-yl)-N-ethyl-N-[2-(2-thienyl)ethyl]prop-2-enamid |
| 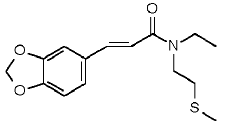 | (E)-3-(1,3-benzodioxol-5-yl)-N-ethyl-N-(2-methyl-sulfanylethyl)prop-2-enamid |
| 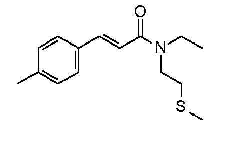 | (E)-N-ethyl-N-(2-methyl-sulfanylethyl)-3-(p-tolyl)prop-2-enamid |
| 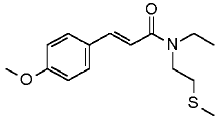 | (E)-N-ethyl-3-(4-methoxyphenyl)-N-(2-methylsulfanylethyl)prop-2-enamid |
| 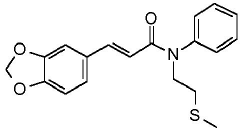 | (E)-3-(1,3-benzodioxol-5-yl)-N-(2-methylsulfanylethyl)-N-phenyl-prop-2-enamid |
| 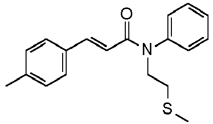 | (E)-N-(2-methylsulfanylethyl)-N-phenyl-3-(p-tolyl)prop-2-enamid |
| 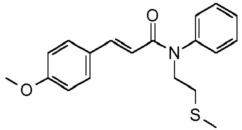 | (E)-3-(4-methoxyphenyl)-N-(2-me-thylsulfanylethyl)-N-phenyl-prop-2-enamid |
| 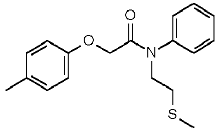 | 2-(4-methylphenoxy)-N-(2-methyl-sulfanylethyl)-N-phenyl-acetamid |
| 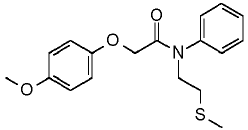 | 2-(4-methoxyphenoxy)-N-(2-me-thylsulfanylethyl)-N-phenyl-acetamid |
| 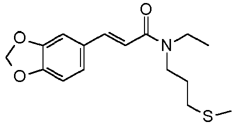 | (E)-3-(1,3-benzodioxol-5-yl)-N-ethyl-N-(3-methylsulfanylpro-pyl)prop-2-enamid |
| 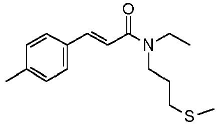 | (E)-N-ethyl-N-(3-methyl-sulfanylpropyl)-3-(p-tolyl)prop-2-enamid |
| 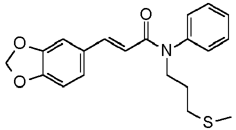 | (E)-3-(1,3-benzodioxol-5-yl)-N-(3-methylsulfanylpropyl)-N-phenyl-prop-2-enamid |
| 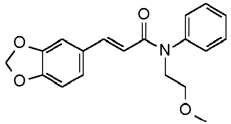 | (E)-3-(1,3-benzodioxol-5-yl)-N-(2-methoxyethyl)-N-phenyl-prop-2-enamid |
| 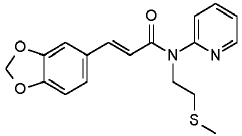 | (E)-3-(1,3-benzodioxol-5-yl)-N-(2-methylsulfanylethyl)-N-(2-pyridyl)prop-2-enamid |
| 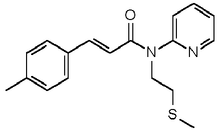 | (E)-N-(2-methylsulfanylethyl)-3-(p-tolyl)-N-(2-pyridyl)prop-2-enamid |
| 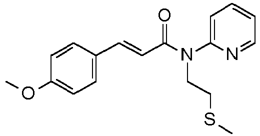 | (E)-3-(4-methoxyphenyl)-N-(2-me-thylsulfanylethyl)-N-(2-pyridyl)prop-2-enamid |
| 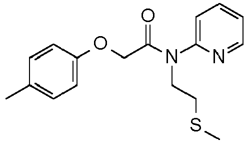 | 2-(4-methylphenoxy)-N-(2-methyl-sulfanylethyl)-N-(2-pyridyl)acetamid |
| 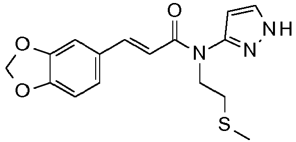 | (E)-3-(1,3-benzodioxol-5-yl)-N-(2-methylsulfanylethyl)-N-(1H-pyrazol-3-yl)prop-2-enamid |
| 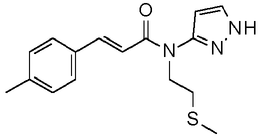 | (E)-N-(2-methylsulfanylethyl)-3-(p-tolyl)-N-(1H-pyrazol-3-yl)prop-2-enamid |
| 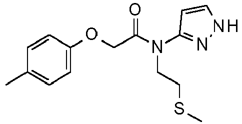 | 2-(4-methylphenoxy)-N-(2-methyl-sulfanylethyl)-N-(1H-pyrazol-3-yl)acetamid |
| 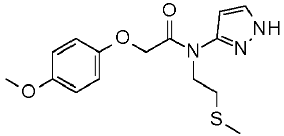 | 2-(4-methoxyphenoxy)-N-(2-me-thylsulfanylethyl)-N-(1H-pyrazol-3-yl)acetamid |

3. Verfahren nach Anspruch 1 oder 2, wobei der Rezeptor mit wenigstens einem Modulator in Kontakt gebracht wird, welcher in einem zellulären Aktivitätstest unter Verwendung von Zellen, welche den humanen TRPM8-Rezeptor rekombinant exprimieren, die Permeabilität dieser Zellen für Ca²⁺ Ionen modulieren.

4. Verfahren nach Anspruch 1, 2 oder 3, wobei der Modulator auf die zelluläre Ca²⁺ lonen-Permeabilität agonistisch oder antagonistisch wirkt.

5. Verfahren nach einem der Ansprüche 1 bis 4, wobei der Modulator ein TRPM8-Rezeptor-Agonist ist.

6. Verwendung eines Modulators gemäß der Definition in Anspruch 5 zur nicht-therapeutischen Induktion von Kältegefühl bei Mensch und/oder Tier.

7. Verwendung eines Modulators gemäß der Definition in Anspruch 5 nach Anspruch 6 zur nicht-therapeutischen Induktion von Kältegefühl durch eine den Modulator enthaltende Verpackung oder ein den Modulator enthaltendes Textil.

8. Verwendung nach Anspruch 6, wobei ein Mittel eingesetzt wird, umfassend wenigstens eine, zwei, drei oder mehr der Modulatoren gemäß der Definition in Anspruch 5, vorzugsweise in einer (Gesamt-)Menge von 0,1 ppm bis 10 Gew.-%, bezogen auf das Gesamtgewicht des Mittels, zum Erzielen einer Kühlwirkung auf Haut oder Schleimhaut, die vorzugsweise verglichen mit der Kühlwirkung eines Mittels gleicher Zusammensetzung, bei dem lediglich der/die Modulatoren gegen Menthancarbonsäure-N-ethylamid in gleicher Konzentration ausgetauscht ist/sind, verlängert ist, vorzugsweise um wenigstens 10 Minuten verlängert ist, und/oder verglichen mit der Kühlwirkung eines Mittels gleicher Zusammensetzung, bei dem lediglich der/die Modulatoren gegen FEMA 4809 oder FEMA 4496 in gleicher Konzentration ausgetauscht ist/sind, früher einsetzt.

9. Verwendung nach Anspruch 7 oder 8, wobei der/die Modulator(en) ausgewählt ist / sind aus Gruppe A wie in Anspruch 2 definiert.

10. Modulator gemäß der Definition nach einem der Ansprüche 1 bis 5 zur Verwendung als Modulator des TRPM8 Rezeptors, vorzugsweise zur Anwendung in einem therapeutischen Verfahren zur Modulation des TRPM8 Rezeptors.

11. Verbindung ausgewählt aus der Gruppe A, bestehend aus den folgenden Verbindungen:
| Struktur | Name |
|---|---|
| 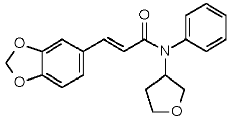 | (E)-3-(1,3-benzodioxol-5-yl)-N-phenyl-N-tetrahydrofuran-3-yl-prop-2-enamid |
| 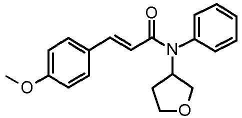 | (E)-3-(4-Methoxyphenyl)-N-phenyl-N-tetrahydrofuran-3-yl-prop-2-enamid |
| 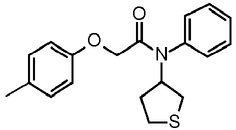 | 2-(4-Methylphenoxy)-N-phenyl-N-tetrahydrothiophen-3-yl-acetamid |
| 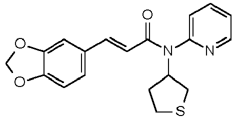 | (E)-3-(1,3-benzodioxol-5-yl)-N-(2-pyridyl)-N-tetrahydrothiophen-3-yl-prop-2-enamid |
| 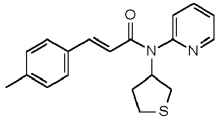 | (E)-3-(p-tolyl)-N-(2-pyridyl)-N-tet-rahydrothiophen-3-yl-prop-2-enamid |
| 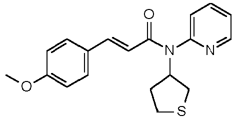 | (E)-3-(4-methoxyphenyl)-N-(2-pyridyl)-N-tetrahydrothiophen-3-yl-prop-2-enamid |
| 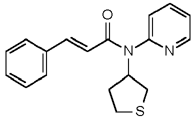 | (E)-3-phenyl-N-(2-pyridyl)-N-tetra-hydrothiophen-3-yl-prop-2-enamid |
| 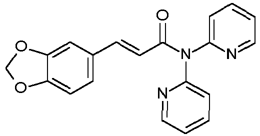 | (E)-3-(1,3-Benzodioxol-5-yl)-N,N-bis(2-pyridyl)prop-2-enamid |
| 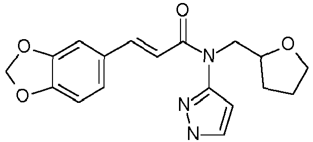 | (E)-3-(1,3-benzodioxol-5-yl)-N-(1H-pyrazol-3-yl)-N-(tetrahydrofu-ran-2-ylmethyl)prop-2-enamid |
| 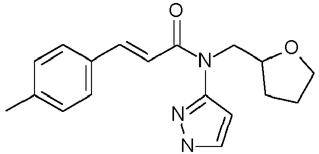 | (E)-3-(p-tolyl)-N-(1H-pyrazol-3-yl)-N-(tetrahydrofuran-2-ylme-thyl)prop-2-enamid |
| 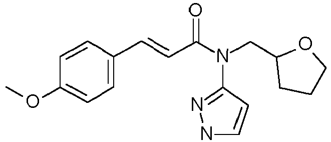 | (E)-3-(4-methoxyphenyl)-N-(1H-pyrazol-3-yl)-N-(tetrahydrofuran-2-ylmethyl)prop-2-enamid |
| 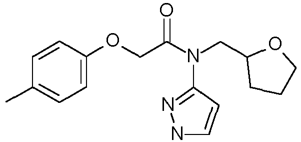 | 2-(4-methylphenoxy)-N-(1H-pyrazol-3-yl)-N-(tetrahydrofuran-2-ylmethyl)acetamid |
| 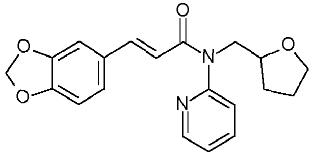 | (E)-3-(1,3-benzodioxol-5-yl)-N-(2-pyridyl)-N-(tetrahydrofuran-2-ylmethyl)prop-2-enamid |
| 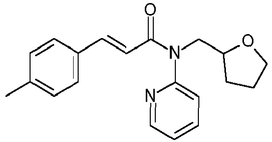 | (E)-3-(p-tolyl)-N-(2-pyridyl)-N-(tet-rahyd rofu ran-2-ylmethyl)prop-2-enamid |
| 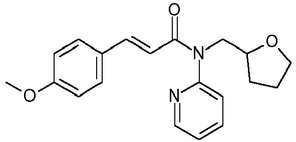 | (E)-3-(4-methoxyphenyl)-N-(2-pyridyl)-N-(tetrahydrofuran-2-ylmethyl)prop-2-enamid |
| 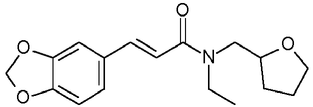 | (E)-3-(1,3-benzodioxol-5-yl)-N-ethyl-N-(tetrahydrofuran-2-ylmethyl)prop-2-enamid |
| 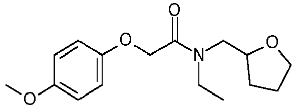 | N-ethyl-2-(4-methoxyphenoxy)-N-(tetrahydrofuran-2-ylmethyl)acetamid |
| 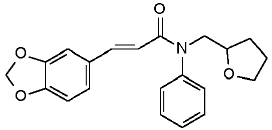 | (E)-3-(1,3-benzodioxol-5-yl)-N-phenyl-N-(tetrahydrofuran-2-ylmethyl)prop-2-enamid |
| 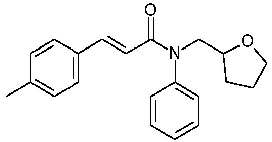 | (E)-N-phenyl-3-(p-tolyl)-N-(tetrahyd rofu ran-2-ylmethyl)prop-2-enamid |
| 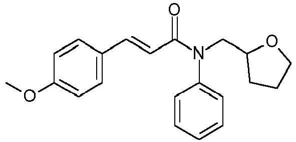 | (E)-3-(4-methoxyphenyl)-N-phenyl-N-(tetrahydrofuran-2-ylmethyl)prop-2-enamid |
| 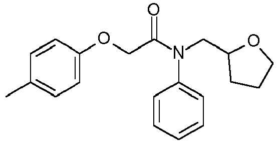 | 2-(4-methylphenoxy)-N-phenyl-N-(tetrahydrofuran-2-ylmethyl)acetamid |
| 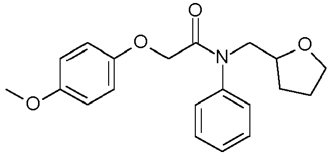 | 2-(4-methoxyphenoxy)-N-phenyl-N-(tetrahydrofuran-2-ylmethyl)acetamid |
| 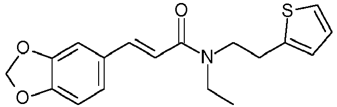 | (E)-3-(1,3-benzodioxol-5-yl)-N-ethyl-N-[2-(2-thienyl)ethyl]prop-2-enamid |
| 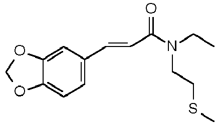 | (E)-3-(1,3-benzodioxol-5-yl)-N-ethyl-N-(2-methyl-sulfanylethyl)prop-2-enamid |
| 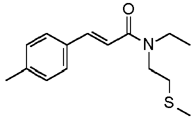 | (E)-N-ethyl-N-(2-methyl-sulfanylethyl)-3-(p-tolyl)prop-2-enamid |
| 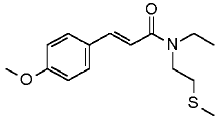 | (E)-N-ethyl-3-(4-methoxyphenyl)-N-(2-methylsulfanylethyl)prop-2-enamid |
| 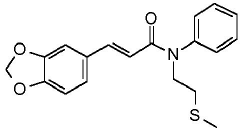 | (E)-3-(1,3-benzodioxol-5-yl)-N-(2-methylsulfanylethyl)-N-phenyl-prop-2-enamid |
| 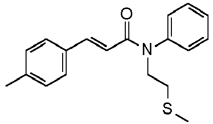 | (E)-N-(2-methylsulfanylethyl)-N-phenyl-3-(p-tolyl)prop-2-enamid |
| 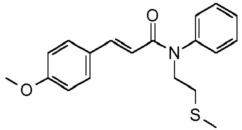 | (E)-3-(4-methoxyphenyl)-N-(2-methylsulfanylethyl)-N-phenyl-prop-2-enamid |
| 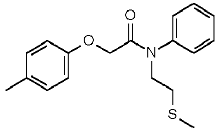 | 2-(4-methylphenoxy)-N-(2-methyl-sulfanylethyl)-N-phenyl-acetamid |
| 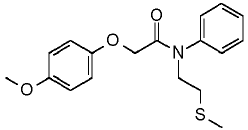 | 2-(4-methoxyphenoxy)-N-(2-methylsulfanylethyl)-N-phenyl-acetamid |
| 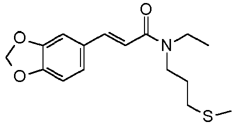 | (E)-3-(1,3-benzodioxol-5-yl)-N-ethyl-N-(3-methylsulfanylpropyl)prop-2-enamid |
| 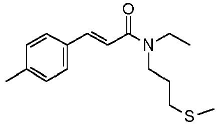 | (E)-N-ethyl-N-(3-methyl-sulfanylpropyl)-3-(p-tolyl)prop-2-enamid |
| 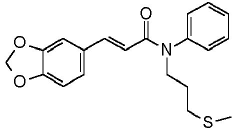 | (E)-3-(1,3-benzodioxol-5-yl)-N-(3-methylsulfanylpropyl)-N-phenyl-prop-2-enamid |
| 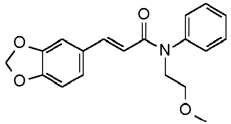 | (E)-3-(1,3-benzodioxol-5-yl)-N-(2-methoxyethyl)-N-phenyl-prop-2-enamid |
| 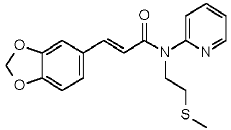 | (E)-3-(1,3-benzodioxol-5-yl)-N-(2-methylsulfanylethyl)-N-(2-pyridyl)prop-2-enamid |
| 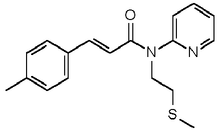 | (E)-N-(2-methylsulfanylethyl)-3-(p-tolyl)-N-(2-pyridyl)prop-2-enamid |
| 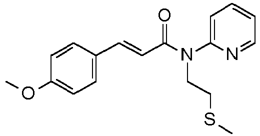 | (E)-3-(4-methoxyphenyl)-N-(2-me-thylsulfanylethyl)-N-(2-pyridyl)prop-2-enamid |
| 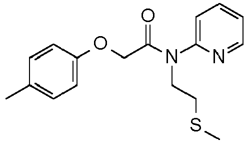 | 2-(4-methylphenoxy)-N-(2-methyl-sulfanylethyl)-N-(2-pyridyl)acetamid |
| 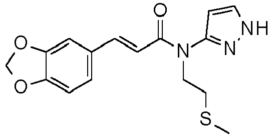 | (E)-3-(1,3-benzodioxol-5-yl)-N-(2-methylsulfanylethyl)-N-(1H-pyrazol-3-yl)prop-2-enamid |
| 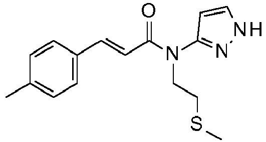 | (E)-N-(2-methylsulfanylethyl)-3-(p-tolyl)-N-(1H-pyrazol-3-yl)prop-2-enamid |
| 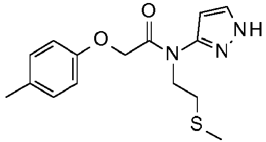 | 2-(4-methylphenoxy)-N-(2-methylsulfanylethyl)-N-(1H-pyrazol-3-yl)acetamid |
| 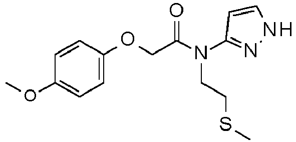 | 2-(4-methoxyphenoxy)-N-(2-methylsulfanylethyl)-N-(1H-pyrazol-3-yl)acetamid |
oder Salz einer solchen Verbindung, insbesondere ausgewählt aus Säureadditionssalzen mit anorganischen oder insbesondere organischen, ein- oder insbesondere mehrwertigen Carbonsäuren.

12. Mittel, umfassend eine Verbindung nach Anspruch 11.

13. Mittel enthaltend wenigstens einen Modulator gemäß der Definition nach einem der Ansprüche 1 bis 5 zur Verwendung als Modulator des TRPM8 Rezeptors, vorzugsweise zur Anwendung in einem therapeutischen Verfahren zur Modulation des TRPM8 Rezeptors.

14. Mittel nach Anspruch 13 zur Verwendung als Modulator des TRPM8 Rezeptors, vorzugsweise zur Anwendung in einem therapeutischen Verfahren zur Modulation des TRPM8 Rezeptors, oder Mittel nach Anspruch 12, wobei das Mittel ausgewählt ist aus der Gruppe bestehend aus
a) Nahrungsmitteln, vorzugsweise Speiseeis, Mousse, Creme, Getränke und Süßwaren,
b) Mundpflegemitteln, vorzugsweise Zahnpasta, Mundwasser und Kaugummi,
c) Körperpflegemitteln, vorzugsweise Haut- oder Haarpflegemitteln, zum Beispiel Sonnencreme, Sonnenbrandcreme, Lotionen und Shampoos, und Pflaster,
d) Schäumen und Gelen.

15. Mittel nach Anspruch 13 oder 14 zur Verwendung als Modulator des TRPM8 Rezeptors, vorzugsweise zur Anwendung in einem therapeutischen Verfahren zur Modulation des TRPM8 Rezeptors, oder Mittel nach Anspruch 12, wobei das Mittel ausgewählt ist aus der Gruppe bestehend aus Aromamischungen und der Ernährung, der Mundhygiene oder dem Genuss dienenden oder kosmetischen Zubereitungen, umfassend eine, zwei, drei oder mehr der Modulatoren gemäß der Definition aus einem der Ansprüche 1 bis 5, wobei diese(r) Modulator(en) in einer (Gesamt-)Menge von 0,05 ppm bis 10 Gew.-%, vorzugsweise von 0,1 ppm bis 10 Gew.-%, bezogen auf das Gesamtgewicht der Aromamischung oder Zubereitung, enthalten ist bzw. sind.

16. Mittel nach Anspruch 13, 14 oder 15 zur Verwendung als Modulator des TRPM8 Rezeptors, vorzugsweise zur Anwendung in einem therapeutischen Verfahren zur Modulation des TRPM8 Rezeptors, oder Mittel nach Anspruch 12, wobei das Mittel zudem umfasst:
(1) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
und/oder
(2) einen oder mehrere Aromastoffe ohne physiologische Kühlwirkung
und/oder
(3) einen oder mehrere trigeminal oder mundwässernd wirksame Stoffe ohne
physiologische Kühlwirkung
und/oder
(4) (iii) eine oder (iv) mehrere Verbindungen, die im Falle (iv) unabhängig voneinander oder gemeinsam zusätzlich einen geschmacksmodulierenden Effekt und/oder einen trigeminalen und/oder einen mundwässernden Reiz verursachen.

17. Verwendung nach Anspruch 6 zur nicht-therapeutischen Induktion von Kältegefühl bei Mensch und/oder Tier zum Erreichen einer physiologischen Kühlwirkung auf der Haut und/oder Schleimhaut, die verglichen mit der Kühlwirkung eines Mittels gleicher Zusammensetzung, bei dem lediglich der/die Modulator(en) gegen Menthancarbonsäure-N-ethylamid in gleicher Konzentration ausgetauscht ist/sind, um wenigstens 10 Minuten verlängert ist, und/oder verglichen mit der Kühlwirkung eines Mittels gleicher Zusammensetzung, bei dem lediglich der/die Modulatoren gegen FEMA 4809 oder FEMA 4496 in gleicher Konzentration ausgetauscht ist/sind, früher einsetzend ist, mit folgendem Schritt:
- Applizieren einer zum Erreichen einer physiologischen Kühlwirkung ausreichenden Menge eines Mittels wie in einem der Ansprüche 12 bis 15 definiert auf die Haut und/oder Schleimhaut.

18. Verwendung nach Anspruch 17, wobei der/die Modulator(en) ausgewählt ist/sind aus der Gruppe A wie in Anspruch 2 definiert.

## Claims

1. A non-therapeutic method of modulation, preferably of *in vitro* and/or *in vivo* modulation, of the cold menthol receptor TRPM8, wherein the receptor is contacted with at least one modulator selected from the group consisting of compounds of the following structure type 1: 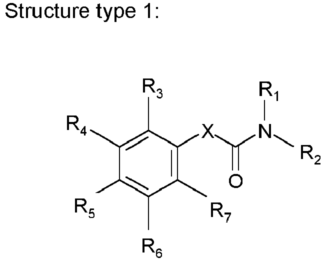 in which
R₁ consists of the following structure: 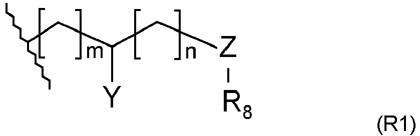 in which
Z is selected from the group consisting of O and S;
n is 0 or 1;
m
- is 1 and Y is hydrogen and R8 is a linear or branched C₁ to C₅ alkyl residue, preferably a C₅ alkyl residue, or Y is NR₉ or CR₉ and R₉ together with R₈ forms a partly or fully saturated 5- or 6-membered ring, or
- is 0, 2 or 3 and Y is hydrogen and R₈ is a linear or branched C₁ to C₅ alkyl residue, preferably a C₅ alkyl residue, or Y is NR₉ or CR₉ and R₉ together with R₈ forms an aromatic, partly or fully saturated 5- or 6-membered ring;
R₂ is a linear or branched C₁ to C₅ alkyl residue, a 5- or 6-membered cycloalkyl residue or a 5- or 6-membered aryl residue, wherein the alkyl residue, the cycloalkyl residue or the aryl residue optionally includes one or two (ring) heteroatoms;
R₃, R₄, R₅, R₆ and R₇ are the same or different and are selected from the group consisting of
hydrogen;
halogens;
linear or branched C₁- to C₆-alkyl groups optionally bearing 1, 2, 3 or 4 identical or different substituents selected from the group consisting of NH₂, OH, SH, halogens or linear or branched C₁- to C₆-alkyl groups;
linear or branched C₁- to C₆-alkoxy groups optionally bearing 1, 2, 3 or 4 identical or different substituents selected from the group consisting of NH₂, OH, SH, halogens or C₁- to C₆-alkoxy groups;
mono- or polycyclic aryl, arylalkyl and heteroaryl groups optionally bearing 1, 2, 3 or 4 identical or different substituents selected from the group consisting of NH₂, OH, SH, halogens, linear or branched C₁- to C₆-alkyl groups; wherein the heteroaryl groups have 1, 2, 3 or 4 ring heteroatoms that are the same or different and are selected from the group consisting of O, N and S, or
two adjacent R₃, R₄, R₅, R₆ and R₇ residues together with the carbon atoms to which they are bonded form a 4-, 5-, 6- or 7-membered, mono- or polyunsaturated heterocyclic ring optionally bearing 1, 2, 3, 4 or 5 identical or different substituents selected from the group consisting of linear or branched C₁- to C₆-alkyl groups, and having 1, 2 or 3 ring heteroatoms that are the same or different and are selected from the group consisting of O, N and S;
X
- is selected from the group consisting of
-C₁- to C₄-alkylene groups; -C₂- to C₄-alkenylene groups, and -A-C₁- to C₄- or -C₁- to C₄-A-alkylene groups or -A-C₂- to C₄- or -C₂- to C₄-A-alkenylene groups, in which A is O, S or NH; or
- is a chemical single bond;
and salts of these compounds, particularly acid addition salts with inorganic or especially organic, mono- and especially polybasic carboxylic acids;
and optionally of these compounds in stereoisomerically pure form or mixtures of stereoisomers thereof.

2. The method according to claim 1, wherein the compounds of structure type 1 are selected from the group A consisting of the following compounds:
| Structure | Name |
|---|---|
| 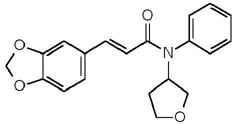 | (E)-3-(1,3-benzodioxol-5-yl)-N-phenyl-N-tetrahydrofuran-3-yl-prop-2-enamide |
| 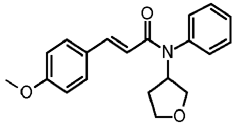 | (E)-3-(4-methoxyphenyl)-N-phenyl-N-tetrahydrofuran-3-yl-prop-2-enamide |
| 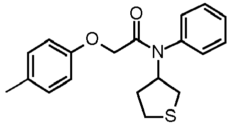 | 2-(4-methylphenoxy)-N-phenyl-N-tetrahydrothiophen-3-ylacetamide |
| 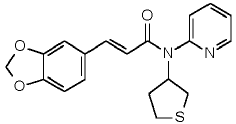 | (E)-3-(1,3-benzodioxol-5-yl)-N-(2-pyridyl)-N-tetrahydrothiophen-3-yl-prop-2-enamide |
| 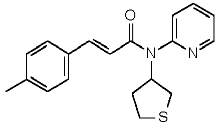 | (E)-3-(p-tolyl)-N-(2-pyridyl)-N-tet-rahydrothiophen-3-ylprop-2-enamide |
| 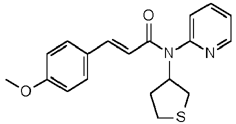 | (E)-3-(4-methoxyphenyl)-N-(2-pyridyl)-N-tetrahydrothiophen-3-yl-prop-2-enamide |
| 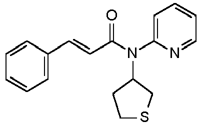 | (E)-3-phenyl-N-(2-pyridyl)-N-tetra-hydrothiophen-3-ylprop-2-enamide |
| 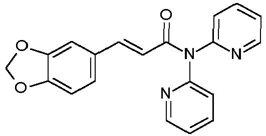 | (E)-3-(1,3-benzodioxol-5-yl)-N,N-bis(2-pyridyl)prop-2-enamide |
| 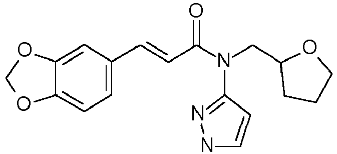 | (E)-3-(1,3-benzodioxol-5-yl)-N-(1H-pyrazol-3-yl)-N-(tetrahydrofuran-2-ylmethyl)prop-2-enamide |
| 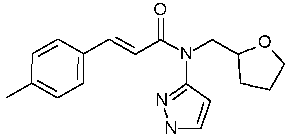 | (E)-3-(p-tolyl)-N-(1H-pyrazol-3-yl)-N-(tetrahydrofuran-2-ylme-thyl)prop-2-enamide |
| 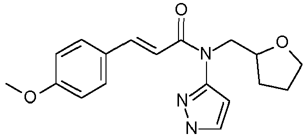 | (E)-3-(4-methoxyphenyl)-N-(1H-pyrazol-3-yl)-N-(tetrahydrofuran-2-ylmethyl)prop-2-enamide |
| 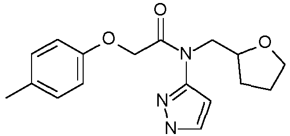 | 2-(4-methylphenoxy)-N-(1H-pyra-zol-3-yl)-N-(tetrahydrofuran-2-ylmethyl)acetamide |
| 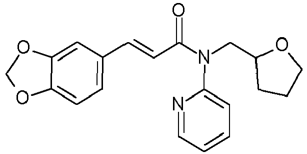 | (E)-3-(1,3-benzodioxol-5-yl)-N-(2-pyridyl)-N-(tetrahydrofuran-2-ylmethyl)prop-2-enamide |
| 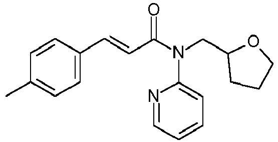 | (E)-3-(p-tolyl)-N-(2-pyridyl)-N-(tetrahydrofuran-2-ylmethyl)prop-2-enamide |
| 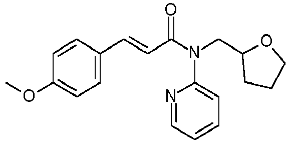 | (E)-3-(4-methoxyphenyl)-N-(2-pyridyl)-N-(tetrahydrofuran-2-ylmethyl)prop-2-enamide |
| 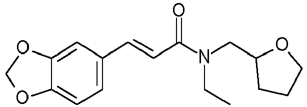 | (E)-3-(1,3-benzodioxol-5-yl)-N-ethyl-N-(tetrahydrofuran-2-ylmethyl)prop-2-enamide |
| 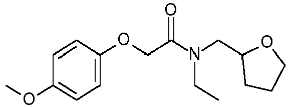 | N-ethyl-2-(4-methoxyphenoxy)-N-(tetrahydrofuran-2-ylmethyl)acetamide |
| 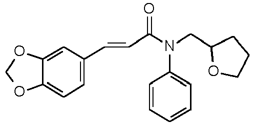 | (E)-3-(1,3-benzodioxol-5-yl)-N-phenyl-N-(tetrahydrofuran-2-ylmethyl)prop-2-enamide |
| 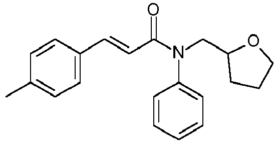 | (E)-N-phenyl-3-(p-tolyl)-N-(tetrahydrofuran-2-ylmethyl)prop-2-enamide |
| 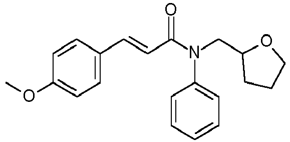 | (E)-3-(4-methoxyphenyl)-N-phenyl-N-(tetrahydrofuran-2-ylmethyl)prop-2-enamide |
| 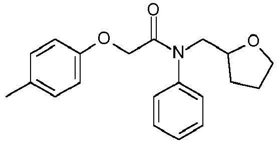 | 2-(4-methylphenoxy)-N-phenyl-N-(tetrahydrofuran-2-ylmethyl)acetamide |
| 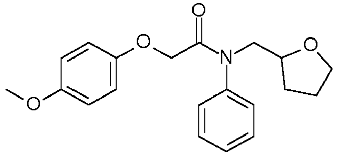 | 2-(4-methoxyphenoxy)-N-phenyl-N-(tetrahydrofuran-2-ylmethyl)acetamide |
| 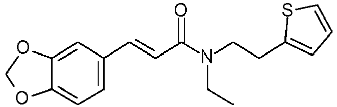 | (E)-3-(1,3-benzodioxol-5-yl)-N-ethyl-N-[2-(2-thienyl)ethyl]prop-2-enamide |
| 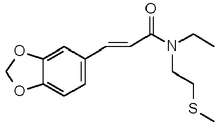 | (E)-3-(1,3-benzodioxol-5-yl)-N-ethyl-N-(2-methyl-sulfanylethyl)prop-2-enamide |
| 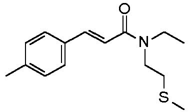 | (E)-N-ethyl-N-(2-methyl-sulfanylethyl)-3-(p-tolyl)prop-2-enamide |
| 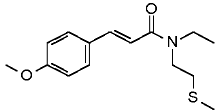 | (E)-N-ethyl-3-(4-methoxyphenyl)-N-(2-methylsulfanylethyl)prop-2-enamide |
| 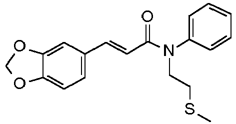 | (E)-3-(1,3-benzodioxol-5-yl)-N-(2-methylsulfanylethyl)-N-phenyl-prop-2-enamide |
| 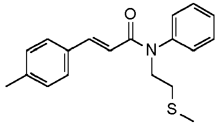 | (E)-N-(2-methylsulfanylethyl)-N-phenyl-3-(p-tolyl)prop-2-enamide |
| 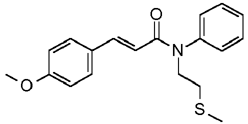 | (E)-3-(4-methoxyphenyl)-N-(2-methylsulfanylethyl)-N-phenyl-prop-2-enamide |
| 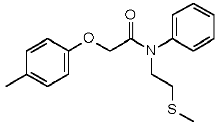 | 2-(4-methylphenoxy)-N-(2-methyl-sulfanylethyl)-N-phenyl-acetamide |
| 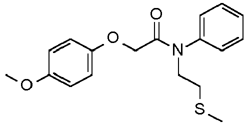 | 2-(4-methoxyphenoxy)-N-(2-methylsulfanylethyl)-N-phenyl-acetamide |
| 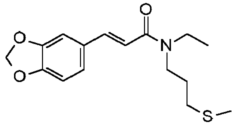 | (E)-3-(1,3-benzodioxol-5-yl)-N-ethyl-N-(3-methylsulfanylpropyl)prop-2-enamide |
| 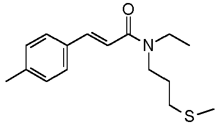 | (E)-N-ethyl-N-(3-methyl-sulfanylpropyl)-3-(p-tolyl)prop-2-enamide |
| 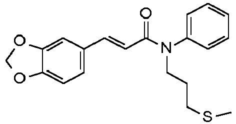 | (E)-3-(1,3-benzodioxol-5-yl)-N-(3-methylsulfanylpropyl)-N-phenyl-prop-2-enamide |
| 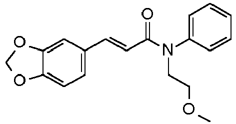 | (E)-3-(1,3-benzodioxol-5-yl)-N-(2-methoxyethyl)-N-phenyl-prop-2-enamide |
| 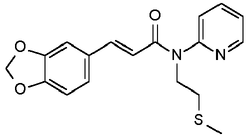 | (E)-3-(1,3-benzodioxol-5-yl)-N-(2-methylsulfanylethyl)-N-(2-pyridyl)prop-2-enamide |
| 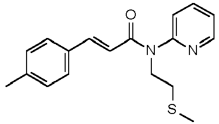 | (E)-N-(2-methylsulfanylethyl)-3-(p-tolyl)-N-(2-pyridyl)prop-2-enamide |
| 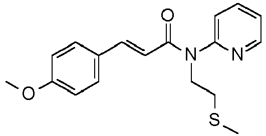 | (E)-3-(4-methoxyphenyl)-N-(2-methylsulfanylethyl)-N-(2-pyridyl)prop-2-enamide |
| 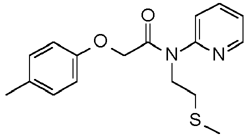 | 2-(4-methylphenoxy)-N-(2-methyl-sulfanylethyl)-N-(2-pyridyl)acetamide |
| 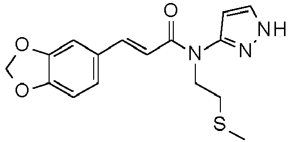 | (E)-3-(1,3-benzodioxol-5-yl)-N-(2-methylsulfanylethyl)-N-(1H-pyrazol-3-yl)prop-2-enamide |
| 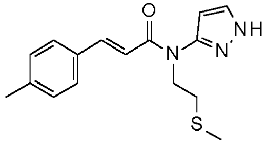 | (E)-N-(2-methylsulfanylethyl)-3-(p-tolyl)-N-(1H-pyrazol-3-yl)prop-2-enamide |
| 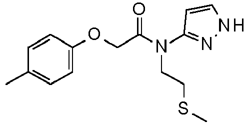 | 2-(4-methylphenoxy)-N-(2-methyl-sulfanylethyl)-N-(1H-pyrazol-3-yl)acetamide |
| 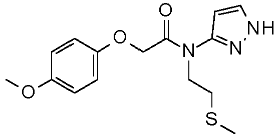 | 2-(4-methoxyphenoxy)-N-(2-methylsulfanylethyl)-N-(1H-pyrazol-3-yl)acetamide |

3. The method according to claim 1 or 2, wherein the receptor is contacted with at least one modulator which, in a cellular activity test using cells that recombinantly express the human TRPM8 receptor, modulates the permeability of these cells for Ca²⁺ ions.

4. The method according to claim 1, 2 or 3, wherein the modulator has an agonistic or antagonistic effect on the cellular Ca²⁺ ion permeability.

5. The method according to any of claims 1 to 4, wherein the modulator is a TRPM8 receptor agonist.

6. The use of a modulator as defined in claim 5 for non-therapeutic induction of a cold sensation in man and/or animals.

7. The use of a modulator as defined in claim 5 according to claim 6 for non-therapeutic induction of a cold sensation by a packing or textile comprising the modulator.

8. The use according to claim 6, wherein a composition comprising at least one, two, three or more of the modulators as defined in claim 5, preferably in a (total) amount of 0.1 ppm to 10% by weight, based on the total weight of the composition, is used to achieve a cooling effect on the skin or mucosa that has preferably been prolonged compared to the cooling effect of a composition of the same constitution in which the modulator(s) has/have merely been exchanged for N-ethyl menthane carboxamide in the same concentration, preferably by at least 10 minutes, and/or a cooling effect that sets in earlier compared to the cooling effect of a composition of the same constitution in which the modulator(s) has/have merely been exchanged for FEMA 4809 or FEMA 4496 in the same concentration.

9. The use according to claim 7 or 8, wherein the modulator(s) is/are selected from group A as defined in claim 2.

10. A modulator as defined in any of claims 1 to 5 for use as a modulator of the TRPM8 receptor, preferably for use in a therapeutic method of modulating the TRPM8 receptor.

11. A compound selected from the group A consisting of the following compounds:
| Structure | Name |
|---|---|
| 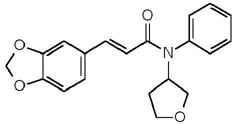 | (E)-3-(1,3-benzodioxol-5-yl)-N-phenyl-N-tetrahydrofuran-3-yl-prop-2-enamide |
| 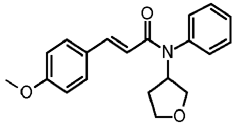 | (E)-3-(4-methoxyphenyl)-N-phenyl-N-tetrahydrofuran-3-yl-prop-2-enamide |
| 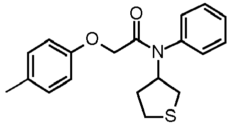 | 2-(4-methylphenoxy)-N-phenyl-N-tetrahydrothiophen-3-ylacetamide |
| 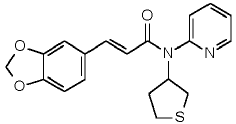 | (E)-3-(1,3-benzodioxol-5-yl)-N-(2-pyridyl)-N-tetrahydrothiophen-3-yl-prop-2-enamide |
| 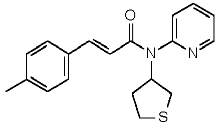 | (E)-3-(p-tolyl)-N-(2-pyridyl)-N-tetrahydrothiophen-3-ylprop-2-enamide |
| 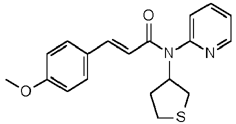 | (E)-3-(4-methoxyphenyl)-N-(2-pyridyl)-N-tetrahydrothiophen-3-yl-prop-2-enamide |
| 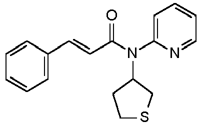 | (E)-3-phenyl-N-(2-pyridyl)-N-tetra-hydrothiophen-3-ylprop-2-enamide |
| 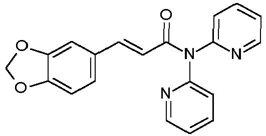 | (E)-3-(1,3-benzodioxol-5-yl)-N,N-bis(2-pyridyl)prop-2-enamide |
| 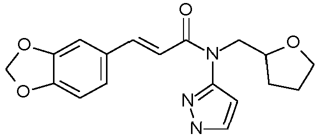 | (E)-3-(1,3-benzodioxol-5-yl)-N-(1H-pyrazol-3-yl)-N-(tetrahydrofuran-2-ylmethyl)prop-2-enamide |
| 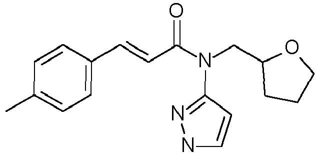 | (E)-3-(p-tolyl)-N-(1H-pyrazol-3-yl)-N-(tetrahydrofuran-2-ylmethyl)prop-2-enamide |
| 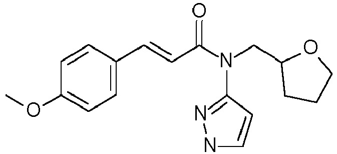 | (E)-3-(4-methoxyphenyl)-N-(1H-pyrazol-3-yl)-N-(tetrahydrofuran-2-ylmethyl)prop-2-enamide |
| 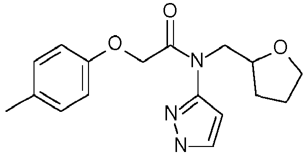 | 2-(4-methylphenoxy)-N-(1H-pyra-zol-3-yl)-N-(tetrahydrofuran-2-ylmethyl)acetamide |
| 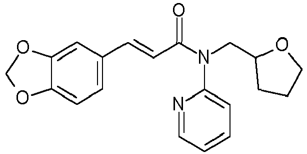 | (E)-3-(1,3-benzodioxol-5-yl)-N-(2-pyridyl)-N-(tetrahydrofuran-2-ylmethyl)prop-2-enamide |
| 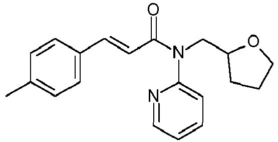 | (E)-3-(p-tolyl)-N-(2-pyridyl)-N-(tetrahydrofuran-2-ylmethyl)prop-2-enamide |
| 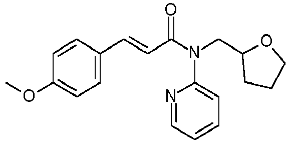 | (E)-3-(4-methoxyphenyl)-N-(2-pyridyl)-N-(tetrahydrofuran-2-ylmethyl)prop-2-enamide |
| 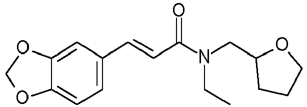 | (E)-3-(1,3-benzodioxol-5-yl)-N-ethyl-N-(tetrahydrofuran-2-ylmethyl)prop-2-enamide |
| 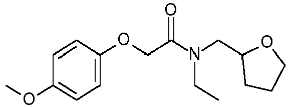 | N-ethyl-2-(4-methoxyphenoxy)-N-(tetrahydrofuran-2-ylmethyl)acetamide |
| 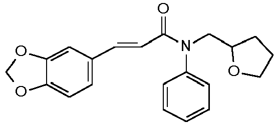 | (E)-3-(1,3-benzodioxol-5-yl)-N-phenyl-N-(tetrahydrofuran-2-ylmethyl)prop-2-enamide |
| 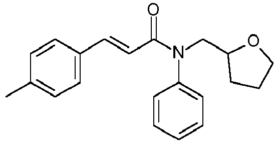 | (E)-N-phenyl-3-(p-tolyl)-N-(tetrahydrofuran-2-ylmethyl)prop-2-enamide |
| 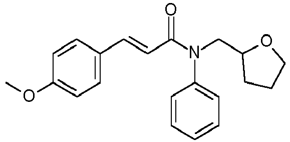 | (E)-3-(4-methoxyphenyl)-N-phenyl-N-(tetrahydrofuran-2-ylmethyl)prop-2-enamide |
| 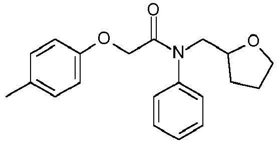 | 2-(4-methoxyphenyl)-N-phe-nyl-N-(tetrahydrofuran-2-ylme-thyl)acet-amide |
| 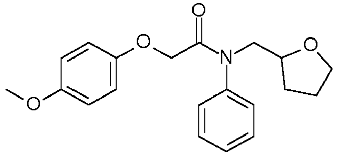 | 2-(4-methoxyphenoxy)-N-phenyl-N-(tetrahydrofuran-2-ylmethyl)acetamide |
| 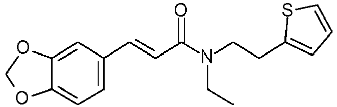 | (E)-3-(1,3-benzodioxol-5-yl)-N-ethyl-N-[2-(2-thienyl)ethyl]prop-2-enamide |
| 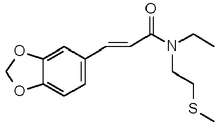 | (E)-3-(1,3-benzodioxol-5-yl)-N-ethyl-N-(2-methylsulfanylethyl)prop-2-enamide |
| 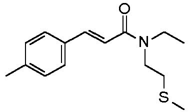 | (E)-N-ethyl-N-(2-methylsulfanylethyl)-3-(p-tolyl)prop-2-enamide |
| 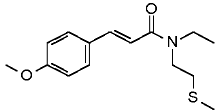 | (E)-N-ethyl-3-(4-methoxyphenyl)-N-(2-methylsulfanylethyl)prop-2-enamide |
| 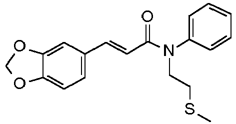 | (E)-3-(1,3-benzodioxol-5-yl)-N-(2-methylsulfanylethyl)-N-phenyl-prop-2-enamide |
| 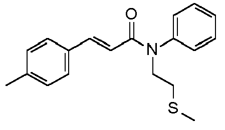 | (E)-N-(2-methylsulfanylethyl)-N-phenyl-3-(p-tolyl)prop-2-enamide |
| 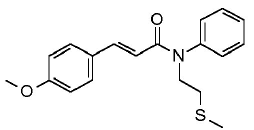 | (E)-3-(4-methoxyphenyl)-N-(2-methylsulfanylethyl)-N-phenyl-prop-2-enamide |
| 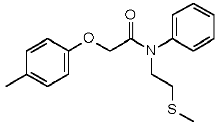 | 2-(4-methylphenoxy)-N-(2-methylsulfanylethyl)-N-phenyl-acetamide |
| 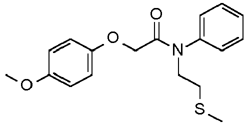 | 2-(4-methoxyphenoxy)-N-(2-methylsulfanylethyl)-N-phenyl-acetamide |
| 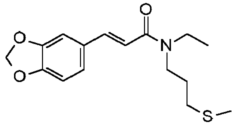 | (E)-3-(1,3-benzodioxol-5-yl)-N-ethyl-N-(3-methylsulfanylpropyl)prop-2-enamide |
| 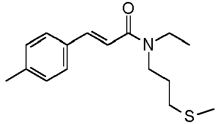 | (E)-N-ethyl-N-(3-methyl-sulfanylpropyl)-3-(p-tolyl)prop-2-enamide |
| 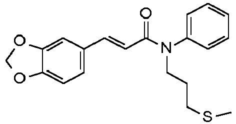 | (E)-3-(1,3-benzodioxol-5-yl)-N-(3-methylsulfanylpropyl)-N-phenyl-prop-2-enamide |
| 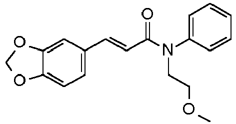 | (E)-3-(1,3-benzodioxol-5-yl)-N-(2-methoxyethyl)-N-phenyl-prop-2-enamide |
| 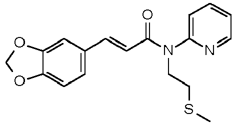 | (E)-3-(1,3-benzodioxol-5-yl)-N-(2-methylsulfanylethyl)-N-(2-pyridyl)prop-2-enamide |
| 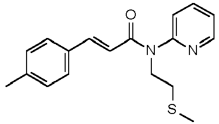 | (E)-N-(2-methylsulfanylethyl)-3-(p-tolyl)-N-(2-pyridyl)prop-2-enamide |
| 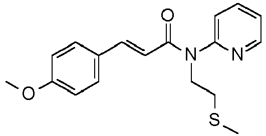 | (E)-3-(4-methoxyphenyl)-N-(2-methylsulfanylethyl)-N-(2-pyridyl)prop-2-enamide |
| 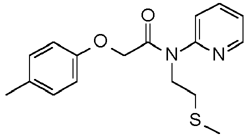 | 2-(4-methylphenoxy)-N-(2-methyl-sulfanylethyl)-N-(2-pyridyl)acetamide |
| 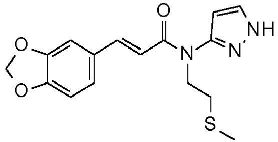 | (E)-3-(1,3-benzodioxol-5-yl)-N-(2-methylsulfanylethyl)-N-(1H-pyrazol-3-yl)prop-2-enamide |
| 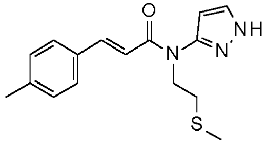 | (E)-N-(2-methylsulfanylethyl)-3-(p-tolyl)-N-(1H-pyrazol-3-yl)prop-2-enamide |
| 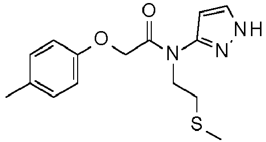 | 2-(4-methylphenoxy)-N-(2-methyl-sulfanylethyl)-N-(1H-pyrazol-3-yl)acetamide |
| 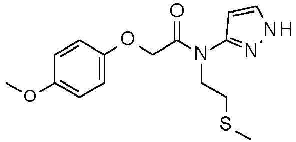 | 2-(4-methoxyphenoxy)-N-(2-me-thylsulfanylethyl)-N-(1H-pyrazol-3-yl)acetamide |
or a salt of such a compound, especially selected from acid addition salts with inorganic or especially organic, mono- or especially polybasic carboxylic acids.

12. Agent comprising a compound according to claim 11.

13. The agent comprising at least one modulator as defined in any of claims 1 to 5 for use as a modulator of the TRPM8 receptor, preferably for use in a therapeutic method of modulating the TRPM8 receptor.

14. The agent according to claim 13 for use as a modulator of the TRPM8 receptor, preferably for use in a therapeutic method of modulating the TRPM8 receptor, or agent according to claim 12, wherein the agent is selected from the group consisting of
a) foods, preferably ice cream, mousse, cream, drinks and confectionery,
b) oral care products, preferably toothpaste, mouthwash and chewing gum,
c) personal care products, preferably skincare or haircare products, for example sun cream, sunburn cream, lotions and shampoos, and patches,
d) foams and gels.

15. The agent according to claim 13 or 14 for use as a modulator of the TRPM8 receptor, preferably for use in a therapeutic method of modulating the TRPM8 receptor, or agent according to claim 12, wherein the agent is selected from the group consisting of aroma mixtures and formulations for nutrition, oral hygiene or enjoyment purposes or cosmetic formulations, comprising one, two, three or more of the modulators as defined in any of claims 1 to 5, wherein said modulator(s) is/are present in a (total) amount of 0.05 ppm to 10% by weight, preferably of 0.1 ppm to 10% by weight, based on the total weight of the aroma mixture or formulation.

16. The agent according to claim 13, 14 or 15 for use as a modulator of the TRPM8 receptor, preferably for use in a therapeutic method of modulating the TRPM8 receptor, or composition according to claim 12, wherein the composition additionally comprises:
(1) one or more further substances having physiological cooling action, wherein the further substance or one, several or all of the further substances (i) cause(s) a flavoring effect or (ii) do(es) not cause a flavoring effect,
and/or
(2) one or more aroma substances with no physiological cooling effect
and/or
(3) one or more substances having a trigeminal or mouthwatering effect with no physiological cooling effect
and/or
(4) (iii) one compound or (iv) two or more compounds which, independently or collectively additionally cause(s) a flavor-modulating effect and/or a trigeminal and/or mouthwatering stimulus.

17. The use according to claim 6 for non-therapeutic induction of a cold sensation in a human and/or animal for achieving a physiological cooling effect on the skin and/or mucosa, which has been prolonged by at least 10 minutes compared to the cooling effect of a composition of the same constitution in which the modulator(s) has/have only been exchanged for N-ethyl menthane carboxamide in the same concentration, and/or a cooling effect that sets in earlier compared to the cooling effect of a composition of the same constitution in which the modulator(s) has/have merely been exchanged for FEMA 4809 or FEMA 4496 in the same concentration, comprising the following step:
- applying an amount of a composition as defined in any of claims 12 to 15, which is sufficient for achieving a physiological cooling effect to the skin and/or mucosa.

18. The use according to claim 17, wherein the modulator(s) is/are selected from the group A as defined in claim 2.

## Revendications

1. Procédé non thérapeutique pour la modulation, de préférence pour la modulation *in vitro* et/ou *in vivo,* du récepteur activé par le froid et le menthol TRMP8, le récepteur étant mis en contact avec au moins un modulateur qui est choisi dans le groupe constitué par les composés du type de structure suivant 1 :
Type de structure 1 : 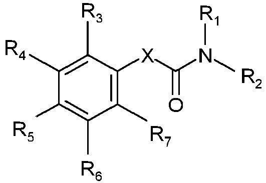
dans lequel
R₁ est constitué par la structure suivante : 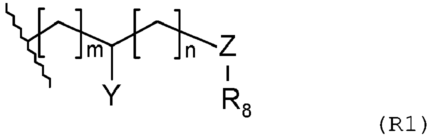
dans laquelle
Z est choisi dans le groupe constitué par O et S ;
n vaut 0 ou 1 ;
m
- vaut 1 et Y représente hydrogène et R₈ représente un radical C₁-C₅-alkyle linéaire ou ramifié, de préférence un radical C₅-alkyle, ou Y représente NR₉ ou CR₉ et R₉ forme, avec R₈, un cycle de 5 ou 6 chaînons, partiellement ou complètement saturé, ou
- vaut 0, 2 ou 3 et Y représente hydrogène et R₈ représente un radical C₁-C₅-alkyle linéaire ou ramifié, de préférence un radical C₅-alkyle, ou Y représente NR₉ ou CR₉ et R₉ forme, avec R₈, un cycle de 5 ou 6 chaînons, aromatique, partiellement ou complètement saturé,
R₂ représente un radical C₁-C₅-alkyle linéaire ou ramifié, un radical cycloalkyle de 5 ou 6 chaînons ou un radical aryle de 5 ou 6 chaînons, le radical alkyle, le radical cycloalkyle ou le radical aryle comportant éventuellement un ou deux hétéroatomes (de cycle) ;
R₃, R₄, R₅, R₆ et R₇ sont identiques ou différents et sont choisis dans le groupe constitué par
hydrogène ;
les halogènes ;
les groupes C₁-C₆-alkyle linéaires ou ramifiés, qui portent le cas échéant 1, 2, 3 ou 4 substituants identiques ou différents, qui sont choisis dans le groupe constitué par NH₂, OH, SH, halogènes ou groupes C₁-C₆-alkyle linéaires ou ramifiés ;
les groupes C₁-C₆-alcoxy linéaires ou ramifiés, qui portent le cas échéant 1, 2, 3 ou 4 substituants identiques ou différents, qui sont choisis dans le groupe constitué par NH₂, OH, SH, halogènes ou groupes C₁-C₆-alcoxy linéaires ou ramifiés ;
un ou plusieurs groupes aryle, arylalkyle et hétéroaryle à plusieurs noyaux, qui portent le cas échéant 1, 2, 3 ou 4 substituants identiques ou différents, qui sont choisis dans le groupe constitué par NH₂, OH, SH, halogènes, groupes C₁-C₆-alkyle linéaires ou ramifiés ; les groupes hétéroaryle présentant 1, 2, 3 ou 4 hétéroatomes de cycle qui sont identiques ou différents et choisis dans le groupe constitué par O, N et S, ou
deux radicaux R₃, R₄, R₅, R₆ et R₇ adjacents, conjointement avec les atomes de carbone auxquels ils sont liés, forment un cycle hétérocyclique de 4, 5, 6 ou 7 chaînons, mono-insaturé ou polyinsaturé, qui porte le cas échéant 1, 2, 3, 4 ou 5 substituants identiques ou différents, qui sont choisis dans le groupe constitué par les groupes C₁-C₆-alkyle linéaires ou ramifiés, et qui présente 1, 2 ou 3 hétéroatomes de cycle, qui sont identiques ou différents et choisis dans le groupe constitué par O, N et S ;
X
- est choisi dans le groupe constitué par :
- les groupes C₁-C₄-alkylène ; les groupes C₂-C₄-alcénylène, ainsi que les groupes -A-C₁-C₄-alkylène ou les groupes -C₁-C₄-A-alkylène ou les groupes -A-C₂-C₄-alcénylène ou les groupes -C₂-C₄-A-alcénylène, dans lesquels A représente O, S ou NH ; ou
- représente une simple liaison chimique ;
ainsi que les sels de ces composés, en particulier les sels d'addition d'acide avec des acides carboxyliques inorganiques ou en particulier organiques, monovalents ou en particulier polyvalents ;
et le cas échéant ces composés sous forme de stéréo-isomères purs ou de mélanges de stéréo-isomères correspondants.

2. Procédé selon la revendication 1, les composés du type de structure 1 étant choisis dans le groupe A constitué par les composés suivants :
| Structure | Nom |
|---|---|
| 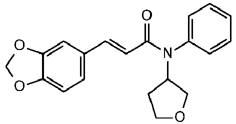 | (E)-3-(1,3-benzodioxol-5-yl)-N-phényl-N-tétrahydrofurann-3-yl-prop-2-énamide |
| 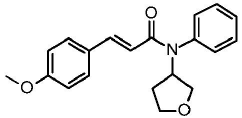 | (E)-3-(4-méthoxyphényl)-N-phényl-N-tétrahydrofurann-3-yl-prop-2-énamide |
| 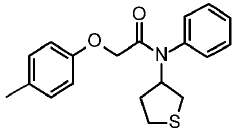 | 2-(4-méthylphénoxy)-N-phényl-tétrahydrothiophén-3-yl-acétamide |
| 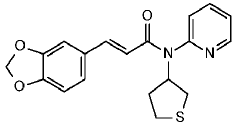 | (E)-3-(1,3-benzodioxol-5-yl)-N-(2-pyridyl)-N-tétrahydrothiophén-3-yl-prop-2-énamide |
| 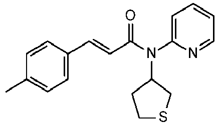 | (E)-3-(p-toluyl)-N-(2-pyridyl)-N-tétrahydrothiophén-3-yl-prop-2-énamide |
| 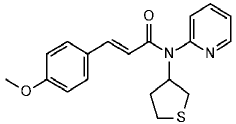 | (E)-3-(4-méthoxyphényl)-N-(2-pyridyl)-N-tétrahydrothiophén-3-yl-prop-2-énamide |
| 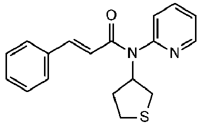 | (E)-3-phényl-N-(2-pyridyl)-N-tétrahydrothiophén-3-yl-prop-2-énamide |
| 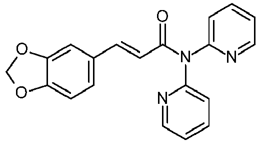 | (E)-3-(1,3-benzodioxol-5-yl)-N,N-bis(2-pyridyl)-prop-2-énamide |
| 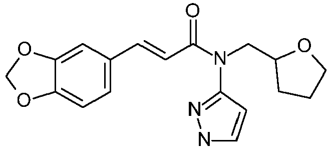 | (E)-3-(1,3-benzodioxol-5-yl)-N-(1H-pyrazol-3-yl)-N-(tétrahydrofurann-2-ylméthyl)prop-2-énamide |
| 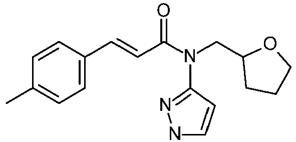 | (E)-3-(p-toluyl)-N-(1H-pyrazol-3-yl)-N-(tétrahydrofurann-2-ylméthyl)prop-2-énamide |
| 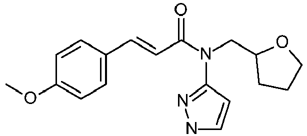 | (E)-3-(4-méthoxyphényl)-N-(1H-pyrazol-3-yl)-N-(tétrahydrofurann-2-ylméthyl)prop-2-énamide |
| 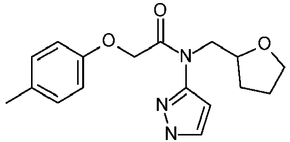 | 2-(4-méthylphénoxy)-N-(1H-pyrazol-3-yl)-N-(tétrahydrofurann-2-ylméthyl)acétamide |
| 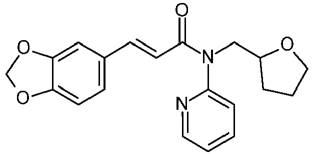 | (E)-3-(1,3-benzodioxol-5-yl)-N-(2-pyridyl)-N-(tétrahydrofurann-2-ylméthyl)prop-2-énamide |
| 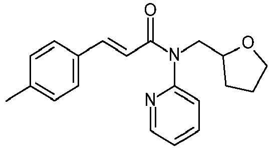 | (E)-3-(p-toluyl)-N-(2-pyridyl)-N-(tétrahydrofurann-2-ylméthyl)prop-2-énamide |
| 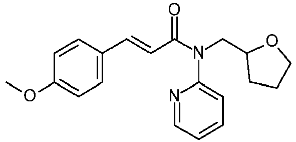 | (E)-3-(4-méthoxyphényl)-N-(2-pyridyl)-N-(tétrahydrofurann-2-ylméthyl)prop-2-énamide |
| 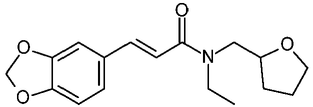 | (E)-3-(1,3-benzodioxol-5-yl)-N-éthyl-N-(tétrahydrofurann-2-ylméthyl)prop-2-énamide |
| 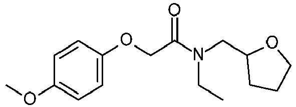 | N-éthyl-2-(4-méthoxyphénoxy)-N-(tétrahydrofurann-2-ylméthyl)acétamide |
| 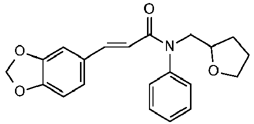 | (E)-3-(1,3-benzodioxol-5-yl)-N-phényl-N-(tétrahydrofurann-2-ylméthyl)prop-2-énamide |
| 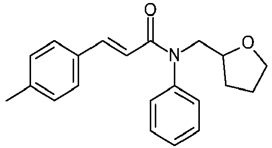 | (E)-N-phényl-3-(p-toluyl)-N-(tétrahydrofurann-2-ylméthyl)prop-2-énamide |
| 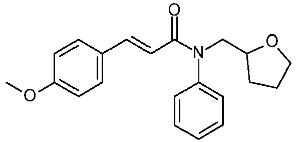 | (E)-3-(4-méthoxyphényl)-N-phényl-N-(tétrahydrofurann-2-ylméthyl)prop-2-énamide |
| 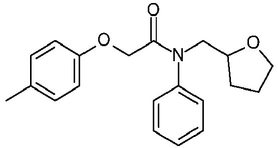 | 2-(4-méthylphénoxy)-N-phényl-N-(tétrahydrofurann-2-ylméthyl)acétamide |
| 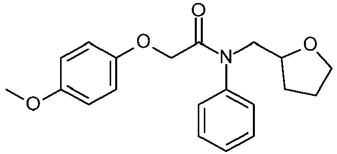 | 2-(4-méthoxyphénoxy)-N-phényl-N-(tétrahydrofurann-2-ylméthyl)acétamide |
| 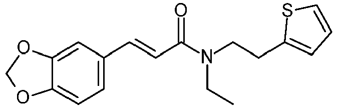 | (E)-3-(1,3-benzodioxol-5-yl)-N-éthyl-N-[2-(2-thiényl)éthyl]prop-2-énamide |
| 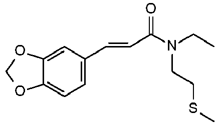 | (E)-3-(1,3-benzodioxol-5-yl)-N-éthyl-N-(2-méthylsulfanyléthyl)-prop-2-énamide |
| 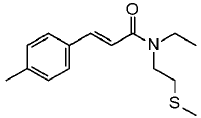 | (E)-N-éthyl-N-(2-méthylsulfanyléthyl)-3-(p-toluyl)prop-2-énamide |
| 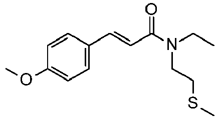 | (E)-N-éthyl-3-(4-méthoxyphényl)-N-(2-méthylsulfanyléthyl)-prop-2-énamide |
| 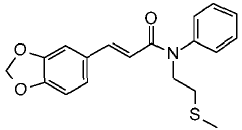 | (E)-3-(1, 3-benzodioxol-5-yl)-N-(2-méthylsulfanyléthyl)-N-phényl-prop-2-énamide |
| 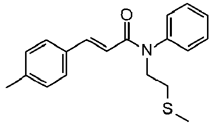 | (E)-N-(2-méthylsulfanyléthyl)-N-phényl-3-(p-toluyl)prop-2-énamide |
| 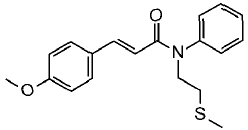 | (E)-3-(4-méthoxyphényl)-N-(2-méthylsulfanyléthyl)-N-phényl-prop-2-énamide |
| 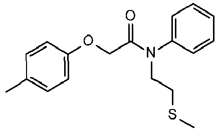 | 2-(4-méthylphénoxy)-N-(2-méthylsulfanyléthyl)-N-phényl-acétamide |
| 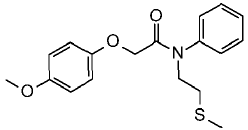 | 2-(4-méthoxyphénoxy)-N-(2-méthylsulfanyléthyl)-N-phényl-acétamide |
| 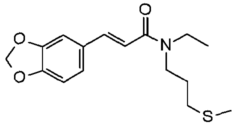 | (E)-3-(1,3-benzodioxol-5-yl)-N-éthyl-N-(3-méthylsulfanylpropyl) prop-2-énamide |
| 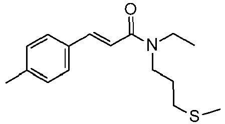 | (E)-N-éthyl-N-(3-méthylsulfanylpropyl) -3-(p-toluyl)prop-2-énamide |
| 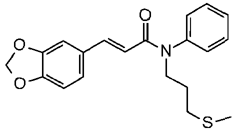 | (E)-3-(1,3-benzodioxol-5-yl)-N-(3-méthylsulfanylpropyl) -N-phényl-prop-2-énamide |
| 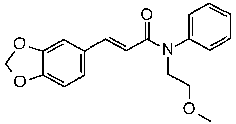 | (E)-3-(1,3-benzodioxol-5-yl)-N-(2-méthoxyéthyl)-N-phényl-prop-2-énamide |
| 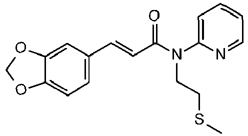 | (E)-3-(1,3-benzodioxol-5-yl)-N-(2-méthylsulfanyléthyl)-N-(2-pyridyl)prop-2-énamide |
| 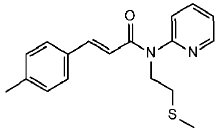 | (E)-N-(2-méthylsulfanyléthyl)-3-(p-toluyl)-N-(2-pyridyl)prop-2-énamide |
| 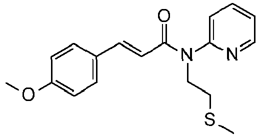 | (E)-3-(4-méthoxyphényl)-N-(2-méthylsulfanyléthyl)-N-(2-pyridyl)prop-2-énamide |
| 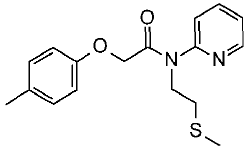 | 2-(4-méthylphénoxy)-N-(2-méthylsulfanyléthyl)-N-(2-pyridyl)acétamide |
| 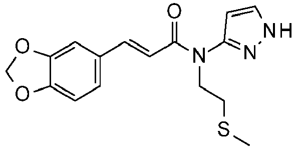 | (E)-3-(1,3-benzodioxol-5-yl)-N-(2-méthylsulfanyléthyl)-N-(1H-pyrazol-3-yl)-prop-2-énamide |
| 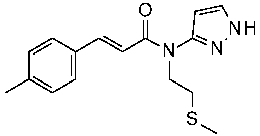 | (E)-N-(2-méthylsulfanyléthyl)-3-(p-toluyl)-N-(1H-pyrazol-3-yl)prop-2-énamide |
| 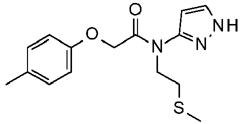 | 2-(4-méthylphénoxy)-N-(2-méthylsulfanyléthyl)-N-(1H-pyrazol-3-yl)acétamide |
| 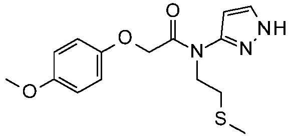 | 2-(4-méthoxyphénoxy)-N-(2-méthylsulfanyléthyl)-N-(1H-pyrazol-3-yl)acétamide |

3. Procédé selon la revendication 1 ou 2, le récepteur étant mis en contact avec au moins un modulateur qui, dans un test d'activité cellulaire avec utilisation de cellules qui expriment le récepteur TRPM8 humain de manière recombinante, module la perméabilité de ces cellules aux ions de Ca²⁺.

4. Procédé selon la revendication 1, 2 ou 3, le modulateur ayant un effet agoniste ou antagoniste sur la perméabilité cellulaire aux ions de Ca²⁺.

5. Procédé selon l'une quelconque des revendications 1 à 4, le modulateur étant un agoniste du récepteur TRPM8.

6. Utilisation d'un modulateur selon la définition dans la revendication 5 pour l'induction non thérapeutique d'une sensation de froid chez l'homme et/ou l'animal.

7. Utilisation selon la revendication 6 d'un modulateur selon la définition dans la revendication 5 pour l'induction non thérapeutique d'une sensation de froid par un emballage contenant le modulateur ou par un textile contenant le modulateur.

8. Utilisation selon la revendication 6, un agent comprenant au moins un, deux, trois modulateurs ou plus selon la définition dans la revendication 5 étant utilisé, de préférence en une quantité (totale) de 0,1 ppm à 10% en poids, par rapport au poids total de l'agent, pour obtenir un effet de refroidissement sur la peau ou les muqueuses, qui, de préférence par rapport à l'effet de refroidissement d'un agent de même composition, dans lequel uniquement le(s) modulateur(s) est/sont remplacé(s) par du N-éthylamide de l'acide menthanecarboxylique à la même concentration, est prolongé de préférence d'au moins 10 minutes et/ou qui, par rapport à l'effet de refroidissement d'un agent de même composition, dans lequel uniquement le(s) modulateur(s) est/sont remplacé(s) par du FEMA 4809 ou du FEMA 4496 à la même concentration, démarre plus rapidement.

9. Utilisation selon la revendication 7 ou 8, le(s) modulateur(s) étant choisi(s) dans le groupe A tel que défini dans la revendication 2.

10. Modulateur selon la définition selon l'une quelconque des revendications 1 à 5 destiné à une utilisation comme modulateur du récepteur TRPM8, de préférence destiné à utilisation dans un procédé thérapeutique pour la modulation du récepteur TRPM8.

11. Composé choisi dans le groupe A, constitué par les composés suivants :
| Structure | Nom |
|---|---|
| 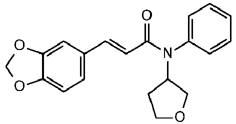 | (E)-3-(1, 3-benzodioxol-5-yl)-N-phényl-N-tétrahydrofurann-3-yl-prop-2-énamide |
| 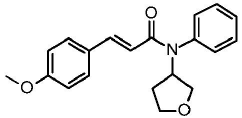 | (E)-3-(4-méthoxyphényl)-N-phényl-N-tétrahydrofurann-3-yl-prop-2-énamide |
| 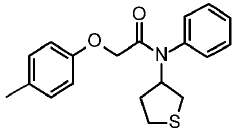 | 2-(4-méthylphénoxy)-N-phényl-N-tétrahydrothiophén-3-yl-acétamide |
| 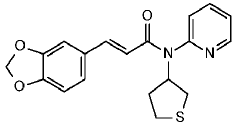 | (E)-3-(1,3-benzodioxol-5-yl)-N-(2-pyridyl)-N-tétrahydrothiophén-3-yl-prop-2-énamide |
| 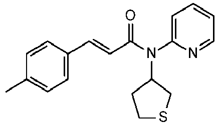 | (E)-3-(p-toluyl)-N-(2-pyridyl)-N-tétrahydrothiophén-3-yl-prop-2-énamide |
| 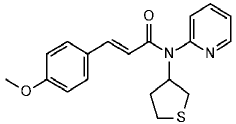 | (E)-3-(4-méthoxyphényl)-N-(2-pyridyl)-N-tétrahydrothiophén-3-yl-prop-2-énamide |
| 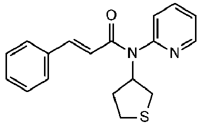 | (E)-3-phényl-N-(2-pyridyl)-N-tétrahydrothiophén-3-yl-prop-2-énamide |
| 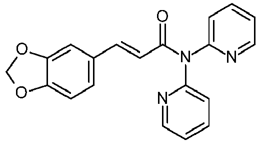 | (E)-3-(1,3-benzodioxol-5-yl)-N,N-bis(2-pyridyl)-prop-2-énamide |
| 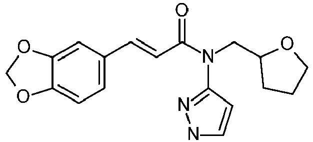 | (E)-3-(1,3-benzodioxol-5-yl)-N-(1H-pyrazol-3-yl)-N-(tétrahydrofurann-2-ylméthyl)prop-2-énamide |
| 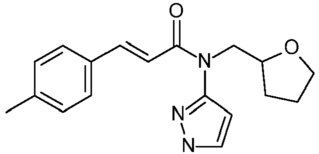 | (E)-3-(p-toluyl)-N-(1H-pyrazol-3-yl)-N-(tétrahydrofurann-2-ylméthyl)prop-2-énamide |
| 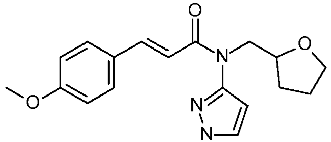 | (E)-3-(4-méthoxyphényl)-N-(1H-pyrazol-3-yl)-N-(tétrahydrofurann-2-ylméthyl)prop-2-énamide |
| 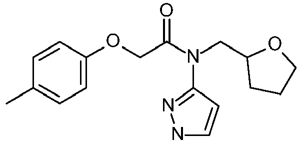 | 2-(4-méthylphénoxy)-N-(1H-pyrazol-3-yl)-N-(tétrahydrofurann-2-ylméthyl)acétamide |
| 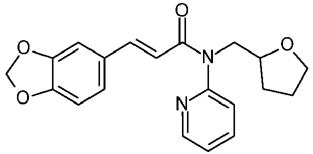 | (E)-3-(1,3-benzodioxol-5-yl)-N-(2-pyridyl)-N-(tétrahydrofurann-2-ylméthyl)prop-2-énamide |
| 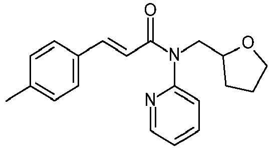 | (E)-3-(p-toluyl)-N-(2-pyridyl)-N-(tétrahydrofurann-2-ylméthyl)prop-2-énamide |
| 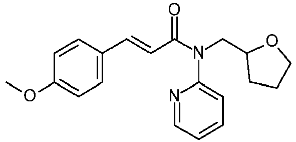 | (E)-3-(4-méthoxyphényl)-N-(2-pyridyl)-N-(tétrahydrofurann-2-ylméthyl)prop-2-énamide |
| 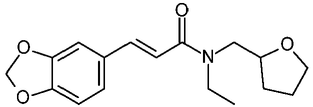 | (E)-3-(1,3-benzodioxol-5-yl)-N-éthyl-N-(tétrahydrofurann-2-ylméthyl)prop-2-énamide |
| 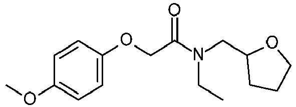 | N-éthyl-2-(4-méthoxyphénoxy)-N-(tétrahydrofurann-2-ylméthyl)acétamide |
| 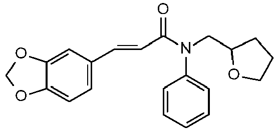 | (E)-3-(1,3-benzodioxol-5-yl)-N-phényl-N-(tétrahydrofurann-2-ylméthyl)prop-2-énamide |
| 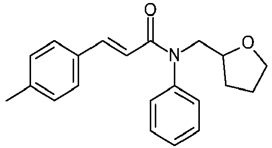 | (E)-N-phényl-3-(p-toluyl)-N-(tétrahydrofurann-2-ylméthyl)prop-2-énamide |
| 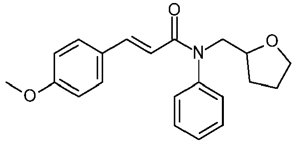 | (E)-3-(4-méthoxyphényl)-N-phényl-N-(tétrahydrofurann-2-ylméthyl)prop-2-énamide |
| 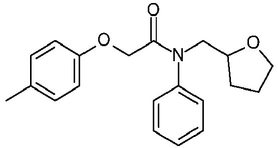 | 2-(4-méthylphénoxy)-N-phényl-N-(tétrahydrofurann-2-ylméthyl)acétamide |
| 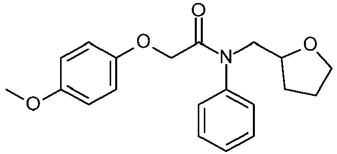 | 2-(4-méthoxyphénoxy)-N-phényl-N-(tétrahydrofurann-2-ylméthyl)acétamide |
| 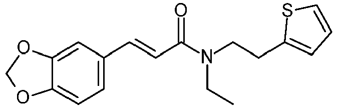 | (E)-3-(1,3-benzodioxol-5-yl)-N-éthyl-N-[2-(2-thiényl)éthyl]prop-2-énamide |
| 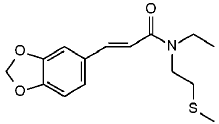 | (E)-3-(1,3-benzodioxol-5-yl)-N-éthyl-N-(2-méthylsulfanyléthyl)-prop-2-énamide |
| 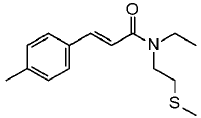 | (E)-N-éthyl-N-(2-méthylsulfanyléthyl)-3-(p-toluyl)prop-2-énamide |
| 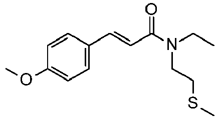 | (E)-N-éthyl-3-(4-méthoxyphényl)-N-(2-méthylsulfanyléthyl)-prop-2-énamide |
| 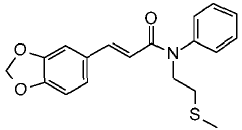 | (E)-3-(1,3-benzodioxol-5-yl)-N-(2-méthylsulfanyléthyl)-N-phényl-prop-2-énamide |
| 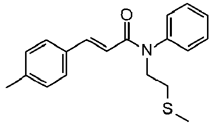 | (E)-N-(2-méthylsulfanyléthyl)-N-phényl-3-(p-toluyl)prop-2-énamide |
| 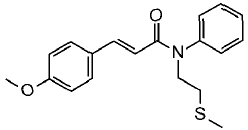 | (E)-3-(4-méthoxyphényl)-N-(2-méthylsulfanyléthyl)-N-phényl-prop-2-énamide |
| 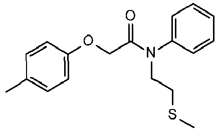 | 2-(4-méthylphénoxy)-N-(2-méthylsulfanyléthyl)-N-phényl-acétamide |
| 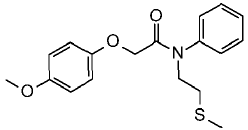 | 2-(4-méthoxyphénoxy)-N-(2-méthylsulfanyléthyl)-N-phényl-acétamide |
| 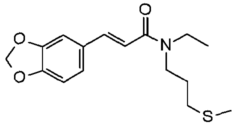 | (E)-3-(1,3-benzodioxol-5-yl)-N-éthyl-N-(3-méthylsulfanylpropyl) prop-2-énamide |
| 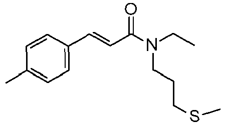 | (E)-N-éthyl-N-(3-méthylsulfanylpropyl) -3-(p-toluyl)prop-2-énamide |
| 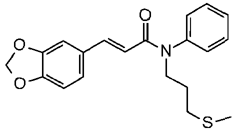 | (E)-3-(1,3-benzodioxol-5-yl)-N-(3-méthylsulfanylpropyl) -N-phényl-prop-2-énamide |
| 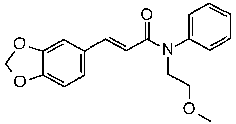 | (E)-3-(1,3-benzodioxol-5-yl)-N-(2-méthoxyéthyl)-N-phényl-prop-2-énamide |
| 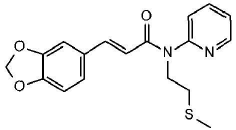 | (E)-3-(1,3-benzodioxol-5-yl)-N-(2-méthylsulfanyléthyl)-N-(2-pyridyl)prop-2-énamide |
| 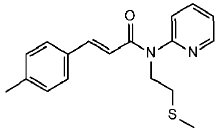 | (E)-N-(2-méthylsulfanyléthyl)-3-(p-toluyl)-N-(2-pyridyl)prop-2-énamide |
| 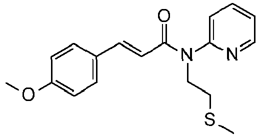 | (E)-3-(4-méthoxyphényl)-N-(2-méthylsulfanyléthyl)-N-(2-pyridyl)prop-2-énamide |
| 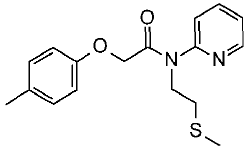 | 2-(4-méthylphénoxy)-N-(2-méthylsulfanyléthyl)-N-(2-pyridyl)acétamide |
| 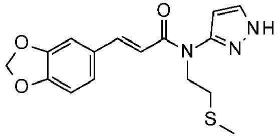 | (E)-3-(1,3-benzodioxol-5-yl)-N-(2-méthylsulfanyléthyl)-N-(1H-pyrazol-3-yl)-prop-2-énamide |
| 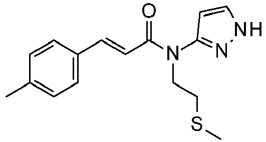 | (E)-N-(2-méthylsulfanyléthyl)-3-(p-toluyl)-N-(1H-pyrazol-3-yl)prop-2-énamide |
| 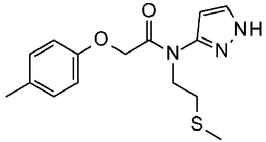 | 2-(4-méthylphénoxy)-N-(2-méthylsulfanyléthyl)-N-(1H-pyrazol-3-yl)acétamide |
| 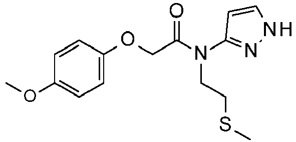 | 2-(4-méthoxyphénoxy)-N- (2-méthylsulfanyléthyl)-N-(1H-pyrazol-3-yl)acétamide |
ou sel d'un tel composé, en particulier choisi parmi les sels d'addition d'acides avec des acides carboxyliques inorganiques ou en particulier organiques, monovalents ou en particulier polyvalents.

12. Agent, comprenant un composé selon la revendication 11.

13. Agent contenant au moins un modulateur selon la définition selon l'une quelconque des revendications 1 à 5 destiné à une utilisation comme modulateur du récepteur TRPM8, de préférence destiné à une utilisation dans un procédé thérapeutique pour la modulation du récepteur TRPM8.

14. Agent selon la revendication 13 destiné à être utilisé comme modulateur du récepteur TRPM8, de préférence destiné à une utilisation dans un procédé thérapeutique pour la modulation du récepteur TRPM8 ou agent selon la revendication 12, l'agent étant choisi dans le groupe constitué par
a) les aliments, de préférence la crème glacée, la mousse, la crème, les boissons et les confiseries,
b) les agents de soins buccaux, de préférence le dentifrice, le bain de bouche et le chewing-gum,
c) les agents de soin corporel, de préférence les agents de soin de la peau ou des cheveux, par exemple les crèmes solaires, les écrans solaires, les lotions et les shampooings et les pansements,
d) les mousses et les gels.

15. Agent selon la revendication 13 ou 14 destiné à être utilisé comme modulateur du récepteur TRPM8, de préférence destiné à une utilisation dans un procédé thérapeutique pour la modulation du récepteur TRPM8 ou agent selon la revendication 12, l'agent étant choisi dans le groupe constitué par les mélanges aromatisants et les préparations servant à l'alimentation, à l'hygiène buccale ou à la dégustation ou les préparations cosmétiques, comprenant un, deux, trois des modulateurs, ou plus, selon la définition de l'une quelconque des revendications 1 à 5, ce(s) modulateur(s) étant contenu(s) en une quantité (totale) de 0,05 ppm à 10% en poids, de préférence de 0,1 ppm à 10% en poids, par rapport au poids total du mélange aromatisant ou de la préparation.

16. Agent selon la revendication 13, 14 ou 15 destiné à être utilisé comme modulateur du récepteur TRPM8, de préférence destiné à une utilisation dans un procédé thérapeutique pour la modulation du récepteur TRPM8 ou agent selon la revendication 12, l'agent comprenant en outre :
(1) une ou plusieurs autres substances pourvues d'un effet de refroidissement physiologique, l'autre substance ou l'une, plusieurs ou l'ensemble des autres substances (i) provoquant un effet gustatif ou (ii) ne provoquant pas d'effet gustatif,
et/ou
(2) une ou plusieurs substances aromatisantes sans effet de refroidissement physiologique
et/ou
(3) une ou plusieurs substances à effet trigéminal ou alléchant sans effet de refroidissement physiologique
et/ou
(4) (iii) un ou (iv) plusieurs composés qui provoque(nt), dans le cas (iv) indépendamment les uns des autres ou conjointement, en plus un effet de modulation du goût et/ou un stimulus trigéminal et/ou alléchant.

17. Utilisation selon la revendication 6 pour l'induction non thérapeutique d'une sensation de froid chez l'homme et/ou l'animal pour obtenir un effet de refroidissement physiologique sur la peau et/ou les muqueuses qui, par rapport à l'effet de refroidissement d'un agent de même composition, dans lequel uniquement le(s) modulateur(s) est/sont remplacé(s) par du N-éthylamide de l'acide menthanecarboxyique à la même concentration, est prolongé d'au moins 10 minutes et/ou qui, par rapport à l'effet de refroidissement d'un agent de même composition, dans lequel uniquement le(s) modulateur(s) est/sont remplacé(s) par du FEMA 4809 ou du FEMA 4496 à la même concentration, démarre plus rapidement, présentant l'étape suivante :
- application d'une quantité suffisante pour obtenir un effet de refroidissement physiologique d'un agent tel que défini dans l'une quelconque des revendications 12 à 15 sur la peau et/ou les muqueuses.

18. Utilisation selon la revendication 17, le(s) modulateur(s) étant choisi(s) dans le groupe A tel que défini dans la revendication 2.
